(19) Europäisches Patentamt
European Patent Office
Office européen des brevets

(11) EP 4 740 967 A1

(12) **EUROPEAN PATENT APPLICATION**
published in accordance with Art. 153(4) EPC

(43) Date of publication:
**13.05.2026 Bulletin 2026/20**

(21) Application number: **24838674.0**

(22) Date of filing: **04.07.2024**

(51) International Patent Classification (IPC):
***A61K 47/68*** (2017.01) ***A61K 47/65*** (2017.01)
***C07D 491/22*** (2006.01) ***C07D 493/22*** (2006.01)
***A61P 35/00*** (2006.01)

(52) Cooperative Patent Classification (CPC):
**A61K 47/64; A61K 47/65; A61K 47/68; A61P 35/00; A61P 35/02; C07D 491/22; C07D 493/22; C07K 5/02; C07K 5/06017; C07K 5/0815; C07K 5/1005**

(86) International application number:
**PCT/CN2024/103480**

(87) International publication number:
**WO 2025/011419 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.07.2023 PCT/CN2023/106385**

(71) Applicant: **Shanghai Escugen Biotechnology Co., Ltd.**
**Shanghai 201206 (CN)**

(72) Inventors:
- **ZENG, Di**
  **Shanghai 201206 (CN)**
- **XU, Chuanying**
  **Shanghai 201206 (CN)**
- **NIAN, Weihong**
  **Shanghai 201206 (CN)**
- **YAN, Hongbin**
  **Shanghai 201206 (CN)**
- **HE, Zhuzi**
  **Shanghai 201206 (CN)**
- **WEI, Hongli**
  **Shanghai 201206 (CN)**
- **ZHENG, Xintong**
  **Shanghai 201206 (CN)**
- **ZHANG, Xinmin**
  **Shanghai 201206 (CN)**
- **HE, Feng**
  **Shanghai 201206 (CN)**
- **ZHOU, Qing**
  **Shanghai 201206 (CN)**

(74) Representative: **Cooley (UK) LLP**
**22 Bishopsgate**
**London EC2N 4BQ (GB)**

Remarks:
The complete document including Reference Table(s) and the Sequence Listing(s) can be downloaded from the EPO website

# (54) LINKER-DRUG MOLECULE AND ANTIBODY DRUG CONJUGATE, PREPARATION METHOD THEREFOR AND USE THEREOF

(57) A compound represented by formula (1), or a tautomer, mesomer, racemate, enantiomer, and diastereomer thereof, or mixture form thereof, or a pharmaceutically acceptable salt, prodrug or solvate thereof, wherein the structure of formula (1) is as shown below: formula (1). According to the compound, a strong hydrophilic sulfonate inner salt and/or phosphate inner salt side chain are introduced at the position of polypeptide (VC, VA, AAA, etc.)-PAB (self-immolative group) of a linker, greatly enhancing the druggability (comprising hydrophilicity and stability) of an antibody drug conjugate corresponding to the compound, reducing the in-vivo drug clearance effect, and improving the tumor treatment effect.

EP 4 740 967 A1

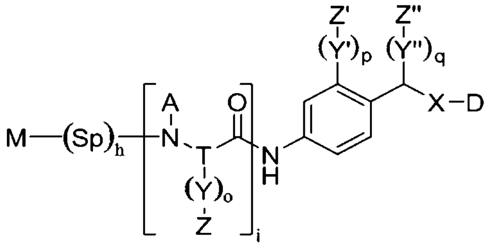

Formula (1)

## Description

TECHNICAL FIELD

The present invention relates to biomedical field, in particular to a method for preparing an linker-drug molecule and an antibody-drug conjugate and use thereof.

CROSS REFERENCE

This application claims the priority of PCT/CN2023/106385 filed on July 7, 2023, the content of which is incorporated herein by reference in its entirety.

SIMULTANEOUSLY SUBMITTED SEQUENCE LISTING DOCUMENT

The content of the attached XML file is incorporated herein by reference in its entirety: sequence listing of computer readable format (CRF) (file name: TFH01015PCT-sequence listing.xml, date: May 29, 2024, size: 6KB)

BACKGROUND

By conjugation of a biologically active drug compound to a tumor targeting antibody, protein, or polypeptide, the cancer treatment window of drug compounds can be increased. This is mainly achieved through two aspects: firstly, the drug conjugate cannot produce toxic side effects before reaching the cancer target lesion due to the inability of the active drug to detach smoothly; secondly, the active drug can increase the concentration of the drug compound at the cancer target lesion through the conjugation of the targeted antibody, protein, or polypeptide to the active drug, because a specially designed linker can be triggered by the special environment of the cancer target lesion to break and thereby release the active drug compound

The specially designed amino acid peptide segments sensitive to tissue proteases in the linker can be efficiently cleaved by tissue proteases in the lysosomes of cancer cells. A drug compound is connected to the amino acid peptide segment of the linker through a chemical self-immolative group, after the polypeptide is cleaved by tissue protease, the chemical self-immolative group releases free drug compounds through the "self-immolative effect", resulting in a tumor killing effect. Among them, the clinical validation data and the approved corresponding drugs obtained from the antibody- or polypeptide-drug conjugates in the form of peptide (VC, VA, AAA, etc.)-PAB (self-immolative group) linkers are the most. However, due to the strong lipophilic characteristics of drug compounds, especially those with important bystander killing effect on tumors, the antibody, protein, or polypeptide drug conjugates, especially when the drug to antibody ratio (DAR) is high, exhibit poor drug properties and low hydrophilicity, thereby forming polymers and accelerating in vivo clearance. By adding hydrophilic group such as polyethylene glycol (PEG) (Mol Cancer Ther, 2017, 16(1): 116-123; Nat. Biotech. 2015, 33:733-735, Toxicol. App. Pharmacol., 2020, 392: 114932; WO2023280227), polylysine (PSAR) (Chem. Sci, 2019, 10(14): 4048-4053; WO 2022228493), sugar alcohol (Cancer Res (2018) 78 (13_Supplement): 755; US 9907854) on the linker to mask drug compounds, the drug formation of antibodies, proteins, or peptide drug conjugates will be improved to some extent. However, the use of PEG polymers to modify proteins has exposed many drawbacks in clinical practice, such as a high proportion of patients exhibiting anti-PEG immunogenicity and related toxic side effects, and excessive modification leading to decreased protein function.

SUMMARY

Based on this, this application introduces a strong hydrophilic sulfonate inner salt and/or phosphate inner salt side chain (sometimes referred to as the linker or the inner salt linkers of this application, i.e., a compound represented by Formula (1) as described below) at the position of the polypeptide (VC, VA, AAA, etc.)-PAB (self-immolative group) of the linker. As both the sulfonate group and the phosphate group have hydrogen bond donor and acceptor, they belong to a strong hydrophilic group, and the sulfonate inner salt or phosphate inner salt has both positive and negative charges, thereby further enhancing the hydrophilicity. At the same time, the water-soluble functional groups in the form of inner salts also have the potential to enhance the tumor targeting and reduce non-specific binding of the antibody-drug conjugate, thus greatly enhancing the drug formation (including hydrophilicity and stability) of the tested antibody-drug conjugate, reducing in vivo drug clearance, and improving tumor treatment efficacy.

The technical solutions of this application are as follows:

1. A compound represented by Formula (1), or a tautomer, mesomer, raceme, enantiomer or diastereomer thereof, or mixture thereof, or a pharmaceutically acceptable salt, prodrug or solvate thereof, wherein Formula (1) has the

following structural formula:

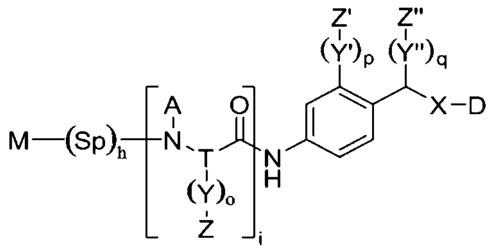

Formula (1)

M is a spacer; Sp is $-L_a-Q_a-$; A is selected from absent, hydrogen or alkyl; T represents a straight or branched ($C_1$-$C_8$) trivalent alkyl group; Y, Y' and Y" are each independently $-L_b-Q_b-$; $L_a$ and $L_b$ are each independently selected from absent, alkylene, alkenylene, alkynylene, alicyclylene, heteroalicyclylene, arylene, heteroarylene or polyethylene glycol group, wherein the alkylene, alkenylene, alkynylene, alicyclylene, heteroalicyclylene, arylene, heteroarylene or polyethylene glycol group is optionally substituted by a substituent;

$Q_a$, and $Q_b$ are each independently selected from absent, -C(=O)NR-, -C(=O)O-, -OC(=O)NR-, $-S(=O)_2NR-$, -NRC(=O)-, -OC(=O)-, -NRC(=O)O-, $-NRS(=O)_2-$, -NRC(=O)NR-, -NHC-(=NH)NH-, -C(=O)-, -OC(=O)O-, -O-, -NR-, -S-, $S(=O)_2-$, S(=O)-, or -Se-, wherein R is selected from hydrogen or optionally substituted alkyl;

Z is selected from absent, hydrogen, optionally substituted alkyl or a sulfonate inner salt;

Z', and Z" are each independently selected from absent, hydrogen, optionally substituted alkyl, a sulfonate inner salt or a phosphate inner salt;

At least one of Z, Z' and Z" is a sulfonate inner salt or a phosphate inner salt;

X comprises a self-immolative unit or is absent; D comprises a drug molecule; i is an integer equal to or greater than 1; h is an integer from 1 to 6; o, p and q are each independently an integer from 0 to 5.

2. The compound, or a tautomer, mesomer, raceme, enantiomer or diastereomer thereof, or mixture thereof, or a pharmaceutically acceptable salt, prodrug or solvate thereof according to item 1, wherein Formula (1) is Formula (1-1):

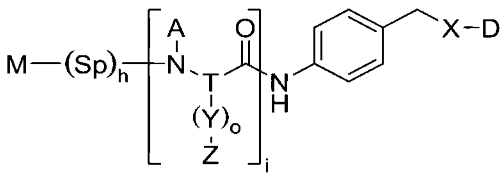

Formula (1-1)

wherein M, Sp, A, T, Y, h, o, i, X, D, and Z are defined as in item 1;
at least one of the i Zs is a sulfonate inner salt.

3. The compound, or a tautomer, mesomer, raceme, enantiomer or diastereomer thereof, or mixture thereof, or a pharmaceutically acceptable salt, prodrug or solvate thereof according to item 2, wherein the Formula (1-1) is Formula (1-1-1):

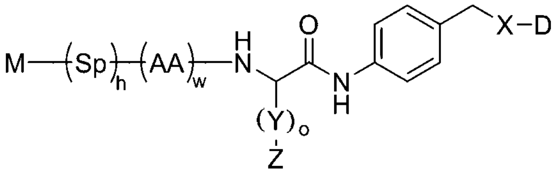

· Formula (1-1-1)

wherein AA is an amino acid residue, W is an integer equal to or greater than 1.

4. The compound, or a tautomer, mesomer, raceme, enantiomer or diastereomer thereof, or mixture thereof, or a pharmaceutically acceptable salt, prodrug or solvate thereof according to item 1, wherein Formula (1) is Formula (1-2):

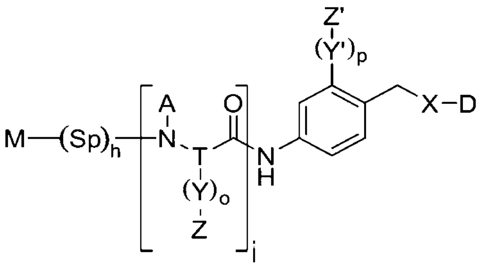

Formula (1-2)

wherein M, Sp, A, T, Y, Z, o, p, i, h, X, Y', and D are defined as in item 1;
Z' is a sulfonate inner salt or a phosphate inner salt.

5. The compound, or a tautomer, mesomer, raceme, enantiomer or diastereomer thereof, or mixture thereof, or a pharmaceutically acceptable salt, prodrug or solvate thereof according to item 4, wherein Formula (1) is Formula (1-2-1):

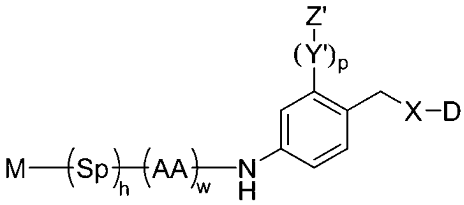

Formula (1-2-1)

wherein AA is an amino acid residue, W is an integer equal to or greater than 1.
6. The compound, or a tautomer, mesomer, raceme, enantiomer or diastereomer thereof, or mixture thereof, or a pharmaceutically acceptable salt, prodrug or solvate thereof according to item 1, wherein Formula (1) is Formula (1-3):

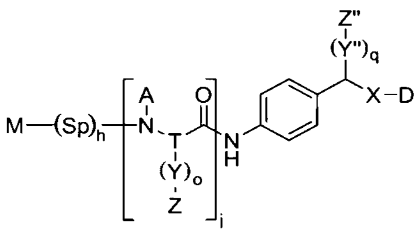

Formula (1-3)

wherein M, Sp, A, Y, T, Z, o, i, h, q, X, Y", and D are defined as in item 1;
Z" is a sulfonate inner salt or a phosphate inner salt.

7. The compound, or a tautomer, mesomer, raceme, enantiomer or diastereomer thereof, or mixture thereof, or a pharmaceutically acceptable salt, prodrug or solvate thereof according to item 6, wherein Formula (1) is Formula (1-3-1):

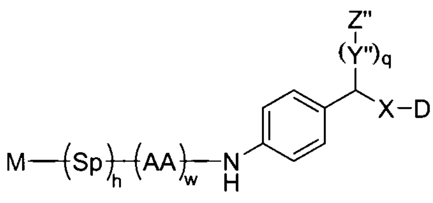

Formula (1-3-1)

wherein AA is an amino acid residue, W is an integer equal to or greater than 1.
8. The compound, or a tautomer, mesomer, raceme, enantiomer or diastereomer thereof, or mixture thereof, or a pharmaceutically acceptable salt, prodrug or solvate thereof according to any one of items 1-7, wherein when A and Z are absent, N, Y and T form a ring to have a structural formula of:

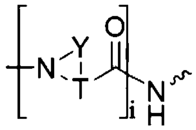,

preferably

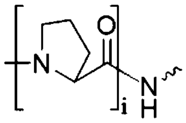.

9. The compound, or a tautomer, mesomer, raceme, enantiomer or diastereomer thereof, or mixture thereof, or a pharmaceutically acceptable salt, prodrug or solvate thereof according to any one of items 3, 5 and 7, wherein the amino acid(s) is one or more selected from the group consisting of: alanine, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, and valine.

10. The compound, or a tautomer, mesomer, raceme, enantiomer or diastereomer thereof, or mixture thereof, or a pharmaceutically acceptable salt, prodrug or solvate thereof according to any one of items 3, 5 and 7, wherein the amino acid(s) is one or more selected from the group consisting of: valine, glutamic acid, citrulline, lysine, alanine, phenylalanine, arginine, glutamine, asparagine, and glycine.

11. The compound, or a tautomer, mesomer, raceme, enantiomer or diastereomer thereof, or mixture thereof, or a pharmaceutically acceptable salt, prodrug or solvate thereof according to any one of items 3, 5 and 7, wherein the amino acid(s) is one or more selected from the group consisting of: valine, citrulline, alanine, phenylalanine, glutamine, and glycine.

12. The compound, or a tautomer, mesomer, raceme, enantiomer or diastereomer thereof, or mixture thereof, or a pharmaceutically acceptable salt, prodrug or solvate thereof according to any one of items 1-11, wherein M is selected from $N_3$, SH, $ONH_2$, $NH_2$, $CO_2H$, (alkyl)-CO-, HCO-, $BrCH_2$-, $ICH_2$-,

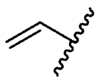,

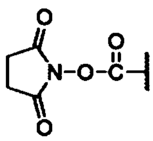 、 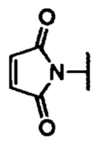, 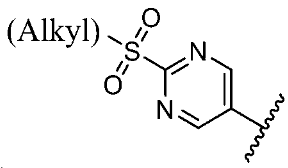, 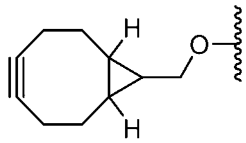 or 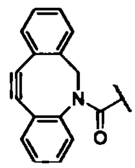.

13. The compound, or a tautomer, mesomer, raceme, enantiomer or diastereomer thereof, or mixture thereof, or a pharmaceutically acceptable salt, prodrug or solvate thereof according to any one of items 1-12, wherein M is

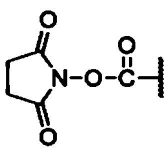 、 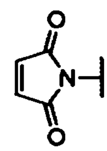, 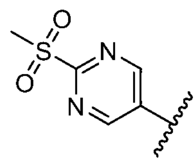 or 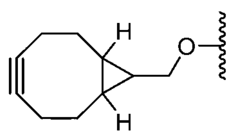.

14. The compound, or a tautomer, mesomer, raceme, enantiomer or diastereomer thereof, or mixture thereof, or a pharmaceutically acceptable salt, prodrug or solvate thereof according to any one of items 1-13, wherein M is

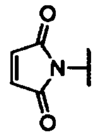.

15. The compound, or a tautomer, mesomer, raceme, enantiomer or diastereomer thereof, or mixture thereof, or a pharmaceutically acceptable salt, prodrug or solvate thereof according to any one of items 1-14, wherein A is hydrogen, methyl, ethyl or propyl.

16. The compound, or a tautomer, mesomer, raceme, enantiomer or diastereomer thereof, or mixture thereof, or a pharmaceutically acceptable salt, prodrug or solvate thereof according to any one of items 1-15, wherein A is hydrogen.

17. The compound, or a tautomer, mesomer, raceme, enantiomer or diastereomer thereof, or mixture thereof, or a pharmaceutically acceptable salt, prodrug or solvate thereof according to any one of items 1-16, wherein the T is any one selected from the group consisting of:

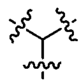, 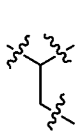, 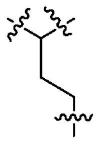, 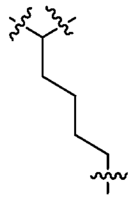.

18. The compound, or a tautomer, mesomer, raceme, enantiomer or diastereomer thereof, or mixture thereof, or a pharmaceutically acceptable salt, prodrug or solvate thereof according to any one of items 1-17, wherein the T is

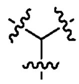.

19. The compound, or a tautomer, mesomer, raceme, enantiomer or diastereomer thereof, or mixture thereof, or a pharmaceutically acceptable salt, prodrug or solvate thereof according to any one of items 1-18, wherein $L_a$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

20. The compound, or a tautomer, mesomer, raceme, enantiomer or diastereomer thereof, or mixture thereof, or a pharmaceutically acceptable salt, prodrug or solvate thereof according to any one of items 1-19, wherein $L_a$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

21. The compound, or a tautomer, mesomer, raceme, enantiomer or diastereomer thereof, or mixture thereof, or a pharmaceutically acceptable salt, prodrug or solvate thereof according to any one of items 1-20, wherein $L_a$ is one, two, three or more selected from the group consisting of: $C_1$-$C_6$ linear or branched alkylene, $C_2$-$C_6$ linear or branched alkenylene, $C_2$-$C_6$ linear or branched alkynylene, and polyethylene glycol group; and wherein the alkylene, alkenylene, or alkynylene is optionally substituted by a substituent.

22. The compound, or a tautomer, mesomer, raceme, enantiomer or diastereomer thereof, or mixture thereof, or a pharmaceutically acceptable salt, prodrug or solvate thereof according to any one of items 1-21, wherein $L_a$ is one, two, three or more selected from the group consisting of: $C_1$-$C_6$ linear or branched alkylene, and polyethylene glycol group;

the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, and n is an integer equal to or greater than 1.

23. The compound, or a tautomer, mesomer, raceme, enantiomer or diastereomer thereof, or mixture thereof, or a pharmaceutically acceptable salt, prodrug or solvate thereof according to any one of items 1-22, wherein $L_a$ is one, two, three or more selected from the group consisting of: ethylidene, linear propylidene, linear butylidene, linear pentylidene, and $-(CH_2CH_2O)_nCH_2CH_2-$; and wherein n is 2, 3, 4, 5, 6, 7 or 8.

24. The compound, or a tautomer, mesomer, raceme, enantiomer or diastereomer thereof, or mixture thereof, or a pharmaceutically acceptable salt, prodrug or solvate thereof according to any one of items 1-23, wherein $L_a$ is ethylidene and/or $-(CH_2CH_2O)_4CH_2CH_2-$ or pentylidene.

25. The compound, or a tautomer, mesomer, raceme, enantiomer or diastereomer thereof, or mixture thereof, or a pharmaceutically acceptable salt, prodrug or solvate thereof according to any one of items 1-24, wherein $L_b$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, or heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

26. The compound, or a tautomer, mesomer, raceme, enantiomer or diastereomer thereof, or mixture thereof, or a pharmaceutically acceptable salt, prodrug or solvate thereof according to any one of items 1-25, wherein $L_b$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

27. The compound, or a tautomer, mesomer, raceme, enantiomer or diastereomer thereof, or mixture thereof, or a pharmaceutically acceptable salt, prodrug or solvate thereof according to any one of items 1-26, wherein $L_b$ is selected from $C_1$-$C_6$ linear or branched alkylene, $C_2$-$C_6$ linear or branched alkenylene, or $C_2$-$C_6$ linear or branched alkynylene, or polyethylene glycol group;

the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; and the alkylene, alkenylene, or alkynylene is optionally substituted by a substituent.

28. The compound, or a tautomer, mesomer, raceme, enantiomer or diastereomer thereof, or mixture thereof, or a pharmaceutically acceptable salt, prodrug or solvate thereof according to any one of items 1-27, wherein $L_b$ is one, two, three or more selected from the group consisting of: $C_1$-$C_6$ linear or branched alkylene, and $C_6$-$C_{10}$ arylene.

29. The compound, or a tautomer, mesomer, raceme, enantiomer or diastereomer thereof, or mixture thereof, or a pharmaceutically acceptable salt, prodrug or solvate thereof according to any one of items 1-28, wherein $L_b$ is one, two, three or more selected from the group consisting of: methylene, ethylene, n-propylidene, isopropylidene, n-butylidene, isobutylidene, sec-butylidene, tert-butylidene, and benzylidene.

30. The compound, or a tautomer, mesomer, raceme, enantiomer or diastereomer thereof, or mixture thereof, or a pharmaceutically acceptable salt, prodrug or solvate thereof according to any one of items 1-29, wherein $Q_a$ is one, two, three or more selected from the group consisting of: -C(=O)NR-, -NR-, -C(=O)O-, -OC(=O)NR-, -NRC(=O)-, -OC(=O)-, -NRC(=O)O-, -NRC(=O)NR-, NHC-(=NH)NH-, -C(=O)-, and -OC(=O)O-; and wherein R is selected from hydrogen, or optionally substituted $C_1$-$C_6$ linear or branched alkyl.

31. The compound, or a tautomer, mesomer, raceme, enantiomer or diastereomer thereof, or mixture thereof, or a pharmaceutically acceptable salt, prodrug or solvate thereof according to item 30, wherein $Q_a$ is one, two, three or more selected from the group consisting of: -C(=O)-, -NR-, -C(=O)NR-, -NRC(=O)NR-, -OC(=O)O-, -OC(=O)NR-, -NRC(=O)- and -OC(=O)-; and wherein R is selected from hydrogen, or optionally substituted $C_1$-$C_6$ linear or branched alkyl.

32. The compound, or a tautomer, mesomer, raceme, enantiomer or diastereomer thereof, or mixture thereof, or a pharmaceutically acceptable salt, prodrug or solvate thereof according to item 30, wherein $Q_a$ is one, two, three or more selected from the group consisting of: -C(=O)-, -NR-, -NRC(=O)-, and -(C=O)NR-; and wherein R is selected from hydrogen, or optionally substituted $C_1$-$C_6$ linear or branched alkyl.

33. The compound, or a tautomer, mesomer, raceme, enantiomer or diastereomer thereof, or mixture thereof, or a pharmaceutically acceptable salt, prodrug or solvate thereof according to item 30, wherein $Q_a$ is -C(=O)-, $-N(CH_3)-$, -NHC(=O)-, -C(=O)-NH- or $-C(=O)-N(CH_3)-$.

34. The compound, or a tautomer, mesomer, raceme, enantiomer or diastereomer thereof, or mixture thereof, or a pharmaceutically acceptable salt, prodrug or solvate thereof according to any one of items 1-33, wherein $Q_b$ is one,

two, three or more selected from the group consisting of: absent, -C(=O)NR-, -C(=O)O-, -OC(=O)NR-, -NRC(=O)-, -OC(=O)-, -NRC(=O)O-, -NRC(=O)NR-, NHC-(=NH)NH-, -C(=O)-, and -OC(=O)O-; and wherein R is selected from hydrogen, or optionally substituted $C_1$-$C_6$ linear or branched alkyl.

35. The compound, or a tautomer, mesomer, raceme, enantiomer or diastereomer thereof, or mixture thereof, or a pharmaceutically acceptable salt, prodrug or solvate thereof according to item 34, wherein $Q_b$ is one, two, three or more selected from the group consisting of: absent, -C(=O)-, -C(=O)NR-, -OC(=O)NR-, -NRC(=O)-, -NRC(=O)O-, and -NRC(=O)NR-; and R is selected from hydrogen, or optionally substituted $C_1$-$C_6$ linear or branched alkyl.

36. The compound, or a tautomer, mesomer, raceme, enantiomer or diastereomer thereof, or mixture thereof, or a pharmaceutically acceptable salt, prodrug or solvate thereof according to item 34, wherein $Q_b$ is one, two, three or more selected from the group consisting of: absent, -C(=O)-, -NRC(=O)-, -NRC(=O)NR-, and -C(=O)NR-; R is hydrogen or methyl.

37. The compound, or a tautomer, mesomer, raceme, enantiomer or diastereomer thereof, or mixture thereof, or a pharmaceutically acceptable salt, prodrug or solvate thereof according to any one of items 1-36, wherein X is selected from:

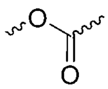 , 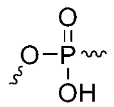 , 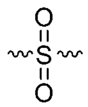 , 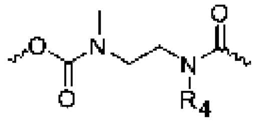 ,

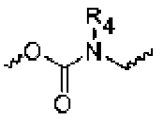 , 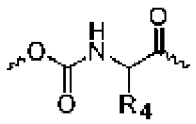 , 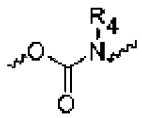 or 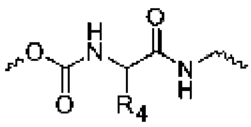 ;

the wavy line at right side of X is connected to D; $R_4$ is selected from hydrogen, $C_1$-$C_6$ linear or branched alkyl, $-(CH_2CH_2O)_mR_2$, $-CH_2CH_2NR_2R_3$, or $-CH_2CH_2SO_2R_2$; $R_2$, and $R_3$ are each independently selected from hydrogen, or $C_1$-$C_6$ linear or branched alkyl; and m is an integer greater than 1.

38. The compound, or a tautomer, mesomer, raceme, enantiomer or diastereomer thereof, or mixture thereof, or a pharmaceutically acceptable salt, prodrug or solvate thereof according to item 37, wherein

X is

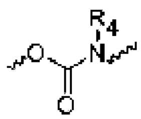 , 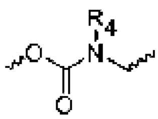 or 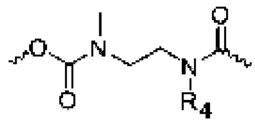

; and

$R_4$ is hydrogen, methyl, $-CH_2CH_2OCH_2CH_2OH$, $-CH_2CH_2NMe_2$, or $-CH_2CH_2SO_2Me$.

39. The compound, or a tautomer, mesomer, raceme, enantiomer or diastereomer thereof, or mixture thereof, or a pharmaceutically acceptable salt, prodrug or solvate thereof according to item 37, wherein
X is

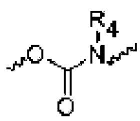 or 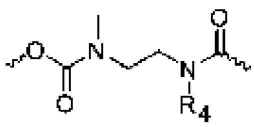 ,

and $R_4$ is hydrogen or methyl.

40. The compound, or a tautomer, mesomer, raceme, enantiomer or diastereomer thereof, or mixture thereof, or a pharmaceutically acceptable salt, prodrug or solvate thereof according to any one of items 1-39, wherein the sulfonate inner salt is

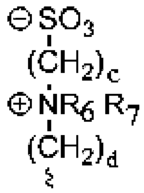 , 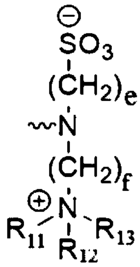 or 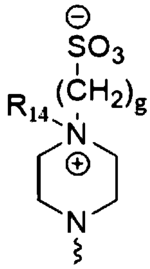 ,

the wavy line is connected to Y, Y' or Y"; wherein c, d, e, f, and g are each independently an integer selected from 1, 2, 3, 4, 5 or 6; and

$R_6$, $R_7$, $R_{11}$, $R_{12}$, $R_{13}$ and $R_{14}$ are each independently selected from absent, hydrogen, $C_1$-$C_6$ linear or branched alkyl.

41. The compound, or a tautomer, mesomer, raceme, enantiomer or diastereomer thereof, or mixture thereof, or a pharmaceutically acceptable salt, prodrug or solvate thereof according to item 40, wherein the sulfonate inner salt is

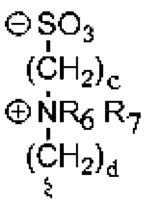 , 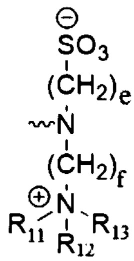 or 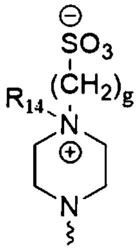 ,

wherein

$R_6$, $R_7$, $R_{11}$, $R_{12}$, $R_{13}$ and $R_{14}$ are each independently selected from absent, methyl or ethyl; and
c, d, e, f, and g are each independently 1, 2, or 3.

42. The compound, or a tautomer, mesomer, raceme, enantiomer or diastereomer thereof, or mixture thereof, or a pharmaceutically acceptable salt, prodrug or solvate thereof according to item 40, wherein the sulfonate inner salt is any one selected from the group consisting of:

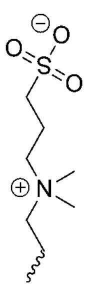 , 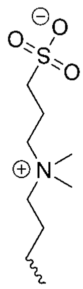 , 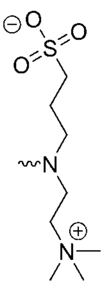 , and 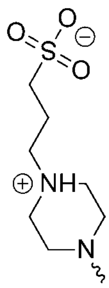 .

43. The compound, or a tautomer, mesomer, raceme, enantiomer or diastereomer thereof, or mixture thereof, or a pharmaceutically acceptable salt, prodrug or solvate thereof according to any one of items 1-42, wherein the phosphate inner salt is

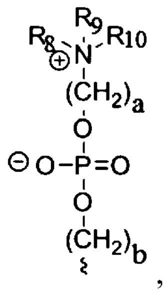

wherein

a and b are each independently an integer selected from 1, 2, 3, 4, 5 or 6; and $R_8$, $R_9$ and $R_{10}$ are each independently selected from hydrogen, or $C_1$-$C_6$ linear or branched alkyl.

44. The compound, or a tautomer, mesomer, raceme, enantiomer or diastereomer thereof, or mixture thereof, or a pharmaceutically acceptable salt, prodrug or solvate thereof according to item 43, wherein
the phosphate inner salt is

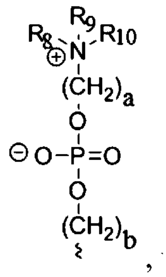

wherein $R_8$, $R_9$, and $R_{10}$ are each independently methyl or ethyl; a is 1, 2, or 3; and b is 1, 2, or 3.

45. The compound, or a tautomer, mesomer, raceme, enantiomer or diastereomer thereof, or mixture thereof, or a pharmaceutically acceptable salt, prodrug or solvate thereof according to item 43, wherein
the phosphate inner salt is

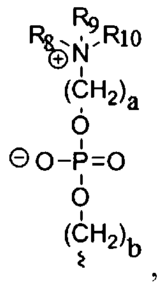

wherein $R_8$, $R_9$, and $R_{10}$ are each independently methyl; a is 2; and b is 1.

46. The compound, or a tautomer, mesomer, raceme, enantiomer or diastereomer thereof, or mixture thereof, or a pharmaceutically acceptable salt, prodrug or solvate thereof according to any one of items 1-45, wherein the D comprises a cytotoxin or immune agonist.

47. The compound, or a tautomer, mesomer, raceme, enantiomer or diastereomer thereof, or mixture thereof, or a pharmaceutically acceptable salt, prodrug or solvate thereof according to item 46, wherein the cytotoxin is one or two selected from the group consisting of: a microtubulin inhibitor, a DNA synthesis inhibitor, a topoisomerase inhibitor, and a RNA polymerase-2 inhibitor.

48. The compound, or a tautomer, mesomer, raceme, enantiomer or diastereomer thereof, or mixture thereof, or a pharmaceutically acceptable salt, prodrug or solvate thereof according to item 46, wherein
the cytotoxin is one, two, or more selected from the group consisting of: auristatin, mertansine DM, calicheamicin, duocarmycin, pyrrolobenzodiazepine, camptothecin derivative, and $\alpha$-amanitin.

49. The compound, or a tautomer, mesomer, raceme, enantiomer or diastereomer thereof, or mixture thereof, or a pharmaceutically acceptable salt, prodrug or solvate thereof according to item 46, wherein the immune agonist is selected from TLR7/8 agonist or STING agonist.

50. The compound, or a tautomer, mesomer, raceme, enantiomer or diastereomer thereof, or mixture thereof, or a pharmaceutically acceptable salt, prodrug or solvate thereof according to any one of items 1-49, wherein i is 2, 3, 4, 5, 6, 7, or 8.

51. The compound, or a tautomer, mesomer, raceme, enantiomer or diastereomer thereof, or mixture thereof, or a pharmaceutically acceptable salt, prodrug or solvate thereof according to any one of items 1-50, wherein h is 1, 2, 3, or 4.

52. The compound, or a tautomer, mesomer, raceme, enantiomer or diastereomer thereof, or mixture thereof, or a pharmaceutically acceptable salt, prodrug or solvate thereof according to any one of items 1-51, wherein o, p, and q are each independently 0, 1, 2 or 3.

53. The compound, or a tautomer, mesomer, raceme, enantiomer or diastereomer thereof, or mixture thereof, or a pharmaceutically acceptable salt, prodrug or solvate thereof according to any one of items 1-52, wherein $L_a$ is one, two, three or more selected from the group consisting of: $C_1$-$C_6$ linear or branched alkylene, and polyethylene glycol group; the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, n is an integer equal to or greater than 1; $Q_a$ is one, two, three or more selected from the group consisting of: -C(=O)-, -NR-, -NR(C=O)-, and -(C=O)NR-; and wherein R is selected from hydrogen, or C $_1$-$C_6$ linear or branched alkyl.

54. The compound, or a tautomer, mesomer, raceme, enantiomer or diastereomer thereof, or mixture thereof, or a pharmaceutically acceptable salt, prodrug or solvate thereof according to item 53, wherein $L_a$ is one, two, three or more selected from the group consisting of: ethylidene, linear propylidene, linear butylidene, linear pentylidene, and $-(CH_2CH_2O)_nCH_2CH_2-$; wherein n is 2, 3, 4, 5, 6, 7 or 8; $Q_a$ is one, two, three or more selected from the group consisting of: -C(=O)-, -NR-, -NR(C=O)-, and -(C=O)NR-; and wherein R is methyl and/or hydrogen.

55. The compound, or a tautomer, mesomer, raceme, enantiomer or diastereomer thereof, or mixture thereof, or a pharmaceutically acceptable salt, prodrug or solvate thereof according to item 53, wherein

$L_a$ is ethylidene and/or $-(CH_2CH_2O)_4CH_2CH_2-$ or pentylidene; and $Q_a$ is -C(=O)-, $-N(CH_3)-$, -NH(C=O)-, -C(=O)NH- or $-C(=O)-N(CH_3)-$.

56. The compound, or a tautomer, mesomer, raceme, enantiomer or diastereomer thereof, or mixture thereof, or a pharmaceutically acceptable salt, prodrug or solvate thereof according to any one of items 1-55, wherein $L_b$ is selected from $C_1$-$C_6$ linear or branched alkylene, $C_2$-$C_6$ linear or branched alkenylene, or $C_2$-$C_6$ linear or branched alkynylene, arylene with 6-10 carbon atoms in one or more rings, or polyethylene glycol group; the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; the alkylene, alkenylene, or alkynylene is optionally substituted by a substituent; $Q_b$ is one, two, three or more selected from the group consisting of: absent, -C(=O)NR-, -C(=O)O-, -OC(=O)NR-, -NRC(=O)-, -OC(=O)-, -NRC(=O)O-, $-NRS(=O)_2-$, -NRC(=O)NR-, -C(=O)-, -OC(=O)O-; and R is selected from hydrogen, or optionally substituted $C_1$-$C_6$ linear or branched alkyl.

57. The compound, or a tautomer, mesomer, raceme, enantiomer or diastereomer thereof, or mixture thereof, or a pharmaceutically acceptable salt, prodrug or solvate thereof according to item 56, wherein $L_b$ is one, two, three or more selected from the group consisting of: $C_1$-$C_6$ linear or branched alkylene, and $C_6$-$C_{10}$ arylene; $Q_b$ is one, two, three or more selected from the group consisting of: absent, -C(=O)-, -C(=O)NR-, -OC(=O)NR-, -NRC(=O)-, -NRC(=O)O-, and -NRC(=O)NR-; and R is selected from hydrogen, or C $_1$-$C_6$ linear or branched alkyl.

58. The compound, or a tautomer, mesomer, raceme, enantiomer or diastereomer thereof, or mixture thereof, or a pharmaceutically acceptable salt, prodrug or solvate thereof according to item 56, wherein $L_b$ is one, two, three or more selected from the group consisting of:methylene, ethylene, n-propylidene, isopropylidene, n-butylidene, isobutylidene, sec-butylidene, tert-butylidene, and benzylidene; $Q_b$ is one, two, three or more selected from the group consisting of: absent, -C(=O)-, -NRC(=O)-, -NRC(=O)NR-, and -C(=O)NR-; and R is hydrogen or methyl.

59. The compound, or a tautomer, mesomer, raceme, enantiomer or diastereomer thereof, or mixture thereof, or a pharmaceutically acceptable salt, prodrug or solvate thereof according to any one of items 1-58, wherein the substituent is any one selected from the group consisting of: deuterium, tritium, halogen, amino, hydroxyl, cyano, nitro, optionally substituted alkyl, alkenyl, alkynyl, optionally substituted heterocyclyl, acyl, optionally substituted amino, optionally substituted aminoformyl, optionally substituted thioaminoformyl, optionally substituted sulfamine, optionally substituted hydroxyl, optionally substituted thioalkyl (SH), and optionally substituted silyl.

60. The compound, or a tautomer, mesomer, raceme, enantiomer or diastereomer thereof, or mixture thereof, or a pharmaceutically acceptable salt, prodrug or solvate thereof according to any one of items 1-59, wherein the compound is any one selected from the group consisting of:

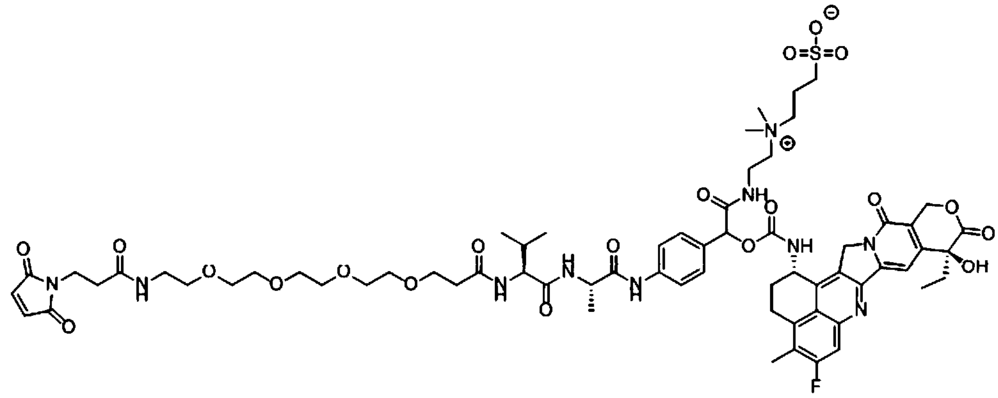

LD-31,

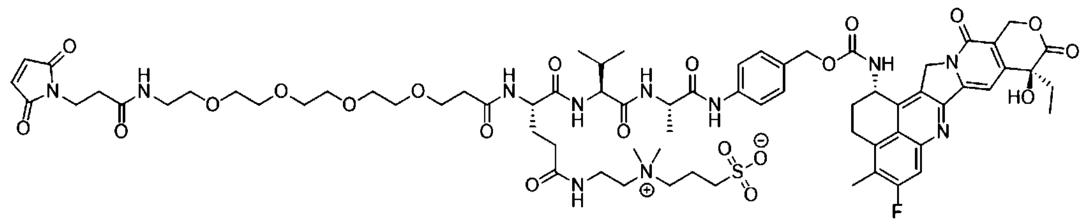

LD-37,

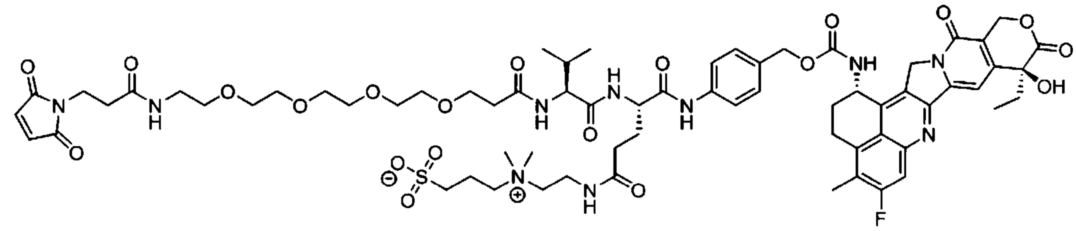

LD-38,

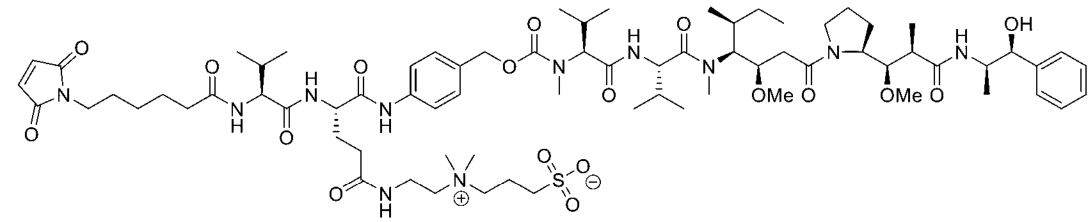

LD-57,

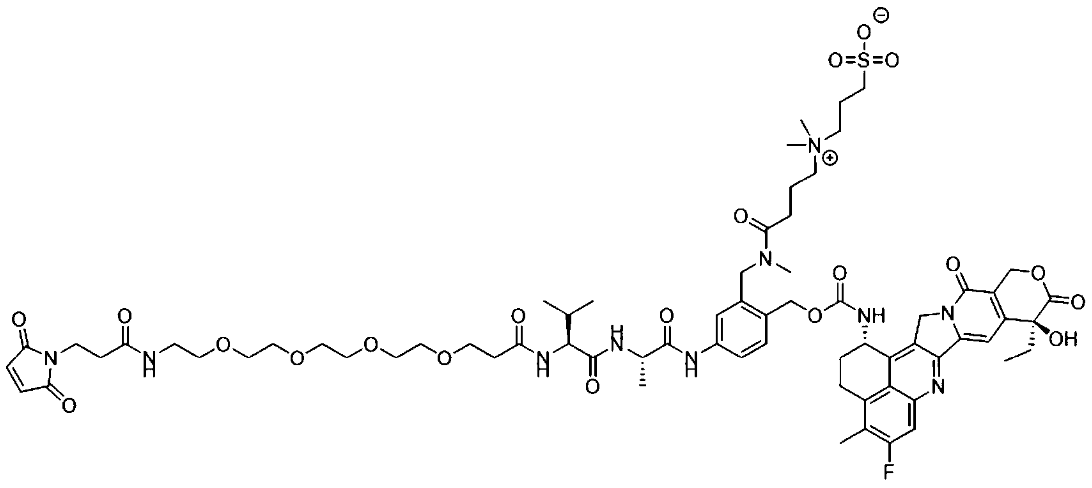

LD-65,

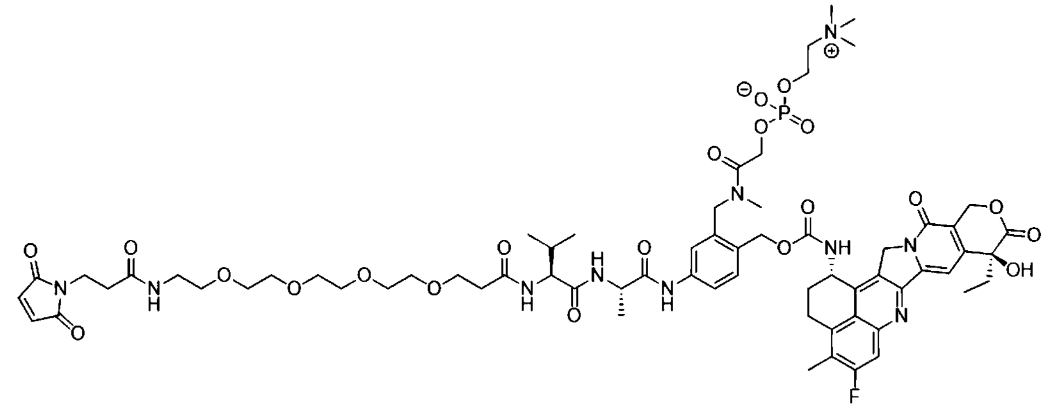

LD-66,

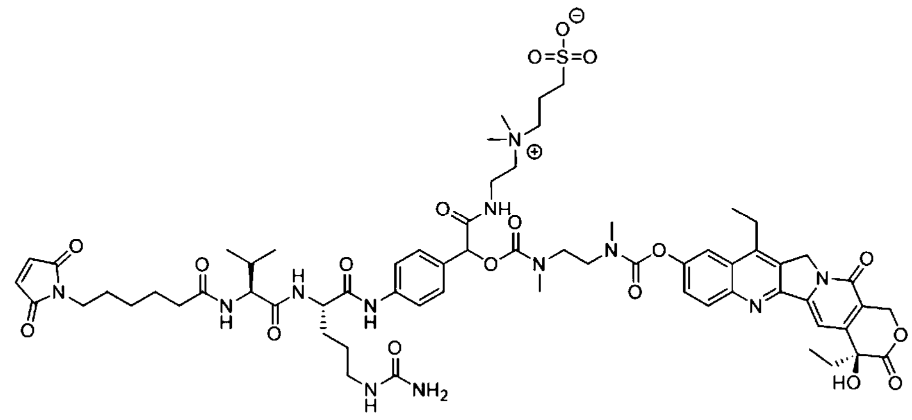

LD-71,

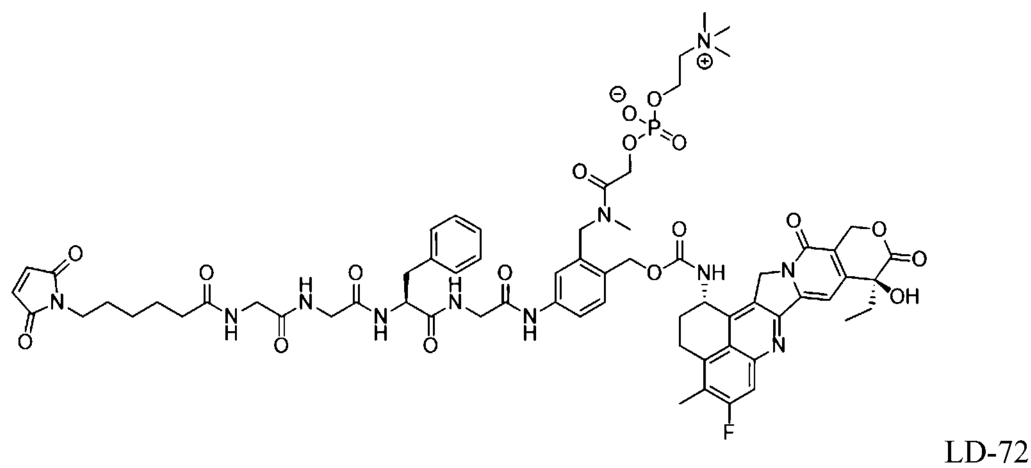

LD-72,

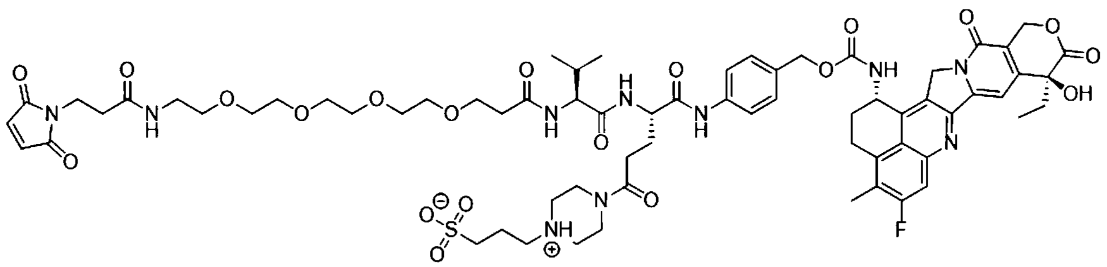

LD-88,

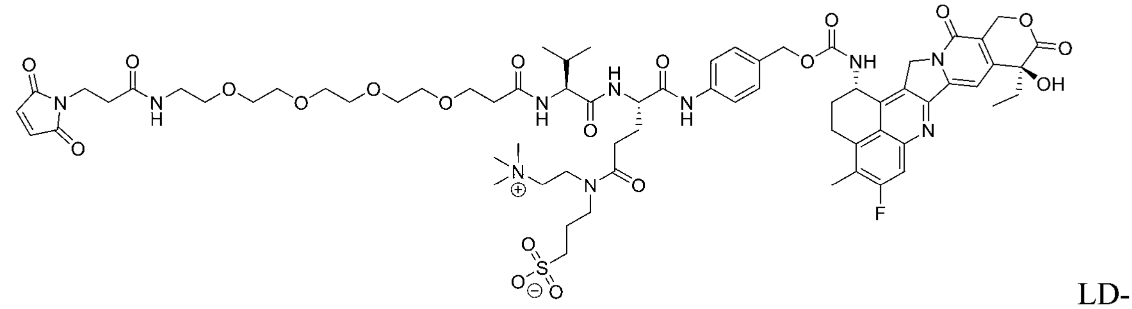

LD-89,

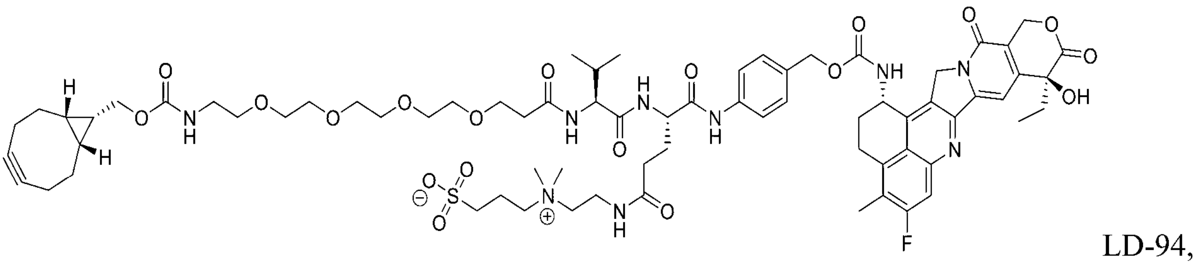

LD-94,

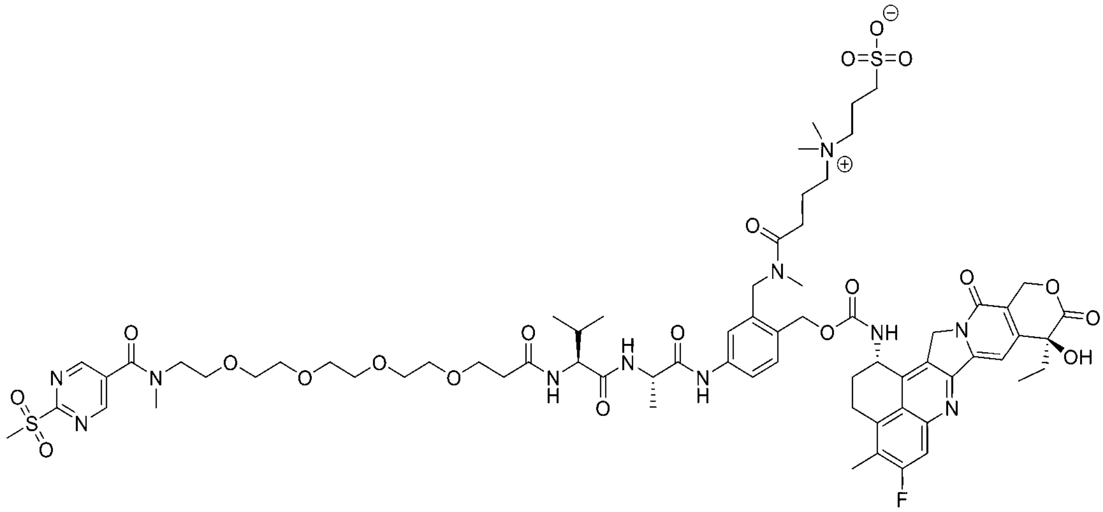

LD-98,

and

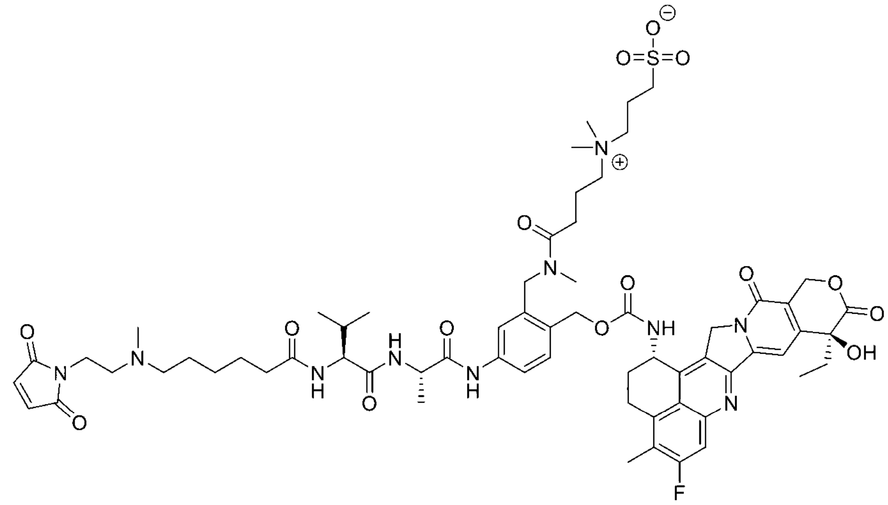

LD-99.

61. A compound represented by Formula (2), or a tautomer, mesomer, raceme, enantiomer or diastereomer thereof, or mixture thereof, or a pharmaceutically acceptable salt, prodrug or solvate thereof, wherein Formula (2) has the following structural formula:

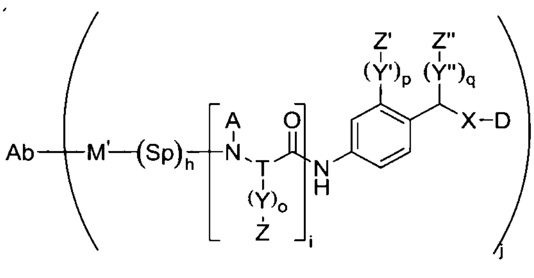

Formula (2)

Ab comprises an antibody or an antigen-binding fragment thereof; M' is a spacer; Sp is $-L_a-Q_a-$; A is selected from absent, hydrogen or alkyl; T represents a straight or branched ($C_1$-$C_8$) trivalent alkyl group; Y, Y' and Y" are each independently $-L_b-Q_b-$; $L_a$ and $L_b$ are each independently selected from absent, alkylene, alkenylene, alkynylene, alicyclylene, heteroalicyclylene, arylene, heteroarylene or polyethylene glycol group, wherein the alkylene, alkenylene, alkynylene, alicyclylene, heteroalicyclylene, arylene, heteroarylene or polyethylene glycol group is optionally substituted by a substituent; $Q_a$, and $Q_b$ are each independently selected from absent, -C(=O)NR-, -C(=O)O-, -OC(=O)NR-, $-S(=O)_2NR-$, -NRC(=O)-, -OC(=O)-, -NRC(=O)O-, $-NRS(=O)_2-$, -NRC(=O)NR-, -NHC-(=NH)NH-,

-C(=O)-, -OC(=O)O-, -O-, -NR-, -S-, $S(=O)_2$-, S(=O)-, or -Se-; wherein R is selected from hydrogen or optionally substituted alkyl; Z is selected from absent, hydrogen, optionally substituted alkyl or a sulfonate inner salt; Z' and Z" are each independently selected from absent, hydrogen, optionally substituted alkyl, a sulfonate inner salt or a phosphate inner salt; At least one of Z, Z' and Z" is a sulfonate inner salt or a phosphate inner salt; X comprises a self-immolative unit or is absent; D comprises a drug molecule; i is an integer equal to or greater than 1; h is an integer from 1 to 6; o, p, and q are each independently an integer from 0 to 5; and j is an integer equal to or greater than 1.

62. The compound, or a tautomer, mesomer, raceme, enantiomer or diastereomer thereof, or mixture thereof, or a pharmaceutically acceptable salt, prodrug or solvate thereof according to item 61, wherein the Formula (2) is Formula (2-1):

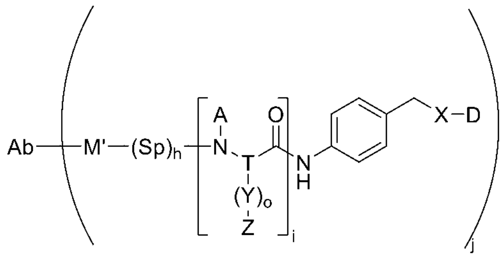

Formula (2-1)

wherein Ab, M', Sp, A, T, Y, h, o, i, j, X, D, and Z are defined as in item 61; at least one of the i Zs is a sulfonate inner salt.

63. The compound, or a tautomer, mesomer, raceme, enantiomer or diastereomer thereof, or mixture thereof, or a pharmaceutically acceptable salt, prodrug or solvate thereof according to item 62, wherein the Formula (2-1) is Formula (2-1-1):

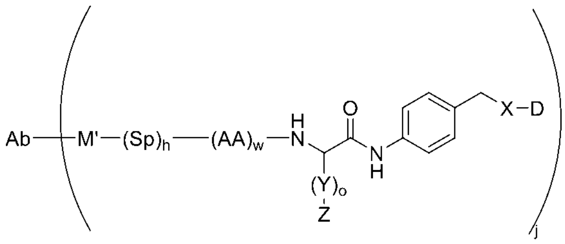

Formula (2-1-1)

wherein AA is an amino acid residue, W is an integer equal to or greater than 1.

64. The compound, or a tautomer, mesomer, raceme, enantiomer or diastereomer thereof, or mixture thereof, or a pharmaceutically acceptable salt, prodrug or solvate thereof according to item 61, wherein the Formula (2) is Formula (2-2):

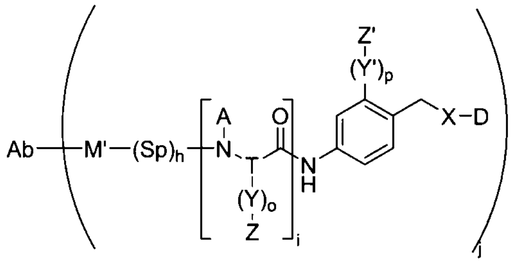

Formula (2-2)

wherein Ab, M', Sp, A, T, Y, Z, o, p, i, h, j, X, Y', and D are defined as in item 61; and Z' is a sulfonate inner salt or a phosphate inner salt.

65. The compound, or a tautomer, mesomer, raceme, enantiomer or diastereomer thereof, or mixture thereof, or a pharmaceutically acceptable salt, prodrug or solvate thereof according to item 64, wherein the Formula (2-2) is Formula (2-2-1):

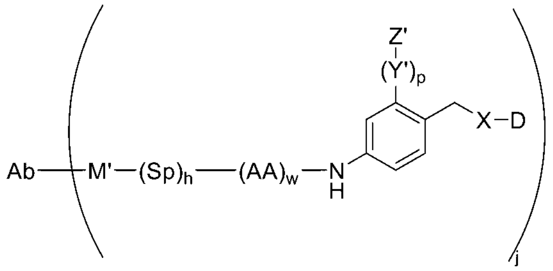

Formula (2-2-1)

wherein AA is an amino acid residue, W is an integer equal to or greater than 1.

66. The compound, or a tautomer, mesomer, raceme, enantiomer or diastereomer thereof, or mixture thereof, or a pharmaceutically acceptable salt, prodrug or solvate thereof according to item 61, wherein the Formula (2) is Formula (2-3):

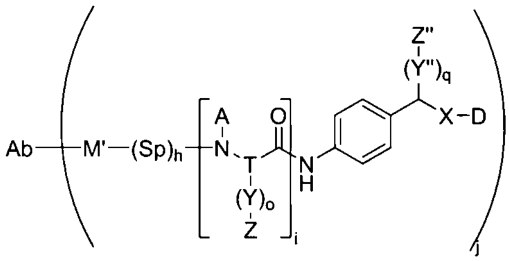

Formula (2-3)

wherein Ab, M', Sp, A, Y, T, Z, o, i, h, j, q, X, Y", and D are defined as in item 61; Z" is a sulfonate inner salt or a phosphate inner salt.

67. The compound, or a tautomer, mesomer, raceme, enantiomer or diastereomer thereof, or mixture thereof, or a pharmaceutically acceptable salt, prodrug or solvate thereof according to item 66, wherein the Formula (2-3) is Formula (2-3-1):

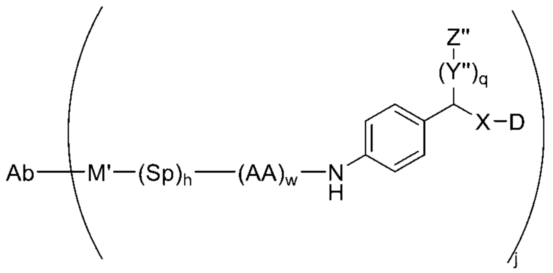

Formula (2-3-1)

wherein AA is an amino acid residue, and W is an integer equal to or greater than 1.

68. The compound, or a tautomer, mesomer, raceme, enantiomer or diastereomer thereof, or mixture thereof, or a pharmaceutically acceptable salt, prodrug or solvate thereof according to any one of items 61-67, wherein when A and Z are absent, N, Y and T form a ring to have a structural formula of:

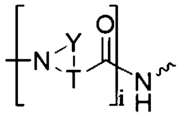

,

preferably

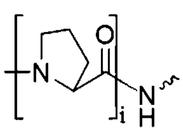

.

69. The compound, or a tautomer, mesomer, raceme, enantiomer or diastereomer thereof, or mixture thereof, or a pharmaceutically acceptable salt, prodrug or solvate thereof according to any one of items 63, 65 or 67, wherein the amino acid(s) is one or more selected from the group consisting of: alanine, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, and valine.

70. The compound, or a tautomer, mesomer, raceme, enantiomer or diastereomer thereof, or mixture thereof, or a pharmaceutically acceptable salt, prodrug or solvate thereof according to any one of items 63, 65 or 67, wherein the amino acid(s) is one or more selected from the group consisting of: valine, glutamic acid, citrulline, lysine, alanine, phenylalanine, arginine, glutamine, asparagine, and glycine.

71. The compound, or a tautomer, mesomer, raceme, enantiomer or diastereomer thereof, or mixture thereof, or a pharmaceutically acceptable salt, prodrug or solvate thereof according to any one of items 63, 65 or 67, wherein the amino acid(s) is one or more selected from the group consisting of: valine, citrulline, alanine, phenylalanine, glutamine, and glycine.

72. The compound, or a tautomer, mesomer, raceme, enantiomer or diastereomer thereof, or mixture thereof, or a pharmaceutically acceptable salt, prodrug or solvate thereof according to any one of items 61-71, wherein M' is any one selected from the group consisting of:

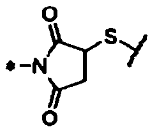, 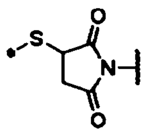, 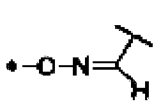, 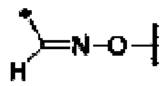, 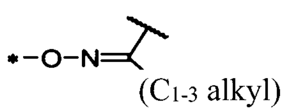,

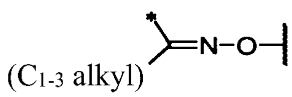, 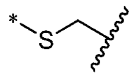, 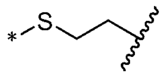, 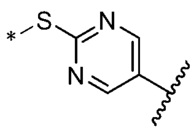, 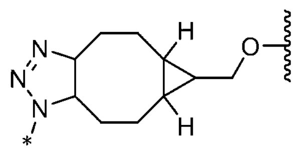,

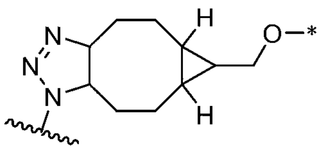, 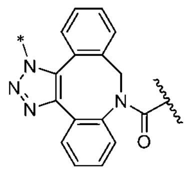, 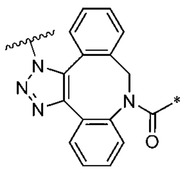, 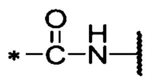 or 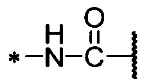;

and

* is connected to Ab.

73. The compound, or a tautomer, mesomer, raceme, enantiomer or diastereomer thereof, or mixture thereof, or a pharmaceutically acceptable salt, prodrug or solvate thereof according to any one of items 61-72, wherein M' is any one selected from the group consisting of:

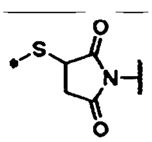, 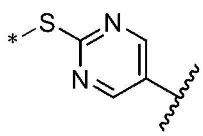, 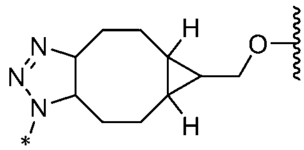, and 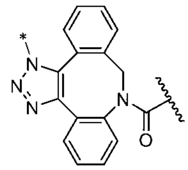.

74. The compound, or a tautomer, mesomer, raceme, enantiomer or diastereomer thereof, or mixture thereof, or a pharmaceutically acceptable salt, prodrug or solvate thereof according to any one of items 61-73, wherein M' is

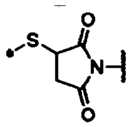

75. The compound, or a tautomer, mesomer, raceme, enantiomer or diastereomer thereof, or mixture thereof, or a pharmaceutically acceptable salt, prodrug or solvate thereof according to any one of items 61-74, wherein A is hydrogen, methyl, ethyl or propyl.

76. The compound, or a tautomer, mesomer, raceme, enantiomer or diastereomer thereof, or mixture thereof, or a pharmaceutically acceptable salt, prodrug or solvate thereof according to any one of items 61-75, wherein A is hydrogen.

77. The compound, or a tautomer, mesomer, raceme, enantiomer or diastereomer thereof, or mixture thereof, or a pharmaceutically acceptable salt, prodrug or solvate thereof according to any one of items 61-76, wherein the T is any one selected from the group consisting of:

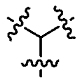, 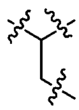, 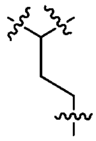, and 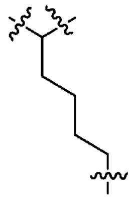.

78. The compound, or a tautomer, mesomer, raceme, enantiomer or diastereomer thereof, or mixture thereof, or a pharmaceutically acceptable salt, prodrug or solvate thereof according to any one of items 61-77, wherein the T is

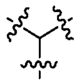.

79. The compound, or a tautomer, mesomer, raceme, enantiomer or diastereomer thereof, or mixture thereof, or a pharmaceutically acceptable salt, prodrug or solvate thereof according to any one of items 61-78, wherein $L_a$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

80. The compound, or a tautomer, mesomer, raceme, enantiomer or diastereomer thereof, or mixture thereof, or a pharmaceutically acceptable salt, prodrug or solvate thereof according to any one of items 61-79, wherein $L_a$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

81. The compound, or a tautomer, mesomer, raceme, enantiomer or diastereomer thereof, or mixture thereof, or a pharmaceutically acceptable salt, prodrug or solvate thereof according to any one of items 61-80, wherein $L_a$ is one, two, three or more selected from the group consisting of: $C_1$-$C_6$ linear or branched alkylene, $C_2$-$C_6$ linear or branched alkenylene, or $C_2$-$C_6$ linear or branched alkynylene, and polyethylene glycol group; and wherein the alkylene, alkenylene, or alkynylene is optionally substituted by a substituent.

82. The compound, or a tautomer, mesomer, raceme, enantiomer or diastereomer thereof, or mixture thereof, or a pharmaceutically acceptable salt, prodrug or solvate thereof according to any one of items 61-81, wherein $L_a$ is one, two, three or more selected from the group consisting of: $C_1$-$C_6$ linear or branched alkylene, and polyethylene glycol group;

the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$,

$-CH_2O(CH_2CH_2O)_n-$, or $-CH_2O(CH_2CH_2O)_nCH_2-$, and n is an integer equal to or greater than 1.

83. The compound, or a tautomer, mesomer, raceme, enantiomer or diastereomer thereof, or mixture thereof, or a pharmaceutically acceptable salt, prodrug or solvate thereof according to any one of items 61-82, wherein $L_a$ is one, two, three or more selected from the group consisting of: ethylidene, linear propylidene, linear butylidene, linear pentylidene, and $-(CH_2CH_2O)_nCH_2CH_2-$; and wherein n is 2, 3, 4, 5, 6, 7 or 8.

84. The compound, or a tautomer, mesomer, raceme, enantiomer or diastereomer thereof, or mixture thereof, or a pharmaceutically acceptable salt, prodrug or solvate thereof according to any one of items 61-83, wherein $L_a$ is ethylidene and/or $-(CH_2CH_2O)_4CH_2CH_2-$ or pentylidene.

85. The compound, or a tautomer, mesomer, raceme, enantiomer or diastereomer thereof, or mixture thereof, or a pharmaceutically acceptable salt, prodrug or solvate thereof according to any one of items 61-84, wherein $L_b$ is one, two, three or more selected from the group consisting of:alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, or heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

86. The compound, or a tautomer, mesomer, raceme, enantiomer or diastereomer thereof, or mixture thereof, or a pharmaceutically acceptable salt, prodrug or solvate thereof according to any one of items 61-85, wherein $L_b$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

87. The compound, or a tautomer, mesomer, raceme, enantiomer or diastereomer thereof, or mixture thereof, or a pharmaceutically acceptable salt, prodrug or solvate thereof according to any one of items 61-86, wherein $L_b$ is selected from $C_1$-$C_6$ linear or branched alkylene, $C_2$-$C_6$ linear or branched alkenylene, or $C_2$-$C_6$ linear or branched alkynylene, or polyethylene glycol group; the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; and the alkylene, alkenylene, or alkynylene is optionally substituted by a substituent.

88. The compound, or a tautomer, mesomer, raceme, enantiomer or diastereomer thereof, or mixture thereof, or a pharmaceutically acceptable salt, prodrug or solvate thereof according to any one of items 61-87, wherein $L_b$ is one, two, three or more selected from the group consisting of: $C_1$-$C_6$ linear or branched alkylene, and $C_6$-$C_{10}$ arylene.

89. The compound, or a tautomer, mesomer, raceme, enantiomer or diastereomer thereof, or mixture thereof, or a pharmaceutically acceptable salt, prodrug or solvate thereof according to any one of items 61-88, wherein $L_b$ is one, two, three or more selected from the group consisting of: methylene, ethylene, n-propylidene, isopropylidene, n-butylidene, isobutylidene, sec-butylidene, tert-butylidene, and benzylidene.

90. The compound, or a tautomer, mesomer, raceme, enantiomer or diastereomer thereof, or mixture thereof, or a pharmaceutically acceptable salt, prodrug or solvate thereof according to any one of items 61-89, wherein $Q_a$ is one, two, three or more selected from the group consisting of: -C(=O)NR-, -NR-, -C(=O)O-, -OC(=O)NR-, -NRC(=O)-, -OC(=O)-, -NRC(=O)O-, -NRC(=O)NR-, NHC-(=NH)NH-, -C(=O)-, -OC(=O)O-; and wherein R is selected from hydrogen, or optionally substituted $C_1$-$C_6$ linear or branched alkyl.

91. The compound, or a tautomer, mesomer, raceme, enantiomer or diastereomer thereof, or mixture thereof, or a pharmaceutically acceptable salt, prodrug or solvate thereof according to item 90, wherein

$Q_a$ is one, two, three or more selected from the group consisting of: -C(=O)-, -NR-, -C(=O)NR-, -NRC(=O)NR-, -OC(=O)O-, -OC(=O)NR-, -NRC(=O)- or -OC(=O)-; and wherein R is selected from hydrogen, or optionally substituted $C_1$-$C_6$ linear or branched alkyl.

92. The compound, or a tautomer, mesomer, raceme, enantiomer or diastereomer thereof, or mixture thereof, or a pharmaceutically acceptable salt, prodrug or solvate thereof according to item 90, wherein $Q_a$ is one, two, three or more selected from the group consisting of: -C(=O)-, -NR-, -NRC(=O)- and -(C=O)NR-; and wherein R is selected from hydrogen, or optionally substituted $C_1$-$C_6$ linear or branched alkyl.

93. The compound, or a tautomer, mesomer, raceme, enantiomer or diastereomer thereof, or mixture thereof, or a pharmaceutically acceptable salt, prodrug or solvate thereof according to item 90, wherein $Q_a$ is -C(=O)-, $-N(CH_3)-$, -NHC(=O)-, -C(=O)-NH- or $-C(=O)-N(CH_3)-$.

94. The compound, or a tautomer, mesomer, raceme, enantiomer or diastereomer thereof, or mixture thereof, or a pharmaceutically acceptable salt, prodrug or solvate thereof according to any one of items 61-93, wherein $Q_b$ is one,

two, three or more selected from the group consisting of: absent, -C(=O)NR-, -C(=O)O-, -OC(=O)NR-, -NRC(=O)-, -OC(=O)-, -NRC(=O)O-, -NRC(=O)NR-, NHC-(=NH)NH-, -C(=O)-, -OC(=O)O-; and wherein R is selected from hydrogen, or optionally substituted $C_1$-$C_6$ linear or branched alkyl.

95. The compound, or a tautomer, mesomer, raceme, enantiomer or diastereomer thereof, or mixture thereof, or a pharmaceutically acceptable salt, prodrug or solvate thereof according to item 94, wherein $Q_b$ is one, two, three or more selected from the group consisting of: absent, -C(=O)-, -C(=O)NR-, -OC(=O)NR-, -NRC(=O)-, -NRC(=O)O-, or -NRC(=O)NR-; and R is selected from hydrogen, or optionally substituted $C_1$-$C_6$ linear or branched alkyl.

96. The compound, or a tautomer, mesomer, raceme, enantiomer or diastereomer thereof, or mixture thereof, or a pharmaceutically acceptable salt, prodrug or solvate thereof according to item 94, wherein $Q_b$ is one, two, three or more selected from the group consisting of: absent, -C(=O), -NRC(=O)-, -NRC(=O)NR-, and -C(=O)NR-; and R is hydrogen or methyl.

97. The compound, or a tautomer, mesomer, raceme, enantiomer or diastereomer thereof, or mixture thereof, or a pharmaceutically acceptable salt, prodrug or solvate thereof according to any one of items 61-96, wherein X is selected from:

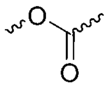 , 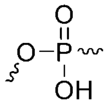 , 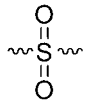 , 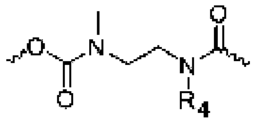 ,

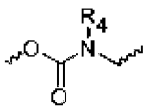 , 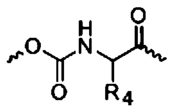 , 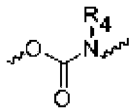 or 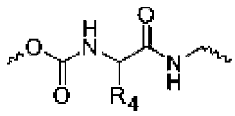 ;

the wavy line at right side of X is connected to D; $R_4$ is selected from hydrogen, $C_1$-$C_6$ linear or branched alkyl, $-(CH_2CH_2O)_mR_2$, $-CH_2CH_2NR_2R_3$, or $-CH_2CH_2SO_2R_2$-; $R_2$, and $R_3$ are each independently selected from hydrogen, or $C_1$-$C_6$ linear or branched alkyl; and m is an integer greater than 1.

98. The compound, or a tautomer, mesomer, raceme, enantiomer or diastereomer thereof, or mixture thereof, or a pharmaceutically acceptable salt, prodrug or solvate thereof according to item 97, wherein

X is

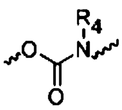 , 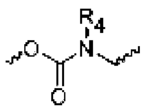 or 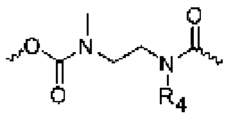

; and

$R_4$ is hydrogen, methyl, $-CH_2CH_2OCH_2CH_2OH$, $-CH_2CH_2NMe_2$, or $-CH_2CH_2SO_2Me$.

99. The compound, or a tautomer, mesomer, raceme, enantiomer or diastereomer thereof, or mixture thereof, or a pharmaceutically acceptable salt, prodrug or solvate thereof according to item 97, wherein

X is

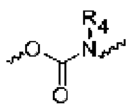 or 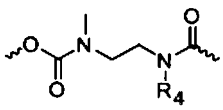 ,

and $R_4$ is hydrogen or methyl.

100. The compound, or a tautomer, mesomer, raceme, enantiomer or diastereomer thereof, or mixture thereof, or a pharmaceutically acceptable salt, prodrug or solvate thereof according to any one of items 61-99, wherein the sulfonate inner salt is

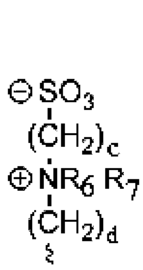, 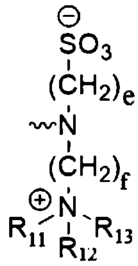 or 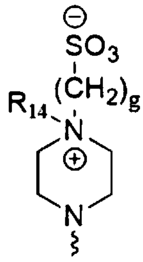,

the wavy line is connected to Y, Y' or Y";
wherein c, d, e, f, and g are each independently an integer selected from 1, 2, 3, 4, 5 or 6; and
$R_6$, $R_7$, $R_{11}$, $R_{12}$, $R_{13}$ and $R_{14}$ are each independently selected from absent, hydrogen, $C_1$-$C_6$ linear or branched alkyl.

101. The compound, or a tautomer, mesomer, raceme, enantiomer or diastereomer thereof, or mixture thereof, or a pharmaceutically acceptable salt, prodrug or solvate thereof according to item 100, wherein the sulfonate inner salt is

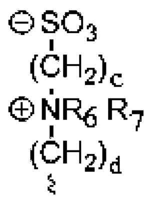, 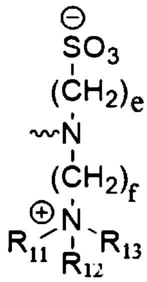 or 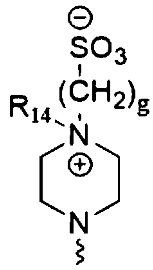,

wherein $R_6$, $R_7$, $R_{11}$, $R_{12}$, $R_{13}$ and $R_{14}$ are each independently selected from absent, methyl or ethyl, and c, d, e, f, and g are each independently 1, 2, or 3.
102. The compound, or a tautomer, mesomer, raceme, enantiomer or diastereomer thereof, or mixture thereof, or a pharmaceutically acceptable salt, prodrug or solvate thereof according to item 100, wherein the sulfonate inner salt is any one selected from the group consisting of:

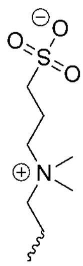, 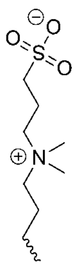, 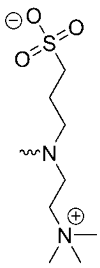, and 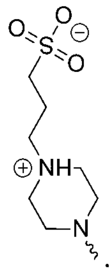.

103. The compound, or a tautomer, mesomer, raceme, enantiomer or diastereomer thereof, or mixture thereof, or a pharmaceutically acceptable salt, prodrug or solvate thereof according to any one of items 61-102, wherein the phosphate inner salt is

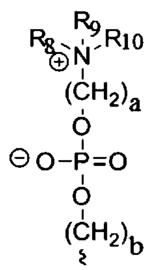,

wherein a and b are each independently an integer selected from 1, 2, 3, 4, 5 or 6; and $R_8$, $R_9$ and $R_{10}$ are each independently selected from hydrogen, or $C_1$-$C_6$ linear or branched alkyl.

104. The compound, or a tautomer, mesomer, raceme, enantiomer or diastereomer thereof, or mixture thereof, or a pharmaceutically acceptable salt, prodrug or solvate thereof according to item 103, wherein the phosphate inner salt is

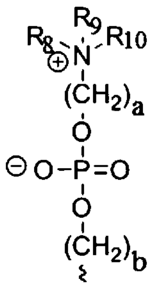

,

wherein $R_8$, $R_9$, and $R_{10}$ are each independently methyl or ethyl; a is 1, 2, or 3; and b is 1, 2, or 3.

105. The compound, or a tautomer, mesomer, raceme, enantiomer or diastereomer thereof, or mixture thereof, or a pharmaceutically acceptable salt, prodrug or solvate thereof according to item 103, wherein the phosphate inner salt is

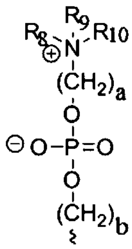

,

wherein $R_8$, $R_9$, and $R_{10}$ are each independently methyl; a is 2; and b is 1.

106. The compound, or a tautomer, mesomer, raceme, enantiomer or diastereomer thereof, or mixture thereof, or a pharmaceutically acceptable salt, prodrug or solvate thereof according to any one of items 61-105, wherein the D comprises a cytotoxin or immune agonist.

107. The compound, or a tautomer, mesomer, raceme, enantiomer or diastereomer thereof, or mixture thereof, or a pharmaceutically acceptable salt, prodrug or solvate thereof according to item 106, wherein the cytotoxin is one or two selected from the group consisting of: a microtubulin inhibitor, a DNA synthesis inhibitor, a topoisomerase inhibitor, and a RNA polymerase-2 inhibitor.

108. The compound, or a tautomer, mesomer, raceme, enantiomer or diastereomer thereof, or mixture thereof, or a pharmaceutically acceptable salt, prodrug or solvate thereof according to item 106, wherein the cytotoxin is one, two, or more selected from the group consisting of: auristatin, mertansine DM, calicheamicin, duocarmycin, pyrrolobenzodiazepine, camptothecin derivative, and $\alpha$-amanitin.

109. The compound, or a tautomer, mesomer, raceme, enantiomer or diastereomer thereof, or mixture thereof, or a pharmaceutically acceptable salt, prodrug or solvate thereof according to item 106, wherein the immune agonist is selected from TLR7/8 agonist or STING agonist.

110. The compound, or a tautomer, mesomer, raceme, enantiomer or diastereomer thereof, or mixture thereof, or a pharmaceutically acceptable salt, prodrug or solvate thereof according to any one of items 61-109, wherein i is 2, 3, 4, 5, 6, 7, or 8.

111. The compound, or a tautomer, mesomer, raceme, enantiomer or diastereomer thereof, or mixture thereof, or a pharmaceutically acceptable salt, prodrug or solvate thereof according to any one of items 61-110, wherein h is 1, 2, 3, or 4.

112. The compound, or a tautomer, mesomer, raceme, enantiomer or diastereomer thereof, or mixture thereof, or a pharmaceutically acceptable salt, prodrug or solvate thereof according to any one of items 61-111, wherein o, p, and q are each independently 0, 1, 2 or 3.

113. The compound, or a tautomer, mesomer, raceme, enantiomer or diastereomer thereof, or mixture thereof, or a pharmaceutically acceptable salt, prodrug or solvate thereof according to any one of items 61-112, wherein $L_a$ is one, two, three or more selected from the group consisting of: $C_1$-$C_6$ linear or branched alkylene, and polyethylene glycol

group; the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, n is an integer equal to or greater than 1; $Q_a$ is one, two, three or more selected from the group consisting of: -C(=O)-, -NR(C=O)-, and -(C=O)NR-; and wherein R is selected from hydrogen, or $C_1$-$C_6$ linear or branched alkyl.

114. The compound, or a tautomer, mesomer, raceme, enantiomer or diastereomer thereof, or mixture thereof, or a pharmaceutically acceptable salt, prodrug or solvate thereof according to any one of items 61-113, wherein $L_a$ is one, two, three or more selected from the group consisting of: ethylidene, linear propylidene, linear butylidene, linear pentylidene, and $-(CH_2CH_2O)_nCH_2CH_2-$; wherein n is 2, 3, 4, 5, 6, 7 or 8; $Q_a$ is one, two, three or more selected from the group consisting of: -C(=O)-, -NR-, -NR(C=O)-, and -(C=O)NR-; and wherein R is methyl and/or hydrogen.

115. The compound, or a tautomer, mesomer, raceme, enantiomer or diastereomer thereof, or mixture thereof, or a pharmaceutically acceptable salt, prodrug or solvate thereof according to item 114, wherein
$L_a$ is ethylidene and/or $-(CH_2CH_2O)_4CH_2CH_2-$ or pentylidene; and $Q_a$ is -C(=O)-, $-N(CH_3)-$, -NH(C=O)-, -C(=O)NH- or $-C(=O)-N(CH_3)-$.

116. The compound, or a tautomer, mesomer, raceme, enantiomer or diastereomer thereof, or mixture thereof, or a pharmaceutically acceptable salt, prodrug or solvate thereof according to any one of items 61-115, wherein $L_b$ is one, two, three or more selected from the group consisting of: $C_1$-$C_6$ linear or branched alkylene, $C_2$-$C_6$ linear or branched alkenylene, or $C_2$-$C_6$ linear or branched alkynylene, arylene with 6-10 carbon atoms in one or more rings, or polyethylene glycol group; the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; the alkylene, alkenylene, or alkynylene is optionally substituted by a substituent; $Q_b$ is one, two, three or more selected from the group consisting of: absent, -C(=O)NR-, -C(=O)O-, -OC(=O)NR-, -NRC(=O)-, -OC(=O)-, -NRC(=O)O-, $-NRS(=O)_2-$, -NRC(=O)NR-, -C(=O)-, -OC(=O)O-; and R is selected from hydrogen, or optionally substituted $C_1$-$C_6$ linear or branched alkyl.

117. The compound, or a tautomer, mesomer, raceme, enantiomer or diastereomer thereof, or mixture thereof, or a pharmaceutically acceptable salt, prodrug or solvate thereof according to item 116, wherein $L_b$ is one, two, three or more selected from the group consisting of: $C_1$-$C_6$ linear or branched alkylene, and $C_6$-$C_{10}$ arylene; $Q_b$ is one, two, three or more selected from the group consisting of: absent, -C(=O)-, -C(=O)NR-, -OC(=O)NR-, -NRC(=O)-, -NRC(=O)O-, and -NRC(=O)NR-; and R is selected from hydrogen, or optionally substituted $C_1$-$C_6$ linear or branched alkyl.

118. The compound, or a tautomer, mesomer, raceme, enantiomer or diastereomer thereof, or mixture thereof, or a pharmaceutically acceptable salt, prodrug or solvate thereof according to item 116, wherein $L_b$ is one, two, three or more selected from the group consisting of:methylene, ethylene, n-propylidene, isopropylidene, n-butylidene, isobutylidene, sec-butylidene, tert-butylidene, and benzylidene; $Q_b$ is one, two, three or more selected from the group consisting of: absent, -C(=O)-, -NRC(=O)-, -NRC(=O)NR-, and -C(=O)NR-; and R is hydrogen or methyl.

119. The compound, or a tautomer, mesomer, raceme, enantiomer or diastereomer thereof, or mixture thereof, or a pharmaceutically acceptable salt, prodrug or solvate thereof according to any one of items 61-118, wherein the substituent is any one selected from the group consisting of: deuterium, tritium, halogen, amino, hydroxyl, cyano, nitro, optionally substituted alkyl, alkenyl, alkynyl, optionally substituted heterocyclyl, acyl, optionally substituted amino, optionally substituted aminoformyl, optionally substituted thioaminoformyl, optionally substituted sulfamine, optionally substituted hydroxyls, optionally substituted thioalkyl (SH), and optionally substituted silyl.

120. The compound, or a tautomer, mesomer, raceme, enantiomer or diastereomer thereof, or mixture thereof, or a pharmaceutically acceptable salt, prodrug or solvate thereof according to any one of items 61-119, wherein j is an integer from 1 to 20.

121. The compound, or a tautomer, mesomer, raceme, enantiomer or diastereomer thereof, or mixture thereof, or a pharmaceutically acceptable salt, prodrug or solvate thereof according to any one of items 61-120, wherein the compound is any one selected from the group consisting of:

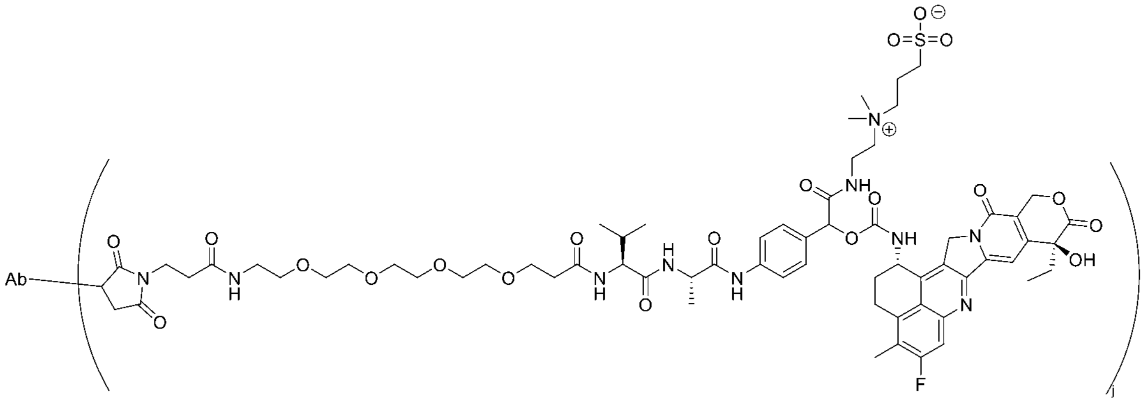

,

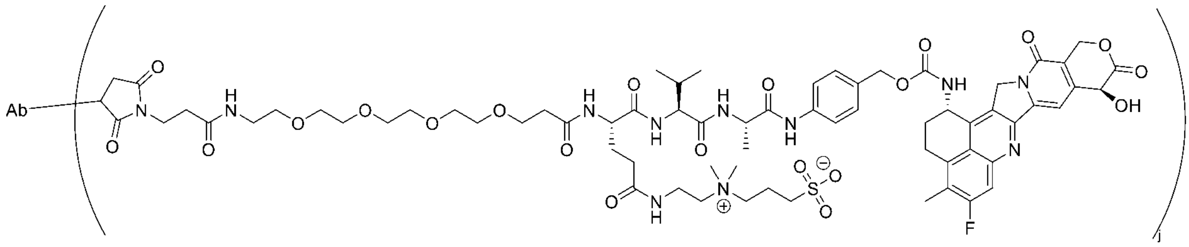

,

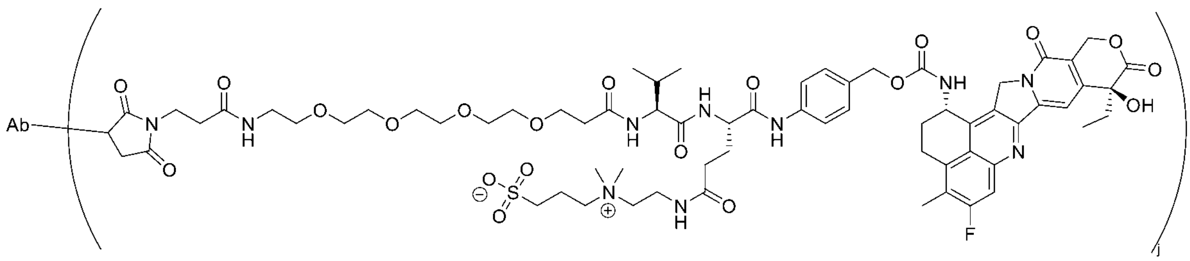

,

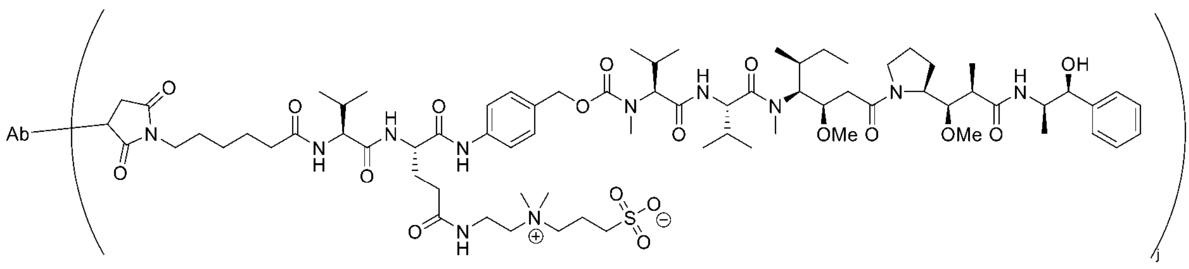

,

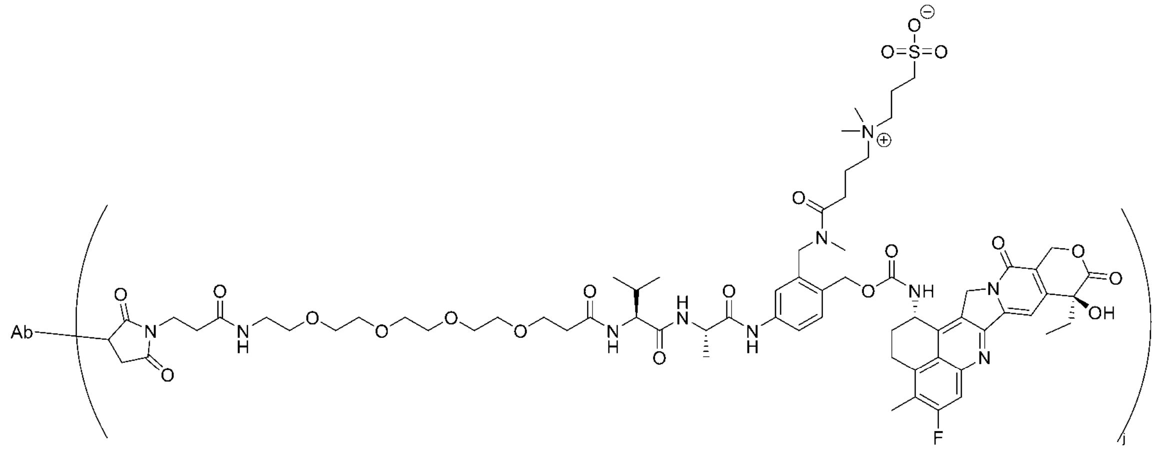

,

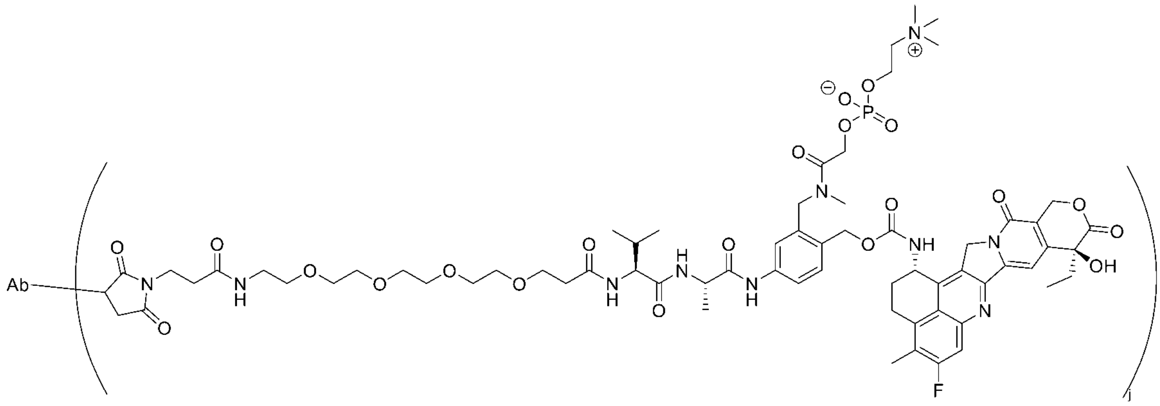

,

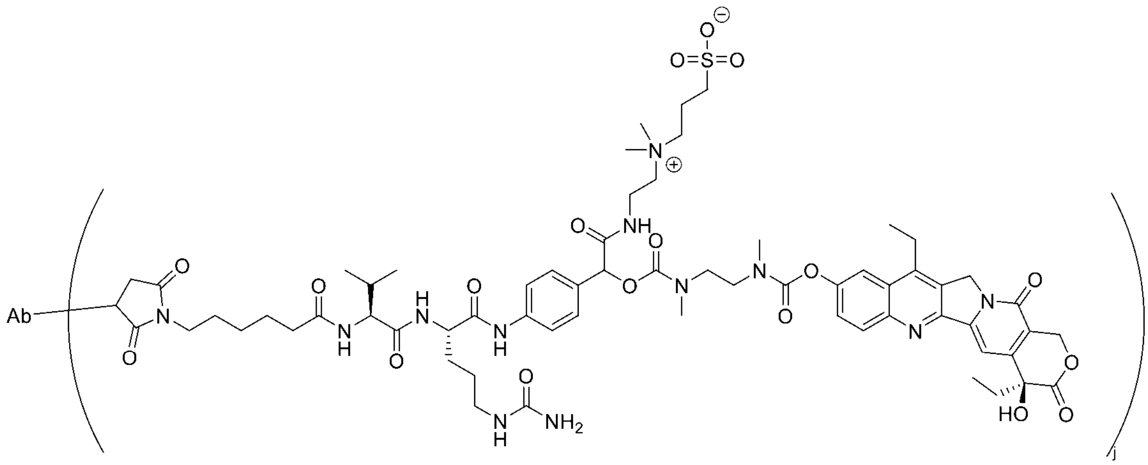

,

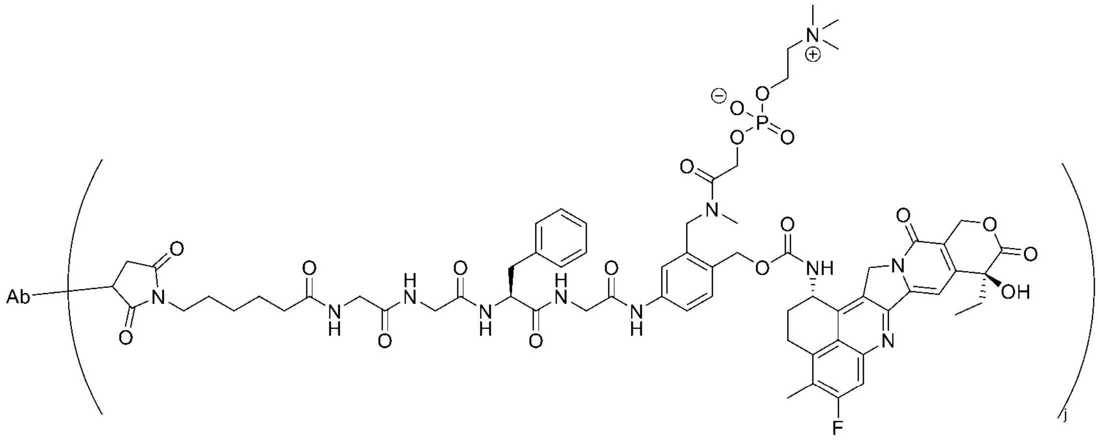

,

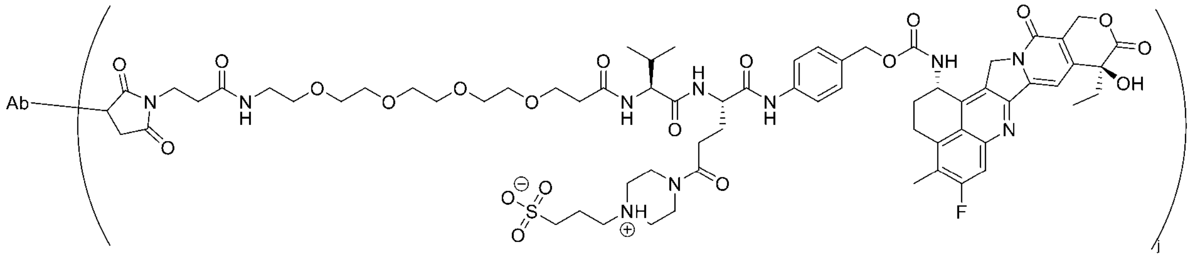

,

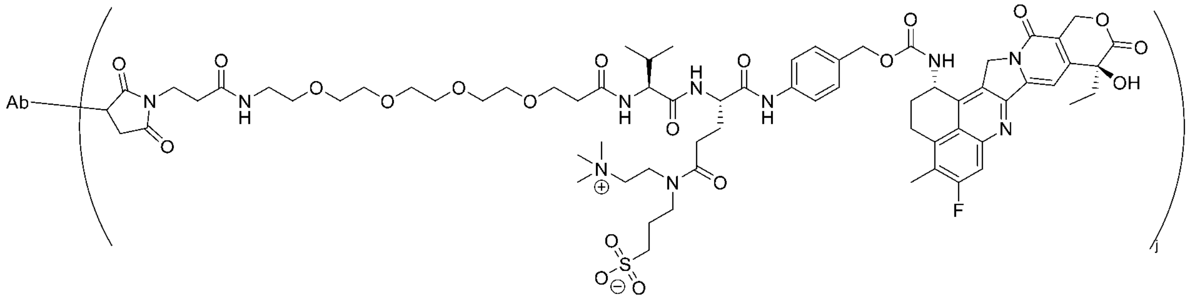

,

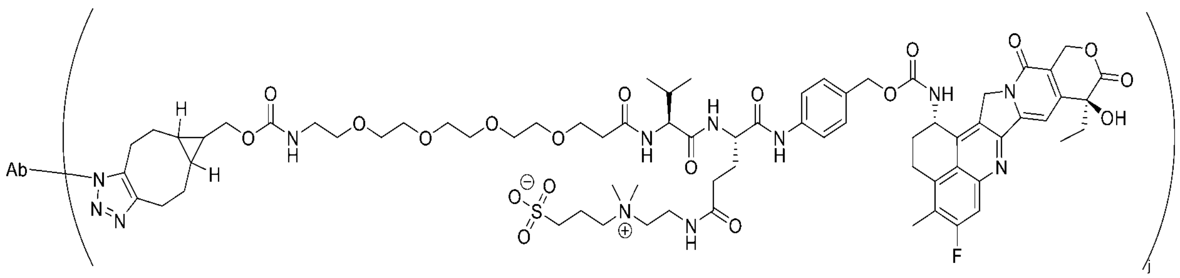

,

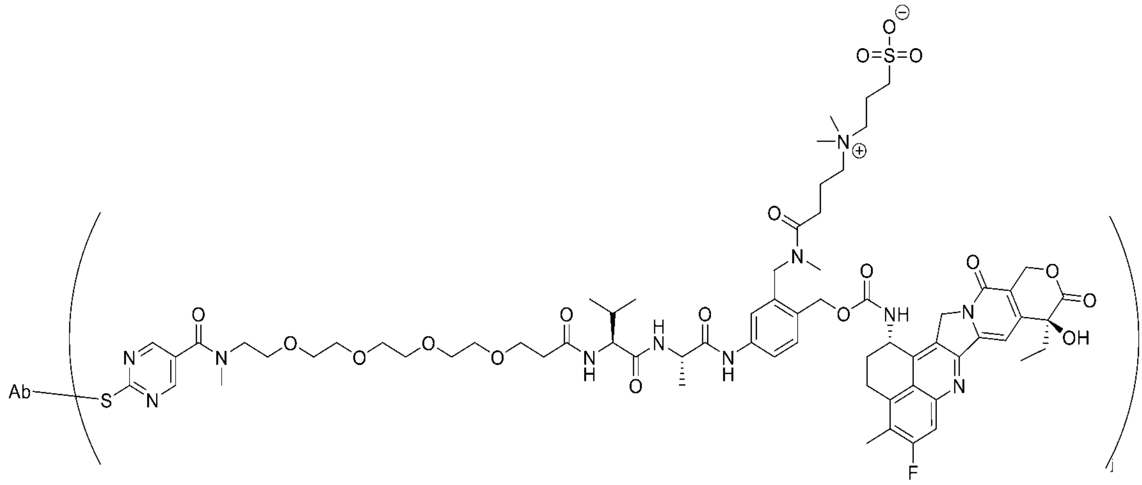

,

and

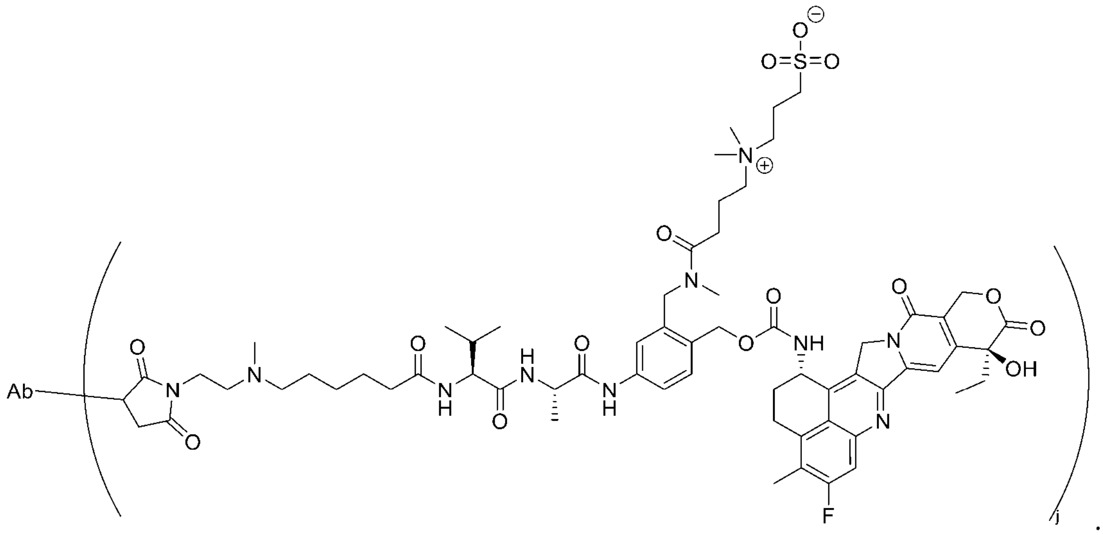

.

122. The compound, or a tautomer, mesomer, raceme, enantiomer or diastereomer thereof, or mixture thereof, or a pharmaceutically acceptable salt, prodrug or solvate thereof according to any one of items 61-121, wherein the antibody is selected from the group consisting of: a mouse-derived antibody, a chimeric antibody, a humanized antibody, and a fully human antibody.
123. The compound, or a tautomer, mesomer, raceme, enantiomer or diastereomer thereof, or mixture thereof, or a pharmaceutically acceptable salt, prodrug or solvate thereof according to any one of items 61-122, wherein the antibody comprises a monoclonal antibody.
124. The compound, or a tautomer, mesomer, raceme, enantiomer or diastereomer thereof, or mixture thereof, or a pharmaceutically acceptable salt, prodrug or solvate thereof according to any one of items 61-123, wherein the antibody comprises a bispecific antibody.
125. The compound, or a tautomer, mesomer, raceme, enantiomer or diastereomer thereof, or mixture thereof, or a pharmaceutically acceptable salt, prodrug or solvate thereof according to any one of items 61-124, wherein the antigen-binding fragment is selected from the group consisting of: Fab, Fab', Fv fragment, $F(ab')_2$, $F(ab)_2$, scFv, di-scFv and VHH.
126. The compound, or a tautomer, mesomer, raceme, enantiomer or diastereomer thereof, or mixture thereof, or a pharmaceutically acceptable salt, prodrug or solvate thereof according to any one of items 61-125, wherein the Ab is selected from the group consisting of: an anti-Claudin18.2 antibody, an anti-human folate receptor antibody, an anti-HER2 antibody, an anti-HER3 antibody, an anti-TROP2 antibody, an anti-B7-H3 antibody, an anti-GPC20 antibody, an anti-CDH6 antibody, an anti-EGFR antibody, an anti-EGFRvIII antibody, an anti-AXL antibody, an anti-Nectin-4 antibody, an anti-Tissue factor antibody, an anti-TIM-1 antibody, an anti-PSMA antibody, an anti-EpCAM antibody, an anti-MUC1 antibody, an anti-STEAP1 antibody, an anti-GPNMB antibody, an anti-FGF2 antibody, an anti-FOLR1

antibody, an anti-c-METantibody, an anti-GFR antibody, an anti-AGS-16, an anti-Guanylyl cyclase C antibody, an anti-Mesothelin antibody, an anti-SLC44A4 antibody, an anti-EphA2 antibody, an anti-AGS-5 antibody, an anti-GPC-3 antibody, an anti-c-KITantibody, an anti-ROR1 antibody, an anti-PD-L1 antibody, an anti-CD27L antibody, an anti-5T4 antibody, an anti-Mucin 16 antibody, an anti-NaPi2b antibody, an anti-STEAP antibody, an anti-SLITRK6 antibody, an anti-ETBR antibody, an anti-BCMA antibody, an anti-CEACAM5 antibody, an anti-SC-16 antibody, an anti-SLC39A6 antibody, an anti-Delta-like protein3 antibody, an anti-Claudin18.2 antibody, an anti-CD19 antibody, an anti-CD20 antibody, an anti-CD22 antibody, an anti-CD30 antibody, an anti-CD33 antibody, an anti-CD37 antibody, an anti-CD45 antibody, an anti-CD56 antibody, an anti-CD66e antibody, an anti-CD70 antibody, an anti-CD73 antibody, an anti-CD74 antibody, an anti-CD79b antibody, an anti-CD138 antibody, an anti-CD147 antibody, an anti-CD166 antibody, an anti-CD223 antibody, an anti-MUC16 antibody, an anti-MSLN antibody, an anti-ENPP3 antibody, an anti-SLTRK6 antibody, an anti-FGFR antibody, an anti-LIV-1 antibody, an anti-Lewis Y antibody, an anti-av-integrin antibody, an anti-ASCT2 antibody, an anti-C4.4a antibody, an anti-CA-IX antibody, an anti-CD324 antibody, an anti-CD352 antibody, an anti-CD44v6 antibody, an anti-CD48a antibody, an anti-CLL-1 antibody, an anti-Cripto antibody, an anti-CS1 antibody, an anti-DPEP3 antibody, an anti-Ephrin-A2 antibody, an anti-Ephrin-A4 antibody, an anti-ETBR antibody, an anti-FGFR2 antibody, an anti-FGFR3 antibody, an anti-FLT3 antibody, an anti-GD3 antibody, an anti-GloboH antibody, an anti-GPC3 antibody, an anti-LAMP-1 antibody, an anti-LRRC15 antibody, an anti-Ly6E antibody, an anti-MFI2 antibody, an anti-NOTCH3 antibody, an anti-p-cadherin antibody, an anti-PRLR antibody and an anti-RNF43 antibody, and antigen-binding fragments of the above antibodies.

127. The compound, or a tautomer, mesomer, raceme, enantiomer or diastereomer thereof, or mixture thereof, or a pharmaceutically acceptable salt, prodrug or solvate thereof according to any one of items 61-126, wherein the heavy chain HCDR1, HCDR2 and HCDR3, and light chain LCDR1, LCDR2 and LCDR3 of Ab comprise the heavy chain HCDR1, HCDR2 and HCDR3, and light chain LCDR1, LCDR2 and LCDR3 of the antibody, respectively.

128. The compound, or a tautomer, mesomer, raceme, enantiomer or diastereomer thereof, or mixture thereof, or a pharmaceutically acceptable salt, prodrug or solvate thereof according to any one of items 61-127, wherein the heavy chain variable region (VH) and light chain variable region (VL) of Ab comprise the heavy chain variable region (VH) and light chain variable region (VL) of the antibody, respectively.

129. The compound, or a tautomer, mesomer, raceme, enantiomer or diastereomer thereof, or mixture thereof, or a pharmaceutically acceptable salt, prodrug or solvate thereof according to any one of items 61-128, wherein the heavy chain and light chain of the Ab comprise the heavy chain and light chain of the antibody, respectively.

130. The compound, or a tautomer, mesomer, raceme, enantiomer or diastereomer thereof, or mixture thereof, or a pharmaceutically acceptable salt, prodrug or solvate thereof according to any one of items 61-129, wherein the Ab is an anti-Claudin18.2 antibody or an antigen-binding fragment thereof.

131. The compound, or a tautomer, mesomer, raceme, enantiomer or diastereomer thereof, or mixture thereof, or a pharmaceutically acceptable salt, prodrug or solvate thereof according to any one of items 61-130, wherein the Ab is an anti-human folate receptor antibody or an antigen-binding fragment thereof.

132, A pharmaceutical composition, comprising the compound, or a tautomer, mesomer, raceme, enantiomer or diastereomer thereof, or mixture thereof, or a pharmaceutically acceptable salt, prodrug or solvate thereof according to any one of items 1-131.

133. Use of the compound, or a tautomer, mesomer, raceme, enantiomer or diastereomer thereof, or mixture thereof, or a pharmaceutically acceptable salt, prodrug or solvate thereof according to any one of items 1-131 in the preparation of a medicament for treating and/or preventing a tumor.

134. The use according to item 133, wherein the tumor is a tumor associated with a target selected from the group consisting of: Claudin18.2, human folate receptor, HER2, HER3, TROP2, B7-H3, GPC20, CDH6, EGFR, EGFRvIII, AXL, Nectin-4, Tissue factor, TIM-1, PSMA, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, PD-L1, CD27L, 5T4, Mucin 16, NaPi2b, STEAP, SLITRK6, ETBR, BCMA, CEACAM5, SC-16, SLC39A6, Delta-like protein3, Claudin18.2, CD19, CD20, CD22, CD30, CD33, CD37, CD45, CD56, CD66e, CD70, CD73, CD74, CD79b, CD138, CD147, CD166, CD223, MUC16, MSLN, ENPP3, SLTRK6, FGFR, LIV-1, Lewis Y, av-integrin, ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, MFI2, NOTCH3, p-cadherin, PRLR and RNF43.

135. The use according to item 133, wherein the tumor associated with the targets comprises a tumor having high expression of the targets and/or a tumor with positive targets.

136. The use according to item 133, wherein the tumor is selected from the group consisting of: a solid tumor, a hematological tumor, and metastatic, refractory or recurrent lesion of cancer.

137. The use according to item 133, wherein the tumor is selected from the group consisting of: esophageal cancer, gastrointestinal cancer, pancreatic cancer, thyroid cancer, colorectal cancer, kidney cancer, lung cancer (such as non-small cell lung cancer), liver cancer, stomach cancer, gastric adenocarcinoma, gastroesophageal junction (GEJ) adenocarcinoma, head and neck cancer, bladder cancer, breast cancer, uterine cancer, cervical cancer, ovarian

cancer, prostatic cancer, testicular cancer, germ cell cancer, bone cancer, skin cancer, thymus cancer, bile duct cancer, gallbladder cancer, melanoma, mesothelioma, lymphoma, myeloma (such as multiple myeloma), sarcoma, glioblastoma and leukemia.

The antibody-drug conjugates provided in this application, as compared to the anionic and cationic hydrophilic groups of PEG, the fluorescent group-antibody conjugate modified with water-soluble groups in the form of sulfonate inner salt greatly increases the specific accumulation at the tumor site. The antibody-drug conjugates of this application introduce a strong hydrophilic sulfonate inner salt and/or phosphate inner salt side chain at the position of the polypeptide (VC, VA, AAA, etc.)-PAB (self-immolative group) of the linker. As both the sulfonate group and the phosphate group have hydrogen bond donor and acceptor, they belong to a strong hydrophilic group, and the sulfonate inner salt or phosphate inner salt has both positive and negative charges, thereby further enhancing the hydrophilicity. At the same time, the water-soluble functional groups in the form of inner salts also have the potential to enhance the tumor targeting and reduce non-specific binding of the antibody-drug conjugate, thus greatly enhancing the drug formation (including hydrophilicity and stability) of the tested antibody-drug conjugate, reducing in vivo drug clearance, and improving tumor treatment efficacy.

## BRIEF DESCRIPTION OF THE DRAWINGS

The drawings are used for a better understanding of this application, and do not constitute an improper limitation on this application, wherein:

Fig. 1 shows the size exclusion chromatography (SEC) analysis of the antibody-drug conjugates from Comparative Example 2 and Experimental Example 6 according to this application.

Fig. 2 shows the size exclusion chromatography (SEC) analysis of the antibody-drug conjugates from Comparative Example 3 and Experimental Example 9 according to this application.

Fig. 3 shows the size exclusion chromatography (SEC) analysis of the antibody-drug conjugates from Comparative Example 1 and Experimental Example 1, Experimental Example 5a, Experimental Example 7, Experimental Example 9, and Experimental Example 11 according to this application.

Fig. 4 shows the hydrophobic interaction chromatography (HIC) analysis of the antibody-drug conjugates from Comparative Example 2 and Experimental Example 6 according to this application.

Fig. 5 shows the hydrophobic interaction chromatography (HIC) analysis of the antibody-drug conjugates from Comparative Example 3 and Experimental Example 9 according to this application.

Fig. 6 shows the hydrophobic interaction chromatography (HIC) analysis of the antibody-drug conjugates from Comparative Example 1 and Experimental Example 1, Experimental Example 5a, Experimental Example 7, Experimental Example 9, and Experimental Example 11 according to this application.

Fig. 7 shows the light chain deconvolution mass spectrum of Experimental Example 5a according to this application.

Fig. 8 shows the heavy chain deconvolution mass spectrum of Experimental Example 5a according to this application.

Fig. 9 shows the reversed phase chromatogram of Experimental Example 5a according to this application.

Fig. 10 shows the in vitro cell killing curves of 9D1 antibody-drug conjugates and the control samples on MC38-18.2 cells according to this application.

Fig. 11 shows the tumor therapeutic effect curves of 9D1 antibody-drug conjugates on MC38-hClaudin18.2 mouse subcutaneous tumor model according to this application.

Fig. 12 shows the tumor therapeutic effect curves of 9D1 antibody-drug conjugates on NUGC4-hClaudin 18.2 mouse subcutaneous tumor model according to this application.

Fig. 13 shows the tumor therapeutic effect curves of Farletuzumab-LD-38 antibody-drug conjugates on OVCAR-3 mouse subcutaneous tumor model according to this application.

Fig. 14 shows the tumor therapeutic effect curves of Farletuzumab-LD-38 antibody-drug conjugates on IGROV1 mouse subcutaneous tumor model according to this application.

Fig. 15 shows the pharmacokinetic curves of 9D1-LD-38 and 9D1-GGFG-Exatecan in rats according to this application.

Fig. 16 shows the killing activity of 9D1-LD-38, 9D1-LD-82, 9D1-LD-88, and 9D1-LD-89 on MC38-hClaudin18.2 cells.

Fig. 17 shows the results of immunogenic cell death of B16F10 hClaudin18.2 cells induced by 9D1-LD-38 and 9D1-GGFG-Dxd.

Fig. 18 shows the bystander killing effect of 9D1-LD-38 and 9D1-GGFG-Dxd on HEK293-hClaudin18.2/HEK293 cells.

Fig. 19 shows the results of the release of free toxin Exatecan after enzymatic digestion of 9D1-LD-38.

Fig. 20 shows the results of the in vitro serum incubation stability of 9D1-LD-38, 9D1-LD-65, 9D1-LD-73, 9D1-LD-74, and 9D1-Deuxtecan (9D1-GGFG-Dxd).

Fig. 21 shows results of the changes of the in vivo DAR values of 9D1-LD-38, 9D1-LD-65, and 9D1-Deuxtecan (9D1-GGFG-Dxd).

Fig. 22 shows the contents of total toxins (conjugated toxin + free toxin) and free toxins in the plasma of tumor bearing mice treated with 9D1-LD-38, 9D1-LD-65, and 9D1-Deuxtecan (9D1-GGFG-Dxd).

Fig. 23 shows the contents of total toxins (conjugated toxin +free toxin) and free toxins in the tissues of tumor bearing mice treated with 9D1-LD-38, 9D1-LD-65, and 9D1-Deuxtecan (9D1-GGFG-Dxd).

Fig. 24 shows the pharmacodynamic results of 9D1 LD-38, 9D1-Deuxtecan (9D1-GGFG-Dxd) and 9D1-vc-PAB-MMAE in p-gp mediated drug-resistant tumor bearing mouse model.

Fig. 25 shows the pharmacodynamic results of 9D1-LD-38 combined with ICI administration in immunocompetent tumor bearing mouse model.

Fig. 26 shows the bystander killing of 9D1-LD-38 on tumor bearing mice.

## DETAILED DESCRIPTION

The following is an explanation of the exemplary Examples of this application, which includes various details of the Examples of this application for better understanding. They should be considered as merely exemplary. Therefore, a person skilled in the art should recognize that various changes and modifications can be made to the Examples described herein without departing from the scope and spirit of this application. Similarly, for clearance and conciseness, the following description omits the description of the well-known functions and structures.

Unless otherwise stated, the definitions of certain terms used in the description and claims of this application are as follows:

"Alkyl" may be branched or unbranched, and preferably has 1 to about 12 carbon atoms. More preferably, the alkyl has about 1 to 6 carbon atoms. Even more preferably, the alkyl group has 1, 2, 3, or 4 carbon atoms. Alkyl includes but is not limited to: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted

"Alkenyl" may be branched or unbranched, with one or more double bonds and 2 to about 12 carbon atoms. More preferably, the alkenyl has 2 to about 6 carbon atoms. Even more preferably, the alkenyl has 2, 3, or 4 carbon atoms. Alkenyl includes but is not limited to: vinyl, 1-propenyl, 2-propenyl, 1-methylvinyl, 1-butenyl, 2-butenyl, and 3-butenyl, etc. The alkenyl may be substituted or unsubstituted

"Alkynyl" may be branched or unbranched, with one or more triple bonds and 2 to about 12 carbon atoms. More preferably, the alkynyl has 2 to about 6 carbon atoms. Even more preferably, the alkynyl has 2, 3, or 4 carbon atoms. The alkynyl includes but is not limited to: acetylene, propylene, 1-butyne, etc.

"Alicyclyl" refers to cyclic substituents that do not possess aromaticity, and may be either saturated or unsaturated hydrocarbon substituents, for example, it may be cycloalkyl, cycloalkenyl, etc.

"Heteroalicyclyl" refer to substituent groups formed by heterocyclic compounds whose ring skeleton comprising at least one heteroatom and without aromaticity, wherein the heteroatom may be oxygen, nitrogen, sulfur atom, etc., including single ring, multi-ring, fused ring, bridged ring, and spiral ring. For example, a 5-7 membered single ring, or a 7-10 membered double ring or triple ring, it may comprise 1, 2, or 3 heteroatoms selected from nitrogen, oxygen, and/or sulfur atom. Heteroalicyclyl includes but is not limited to: morpholinyl, oxetanyl, thiomorpholinyl, tetrahydrofuranyl, tetrahydropyranyl, 1,1-dioxo-thiomorpholinyl, piperidinyl, 2-oxo-piperidinyl, pyrrolidinyl, 2-oxo-pyrrolidinyl, piperazin-2-one, 8-oxa-3-aza bicyclic[3.2.1]octyl, piperazinyl, and hexahydropyrimidine. Heteroalicyclyl may be substituted or unsubstituted.

"Aryl" refers to a carbon ring aromatic system comprising one or two rings, wherein the rings may be connected together in a fused manner. The term "aryl" includes single or double ring aryls, such as phenyl, naphthyl, and tetrahydronaphthyl. Preferably, the aryl is C6-C10 aryl; more preferably, the aryl is phenyl, naphthyl; most preferably, the aryl is naphthyl. The aryl may be substituted or unsubstituted.

"Heteroaryl" refers to an aromatic group consisting of a 5-6 membered single ring or a 8-10 membered double ring, which may comprise 1 to 4 heteroatoms selected from nitrogen, oxygen, and/or sulfur. Examples of "heteroaryl " include but are not limited to: furanyl, pyridinyl, 2-oxo-1,2-dihydropyridyl, pyridazinyl, pyrimidinyl, pyrazinyl, thiophenyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothiophenyl, benzoimidazolyl, indolyl, isoindolyl, 1,3-dioxo-isoindolyl, quinolinyl, indazolyl, benzoisothiazolyl, benzoxazolyl, benzoisoxazolyl. Heteroaryl may be substituted or unsubstituted.

"Substituted" refers to one or more hydrogen atoms, preferably up to 5, and more preferably 1-3 hydrogen atoms in a functional group are independently replaced by a corresponding number of substituents. It goes without saying that, substituents are only located in their possible chemical positions, and a person skilled in the art can determine (through experiments or theory) possible or impossible substitutions without excessive effort. For example, an amino

or hydroxyl with free hydrogen may be unstable when combined with a carbon atom with unsaturated (such as olefinic) bonds.

The term "substituted" as described herein, unless otherwise specified, refers to a group that may be substituted by one or more groups selected from the group consisting of: alkyl, alkoxyl, alkylthio, alkylamino, halogen, sulfenyl, hydroxyl, nitro, cyano, cycloalkyl, heterocyclyl, aryl, heteroaryl, cycloalkoxyl, heterocycloalkoxyl, cycloalkylthio, heterocycloalkylthio, amino, haloalkyl, hydroxyalkyl substituents.

Whenever a functional group is described as "optionally substituted by a substituent," it can be either unsubstituted or substituted by one or more substituents. Similarly, when a group is described as "unsubstituted or substituted", if it is substituted, the substituent can be selected from one or more of the listed substituents. If no substituent is specified, it means that the "optionally substituted" or "substituted" groups can be replaced by one or more groups, which are individually or independently selected from: alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxyl, protected hydroxyl, alkoxy, aryloxy, acyl, mercapto, alkylthio, arylthio, cyano, halogen, thiocarbonyl, O-carbamyl, N-carbamyl, O-thiocarbamyl, N-thiocarbamyl, C-amide, N-amide, S-sulfonamide, N-sulfonamide, C-carboxyl, protected C-carboxyl, O-carboxyl, isocyanate, thiocyanate, isothiocyanate, nitro, silicyl, sulfenyl, sulfinyl, sulfonyl, haloalkyl, haloalkoxy, trihalomethanesulfonyl, trihalomethanesulfamine, amino, monosubstituted amino, and disubstituted amino and their protected derivatives

Halogen substituents include F, Cl, Br, and I. "Hydroxyl" refers to -OH group. Halogens refer to fluorine, chlorine, bromine, and iodine.

"Amino" refers to $-NH_2$. "Cyano" refers to -CN. "Nitro" refers to $-NO_2$. "Carboxyl" refers to -C(O)OH. "Acyl" refers to -C(O)R.

The terms "hydrogen" or "H" in the chemical structure used herein, unless otherwise specified, should be understood to include not only $^1H$, but also deuterium ($^2H$, D), tritium ($^3H$, T), or their mixture. Deuterinated isotope derivatives refer to the corresponding compounds obtained by replacing one or more H atoms with D atoms in a compound.

"Pharmaceutical composition" refers to a mixture of one or more compounds described herein, or their physiologically and pharmaceutically acceptable salts or the precursor drugs with other chemical components, as well as other components such as physiologically and pharmaceutically acceptable carriers and excipients. The object of a pharmaceutical composition is to promote the administration of a drug to living organisms, facilitate the absorption of active ingredients, and thus exert biological activity.

The present application provides a compound represented by Formula (1), or a tautomer, mesomer, raceme, enantiomer or diastereomer thereof, or mixture thereof, or a pharmaceutically acceptable salt, prodrug or solvate thereof, wherein Formula (1) has the following structural formula:

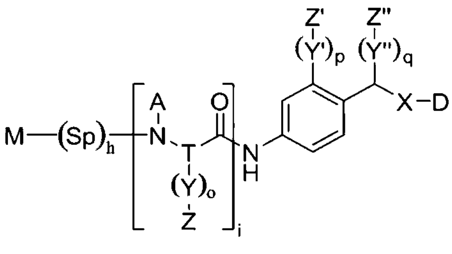

Formula (1)

M is a spacer; Sp is $-L_a-Q_a-$; A is selected from absent, hydrogen or alkyl; T represents a straight or branched ($C_1$-$C_8$) trivalent alkyl group; Y, Y' and Y" are each independently $-L_b-Q_b-$; $L_a$ and $L_b$ are each independently selected from absent, alkylene, alkenylene, alkynylene, alicyclylene, heteroalicyclylene, arylene, heteroarylene or polyethylene glycol group, wherein the alkylene, alkenylene, alkynylene, alicyclylene, heteroalicyclylene, arylene, heteroarylene or polyethylene glycol group is optionally substituted by a substituent; $Q_a$, and $Q_b$ are each independently selected from absent, -C(=O)NR-, -C(=O)O-, -OC(=O)NR-, $-S(=O)_2NR-$, -NRC(=O)-, -OC(=O)-, -NRC(=O)O-, $-NRS(=O)_2-$, -NRC(=O)NR-, -NHC-(=NH)NH-, -C(=O)-, -OC(=O)O-, -O-, -NR-, -S-, $S(=O)_2-$, S(=O)-, or -Se-, wherein R is selected from hydrogen or optionally substituted alkyl; Z is selected from absent, hydrogen, optionally substituted alkyl or a sulfonate inner salt; Z' and Z" are each independently selected from absent, hydrogen, optionally substituted alkyl, a sulfonate inner salt or a phosphate inner salt; At least one of Z, Z' and Z" is a sulfonate inner salt or a phosphate inner salt; X comprises a self-immolative unit or is absent; D comprises a drug molecule; i is an integer equal to or greater than 1, for example, it may be 1, 2, 3, 4, 5, 6, 7, 8, 9, 10, 11, 12, 13, 14, 15, etc. h is an integer from 1 to 6, for example, it may be 1, 2, 3, 4, 5, 6, etc. o, p, and q are each independently an integer from 0 to 5, for example, it may be 0, 1, 2, 3, 4, 5, etc.

When h is greater than 1, multiple Sp may have different structures, or identical structures, i.e., multiple $L_a$ may have different structures, or identical structures; and multiple $Q_a$ may have different structures, or identical structures. The number of $L_a$ and $Q_a$ may be different or identical.

When i is greater than 1, multiple A may be different or identical, multiple T may be different or identical, multiple Y may be different or identical, and multiple Z may be different or identical.

When p is greater than 1, multiple Y' may have different structures, or identical structures.

When q is larger than 1, multiple Y" may have different structures, or identical structures.

In the present application, the Formula (1) is Formula (1-1):

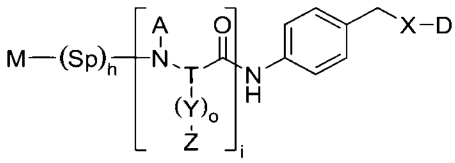

Formula (1-1)

wherein M, Sp, A, T, Y, h, o, i, X, D, and Z are defined as the above; and at least one of the i Zs is a sulfonate inner salt.

In the present application, the Formula (1-1) is Formula (1-1-1):

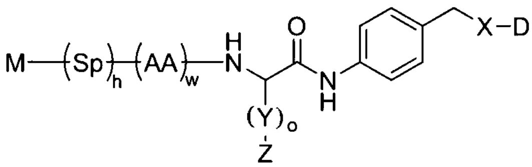

· Formula (1-1-1)

wherein AA is an amino acid residue, and w is an integer equal to or greater than 1, for example, it may be 1, 2, 3, 4, 5, 6, etc. When w is greater than 1, multiple AAs may be identical amino acid residues, or different amino acid residues.

In the present application, the Formula (1) is Formula (1-2):

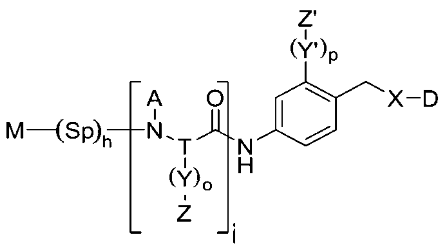

Formula (1-2)

wherein M, Sp, A, T, Y, Z, o, p, i, h, X, Y', and D are defined as the above; and Z' is a sulfonate inner salt or a phosphate inner salt.

In the present application, the Formula (1) is Formula (1-2-1):

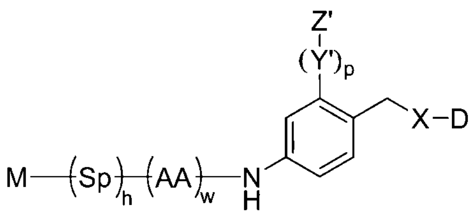

Formula (1-2-1)

wherein AA is an amino acid residue, and w is an integer equal to or greater than 1, for example, it may be 1, 2, 3, 4, 5, 6, etc. When w is greater than 1, multiple AAs may be identical amino acid residues, or different amino acid residues.

In the present application, the Formula (1) is Formula (1-3):

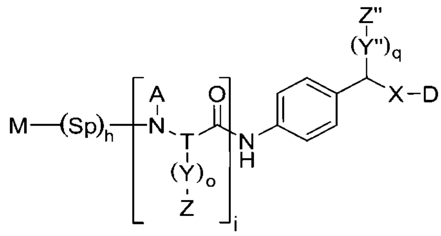

Formula (1-3)

wherein M, Sp, A, Y, T, Z, o, i, h, q, X, Y", and D are defined as the above; and Z" is a sulfonate inner salt or a phosphate inner salt.

In the present application, when Z is hydrogen or optionally substituted alkyl,

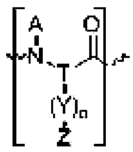

is an amino acid residue, also referred to as AA.

In the present application, when Z is a sulfonate inner salt,

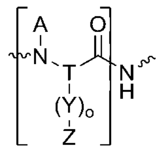

is

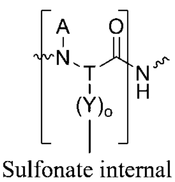

Sulfonate internal salt.

In the present application, when A and Z are absent, N, Y and T form a ring, and

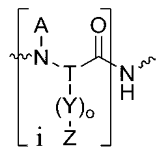

is

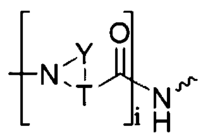

,

preferably

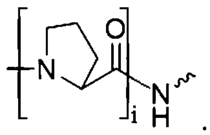

In the present application, when Z is hydrogen or optionally substituted alkyl, the Formula (1) is Formula (1-3-1):

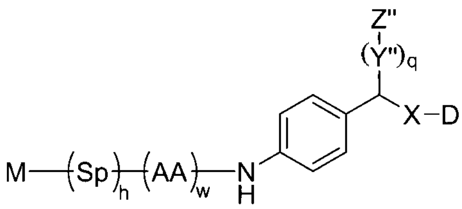

Formula (1-3-1)

wherein AA is an amino acid residue, and w is an integer equal to or greater than 1, for example, it may be 1, 2, 3, 4, 5, 6, etc. When w is greater than 1, multiple AAs may be identical amino acid residues, or different amino acid residues.

In the present application, the amino acid(s) is one or more selected from the group consisting of: alanine, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, and valine.

In the present application, the amino acid(s) is one or more selected from the group consisting of: valine, glutamic acid, citrulline, lysine, alanine, phenylalanine, arginine, glutamine, asparagine, and glycine.

In the present application, the amino acid(s) is one or more selected from the group consisting of: valine, citrulline, alanine, phenylalanine, glutamine, and glycine.

When w is greater than 1, multiple AAs may be different or identical, the details are as follows:

In some embodiments, when w is 2, two AAs may be each independently valine residue and citrulline residue.

In some embodiments, when w is greater than 2, w AAs may be each independently valine residue and citrulline residue, wherein the number of valine residues may be 1, 2, or more; or the number of citrulline residues may be 1, 2, or more; for example, when w is 3, three AAs are each independently two valine residues and one citrulline residue. For another example, when w is 3, three AAs are each independently one valine residue and two citrulline residues.

In some embodiments, when w is 2, two AAs may be each independently valine residue and alanine residue.

In some embodiments, when w is greater than 2, w AAs may be each independently valine residue and alanine residue, wherein the number of valine residues may be 1, 2, or more; or the number of alanine residues may be 1, 2, or more; for example, when w is 3, three AAs are each independently two valine residues and one alanine residue. For another example, when w is 3, three AAs are each independently one valine residue and two alanine residues.

In some embodiments, when w is 2, two AAs may be each independently valine residue and phenylalanine residue.

In some embodiments, when w is greater than 2, w AAs may be each independently valine residue and phenylalanine residue, wherein the number of valine residues may be 1, 2, or more; or the number of phenylalanine residues may be 1, 2, or more; for example, when w is 3, three AAs are each independently two valine residues and one phenylalanine residue. For another example, when w is 3, three AAs are each independently one valine residue and two phenylalanine residue.

In some embodiments, when w is 2, two AAs may be each independently valine residue and glutamine residue.

In some embodiments, when w is greater than 2, w AAs may be each independently valine residue and glutamine residue, wherein the number of valine residues may be 1, 2, or more; or the number of glutamine residues may be 1, 2, or more; for example, when w is 3, three AAs are each independently two valine residues and one glutamine residue. For another example, when w is 3, three AAs are each independently one valine residue and two glutamine residues.

In some embodiments, when w is 2, two AAs may be each independently valine residue and glycine residue.

In some embodiments, when w is greater than 2, w AAs may be each independently valine residue and glycine residue, wherein the number of valine residues may be 1, 2, or more; or the number of glycine residues may be 1, 2, or more; for example, when w is 3, three AAs are each independently two valine residues and one glycine residue. For another example, when w is 3, three AAs are each independently one valine residue and two glycine residues.

In some embodiments, when w is 2, two AAs may be each independently citrulline residue and alanine residue.

In some embodiments, when w is greater than 2, w AAs may be each independently citrulline residue and alanine residue, wherein the number of citrulline residues may be 1, 2, or more; or the number of alanine residues may be 1, 2, or more; for example, when w is 3, three AAs are each independently two citrulline residues and one alanine residue. For

another example, when w is 3, three AAs are each independently one citrulline residue and two alanine residues.

In some embodiments, when w is greater than 1, w AAs may be each independently citrulline residue and phenylalanine residue, wherein the number of citrulline residues may be 1, 2, or more; or the number of phenylalanine residues may be 1, 2, or more.

In some embodiments, when w is greater than 1, w AAs may be each independently citrulline residue and glutamine residue, wherein the number of citrulline residues may be 1, 2, or more; or the number of glutamine residues may be 1, 2, or more.

In some embodiments, when w is greater than 1, w AAs may be each independently citrulline residue and glycine residue, wherein the number of citrulline residues may be 1, 2, or more; or the number of glycine residues may be 1, 2, or more.

In some embodiments, when w is greater than 1, w AAs may be each independently alanine residue and phenylalanine residue, wherein the number of alanine residues may be 1, 2, or more; or the number of phenylalanine residues may be 1, 2, or more.

In some embodiments, when w is greater than 1, w AAs may be each independently alanine residue and glutamine residue, wherein the number of alanine residues may be 1, 2, or more; or the number of glutamine residues may be 1, 2, or more.

In some embodiments, when w is greater than 1, w AAs may be each independently alanine residue and glycine residue, wherein the number of alanine residues may be 1, 2, or more; or the number of glycine residues may be 1, 2, or more.

In some embodiments, when w is greater than 1, w AAs may be each independently phenylalanine residue and glutamine residue, wherein the number of phenylalanine residues may be 1, 2, or more; or the number of glutamine residues may be 1, 2, or more.

In some embodiments, when w is greater than 1, w AAs may be each independently phenylalanine residue and glycine residue, wherein the number of phenylalanine residues may be 1, 2, or more; or the number of glycine residues may be 1, 2, or more.

In some embodiments, when w is greater than 1, w AAs may be each independently glutamine residue and glycine residue, wherein the number of glutamine residues may be 1, 2, or more; or the number of glycine residues may be 1, 2, or more.

In some embodiments, when w is greater than 1, w AAs may be each independently valine residue, citrulline residue and alanine residue, wherein the number of valine residues may be 1, 2, or more; the number of citrulline residues may be 1, 2, or more; or the number of alanine residues may be 1, 2, or more.

In some embodiments, when w is greater than 1, w AAs may be each independently valine residue, citrulline residue and phenylalanine residue, wherein the number of valine residues may be 1, 2, or more; the number of citrulline residues may be 1, 2, or more; or the number of phenylalanine residues may be 1, 2, or more.

In some embodiments, when w is greater than 1, w AAs may be each independently valine residue, citrulline residue and glutamine residue, wherein the number of valine residues may be 1, 2, or more; the number of citrulline residues may be 1, 2, or more; or the number of glutamine residues may be 1, 2, or more.

In some embodiments, when w is greater than 1, w AAs may be each independently valine residue, citrulline residue and glycine residue, wherein the number of valine residues may be 1, 2, or more; the number of citrulline residues may be 1, 2, or more; or the number of glycine residues may be 1, 2, or more.

In some embodiments, when w is greater than 1, w AAs may be each independently valine residue, alanine residue and phenylalanine residue, wherein the number of valine residues may be 1, 2, or more; the number of alanine residues may be 1, 2, or more; or the number of phenylalanine residues may be 1, 2, or more.

In some embodiments, when w is greater than 1, w AAs may be each independently valine residue, alanine residue and glutamine residue, wherein the number of valine residues may be 1, 2, or more; the number of alanine residues may be 1, 2, or more; or the number of glutamine residues may be 1, 2, or more.

In some embodiments, when w is greater than 1, w AAs may be each independently valine residue, alanine residue and glycine residue, wherein the number of valine residues may be 1, 2, or more; the number of alanine residues may be 1, 2, or more; or the number of glycine residues may be 1, 2, or more.

In some embodiments, when w is greater than 1, w AAs may be each independently valine residue, phenylalanine residue and glutamine residue, wherein the number of valine residues may be 1, 2, or more; the number of phenylalanine residues may be 1, 2, or more; or the number of glutamine residues may be 1, 2, or more.

In some embodiments, when w is greater than 1, w AAs may be each independently valine residue, phenylalanine residue and glycine residue, wherein the number of valine residues may be 1, 2, or more; the number of phenylalanine residues may be 1, 2, or more; or the number of glycine residues may be 1, 2, or more.

In some embodiments, when w is greater than 1, w AAs may be each independently valine residue, glutamine residue and glycine residue, wherein the number of valine residues may be 1, 2, or more; the number of glutamine residues may be 1, 2, or more; or the number of glycine residues may be 1, 2, or more.

In some embodiments, when w is greater than 1, w AAs may be each independently citrulline residue, alanine residue and phenylalanine residue, wherein the number of citrulline residues may be 1, 2, or more; the number of alanine residues may be 1, 2, or more; or the number of phenylalanine residues may be 1, 2, or more.

In some embodiments, when w is greater than 1, w AAs may be each independently citrulline residue, alanine residue and glutamine residue, wherein the number of citrulline residues may be 1, 2, or more; the number of alanine residues may be 1, 2, or more; or the number of glutamine residues may be 1, 2, or more.

In some embodiments, when w is greater than 1, w AAs may be each independently citrulline residue, alanine residue and glycine residue, wherein the number of citrulline residues may be 1, 2, or more; the number of alanine residues may be 1, 2, or more; or the number of glycine residues may be 1, 2, or more.

In some embodiments, when w is greater than 1, w AAs may be each independently alanine residue, phenylalanine residue and glutamine residue, wherein the number of alanine residues may be 1, 2, or more; the number of phenylalanine residues may be 1, 2, or more; or the number of glutamine residues may be 1, 2, or more.

In some embodiments, when w is greater than 1, w AAs may be each independently alanine residue, phenylalanine residue and glycine residue, wherein the number of alanine residues may be 1, 2, or more; the number of phenylalanine residues may be 1, 2, or more; or the number of glycine residues may be 1, 2, or more.

In some embodiments, when w is greater than 1, w AAs may be each independently phenylalanine residue, glutamine residue and glycine residue, wherein the number of phenylalanine residues may be 1, 2, or more; the number of glutamine residues may be 1, 2, or more; or the number of glycine residues may be 1, 2, or more.

In some embodiments, when w is greater than 1, w AAs may be each independently valine residue, citrulline residue, alanine residue and phenylalanine residue, wherein the number of valine residues may be 1, 2, or more; the number of citrulline residues may be 1, 2, or more; the number of alanine residues may be 1, 2, or more; or the number of phenylalanine residues may be 1, 2, or more.

In some embodiments, when w is greater than 1, w AAs may be each independently valine residue, citrulline residue, alanine residue and glutamine residue, wherein the number of valine residues may be 1, 2, or more; the number of citrulline residues may be 1, 2, or more; the number of alanine residues may be 1, 2, or more; or the number of glutamine residues may be 1, 2, or more.

In some embodiments, when w is greater than 1, w AAs may be each independently valine residue, citrulline residue, alanine residue and glycine residue, wherein the number of valine residues may be 1, 2, or more; the number of citrulline residues may be 1, 2, or more; the number of alanine residues may be 1, 2, or more; or the number of glycine residues may be 1, 2, or more.

In some embodiments, when w is greater than 1, w AAs may be each independently citrulline residue, alanine residue, phenylalanine residue and glutamine residue, wherein the number of citrulline residues may be 1, 2, or more; the number of alanine residues may be 1, 2, or more; the number of phenylalanine residues may be 1, 2, or more; or the number of glutamine residues may be 1, 2, or more.

In some embodiments, when w is greater than 1, w AAs may be each independently citrulline residue, alanine residue, phenylalanine residue and glycine residue, wherein the number of citrulline residues may be 1, 2, or more; the number of alanine residues may be 1, 2, or more; the number of phenylalanine residues may be 1, 2, or more; or the number of glycine residues may be 1, 2, or more.

In some embodiments, when w is greater than 1, w AAs may be each independently alanine residue, phenylalanine residue, glutamine residue and glycine residue, wherein the number of alanine residues may be 1, 2, or more; the number of phenylalanine residues may be 1, 2, or more; the number of glutamine residues may be 1, 2, or more; or the number of glycine residues may be 1, 2, or more.

In some embodiments, when w is greater than 1, w AAs may be each independently valine residue, citrulline residue, alanine residue, phenylalanine residue and glutamine residue, wherein the number of valine residues may be 1, 2, or more; the number of citrulline residues may be 1, 2, or more; the number of alanine residues may be 1, 2, or more; the number of phenylalanine residues may be 1, 2, or more; or the number of glutamine residues may be 1, 2, or more.

In some embodiments, when w is greater than 1, w AAs may be each independently valine residue, citrulline residue, alanine residue, phenylalanine residue and glycine residue, wherein the number of valine residues may be 1, 2, or more; the number of citrulline residues may be 1, 2, or more; the number of alanine residues may be 1, 2, or more; the number of phenylalanine residues may be 1, 2, or more; or the number of glycine residues may be 1, 2, or more.

In some embodiments, when w is greater than 1, w AAs may be each independently citrulline residue, alanine residue, phenylalanine residue, glutamine residue and glycine residue, wherein the number of citrulline residues may be 1, 2, or more; the number of alanine residues may be 1, 2, or more; the number of phenylalanine residues may be 1, 2, or more; the number of glutamine residues may be 1, 2, or more; or the number of glycine residues may be 1, 2, or more.

In the present application, M is $N_3$, SH, $ONH_2$, $NH_2$, $CO_2H$, (alkyl)-CO-, HCO-, $BrCH_2$-, $ICH_2$-,

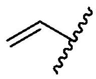, 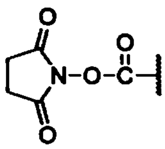、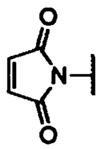, 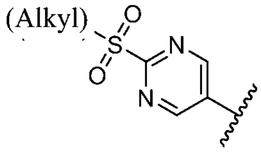, 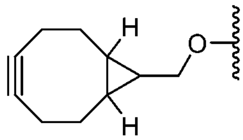 or 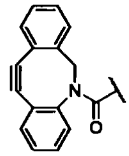.

Further, M is

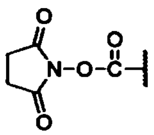、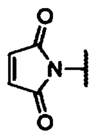, 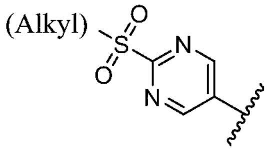 or 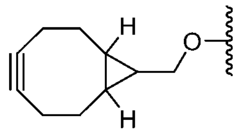.

In some embodiments, M is

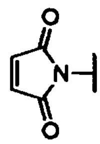.

In some embodiments, M is

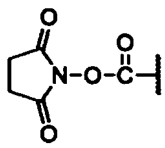.

In some embodiments, M is

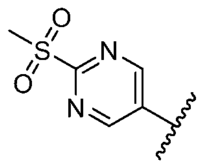.

In some embodiments, M is

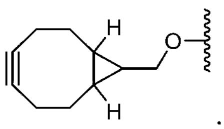.

In the present application, A is hydrogen, methyl, ethyl or propyl.

In some embodiments, A is hydrogen. In some embodiments, A is methyl. In some embodiments, A is ethyl. In some embodiments, A is propyl.

In the present application, the T is any one selected from the group consisting of:

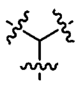, 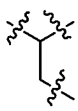, 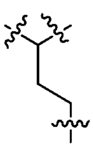, and 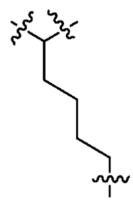.

In some embodiments, T is

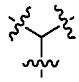.

In the present application, $L_a$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

Further, $L_a$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur. Further, $L_a$ is one, two, three or more selected from the group consisting of: $C_1$-$C_6$ linear or branched alkylene, $C_2$-$C_6$ linear or branched alkenylene, or $C_2$-$C_6$ linear or branched alkynylene, and polyethylene glycol group; and wherein the alkylene, alkenylene, or alkynylene is optionally substituted by a substituent.

Further, $L_a$ is one, two, three or more selected from the group consisting of: $C_1$-$C_6$ linear or branched alkylene, and polyethylene glycol group; the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, and n is an integer equal to or greater than 1.

Further, $L_a$ is one, two, three or more selected from the group consisting of: ethylidene, linear propylidene, linear butylidene, linear pentylidene, and $-(CH_2CH_2O)_nCH_2CH_2-$; and wherein n is 2, 3, 4, 5, 6, 7 or 8.

Further, $L_a$ is ethylidene and/or $-(CH_2CH_2O)_4CH_2CH_2-$ or pentylidene.

In some embodiments, the number of $L_a$ is 2, wherein one is ethylidene, and the other is $-(CH_2CH_2O)_4CH_2CH_2-$.

In some embodiments, when the number of $L_a$ is 1, it is ethylidene. In some embodiments, when the number of $L_a$ is 1, it is pentylidene. In some embodiments, when the number of $L_a$ is 1, it is $-(CH_2CH_2O)_4CH_2CH_2-$.

In the present application, $L_b$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, or heteroarylene with 6-18 carbon atoms and 1-4 heteroatoms in one or more rings, and polyethylene glycol group; the alkylene, alkenylene, alkynylene, alicyclylene, heteroalicyclylene, arylene, or heteroarylene is optionally substituted by a substituent; and the heteroatom is selected from nitrogen, oxygen, or sulfur.

Further, $L_b$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group;
the alkylene, alkenylene, alkynylene, alicyclylene, heteroalicyclylene, arylene, or heteroarylene is optionally substituted by a substituent; and the heteroatom is selected from nitrogen, oxygen, or sulfur.

Further, $L_b$ is selected from $C_1$-$C_6$ linear or branched alkylene, $C_2$-$C_6$ linear or branched alkenylene, or $C_2$-$C_6$ linear or branched alkynylene, or polyethylene glycol group;
the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; and the alkylene, alkenylene, or alkynylene is optionally substituted by a substituent.

Further, $L_b$ is one, two, three or more selected from the group consisting of: $C_1$-$C_6$ linear or branched alkylene, and $C_6$-$C_{10}$ arylene.

Further, $L_b$ is one, two, three or more selected from the group consisting of: methylene, ethylene, n-propylidene, isopropylidene, n-butylidene, isobutylidene, sec-butylidene, tert-butylidene, and benzylidene.

When the number of $L_b$ is more than 1, multiple $L_b$ may have different structures or identical structures; the details are as follows:
In some embodiments, when the number of $L_b$ is 1 or more, each $L_b$ is independently methylene. In some embodiments, when the number of $L_b$ is 1 or more, each $L_b$ is independently ethylidene. In some embodiments, when the number of $L_b$ is 1 or more, each $L_b$ is independently n-propylidene. In some embodiments, when the number of $L_b$ is 1 or more, each $L_b$ is independently isopropylidene. In some embodiments, when the number of $L_b$ is 1 or more, each $L_b$ is independently n-butylidene. In some embodiments, when the number of $L_b$ is 1 or more, each $L_b$ is independently isobutylidene. In some embodiments, when the number of $L_b$ is 1 or more, each $L_b$ is independently sec-butylidene. In some embodiments, when the number of $L_b$ is 1 or more, each $L_b$ is independently tert-butylidene. In some embodiments, when the number of $L_b$ is 1 or more, each $L_b$ is independently benzylidene.

In some embodiments, when the number of $L_b$ is more than 1, multiple $L_b$ are each independently methylene and ethylidene, wherein the number of methylene may be 1, 2 or more; the number of ethylidene may be 1, 2 or more; for another example, when the number of $L_b$ is 3, the three $L_b$ are each independently two methylene and one ethylidene. For another example, when the number of $L_b$ is 3, the three $L_b$ are each independently one methylene, and two ethylidene.

In some embodiments, when the number of $L_b$ is more than 1, multiple $L_b$ are each independently methylene and n-propylidene, wherein the number of methylene may be 1, 2 or more; the number of n-propylidene may be 1, 2 or more; for another example, when the number of $L_b$ is 3, the three $L_b$ are each independently two methylene, and one n-propylidene. For another example, when the number of $L_b$ is 3, the three $L_b$ are each independently one methylene, and two n-propylidene.

In some embodiments, when the number of $L_b$ is greater than 1, multiple $L_b$ are each independently methylene and isopropylidene, wherein the number of methylene may be 1, 2 or more; and the number of isopropylidene may be 1, 2 or more.

In some embodiments, when the number of $L_b$ is greater than 1, multiple $L_b$ are each independently methylene and n-butylidene, wherein the number of methylene may be 1, 2 or more; and the number of n-butylidene may be 1, 2 or more.

In some embodiments, when the number of $L_b$ is greater than 1, multiple $L_b$ are each independently methylene and isobutylidene, wherein the number of methylene(s) may be 1, 2 or more; and the number of isobutylidene may be 1, 2 or more.

In some embodiments, when the number of $L_b$ is greater than 1, multiple $L_b$ are each independently methylene and sec-butylidene, wherein the number of methylene may be 1, 2 or more; and the number of sec-butylidene may be 1, 2 or more.

In some embodiments, when the number of $L_b$ is greater than 1, multiple $L_b$ are each independently methylene and tert-butylidene, wherein the number of methylene may be 1, 2 or more; and the number of tert-butylidene may be 1, 2 or more.

In some embodiments, when the number of $L_b$ is greater than 1, multiple $L_b$ are each independently methylene and benzylidene, wherein the number of methylene may be 1, 2 or more; and the number of benzylidene may be 1, 2 or more.

In some embodiments, when the number of $L_b$ is greater than 1, multiple $L_b$ are each independently ethylidene and n-propylidene, wherein the number of ethylidene may be 1, 2 or more and the number of n-propylidene may be 1, 2 or more.

In some embodiments, when the number of $L_b$ is greater than 1, multiple $L_b$ are each independently ethylidene and isopropylidene, wherein the number of ethylidene may be 1, 2 or more, and the number of isopropylidene may be 1, 2 or more.

In some embodiments, when the number of $L_b$ is greater than 1, multiple $L_b$ are each independently ethylidene and n-butylidene, wherein the number of ethylidene may be 1, 2 or more; and the number of n-butylidene may be 1, 2 or more.

In some embodiments, when the number of $L_b$ is greater than 1, multiple $L_b$ are each independently ethylidene and isobutylidene, wherein the number of ethylidene may be 1, 2 or more; and the number of isobutylidene may be 1, 2 or more.

In some embodiments, when the number of $L_b$ is greater than 1, multiple $L_b$ are each independently ethylidene and sec-butylidene, wherein the number of ethylidene may be 1, 2 or more; and the number of sec-butylidene may be 1, 2 or more.

In some embodiments, when the number of $L_b$ is greater than 1, multiple $L_b$ are each independently ethylidene and tert-butylidene, wherein the number of ethylidene may be 1, 2 or more; and the number of tert-butylidene may be 1, 2 or more.

In some embodiments, when the number of $L_b$ is greater than 1, multiple $L_b$ are each independently ethylidene and benzylidene, wherein the number of ethylidene may be 1, 2 or more; and the number of benzylidene may be 1, 2 or more.

In some embodiments, when the number of $L_b$ is greater than 1, multiple $L_b$ are each independently n-propylidene and isopropylidene, wherein the number of n-propylidene may be 1, 2 or more; and the number of isopropylidene may be 1, 2 or more.

In some embodiments, when the number of $L_b$ is greater than 1, multiple $L_b$ are each independently n-propylidene and n-butylidene, wherein the number of n-propylidene may be 1, 2 or more; and the number of n-butylidene may be 1, 2 or more.

In some embodiments, when the number of $L_b$ is greater than 1, multiple $L_b$ are each independently n-propylidene and isobutylidene, wherein the number of n-propylidene may be 1, 2 or more; and the number of isobutylidene may be 1, 2 or more.

In some embodiments, when the number of $L_b$ is greater than 1, multiple $L_b$ are each independently n-propylidene and sec-butylidene, wherein the number of n-propylidene may be 1, 2 or more; and the number of sec-butylidene may be 1, 2 or more.

In some embodiments, when the number of $L_b$ is greater than 1, multiple $L_b$ are each independently n-propylidene and tert-butylidene, wherein the number of n-propylidene may be 1, 2 or more; and the number of tert-butylidene may be 1, 2 or more.

In some embodiments, when the number of $L_b$ is greater than 1, multiple $L_b$ are each independently n-propylidene and benzylidene, wherein the number of n-propylidene may be 1, 2 or more; and the number of benzylidene may be 1, 2 or more.

In some embodiments, when the number of $L_b$ is greater than 1, multiple $L_b$ are each independently isopropylidene and n-butylidene, wherein the number of isopropylidene may be 1, 2 or more; and the number of n-butylidene may be 1, 2 or more.

In some embodiments, when the number of $L_b$ is greater than 1, multiple $L_b$ are each independently isopropylidene and isobutylidene, wherein the number of isopropylidene may be 1, 2 or more; and the number of isobutylidene may be 1, 2 or more.

In some embodiments, when the number of $L_b$ is greater than 1, multiple $L_b$ are each independently isopropylidene and sec-butylidene, wherein the number of isopropylidene may be 1, 2 or more; and the number of sec-butylidene may be 1, 2 or more.

In some embodiments, when the number of $L_b$ is greater than 1, multiple $L_b$ are each independently isopropylidene and tert-butylidene, wherein the number of isopropylidene may be 1, 2 or more; and the number of tert-butylidene may be 1, 2 or more.

In some embodiments, when the number of $L_b$ is greater than 1, multiple $L_b$ are each independently isopropylidene and benzylidene, wherein the number of isopropylidene may be 1, 2 or more; and the number of benzylidene may be 1, 2 or more.

In some embodiments, when the number of $L_b$ is greater than 1, multiple $L_b$ are each independently methylene, ethylidene and n-propylidene, wherein the number of methylene may be 1, 2 or more; the number of ethylidene may be 1, 2 or more; and the number of n-propylidene may be 1, 2 or more.

In some embodiments, when the number of $L_b$ is greater than 1, multiple $L_b$ are each independently methylene, ethylidene and isopropylidene, wherein the number of methylene may be 1, 2 or more; the number of ethylidene may be 1, 2 or more; and the number of isopropylidene may be 1, 2 or more.

In some embodiments, when the number of $L_b$ is greater than 1, multiple $L_b$ are each independently methylene, ethylidene, n-propylidene, and isopropylidene, wherein the number of methylene may be 1, 2 or more; the number of ethylidene may be 1, 2 or more; the number of n-propylidene may be 1, 2 or more; and and the number of isopropylidene may be 1, 2 or more.

In some embodiments, when the number of $L_b$ is greater than 1, multiple $L_b$ are each independently methylene, ethylidene, n-propylidene, isopropylidene, and n-butylidene, wherein the number of methylene may be 1, 2 or more; the number of ethylidene may be 1, 2 or more; the number of n-propylidene may be 1, 2 or more; the number of isopropylidene may be 1, 2 or more; and the number of n-butylidene may be 1, 2 or more.

In the present application, $Q_a$ is one, two, three or more selected from the group consisting of: -C(=O)NR-, -NR-, -C(=O)O-, -OC(=O)NR-, -NRC(=O)-, -OC(=O)-, -NRC(=O)O-, -NRC(=O)NR-, NHC-(=NH)NH-, -C(=O)-, and -OC(=O)O-; wherein R is selected from hydrogen, or optionally substituted $C_1$-$C_6$ linear or branched alkyl.

Further, $Q_a$ is one, two, three or more selected from the group consisting of: -C(=O)-, -NR-, -C(=O)NR-, -NRC(=O)NR-, -OC(=O)O-, -OC(=O)NR-, -NRC(=O)-, and -OC(=O)-; wherein R is selected from hydrogen, or optionally substituted $C_1$-$C_6$ linear or branched alkyl.

Further, $Q_a$ is one, two, three or more selected from the group consisting of: -C(=O)-, -NR-, -NRC(=O)-, and -(C=O)NR-; wherein R is selected from hydrogen, or optionally substituted $C_1$-$C_6$ linear or branched alkyl.

Further, $Q_a$ is -C(=O)-, -N($CH_3$)-, -NHC(=O)-, -C(=O)-NH-, or -C(=O)-N($CH_3$)-.

In some embodiments, when the number of $Q_a$ is one, it is -C(=O)-.

In some embodiments, when the number of $Q_a$ is two, one is -C(=O)-, and the other is -C(=O)NH-.

In some embodiments, when the number of $Q_a$ is two, one is -C(=O)-, the othe is -C(=O)-NR-, and R is methyl.

In some embodiments, when the number of $Q_a$ is two, one is -C(=O)-, the othe is -NR-, and R is methyl.

In the present application, $Q_b$ is one, two, three or more selected from the group consisting of: absent, -C(=O)NR-, -C(=O)O-, -OC(=O)NR-, -NRC(=O)-, -OC(=O)-, -NRC(=O)O-, -NRC(=O)NR-, NHC-(=NH)NH-, -C(=O)-, and -OC(=O)O-; wherein R is selected from hydrogen, or optionally substituted $C_1$-$C_6$ linear or branched alkyl.

Further, $Q_b$ is one, two, three or more selected from the group consisting of: absent, -C(=O)NR-, -OC(=O)NR-,

-NRC(=O)-, -NRC(=O)O-, and -NRC(=O)NR-; and R is selected from hydrogen, or optionally substituted $C_1$-$C_6$ linear or branched alkyl.

Further, $Q_b$ is one, two, three or more selected from the group consisting of: absent, -C(=O)-, -NRC(=O)-, -NRC(=O)NR-, and -C(=O)NR-; and R is hydrogen or methyl.

In some embodiments, $Q_b$ is absent. In some embodiments, $Q_b$ is -C(=O)-. In some embodiments, $Q_b$ is -NRC(=O)-, R is hydrogen. In some embodiments, $Q_b$ is -NRC(=O)-, R is methyl. In some embodiments, $Q_b$ is -NRC(=O)NR-, R is hydrogen. In some embodiments, $Q_b$ is -NRC(=O)NR-, and R is methyl. In some embodiments, $Q_b$ is -C(=O)NR-, and R is hydrogen. In some embodiments, $Q_b$ is -C(=O)NR-, and R is methyl. In the present application, X is selected from:

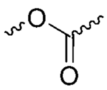 , 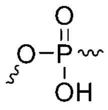 , 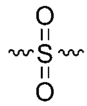 , 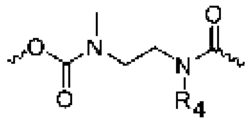 ,

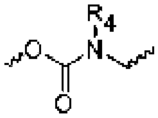 , 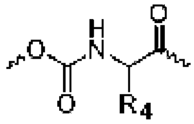 , 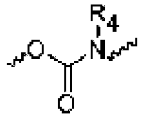 or 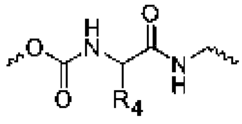 ;

the wavy line at right side of X is connected to D;
$R_4$ is selected from hydrogen, $C_1$-$C_6$ linear or branched alkyl, $-(CH_2CH_2O)_mR_2$, $-CH_2CH_2NR_2R_3$, or $-CH_2CH_2SO_2R_2$; $R_2$, and $R_3$ are each independently selected from hydrogen, or $C_1$-$C_6$ linear or branched alkyl; and m is an integer greater than 1.

Further, X is

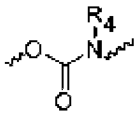 , 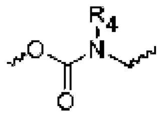 or 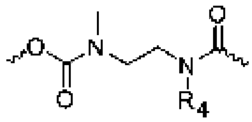 ;

$R_4$ is hydrogen, methyl, $-CH_2CH_2OCH_2CH_2OH$, $-CH_2CH_2NMe_2$, or $-CH_2CH_2SO_2Me$.

In some embodiments, X is

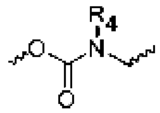 ,

and $R_4$ is hydrogen.

In some embodiments, X is

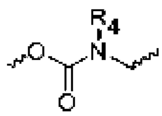 ,

and $R_4$ is methyl.

In some embodiments, X is

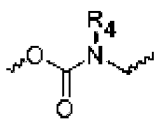

, and $R_4$ is $-CH_2CH_2OCH_2CH_2OH$.

In some embodiments, X is

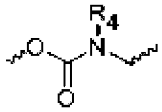

,

and $R_4$ is $-CH_2CH_2NMe_2$.

In some embodiments, X is

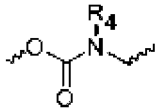

,

$R_4$ is $-CH_2CH_2SO_2Me$.

In some embodiments, X is

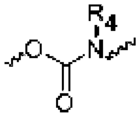

,

and $R_4$ is hydrogen.

In some embodiments, X is

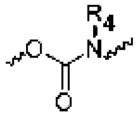

,

$R_4$ is methyl. In some embodiments, X is

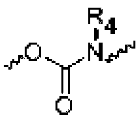

, and $R_4$ is $-CH_2CH_2OCH_2CH_2OH$.

In some embodiments, X is

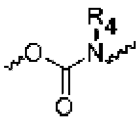

, $R_4$ is $-CH_2CH_2NMe_2$.

In some embodiments, X is

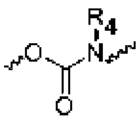

, and $R_4$ is $-CH_2CH_2SO_2Me$.

In some embodiments, X is

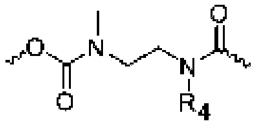

,

and $R_4$ is hydrogen.

In some embodiments, X is

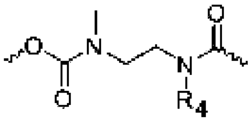

,

and $R_4$ is methyl.

In some embodiments, X is

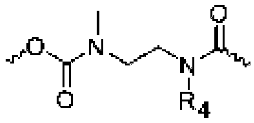

,

and $R_4$ is $-CH_2CH_2OCH_2CH_2OH$.

In some embodiments, X is

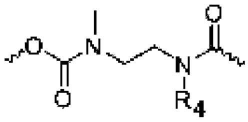

,

and $R_4$ is $-CH_2CH_2NMe_2$.

In some embodiments, X is

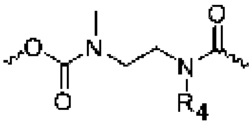

, and $R_4$ is $-CH_2CH_2SO_2Me$.

In the present application, the sulfonate inner salt is

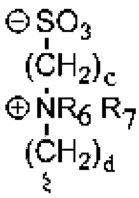

,

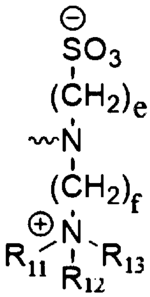

or

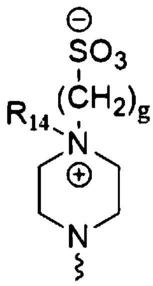

,

the wavy line is connected to Y, Y' or Y";

wherein c, d, e, f, and g are each independently an integer selected from 1, 2, 3, 4, 5 or 6; and $R_6$, $R_7$, $R_{11}$, $R_{12}$, $R_{13}$ and $R_{14}$ are each independently selected from absent, hydrogen, $C_1$-$C_6$ linear or branched alkyl.

Further, the sulfonate inner salt is

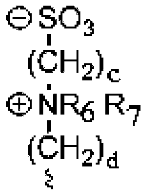, 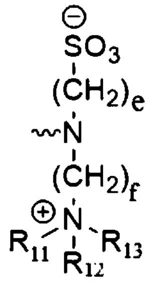 or 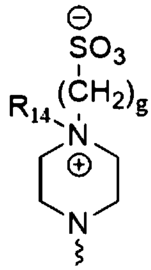,

wherein $R_6$, $R_7$, $R_{11}$, $R_{12}$, $R_{13}$ and $R_{14}$ are each independently selected from absent, methyl or ethyl; and c, d, e, f, and g are each independently 1, 2, or 3.

Further, the sulfonate inner salt has a structural formula selected from the group consisting of:

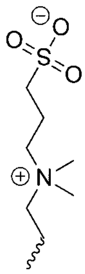, 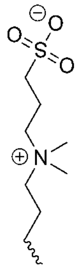, 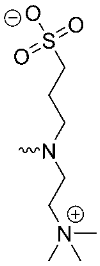, and 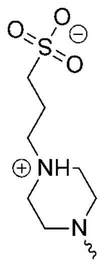.

In some embodiments, the sulfonate inner salt is

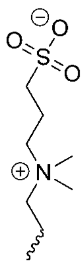.

In some embodiments, the sulfonate inner salt is

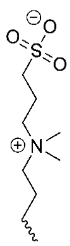.

In some embodiments, the sulfonate inner salt is

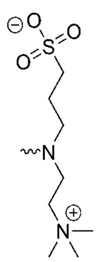.

In some embodiments, the sulfonate inner salt is

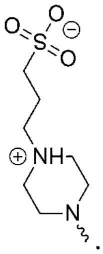

In the present application, the phosphate inner salt is

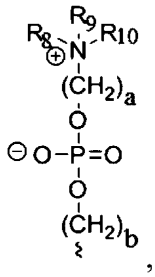

wherein a and b are each independently an integer selected from 1, 2, 3, 4, 5 or 6; and $R_8$, $R_9$ and $R_{10}$ are each independently selected from hydrogen, or $C_1$-$C_6$ linear or branched alkyl.

Further, the phosphate inner salt is

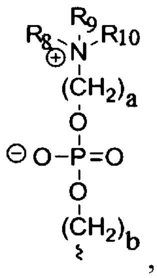

wherein $R_8$, $R_9$, and $R_{10}$ are each independently methyl or ethyl; a is 1, 2, or 3; b is 1, 2, or 3.

Further, the phosphate inner salt is

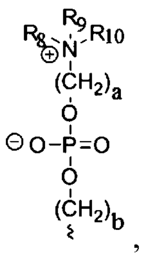

wherein $R_8$, $R_9$, and $R_{10}$ are each independently methyl; a is 2; and b is 1.

In the present application, the D comprises a cytotoxin or immune agonist.

In the present application, the cytotoxin is one, or two selected from the group consisting of: a microtubulin inhibitor, a DNA synthesis inhibitor, a topoisomerase inhibitor, and a RNA polymerase-2 inhibitor.

In the present application, the cytotoxin is one, two, or more selected from the group consisting of: auristatin, mertansine DM, calicheamicin, duocarmycin, pyrrolobenzodiazepine, camptothecin derivative, and α-amanitin.

In the present application, the immune agonist is selected from TLR7/8 agonist or STING agonist.

In the present application, i is an integer from 2 to 8, for example, it may be 2, 3, 4, 5, 6, 7 or 8.

In the present application, h is an integer from 1 to 4, for example, it may be 1, 2, 3 or 4.

In the present application, o, p, and q are each independently an integer from 0 to 3, for example, it may be 0, 1, 2 or 3.

In the present application, $L_a$ is one, two, three or more selected from the group consisting of: $C_1$-$C_6$ linear or branched alkylene, and polyethylene glycol group; the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, n is an integer equal to or greater than 1; $Q_a$ is one, two, three or more selected from the group consisting of: -C(=O)-, -NR(C=O)- and -(C=O)NR-; and wherein R is selected from hydrogen, or C $_1$-$C_6$ linear or branched alkyl.

Further, $L_a$ is one, two, three or more selected from the group consisting of: ethylidene, linear propylidene, linear butylidene, linear pentylidene, and $-(CH_2CH_2O)_nCH_2CH_2-$; wherein n is 2, 3, 4, 5, 6, 7 or 8; $Q_a$ is one, two, three or more selected from the group consisting of: -C(=O)-, -NR(C=O)-, and -(C=O)NR-; and wherein R is methyl and/or hydrogen.

Further, $L_a$ is ethylidene and/or $-(CH_2CH_2O)_4CH_2CH_2-$ or pentylidene; $Q_a$ is -C(=O)-, -NR-, -NH(C=O)- and/or -C(=O)NH-.

In some embodiments, the number of $L_a$ is two, wherein one is ethylidene, and the other is $-(CH_2CH_2O)_4CH_2CH_2-$; the number of $Q_a$ is two, wherein one is -C(=O)-, and the other is -C(=O)NH-.

In some embodiments, the number of $L_a$ is one, it is a pentylidene; the number of $Q_a$ is one, it is -C(=O)-.

In some embodiments, the number of $L_a$ is one, it is $-(CH_2CH_2O)_4CH_2CH_2-$; the number of $Q_a$ is two, one is -C(=O)-, and the other is -C(=O)NH-.

In some embodiments, the number of $L_a$ is one, it is $-(CH_2CH_2O)_4CH_2CH_2-$; the number of $Q_a$ is two, one is -C(=O)-, and the other is $-C(=O)N(CH_3)-$.

In some embodiments, the number of $L_a$ is two, they are each independently ethylidene and pentylidene; the number of $Q_a$ is two, one is -C(=O)-, and the other is $-N(CH_3)-$.

In the present application, $L_b$ is selected from $C_1$-$C_6$ linear or branched alkylene, $C_2$-$C_6$ linear or branched alkenylene, or $C_2$-$C_6$ linear or branched alkynylene, arylene with 6-10 carbon atoms in one or more rings, or polyethylene glycol group,

the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; and the alkylene, alkenylene, or alkynylene is optionally substituted by a substituent;

$Q_b$ is one, two, three or more selected from the group consisting of: absent, -C(=O)NR-, -C(=O)O-, -OC(=O)NR-, -NRC(=O)-, -OC(=O)-, -NRC(=O)O-, $-NRS(=O)_2-$, -NRC(=O)NR-, -C(=O)-, and -OC(=O)O-; and R is selected from hydrogen, or optionally substituted $C_1$-$C_6$ linear or branched alkyl.

Further, $L_b$ is one, two, three or more selected from the group consisting of: $C_1$-$C_6$ linear or branched alkylene, or $C_6$-$C_{10}$ arylene; $Q_b$ is one, two, three or more selected from the group consisting of: absent, -C(=O)NR-, -C(=O)-, -OC(=O) NR-, -NRC(=O)-, -NRC(=O)O-, and -NRC(=O)NR-; and R is selected from hydrogen, or $C_1$-$C_6$ linear or branched alkyl.

Further, $L_b$ is one, two, three or more selected from the group consisting of: methylene, ethylene, n-propylidene, isopropylidene, n-butylidene, isobutylidene, sec-butylidene, tert-butylidene, and benzylidene;

$Q_b$ is one, two, three or more selected from the group consisting of: absent, -C(=O)-, -NRC(=O)-, -NRC(=O)NR-, and -C(=O)NR-; and R is hydrogen or methyl.

In some embodiments, in the Y, when the number of $L_b$ is two, wherein one is methylene, and the other is isopropylidene; and $Q_b$ is absent.

In some embodiments, in the Y, when the number of $L_b$ is three, they are each independently methylene, isopropylidene and ethylidene; the number of $Q_b$ is one, it is -C(=O)NR-; and R is hydrogen.

In some embodiments, in the Y, when the number of $L_b$ is two, wherein one is an ethylidene, and the other is isopropylidene; the number of $Q_b$ is one, it is -C(=O)NR-; and R is hydrogen.

In some embodiments, in the Y, when the number of $L_b$ is two, wherein one is n-propylidene, and the other is isopropylidene; the number of $Q_b$ is one, it is -NRC(=O)NR-; and R is hydrogen.

In some embodiments, in the Y, when the number of $L_b$ is one, it is benzylidene; and $Q_b$ is absent.

In some embodiments, in the Y, when the number of $L_b$ is two, they are each independently ethylidene and isopropylidene; and the number of $Q_b$ is one, it is -C(=O)-.

In the present application, the substitutent is any one selected from the group consisting of: deuterium, tritium, halogen, amino, hydroxyl, cyano, nitro, optionally substituted alkyl, alkenyl, alkynyl, optionally substituted heterocyclyl, acyl, optionally substituted amino, optionally substituted aminoformyl, optionally substituted thioaminoformyl, optionally substituted sulfamine, optionally substituted hydroxyls, optionally substituted thioalkyl (SH), and optionally substituted silyl.

In the present application, the compound is any one selected from the group consisting of:

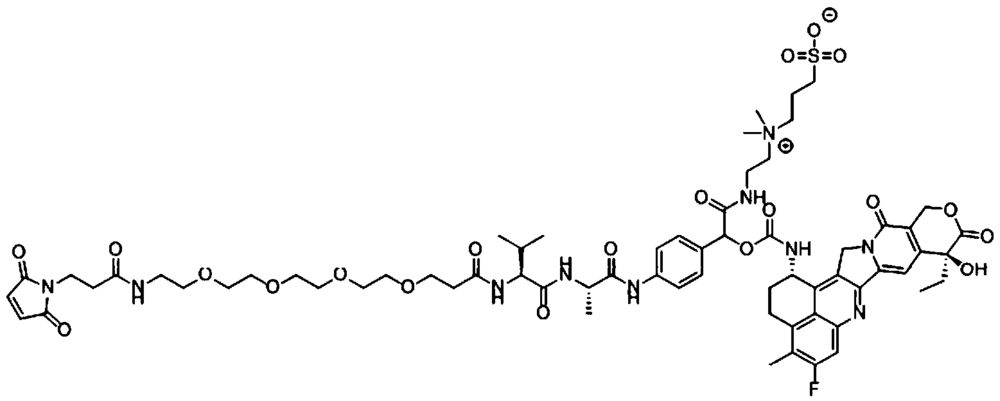

LD-31,

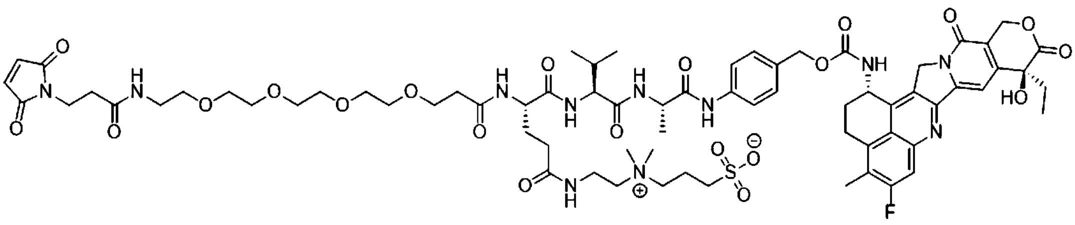

LD-37,

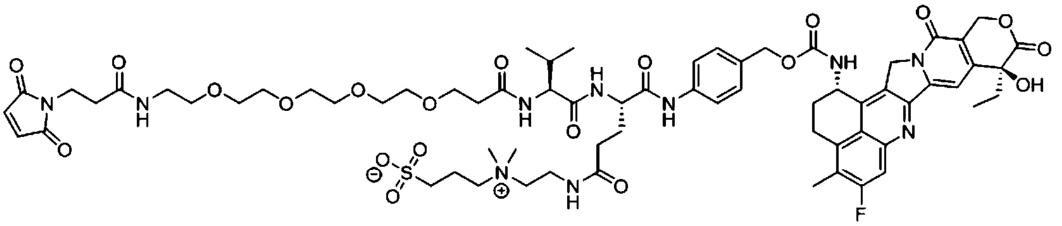

LD-38,

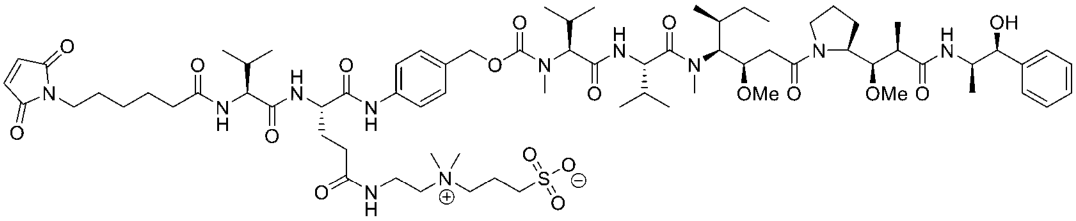

LD-57,

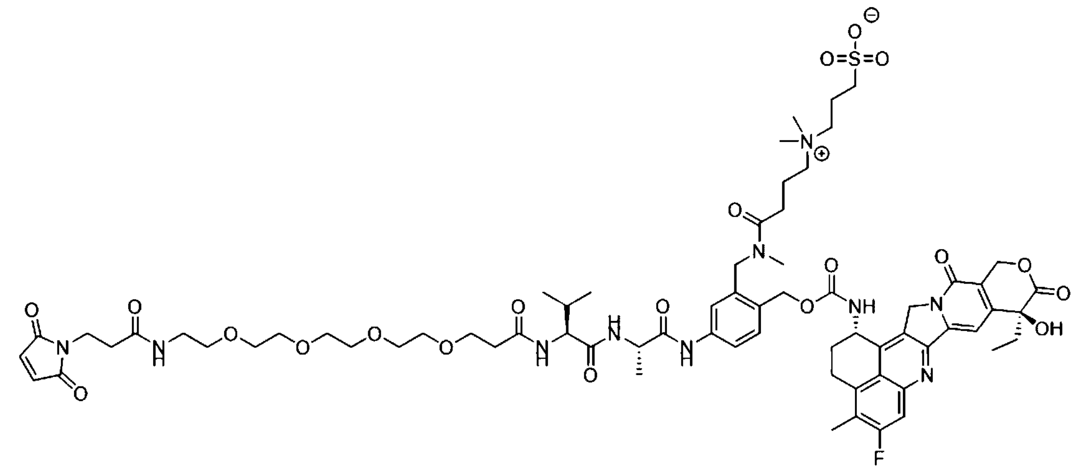

LD-65,

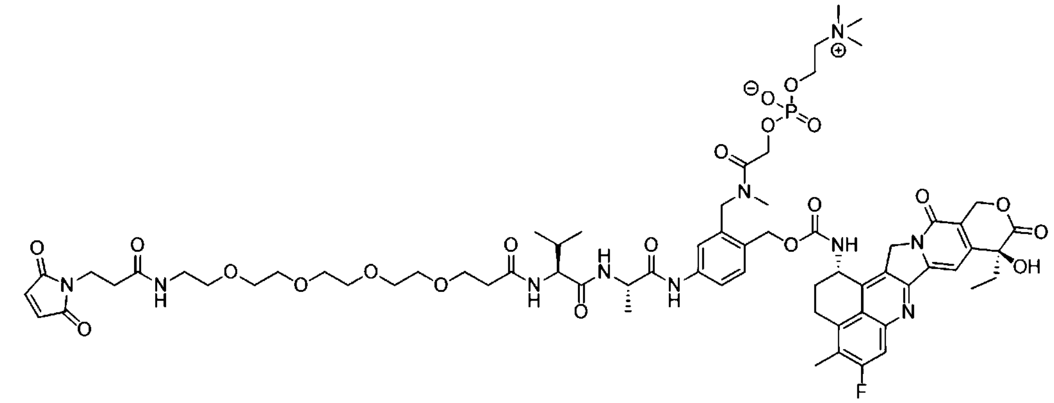

LD-66,

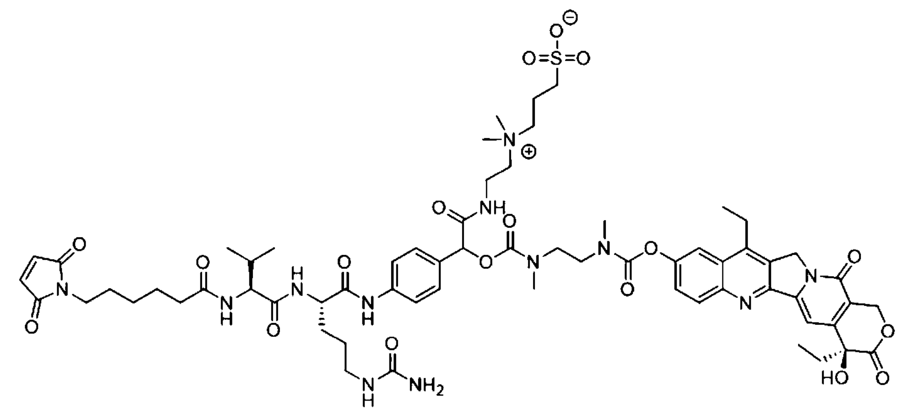

LD-71,

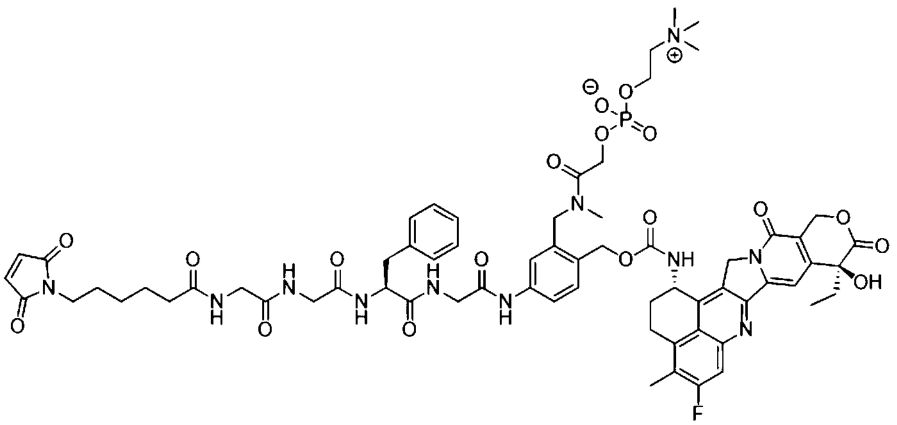

LD-72,

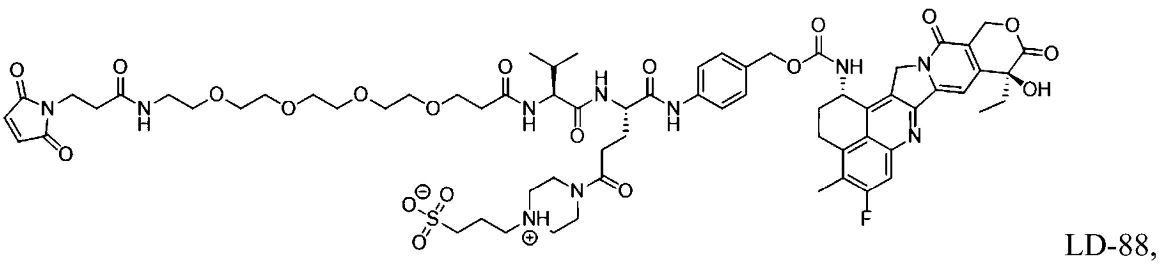

LD-88,

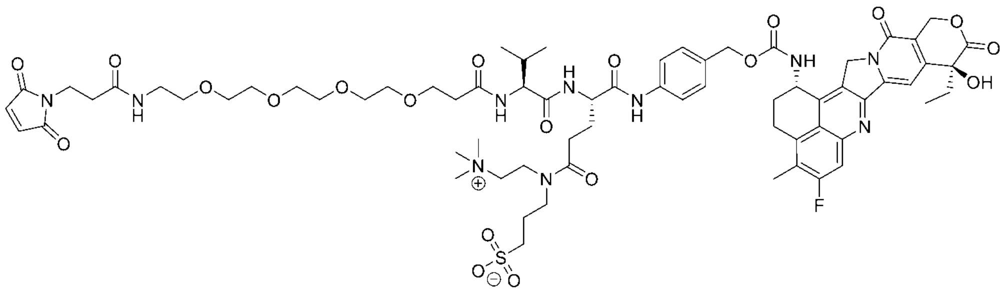

LD-89 ,

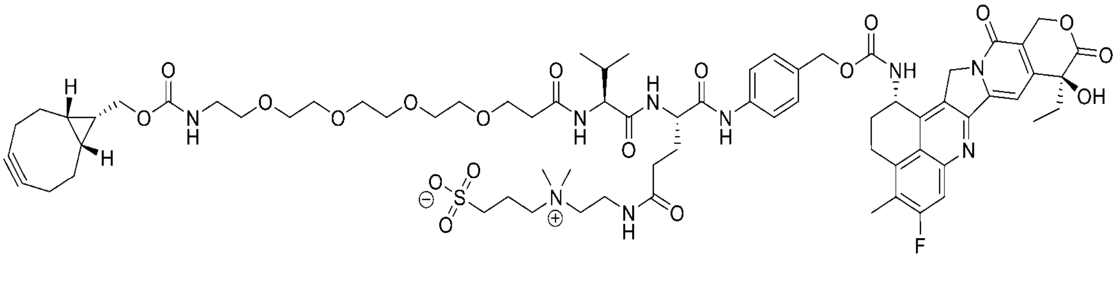

LD-94,

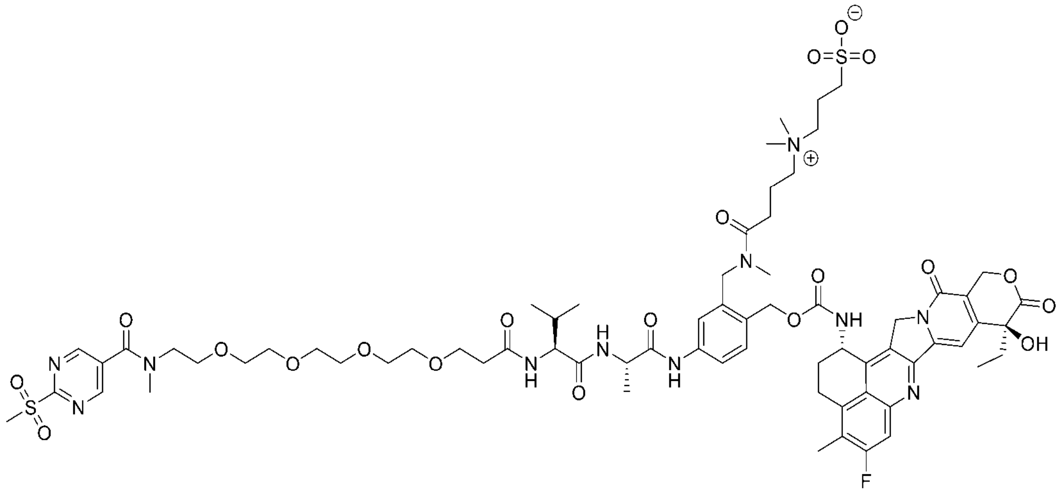

LD-98,

and

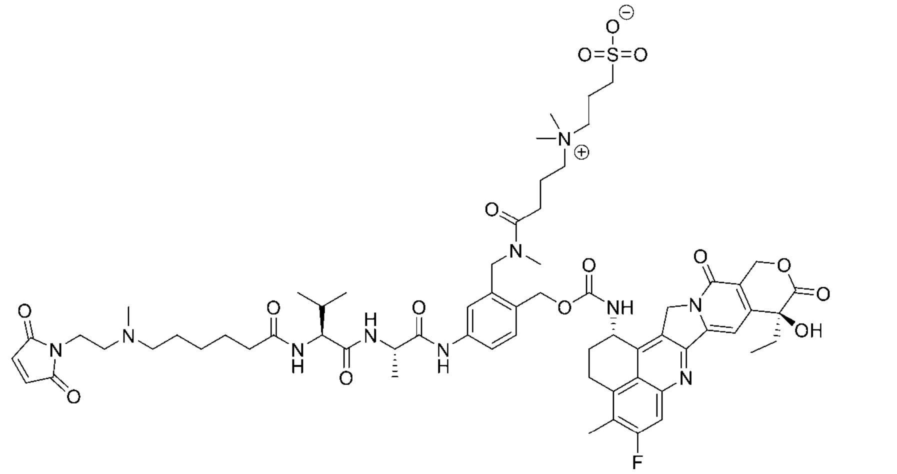

LD-99.

The present application also provides a compound represented by Formula (2), or a tautomer, mesomer, raceme, enantiomer or diastereomer thereof, or mixture thereof, or a pharmaceutically acceptable salt, prodrug or solvate thereof, wherein Formula (2) has the following structural formula:

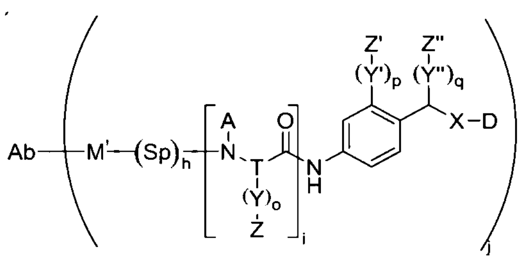

Formula (2)

wherein Ab comprises an antibody or an antigen-binding fragment thereof; M' is a spacer; Sp is $-L_a-Q_a-$; A is selected from absent, hydrogen or alkyl; T represents a straight or branched ($C_1$-$C_8$) trivalent alkyl group; Y, Y' and Y" are each independently $-L_b-Q_b-$; $L_a$, $L_b$ are each independently selected from absent, alkylene, alkenylene, alkynylene, alicyclylene, heteroalicyclylene, arylene, heteroarylene or polyethylene glycol group, wherein the alkylene, alkenylene, alkynylene, alicyclylene, heteroalicyclylene, arylene, heteroarylene or polyethylene glycol group is optionally substituted by a substituent; $Q_a$, and $Q_b$ are each independently selected from absent, -C(=O)NR-, -C(=O)O-, -OC(=O)NR-, $-S(=O)_2NR-$, -NRC(=O)-, -OC(=O)-, -NRC(=O)O-, $-NRS(=O)_2-$, -NRC(=O)NR-, -NHC-(=NH)NH-, -C(=O)-, -OC(=O)O-, -O-, -NR-, -S-, $S(=O)_2-$, S(=O)-, or -Se-; wherein R is selected from hydrogen or optionally substituted alkyl; Z is selected from absent, hydrogen, optionally substituted alkyl or a sulfonate inner salt; Z', and Z" are each independently selected from absent,

hydrogen, optionally substituted alkyl, a sulfonate inner salt or a phosphate inner salt; At least one of Z, Z' and Z" is a sulfonate inner salt or a phosphate inner salt; X comprises a self-immolative unit or is absent; D comprises a drug molecule; and i is an integer equal to or greater than 1, for example, it may be 1, 2, 3, 4, 5, 6, 7, 8, 9, 10, 11, 12, 13, 14, 15, etc.

h is an integer from 1 to 6, for example, it may be 1, 2, 3, 4, 5, 6, etc. o, p, and q are each independently an integer from 0 to 5, for example, it may be 0, 1, 2, 3, 4, 5, etc. j is an integer equal to or greater than 1, for example, it may be 0, 1, 2, 3, 4, 5, 6, 7, 8, 9, 10, 11, 12, etc.

When h is greater than 1, multiple Sp may have different structures, or identical structures, i.e., multiple $L_a$ may have different structures, or identical structures; and multiple $Q_a$ may have different structures, or identical structures. The number of $L_a$ and $Q_a$ may be different or identical.

When i is greater than 1, multiple As may be different or identical, multiple T may be different or identical, multiple Y may be different or identical, multiple o may be different or identical, and multiple Z may be different or identical.

When p is greater than 1, multiple Y's may have different structures, or identical structures.

When j is larger than 1, the portion in the bracket may have different structures, or identical structures.

In the present application, the Formula (2) is Formula (2-1):

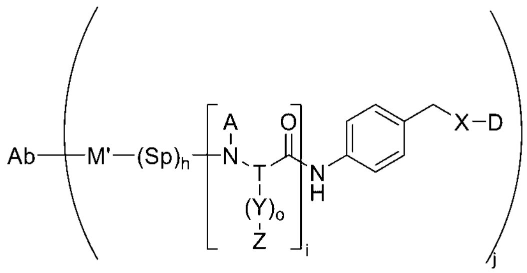

Formula (2-1)

wherein Ab, M', Sp, A, T, Y, h, o, i, j, X, D, and Z are defined as in Formula (1) described above.

At least one of the i Zs is a sulfonate inner salt.

Further, the Formula (2-1) is Formula (2-1-1):

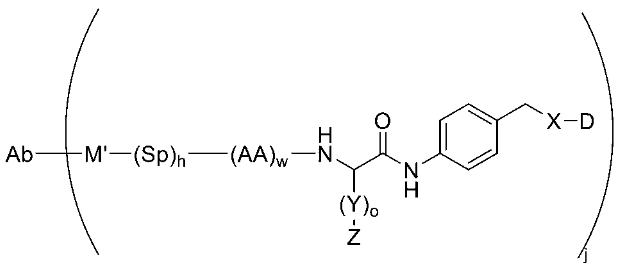

Formula (2-1-1)

wherein AA is an amino acid residue,

and w is an integer equal to or greater than 1, for example, it may be 1, 2, 3, 4, 5, 6, etc. When w is greater than 1, multiple AAs may be identical amino acid residues, or different amino acid residues.

In the present application, the Formula (2) is Formula (2-2):

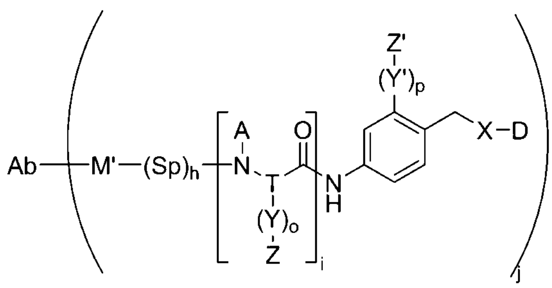

Formula (2-2)

wherein Ab, M', Sp, A, T, Y, Z, o, p, i, h, j, X, Y', and D are defined as in Formula (1) described above; and Z' is a sulfonate inner salt or a phosphate inner salt.

Further, the Formula (2-2) is Formula (2-2-1):

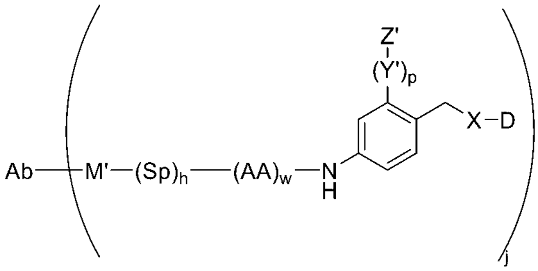

Formula (2-2-1)

wherein AA is an amino acid residue, and w is an integer equal to or greater than 1, for example, it may be 1, 2, 3, 4, 5, 6, etc. When w is greater than 1, multiple AAs may be identical amino acid residues, or different amino acid residues.

In the present application, the Formula (2) is Formula (2-3):

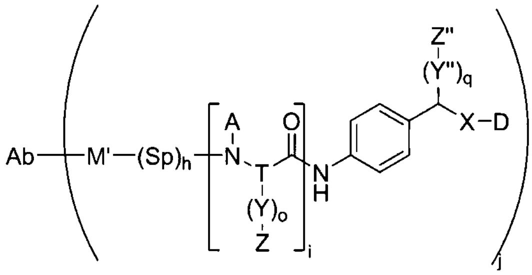

Formula (2-3)

wherein Ab, M', Sp, A, Y, T, Z, o, i, h, j, q, X, Y", and D are defined as in Formula (1) described above; and Z" is a sulfonate inner salt or a phosphate inner salt.

Further, the Formula (2-3) is Formula (2-3-1):

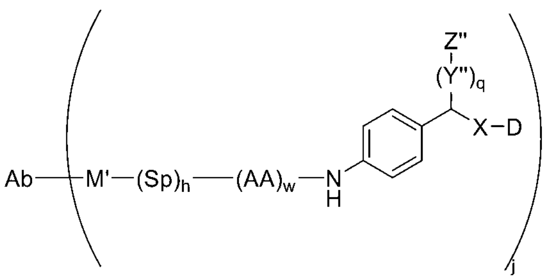

Formula (2-3-1),

wherein AA is an amino acid residue, and w is an integer equal to or greater than 1, for example, it may be 1, 2, 3, 4, 5, 6, etc. When w is greater than 1, multiple AAs may be identical amino acid residues, or different amino acid residues.

All definitions described herein about Formula (1) apply to Formula (2), all definitions described herein about Formula (1-1) apply to Formula (2-1), all definitions described herein about Formula (1-1-1) apply to Formula (2-1-1), all definitions described herein about Formula (1-2) apply to Formula (2-2), all definitions described herein about Formula (1-2-1) apply to Formula (2-2-1), all definitions described herein about Formula (1-3) apply to Formula (2-3), and all definitions described herein about Formula (1-3-1) apply to Formula (2-3-1). Therefore, for Sp, A, Y, T, Z, o, i, h, j, q, X, Y', Z', Y", Z" and D in Formula (2), Formula (2-1), Formula (2-1-1), Formula (2-2), Formula (2-2-1), Formula (2-3) and Formula (2-3-1), the description in Formula (1) may be referred to, and no further limitations will be made herein.

In the present application, M' is any one selected from the group consisting of:

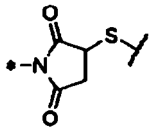 , 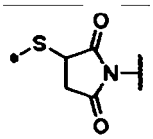 , 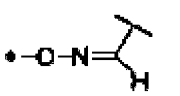 , 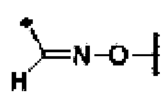 , 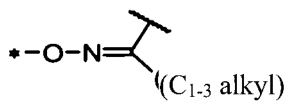 ,

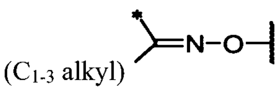, 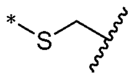, 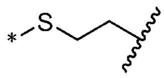, 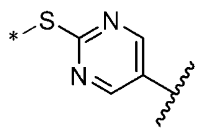, 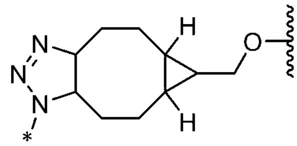,

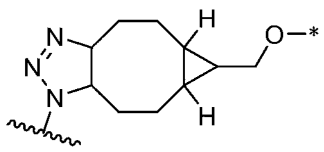, 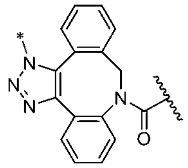, 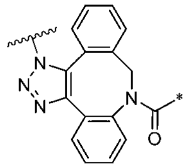, 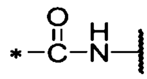 or 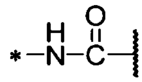;

and

* is connected to Ab.

Further, M' is any one selected from the group consisting of:

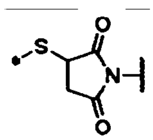, 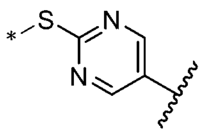, 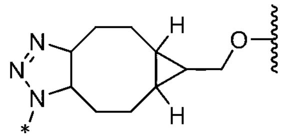, and 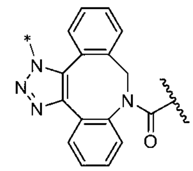.

In some embodiments, M' is

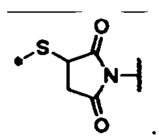.

In the present application, the compound is any one selected from the group consisting of:

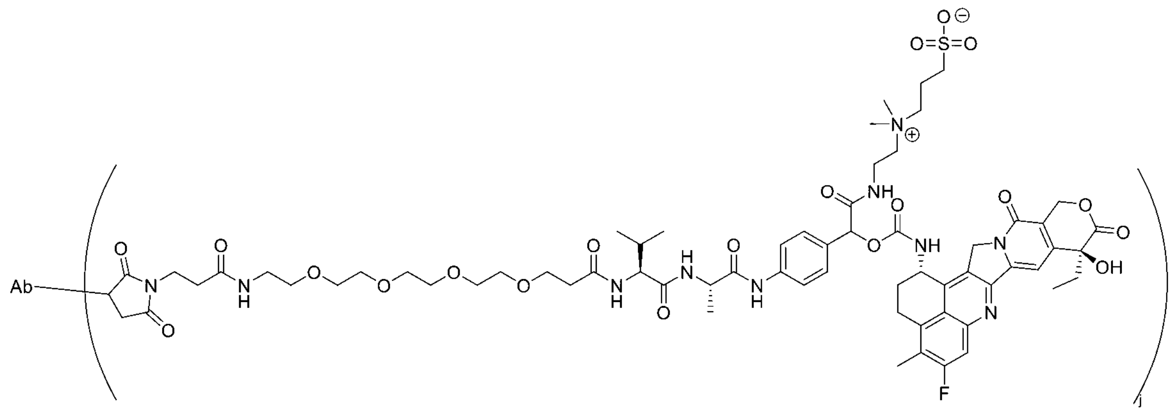,

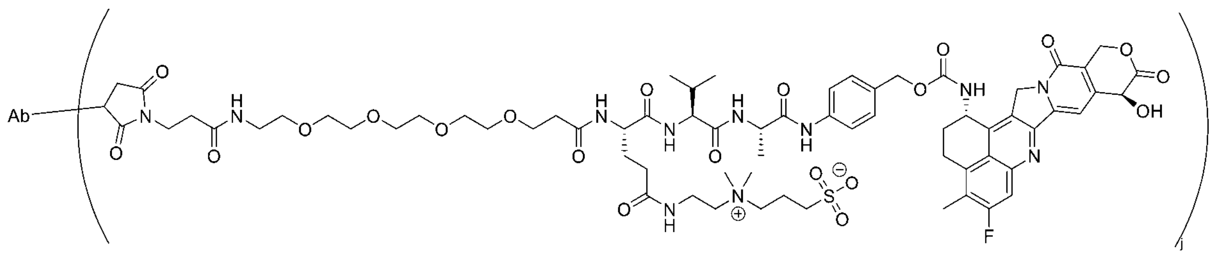,

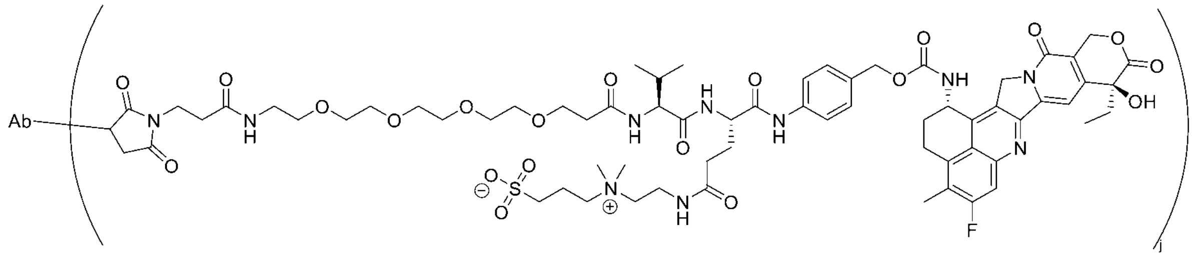

,

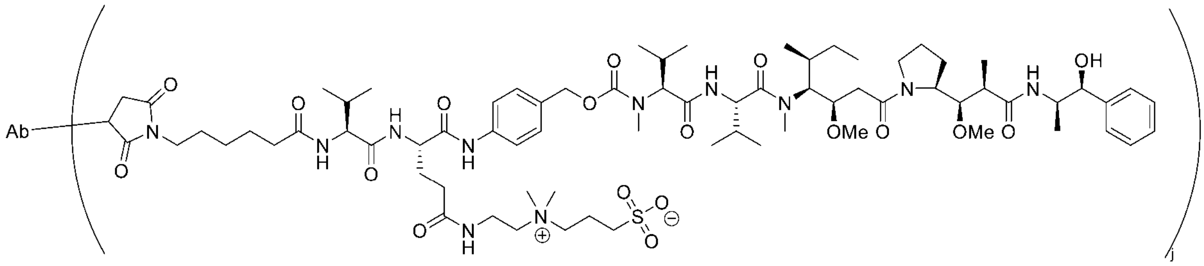

,

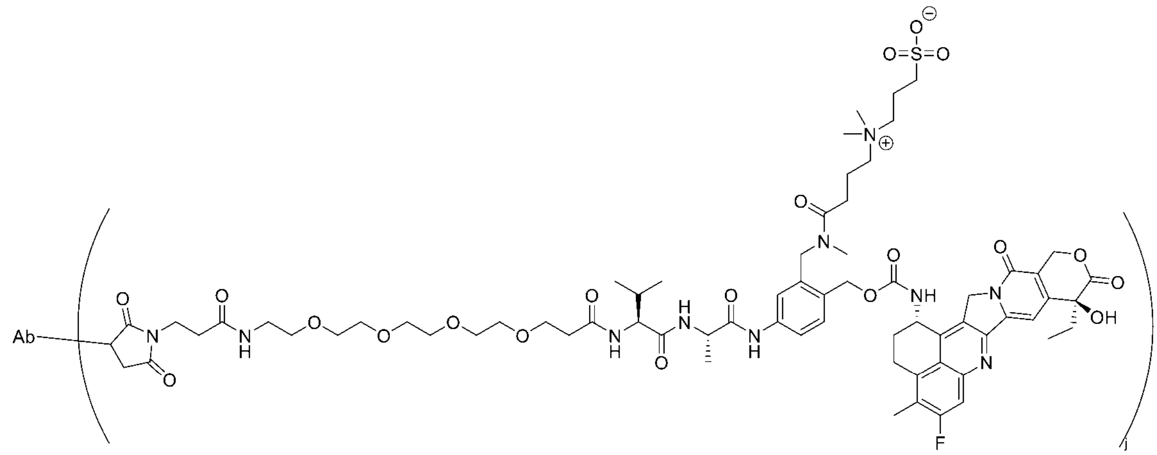

,

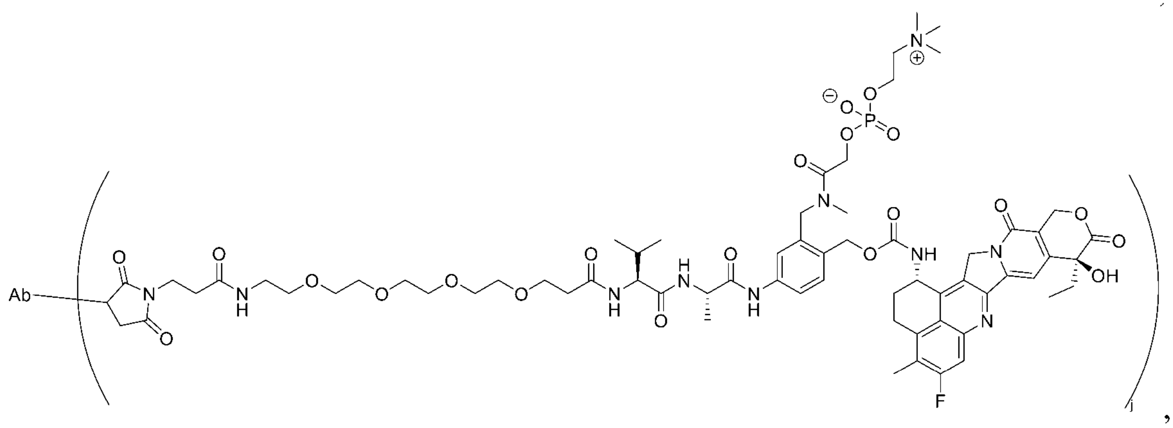

,

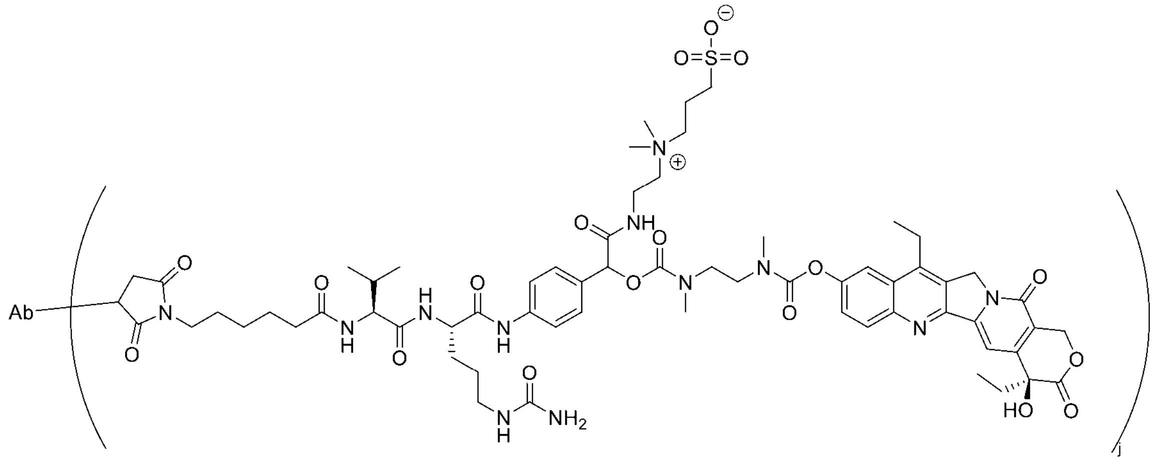

,

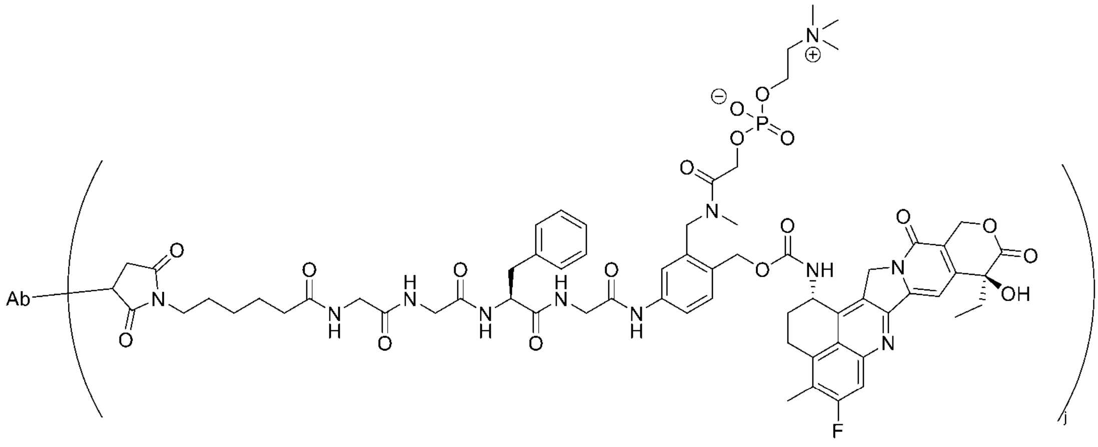

,

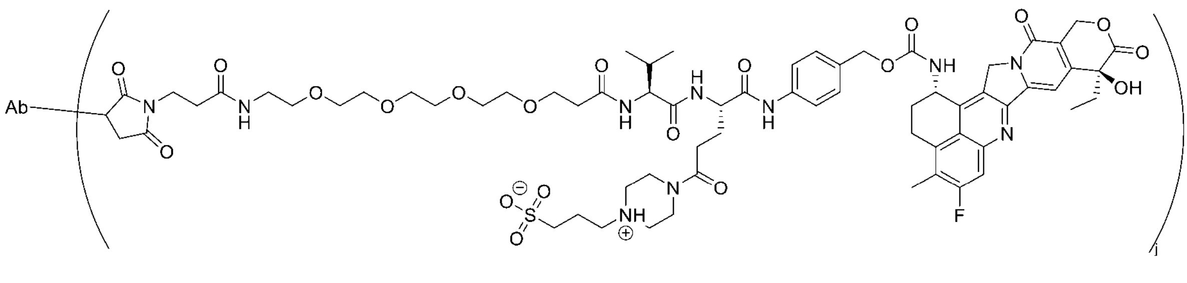

,

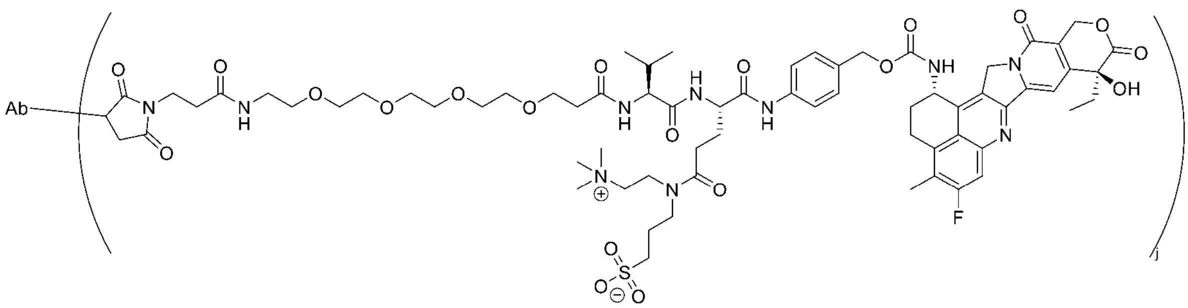

,

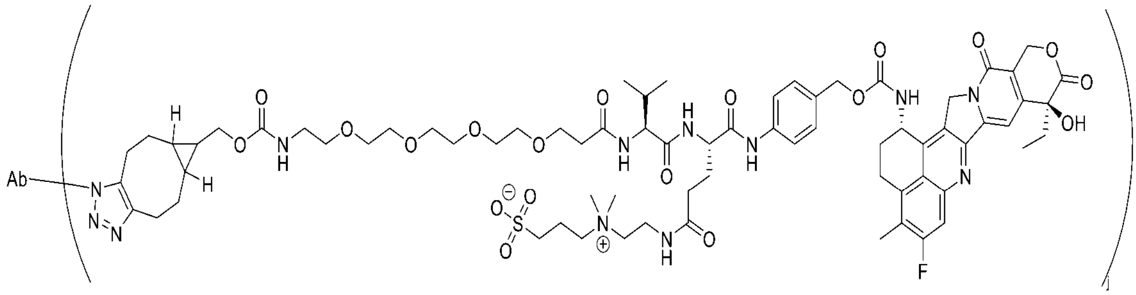

,

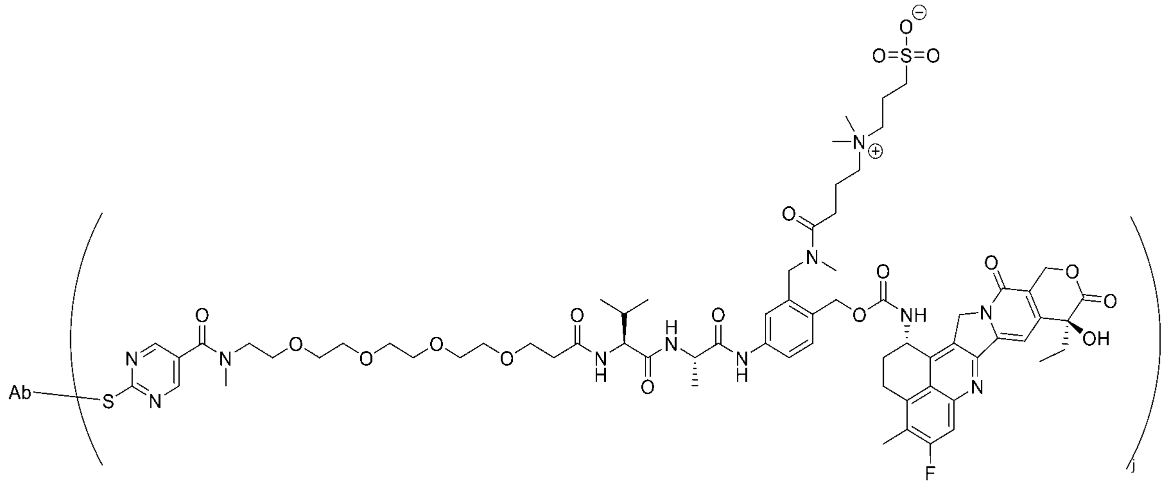

,

and

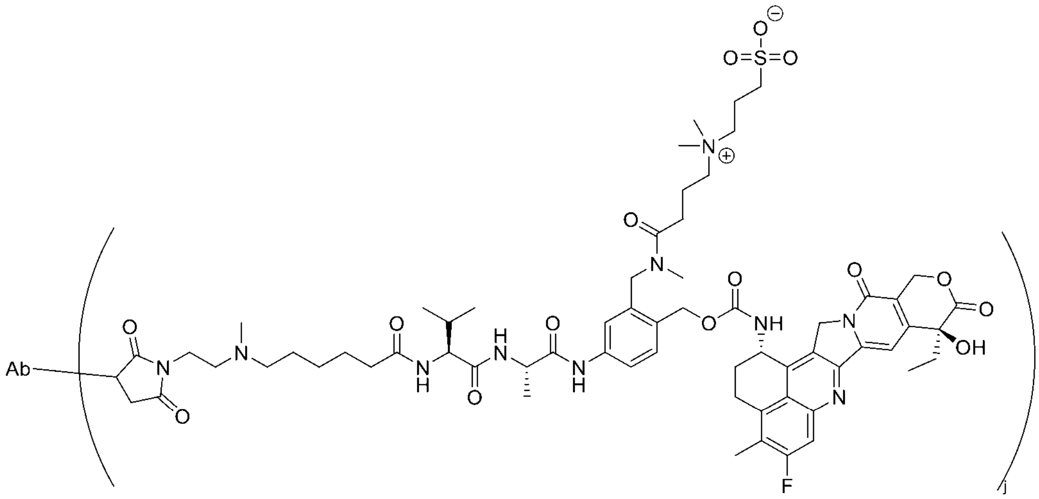

.

In the present application, the antibody is selected from the group consisting of: a mouse-derived antibody, a chimeric antibody, a humanized antibody, and a fully human antibody. In the present application, the antibody comprises a monoclonal antibody. In the present application, the antibody comprises a bispecific antibody. In the present application, the antigen-binding fragment is selected from the group consisting of: Fab, Fab', Fv fragment, $F(ab')_2$, $F(ab)_2$, scFv, di-scFv and VHH.

In the present application, the Ab is selected from the group consisting of: an anti-Claudin18.2 antibody, an anti-human folate receptor antibody, an anti-HER2 antibody, an anti-HER3 antibody, an anti-TROP2 antibody, an anti-B7-H3 antibody, an anti-GPC20 antibody, an anti-CDH6 antibody, an anti-EGFR antibody, an anti-EGFRvIII antibody, an anti-AXL antibody, an anti-Nectin-4 antibody, an anti-Tissue factor antibody, an anti-TIM-1 antibody, an anti-PSMA antibody, an anti-EpCAM antibody, an anti-MUC1 antibody, an anti-STEAP1 antibody, an anti-GPNMB antibody, an anti-FGF2 antibody, an anti-FOLR1 antibody, an anti-c-MET antibody, an anti-GFR antibody, an anti-AGS-16, an anti-Guanylyl cyclase C

antibody, an anti-Mesothelin antibody, an anti-SLC44A4 antibody, an anti-EphA2 antibody, an anti-AGS-5 antibody, an anti-GPC-3 antibody, an anti-c-KIT antibody, an anti-ROR1 antibody, an anti-PD-L1 antibody, an anti-CD27L antibody, an anti-5T4 antibody, an anti-Mucin 16 antibody, an anti-NaPi2b antibody, an anti-STEAP antibody, an anti-SLITRK6 antibody, an anti-ETBR antibody, an anti-BCMA antibody, an anti-CEACAM5 antibody, an anti-SC-16 antibody, an anti-SLC39A6 antibody, an anti-Delta-like protein3 antibody, an anti-Claudin18.2 antibody, an anti-CD19 antibody, an anti-CD20 antibody, an anti-CD22 antibody, an anti-CD30 antibody, an anti-CD33 antibody, an anti-CD37 antibody, an anti-CD45 antibody, an anti-CD56 antibody, an anti-CD66e antibody, an anti-CD70 antibody, an anti-CD73 antibody, an anti-CD74 antibody, an anti-CD79b antibody, an anti-CD138 antibody, an anti-CD147 antibody, an anti-CD166 antibody, an anti-CD223 antibody, an anti-MUC16 antibody, an anti-MSLN antibody, an anti-ENPP3 antibody, an anti-SLTRK6 antibody, an anti-FGFR antibody, an anti-LIV-1 antibody, an anti-Lewis Y antibody, an anti-av-integrin antibody, an anti-ASCT2 antibody, an anti-C4.4a antibody, an anti-CA-IX antibody, an anti-CD324 antibody, an anti-CD352 antibody, an anti-CD44v6 antibody, an anti-CD48a antibody, an anti-CLL-1 antibody, an anti-Cripto antibody, an anti-CS1 antibody, an anti-DPEP3 antibody, an anti-Ephrin-A2 antibody, an anti-Ephrin-A4 antibody, an anti-ETBR antibody, an anti-FGFR2 antibody, an anti-FGFR3 antibody, an anti-FLT3 antibody, an anti-GD3 antibody, an anti-GloboH antibody, an anti-GPC3 antibody, an anti-LAMP-1 antibody, an anti-LRRC15 antibody, an anti-Ly6E antibody, an anti-MFI2 antibody, an anti-NOTCH3 antibody, an anti-p-cadherin antibody, an anti-PRLR antibody and an anti-RNF43 antibody, and antigen-binding fragments of the above antibodies.

In the present application, the heavy chain HCDR1, HCDR2 and HCDR3, and light chain LCDR1, LCDR2 and LCDR3 of Ab comprise the heavy chain HCDR1, HCDR2 and HCDR3, and light chain LCDR1, LCDR2 and LCDR3 of the antibody, respectively.

In the present application, the heavy chain variable region (VH) and light chain variable region (VL) of Ab comprise the heavy chain variable region (VH) and light chain variable region (VL) of the antibody, respectively. In the present application, the heavy chain and light chain of the Ab comprise the heavy chain and light chain of the antibody, respectively. In the present application, the Ab is an anti-Claudin18.2 antibody or an antigen-binding fragment thereof. In the present application, the Ab is an anti-human folate receptor antibody, or an antigen-binding fragment thereof.

The present application also provides a pharmaceutical composition, wherein it comprises the compound, or a tautomer, mesomer, raceme, enantiomer or diastereomer thereof, or mixture thereof, or a pharmaceutically acceptable salt, prodrug or solvate thereof as described above.

The present application also provides use of the compound, or a tautomer, mesomer, raceme, enantiomer or diastereomer thereof, or mixture thereof, or a pharmaceutically acceptable salt, prodrug or solvate thereof as described above in the preparation of a medicament for treating and/or preventing a tumor.

In the present application, the tumor is a tumor associated with a target selected from the group consisting of: Claudin18.2, human folate receptor, HER2, HER3, TROP2, B7-H3, GPC20, CDH6, EGFR, EGFRvIII, AXL, Nectin-4, Tissue factor, TIM-1, PSMA, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, PD-L1, CD27L, 5T4, Mucin 16, NaPi2b, STEAP, SLITRK6, ETBR, BCMA, CEACAM5, SC-16, SLC39A6, Delta-like protein3, Claudin18.2, CD19, CD20, CD22, CD30, CD33, CD37, CD45, CD56, CD66e, CD70, CD73, CD74, CD79b, CD138, CD147, CD166, CD223, MUC16, MSLN, ENPP3, SLTRK6, FGFR, LIV-1, Lewis Y, av-integrin, ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, MFI2, NOTCH3, p-cadherin, PRLR and RNF43.

In the present application, the tumor associated with the targets comprises a tumor having high expression of the targets and/or a tumor with positive targets.

In the present application, the tumor is selected from the group consisting of: a solid tumor, a hematological tumor, and metastatic, refractory or recurrent lesion of cancer.

In the present application, the tumor is selected from the group consisting of: esophageal cancer, gastrointestinal cancer, pancreatic cancer, thyroid cancer, colorectal cancer, kidney cancer, lung cancer (such as non-small cell lung cancer), liver cancer, stomach cancer, gastric adenocarcinoma, gastroesophageal junction (GEJ) adenocarcinoma, head and neck cancer, bladder cancer, breast cancer, uterine cancer, cervical cancer, ovarian cancer, prostatic cancer, testicular cancer, germ cell cancer, bone cancer, skin cancer, thymus cancer, bile duct cancer, gallbladder cancer, melanoma, mesothelioma, lymphoma, myeloma (such as multiple myeloma), sarcoma, glioblastoma and leukemia.

EXAMPLES

The experimental methods used in the following Examples, unless otherwise specified, are each independently a conventional method.

All the materials, reagents, etc., used in the following Examples are commercial available, unless otherwise specified.

In the following Examples, all the following reagents are commercial available: Fmoc-L-glutamic acid-5-tert-butyl

ester, compound 12-1, HATU (2-(7-azobenzotriazole)-N,N,N',N'-tetramethylurea hexafluorophosphate), DIEA (N,N-diisopropylethylamine), di-(p-nitrobenzene)carbonate, Exatecan mesulfonate, Mal-amido-PEG4-acid (maleimide-PEG4-propionic acid), DCM (dichloromethane), TFA (trifluoroacetic acid), 6-maleimide-hexanoic acid, Fmoc-L-valyl-L-alanine, di-(p-nitrophenyl)carbonate, Fmoc-L- glutamic acid-5-tert-butyl ester, 4-aminobenzyl alcohol, Fmoc-L-valine, MMAE (monomethyl auristatin E), HOBT (1-hydroxybenzotriazole), methyl tert-butyl ether, compound 65-1, $BH_3$-Me2S, DMAP (4-dimethylaminopyridine), $(BOC)_2O$, EA (ethyl acrylate), TBAF (tetra-n-butylammonium fluoride), EEDQ (2-ethoxy-1-ethoxycarbonyl-1,2-dihydroquinoline), compound 71-1,6-(maleimide) succinimide adipate, compound 72-1, HOSu (N-hydroxysuccinimide), EDCI (carbodiimide), Fmoc-glycine, nitrophenylchloroformate, compound 73-1, BCN-PEG4-acid (rel-1-(((1R, 8S, 9s)-bicyclo[6.1.0] non-4-yn-9-yl)-3-oxo-2,7,10,13,16-pentaoxa-4-aza-nonadecane-19-acid, Fmoc-NMe-PEG4-C2-Acid, N,N-diisopropylethylamine, 4-methylsulfonylbenzoic acid, DIPEA (N,N-diisopropylethylamine), external-3,6-epoxy-1,2,3,6-tetrahydrophthalic anhydride, N-Boc-N-methylethylenediamine.

**Example 1 (Preparation of LD-12)**

The synthetic scheme of LD-12 is as follows:

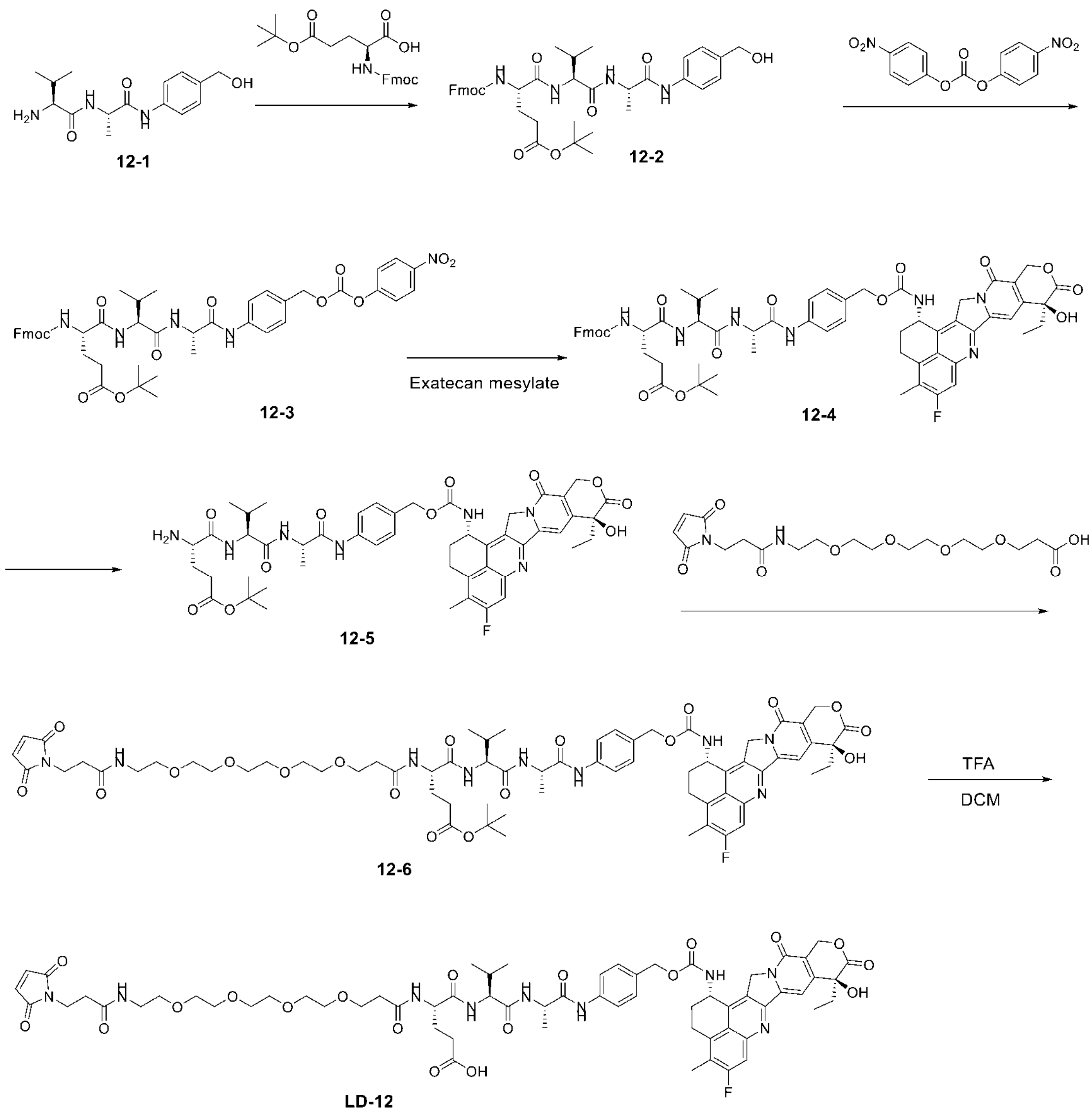

12-1 12-2 12-3 12-4 12-5 12-6 LD-12

Synthesis of 12-2

Fmoc-L-glutamic acid-5-tert-butyl ester (600 mg, 1.41 mmoL) and Compound 12-1 (414 mg, 1.41 mmoL) were dissolved in 18 mL of anhydrous DMF, adding HATU (644 mg, 1.69 mmoL), and DIEA (365 mg, 2.82 mmoL) to stir continuously for 18 hours at room temperature. LCMS showed that the reaction was performed completely. After adding

water and extracting with ethyl acetate for three times, the organic phases were combined, washing with saturated saline solution, drying over anhydrous sodium sulfate, then filtering and concentrating to obtain 0.9g of a crude product.

Synthesis of 12-3

Compound 12-2 (300 mg, 0.43 mmoL), bis(p-nitrophenyl) carbonate (261 mg, 0.86 mmoL) and DIEA (166 mg, 1.28 mmoL) were dissolved in 4 mL of anhydrous DMF to stir for 18 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using thin-layer TLC plate (MeOH/DCM, 0% to 3%) to obtain 0.3g of a final product. MS+Na(+)889.4.

Synthesis of 12-4

Compound 12-3 (350 mg, 0.425 mmoL), DIEA(207 mg, 1.61 mmoL) and exatecan mesylate (600 mg, 0.69 mmoL) were dissolved in 2 mL of anhydrous DMF to react for 6 hours at room temperature. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 470mg of a final product. MS (+)1162.4.

Synthesis of 12-5

Compound 12-4 (100 mg, 0.086 mmoL) was dissolved in 2 mL of anhydrous DMF, adding piperidine (0.05mL) to stir for 2 hours at room temperature, after concentration at reduced pressure, beating with methyl tert-butyl ether and filtering to obtain 80mg of a crude product, which was directly used for the next reaction. MS (+)940.4.

Synthesis of 12-6

Compound 12-5 (46 mg, 0.11 mmoL) was dissolved in 2 mL of anhydrous DMF, adding HATU (48.5 mg, 0.13 mmoL) to stir for 5 minutes at room temperature, then adding Mal-amido-PEG4-acid (80 mg, 0.085 mmoL) and DIEA (33 mg, 0.26 mmoL) in sequence to stir continuously for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using thin-layer TLC plate (MeOH/DCM, 0% to 3%) to obtain 30mg of a final product. MS (+)1339.2.

Synthesis of LD-12

Compound 12-6 (30 mg, 0.022 mmoL) was dissolved in 2 mL of DCM, adding 0.3mL of TFA to stir for 2 hours at room temperature. LCMS showed that the reaction was performed completely. After concentration under reduced pressure, reversed phase preparation was performed (mobile phase, containing 0.1% formic acid) to obtain 7.7 mg of a product.

LCMS:m/z(ES+)(M+H)+=1282.4, Rt=1.385 min

1H NMR (400 MHz, DMSO-d6) δ 10.00 (s, 1H), 8.23 (d, J = 6.8 Hz, 1H), 8.11-8.03 (m, 3H), 7.79 (d, J = 10.8 Hz, 1H), 7.72 (d, J = 8.4 Hz, 1H), 7.60 (d, J = 8.4 Hz, 2H), 7.38 (d, J = 8.4 Hz, 2H), 7.33 (s, 1H), 7.00 (s, 2H), 6.55 (s, 1H), 5.46 (s, 2H), 5.36-5.25 (m, 3H), 5.09 (s, 2H), 4.41-4.32 (m, 2H), 4.23-4.17 (m, 1H), 3.60 (t, J = 7.2 Hz,2H), 3.50-3.49 (m, 16H), 3.37-3.33 (m, 2H), 3.16-3.14 (m, 2H), 2.45-2.32 (m, 7H), 2.27-2.10 (m, 4H), 2.01-1.96 (m, 1H), 1.96-1.81 (m, 3H), 1.74 (d, J = 6.8 Hz, 1H), 1.31 (d, J = 7.2 Hz, 3H), 0.91-0.83 (m, 9H).

Example 2 (Preparation of LD-27)

The process flow for preparation of LD-27 is as follows:

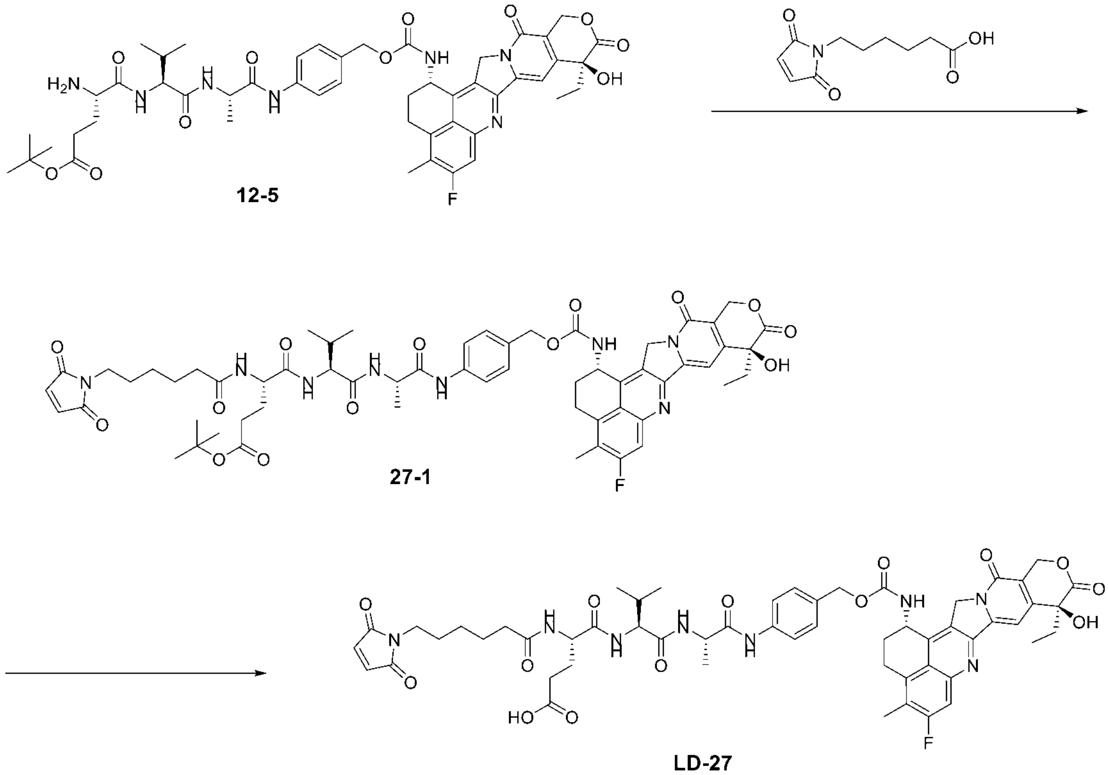

12-5

27-1

LD-27

Synthesis of 27-1

Compound 12-5 (127.34 mg, 0.306 mmoL, synthesis of Compound 12-5 refer to Example 1) and 6-maleimidohexanoic acid (80 mg, 0.085 mmoL) were dissolved in 4 mL of anhydrous DMF, adding HATU (49 mg, 0.128 mmoLl) and DIEA (22 mg, 0.170 mmoL) to stir continuously for 3 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain 80mg of a crude product. MS(+)=1133.4.

Synthesis of LD-27

Compound 27-1 (70 mg, 0.062 mmoL) was dissolved in 5mL of DCM, adding 0.8mL of TFA to stir for 2 hours at room temperature. LCMS showed that the reaction was performed completely. After concentration under reduced pressure, reversed phase preparation was performed (mobile phase, containing 0.1% formic acid) to obtain 22.8 mg of a product.

LCMS:m/z(ES+)(M+H)+=1077.4, Rt=1.454 min

1H NMR (400 MHz, DMSO-d6) δ 12.08 (s, 1H), 9.97 (s, 1H), 8.21 (d, J = 6.8Hz, 1H), 8.06-7.98 (m, 2H), 7.77 (d, J = 10.8 Hz, 1H), 7.65-7.57 (m, 3H), 7.44 - 7.26 (m, 3H), 6.99 (s, 2H), 6.51 (s, 1H), 5.45 (s, 2H), 5.28 (d, J = 4.4 Hz, 3H), 5.08 (s, 2H), 4.42 - 4.15 (m, 3H), 3.26-3.08 (m, 2H), 2.37 (s, 3H), 2.29 - 1.63 (m, 12H), 1.51-1.42 (m, 4H), 1.36 - 1.12 (m, 6H), 0.89-0.81 (m, 9H).

**Example 3 (Preparation of LD-31)**

The synthetic scheme of LD-31 is as follows:

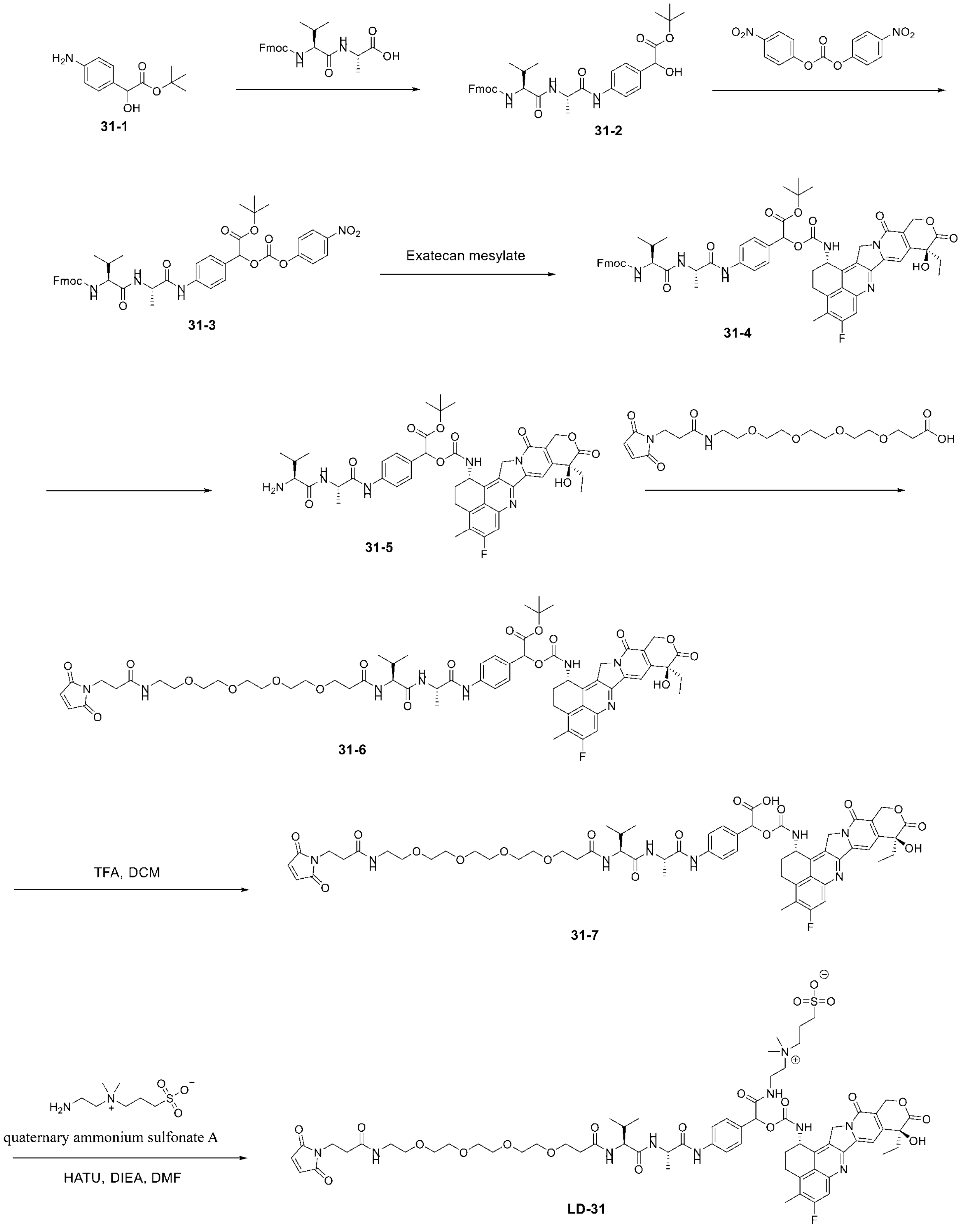

31-1 31-2 31-3 31-4 31-5 31-6 31-7 LD-31

Synthesis of 31-2

Fmoc-L-valyl-L-alanine (220 mg, 0.54 mmoL) was dissolved in 5 mL of anhydrous DMF, adding HATU (244.5 mg, 0.643 mmol) and DIEA (138.5 mg, 1.07 mmoL) to stir for 15 minutes at room temperature, then Compound 31-1 (119.67 mg, 0.536 mmoL, provided by Shanghai Haoyuan Pharmaceutical Co., Ltd., for synthetic method, referring to WO2022058548A1) was added into the reaction solution to stir for 2 hours at room temperature. LCMS detection showed

that the reaction was performed completely. After concentration, a crude product was obtained. Purification was performed by using normal phase silica gel column (MeOH/DCM, 0% to 10%) to obtain 320mg of a final product. MS+Na (+)=638.4.

Synthesis of 31-3

Compound 31-2 (320 mg, 0.520 mmoL) was dissolved in 5 mL of DCM, adding successively bis(p-nitrophenyl) carbonate (474.30 mg, 1.559 mmoL) and DIEA (0.344 mL, 2.079 mmoL) to stir for 2 hours at room temperature. LCMS detection showed that the reaction was performed completely. After adding saturated saline solution and extracting with ethyl acetate, the organic phase was dried over anhydrous sodium sulfate, then filtering. After concentration, a crude product was obtained. Purification was performed by using normal phase silica gel column (MeOH/DCM, 0% to 10%) to obtain 340mg of a final product. MS+Na (+)=803.2.

Synthesis of 31-4

Compound 31-3 (340 mg, 0.462 mmoL) was dissolved in 2 mL of anhydrous DMF, adding exatecan mesylate (245.61 mg, 0.666 mmoL), DIEA (119.43 mg, 0.924 mmoL) and DMAP (5.65 mg, 122.17 mmoL) successively to stir for 1 hour at room temperature. LCMS detection showed that the reaction was performed completely. After adding saturated saline solution, a solid crude product was precipitated, beating with methyl tert-butyl ether to obtain 157mg of Compound 31-4, MS(+)=1078.2.

Synthesis of 31-5

Compound 31-4 (440 mg, 0.408 mmoL) was dissolved in 4 mL of anhydrous DMF, adding piperidine (69.56mg) into the reaction solution to stir for 1 hour at room temperature. After concentration, 292 mg of a crude product was obtained and directly used for the next reaction. MS(+)= 855.2.

Synthesis of 31-6

Compound 31-5 (142.23 mg, 0.342 mmoL) and Mal-amido-PEG4-acid (292 mg, 0.342 mmoL) were dissolved in 4 mL of anhydrous DMF, adding DIEA (88.28 mg, 0.683 mmoL) and HATU (155.84 mg, 0.410 mmoL) to stir for 2 hours at room temperature. LCMS showed that the reaction was performed completely. After adding saturated saline solution, a solid crude product was precipitated, beating with methyl tert-butyl ether to obtain 350mg of a crude product. MS (+) =1254.4.

Synthesis of 31-7

Compound 31-6 (90mg of a crude product) was dissolved in 2.5 mL of anhydrous DMF, adding TFA (0.5mL) into the reaction solution to stir for 1 hour at room temperature. After concentration, 80 mg of a crude product was obtained and directly used for the next reaction. MS(+)=1198.4.

Synthesis of LD-31

Compound 31-7 (76.62 mg, 0.064 mmoL) was dissolved in 2 mL of anhydrous DMF, adding DIEA (16.54 mg, 0.128 mmoL) and HATU (29.20 mg, 0.077 mmoL) to stir for 15 minutes at room temperature, then adding quaternary ammonium sulfonate A (14.81 mg, 0.070 mmoL, CAS#: 78276-19-4, provided by Shanghai Haoyuan Pharmaceutical Co., Ltd., for synthetic method, referring to WO2011146595A2) to stir for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Reversed phase preparation was performed (formic acid) to obtain LD-31 as white solid (P1:12.5 mg, P2:11.3 mg).

LCMS: m/z (ES+) (M+H)+ = 1390.6, Rt = 1.242 min (P1), 1.279 min (P2).

**Example 4 (Preparation of LD-37)**

The synthetic scheme of LD-37 is as follows:

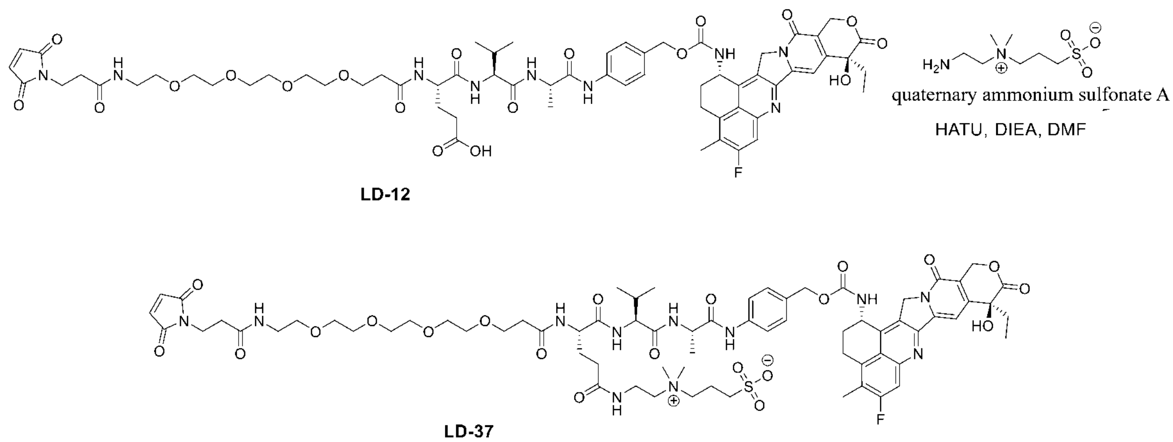


LD-12

LD-37

Synthesis of LD-37

Compound LD-12 (49 mg, 0.038 mmoL, synthesis of LD-12 refer to Example 1) was dissolved in 2 mL of anhydrous DMF, adding DIEA (9.88 mg, 0.076 mmoL) and HATU (17.43 mg, 0.046 mmoL) to stir for 15 minutes at room temperature, adding quaternary ammonium sulfonate A (8.04 mg, 0.038 mmoL, CAS#: 78276-19-4, provided by Shanghai Haoyuan Pharmaceutical Co., Ltd., for synthetic method, referring to WO2011146595A2) to stir for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Reversed phase preparation (mobile phase, containing 0.1% formic acid) was performed to obtain LD-37 as white solid (7.35 mg 13.04% yield).

LCMS: m/z (ES+) (M+H)+ = 1475.6, Rt = 1.344 min

1H NMR (400 MHz, DMSO) $\delta$ 10.03 (s, 1H), 8.24 (d, J = 6.4 Hz, 1H), 8.16 - 8.07 (m, 3H), 8.03 (t, J = 6.0 Hz, 1H), 7.80 (d, J = 10.8Hz, 1H), 7.71 (d, J = 9.2 Hz, 1H), 7.62 (d, J = 8.0 Hz, 2H), 7.38 (d, J = 8.4 Hz, 2H), 7.33 (s, 1H), 7.02 (s, 2H), 6.53 (s, 1H), 5.47 (s, 2H), 5.31 (s, 3H), 5.10 (s, 2H), 4.41 (d, J = 6.8 Hz, 1H), 4.31 (s, 1H), 4.24 - 4.20 (m, 1H), 3.61 (t, J = 7.2 Hz, 5H), 3.46-3.51 (m, 16H), 3.16 (dd, J = 11.2, 5.6 Hz, 4H), 3.05 (s, 6H), 2.69 (s, 3H), 2.40 (s, 4H), 2.35 (s, 6H), 2.18 (s, 4H), 2.01 (s, 2H), 1.90 (d, J = 9.2 Hz, 2H), 1.79 - 1.69 (m, 1H), 1.32 (d, J = 7.2 Hz, 3H), 0.89 (d, J = 6.8 Hz, 6H), 0.84 (d, J = 6.8 Hz, 3H).

**Example 5 (Preparation of LD-38)**

The synthetic scheme of LD-38 is as follows:

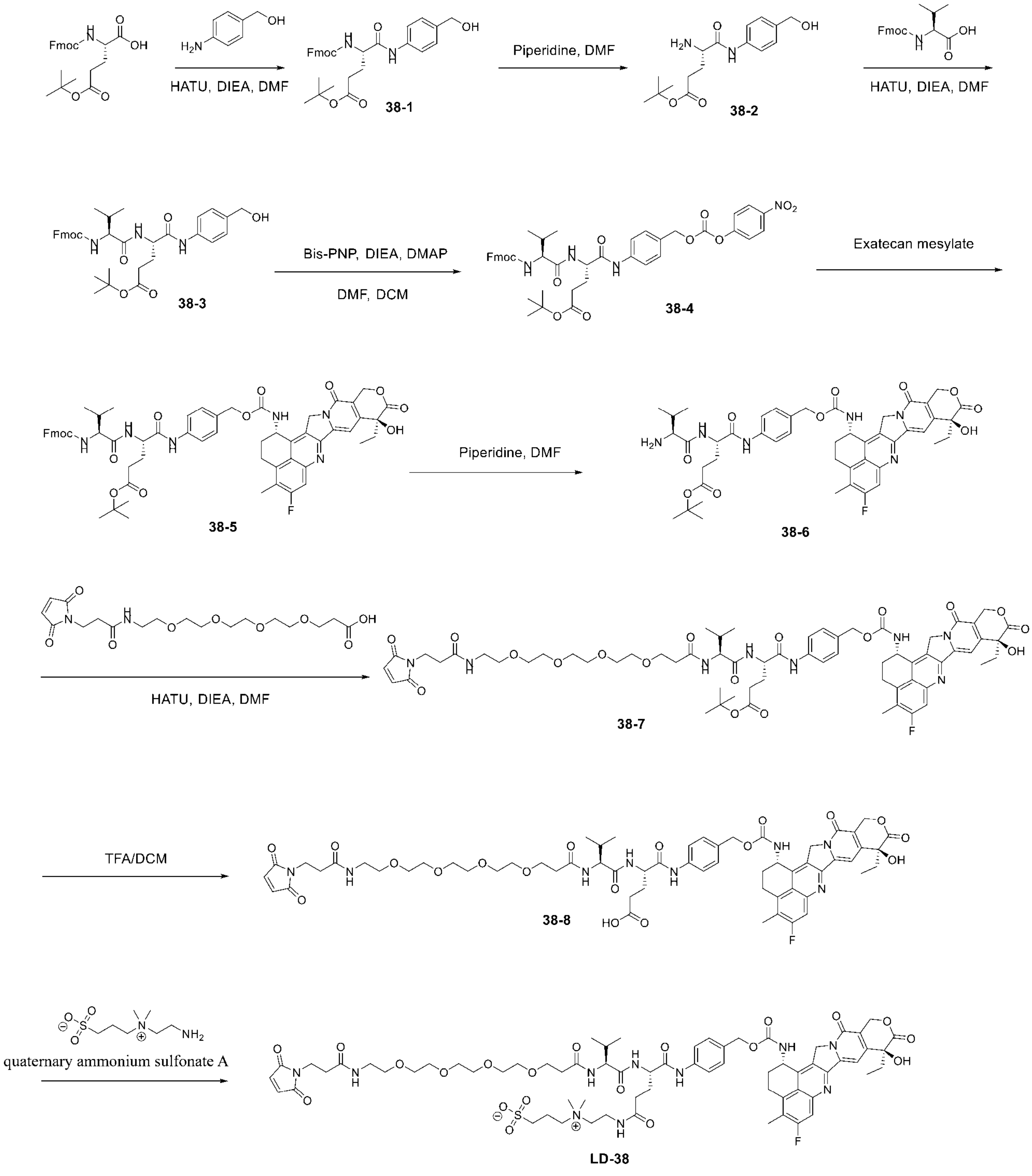


Synthesis of 38-1

Fmoc-L-glutamic acid-5-tert-butyl ester (144.7mg, 1.175 mmoL) and 4-aminobenzyl alcohol (500mg, 1.175 mmoL) were dissolved in 3 mL of anhydrous DMF, adding DIEA (303.7mg, 2.350 mmoL) and HATU (536.19mg, 1.410 mmoL) to stir for 16 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using a normal phase silica gel column (EA/PE, 0% to 45%) to obtain 530mg of a final product. MS(+)=475.2.

Synthesis of 38-2

Compound 38-1 (530mg, 0.999 mmoL) was dissolved in 3 mL of anhydrous DMF, adding piperidine (0.2mL) into

the reaction solution to stir for 1 hour at room temperature. After concentration, 600mg of a crude product was obtained and directly used for the next reaction. MS(+)=309.2.

Synthesis of 38-3

Compound 38-2 (600mg of a crude product) and Fmoc-L-valine (500mg, 1.175 mmoL) was dissolved in 3 mL of anhydrous DMF, adding DIEA (334.13 mg, 2.585 mmoL) and HATU (589.82 mg, 1.551 mmoL) to stir for 16 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (EA/PE, 40% to 60%) to obtain 450mg of a final product. MS(+)=630.4.

Synthesis of 38-4

Compound 38-3 (450mg, 0.715 mmoL) were dissolved in 1mL of anhydrous DCM and 4mL of anhydrous DMF, adding bis(p-nitrophenyl) carbonate (869.5mg, 2.858 mmoL) and 0.354mL of DIEA successively to react for 12 hours at room temperature. After adding water into the reaction solution and extracting with ethyl acetate for three times, the organic phases were combined, washing with saturated saline solution, drying over anhydrous sodium sulfate, then filtering and concentrating to obtain a crude product. Purification was performed by using normal phase silica gel column (EA/PE, 30% to 70%) to obtain 200mg of a final product. MS(+)=543.2.

Synthesis of 38-5

Compound 38-4 (100mg, 0.126 mmoL) and exatecan mesylate (54.78mg, 0.126 mmoL) were dissolved in 2 mL of anhydrous DMF, adding DMAP(0.15mg, 0.0001 mmoL) and DIEA(32.52mg, 0.252 mmoL) successively to react for 1 hour at room temperature. After adding water into the reaction solution and extracting with ethyl acetate for three times, the organic phases were combined, washing with saturated saline solution, drying over anhydrous sodium sulfate, then filtering and concentrating to obtain a crude product. Purification was performed by using normal phase silica gel column (EA/PE, 30% to 70%) to obtain 100mg of final pruduct. MS(+)=1091.4.

Synthesis of 38-6

Compound 38-5 (100mg, 0.073 mmoL) was dissolved in 3 mL of anhydrous DMF, adding piperidine (0.3 mL) into the reaction solution to stir for 1 hour at room temperature. 110mg of a crude product was obtained and directly used for the next reaction. MS(+)=870.8.

Synthesis of 38-7

Compound 38-6 (110mg of a crude product) and Mal-amido-PEG4-acid (30.19mg, 0.073 mmol) were dissolved in 2 mL of anhydrous DMF, adding DIEA (18.74 mg, 0.145 mmoL) and HATU (33.08 mg, 0.087 mmoL) to stir for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 5% to 20%) to obtain 90mg of a final product. MS(+)=1267.6.

Synthesis of 38-8

Compound 38-7 (90mg, 0.071 mmoL) was dissolved in 3 mL of anhydrous DCM, adding 0.3 mL TFA into the reaction solution to stir for 2 hours at room temperature. 80mg of a crude product was obtained and directly used for the next reaction. MS(+)=2111.4.

Synthesis of LD-38

Compound 38-8 (80mg, crude product) and quaternary ammonium sulfonate A (13.89mg, 0.066mmol, CAS#: 78276-19-4, provided by Shanghai Haoyuan Pharmaceutical Co., Ltd., for synthetic method, referring to WO2011146595A2) were dissolved in 2 mL of anhydrous DMF, adding DIEA (17.07 mg, 0.132 mmoL) and HATU (30.14 mg, 0.079 mmoL) to stir for 1 hour at room temperature. LCMS showed that the reaction was performed completely. Reversed phase preparation (mobile phase, containing 0.1% formic acid) was performed to obtain 13.5 mg as white solid.

LCMS: m/z (ES+) (M+H)+ = 1404.6, Rt = 1.314 min
1H NMR (400 MHz, DMSO) δ 9.89 (s, 1H), 8.18 (d, J = 7.2 Hz, 2H), 8.10 - 7.93 (m, 3H), 7.78 (d, J = 10.8 Hz, 1H), 7.63 (d, J = 8.4 Hz, 2H), 7.37 (d, J = 8.4 Hz, 2H), 7.32 (s, 1H), 6.99 (s, 2H), 6.53 (s, 1H), 5.45 (s, 2H), 5.29 (d, J = 4.Hz, 3H), 5.08 (s, 2H), 4.32 (dd, J = 13.2, 7.6 Hz, 1H), 4.20 - 4.14 (m, 1H), 3.59 (dd, J = 14.8, 7.6 Hz, 6H), 3.40-3.51 (m, 18H), 3.14-3.20 (m, 4H), 3.03 (s, 7H), 2.42 - 2.30 (m, 7H), 2.20-2.31 (m, 4H), 2.03 - 1.82 (m, 8H), 0.87-0.91 (m, 9H).

**Example 6 (Preparation of LD-57)**

The synthetic scheme of LD-57 is as follows:

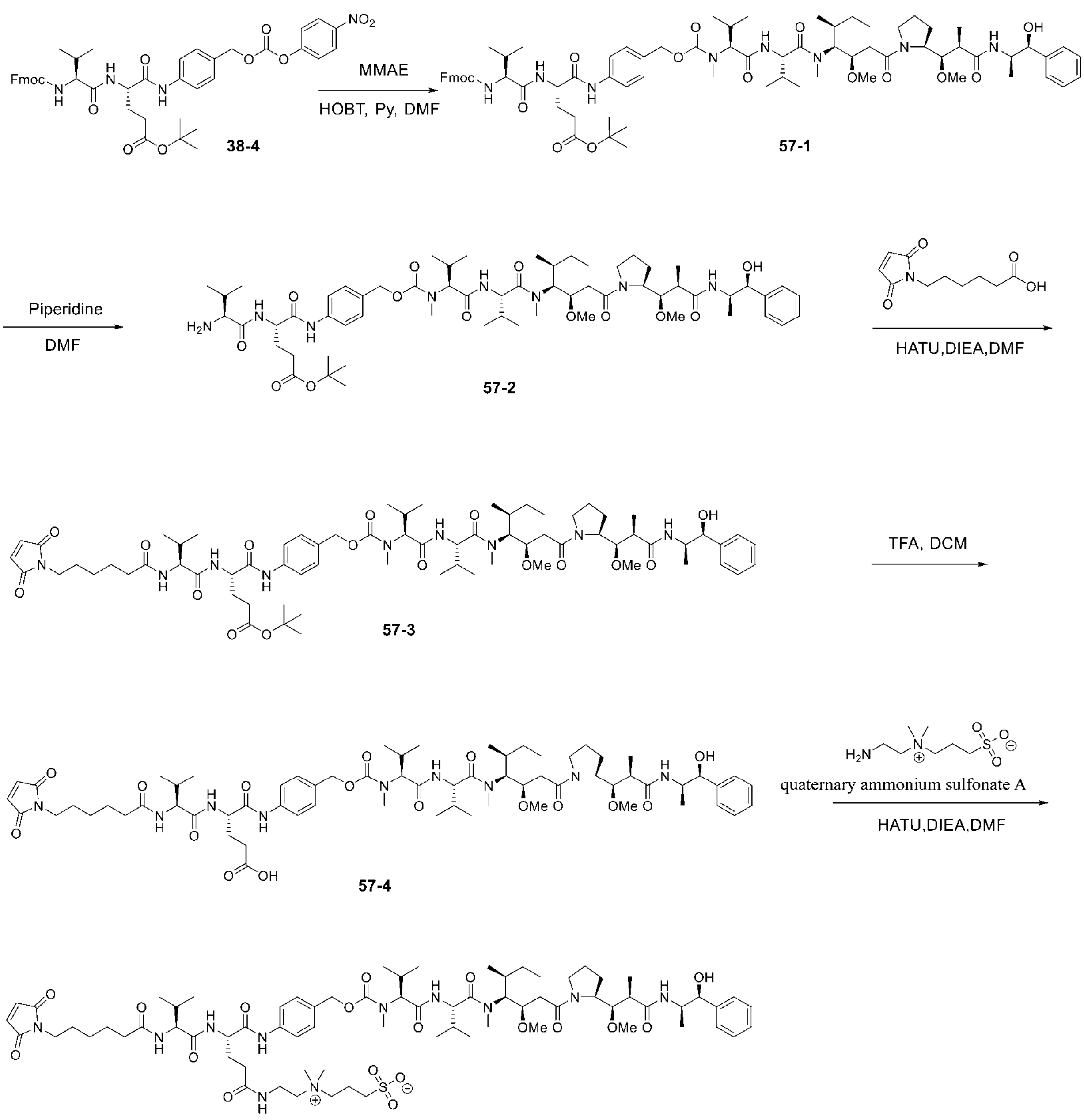

38-4
57-1
57-2
57-3
57-4
LD-57

Synthesis of 57-1

Compound 38-4 (288mg, 0.363 mmoL, the synthetic method refer to Example 5) and MMAE (200mg, 0.279 mmoL) were dissolved in 5mL of anhydrous DMF, adding pyridine (1 mL) and HOBT (59mg, 0.279 mmoL) successively to react for 18 hours at room temperature. After concentration, a crude product was obtained. Purification was performed by using normal phase silica gel column (MeOH/DCM, 0% to 10%) to obtain 320mg of a final product. MS(+)=1374.2.

Synthesis of 57-2

Compound 57-1 (260mg, 0.189 mmoL) was dissolved in 3 mL of anhydrous DMF, adding piperidine (0.05mL) into the reaction solution to stir for 1 hour at room temperature, after concentration, beating with methyl tert-butyl ether to obtain 215mg of a crude product, which was directly used for the next reaction. MS(+)=1152.5.

Synthesis of 57-3

Compound 57-2 (215mg of a crude product) and 6-maleimidohexanoic acid (47.3mg, 0.224mmoL) were dissolved in 3 mL of anhydrous DMF, adding DIEA (0.092 mL, 0.559 mmoL) and HATU (142 mg, 0.374 mmol) to stir for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 170mg of a final product. MS(+)=1345.7.

Synthesis of 57-4

Compound 57-3 (150 mg, 0.112 mmoL) was dissolved in 3 mL of anhydrous DCM, adding TFA (0.6mL) into the reaction solution to stir for 2 hours at room temperature. 150mg of a crude product was obtained and directly used for the next reaction. MS(+)=1289.5.

Synthesis of LD-57

Compound 57-4 (143 mg, 0.111 mmoL) and quaternary ammonium sulfonate A (70 mg, 0.333mmoL, CAS#: 78276-19-4, provided by Shanghai Haoyuan Pharmaceutical Co., Ltd., for synthetic method, referring to WO2011146595A2) were dissolved in 2 mL of anhydrous DMF, adding DIEA (0.092 mL, 0.554 mmoL) and HATU(126.5 mg, 0.333 mmoL) to stir for 3 hours at room temperature. LCMS showed that the reaction was performed completely. Reversed phase preparation (mobile phase, containing 0.1% formic acid) was performed to obtain 30 mg of white solid.

LCMS:m/z(ES+)(M+H)+=1481.2, Rt=1.475 min
1H NMR (400 MHz, DMSO) δ 9.89 (s, 1H), 8.40 - 8.03 (m, 3H), 7.92 (t, J = 7.6 Hz, 1H), 7.63 (d, J = 6.4 Hz, 2H), 7.34 - 7.13 (m, 8H), 7.02 (s, 2H), 5.06-5.10 (m, 3H), 4.85 - 3.92 (m, 10H), 3.63 - 3.43 (m, 9H), 3.30 - 2.97 (m, 18H), 2.89 (d, J = 14.4 Hz, 3H), 2.37 - 1.68 (m, 17H), 1.61 - 1.45 (m, 6H), 1.40 - 0.70 (m, 36H).

**Example 7 (Preparation of LD-65)**

The synthetic scheme of LD-67 is as follows:

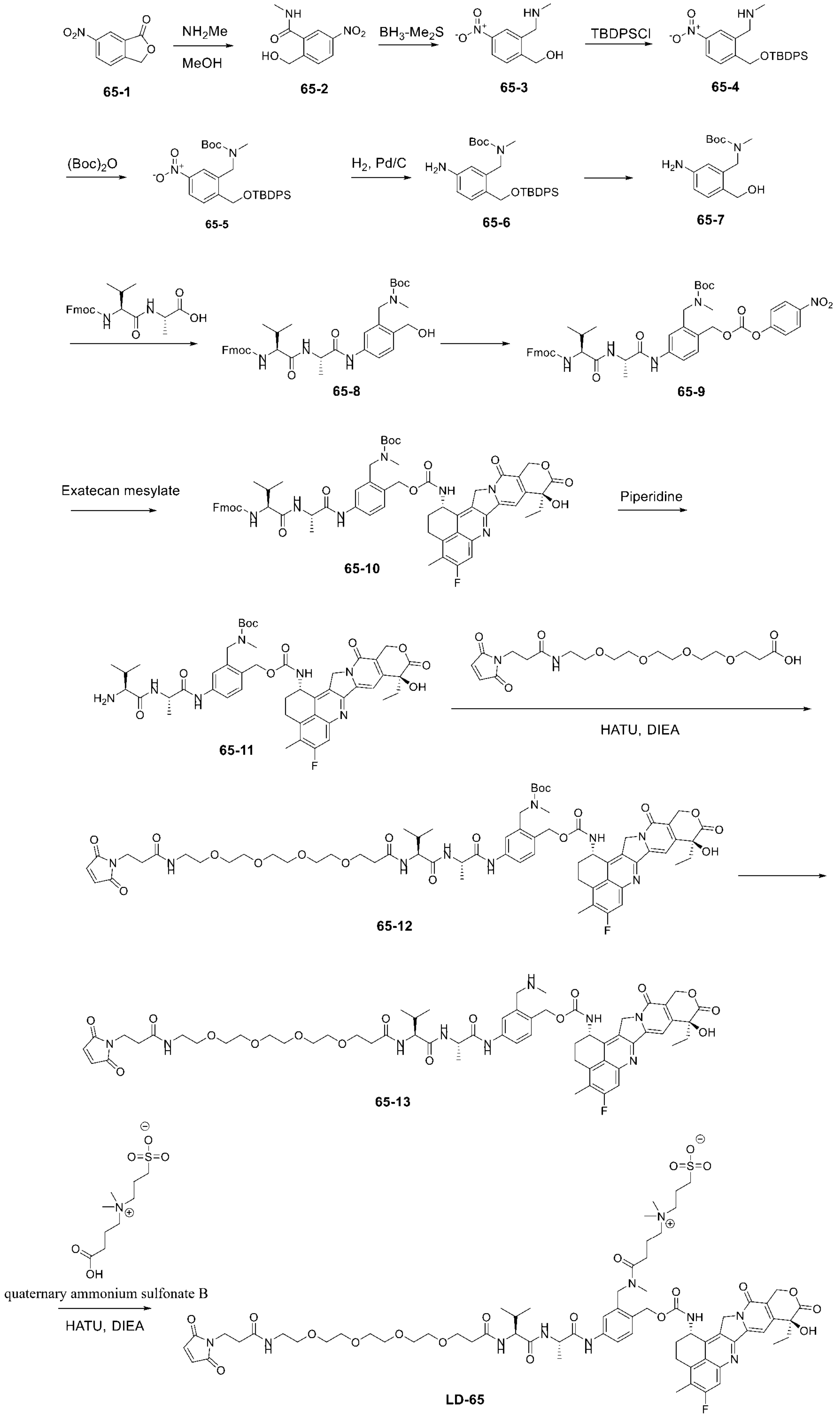


65-1 65-2 65-3 65-4 65-5 65-6 65-7 65-8 65-9 65-10 65-11 65-12 65-13 LD-65

Synthesis of 65-2

Compound 65-1 (500 mg, 2.791 mmol) was dissolved in 5mL of methanol, adding methylamine methanol solution (1.73g, 55.825 mmol) to react for 1 hour at room temperature, then filtering to obtain a crude product, washing with water for two times. After drying under reduced pressure, 550mg of a final product was obtained. MS(+)211.2.

Synthesis of 65-3

Compound 65-2 (500 mg, 2.379 mmoL) was dissolved in 50 mL of anhydrous THF, adding $BH_3$-$Me_2S$ (457mg, 5.947 mmol) (10.0 M in THF) slowly dropwise under ice bath to perform reflux for 5 hours. LCMS showed that the reaction was performed completely. 4M HCl (12 mL) methanol solution was added under ice bath into the reaction solution, then heating up to 65°C to react continuously for 8 hours, filtering under reduced pressure to obtain 350mg of a crude product. MS(+)197.08.

Synthesis of 65-4

Compound 65-3 (350mg of a crude product) and imidazole (485.77mg, 7.135 mmoL) were dissolved in 5 mL of anhydrous CMF, adding TBDPS-Cl (735.44mg, 2.676 mmoL) to stir for 2 hours at 0°C. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (EA/PE, 10% to 50%) to obtain 710mg of a final product. MS(+)435.20.

Synthesis of 65-5

Compound 64-4 (1.5 g, 3.451 mmoL) and DMAP (0.21 g, 1.726 mmoL) were dissolved in DCM (20 mL), adding $(BOC)_2O$ (1.51 g, 6.903 mmoL) to stir for 2 hours. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (EA/PE, 10% to 50%) to obtain 1.87g of a final product. MS+Na(+) 557.3.

Synthesis of 65-6

Compound 65-5 (1.7 g, 3.179 mmoL) and Pd/C 5% (0.15 g, 1.410 mmoL) were dissolved in EA (30 mL), stirring the reaction solution for 16 hours under hydrogen atmosphere, filtering and spin-drying to obtain 1.7g of a final product. MS+Na(+)527.6.

Synthesis of 65-7

Compound 65-6 (350 mg, 0.693 mmol) was dissolved in anhydrous THF (4 mL), adding TBAF (1.387 mL, 1.387 mmoL) to stir for 4 hours at room temperature. The reaction solution was spin-dried, and purification was performed by using normal phase silica gel column (MeOH/DCM, 0% to 10%) to obtain 170mg of a final product. MS+Na(+)=289.2.

Synthesis of 65-8

Compound 65-7 (170 mg, 0.638 mmoL), Fmoc-L-valyl-L-alanine (392.99 mg, 0.957 mmol) and EEDQ (236.48 mg, 0.957mmoL) were dissolved in 2mL of DCM and 1mL of methanol to stir for 16 hours at room temperature. The reaction solution was spin-dried, and purification was performed by using normal phase silica gel column (MeOH/DCM, 0% to 10%) to obtain 367mg of a final product. MS(+)Na(+)=681.4.

Synthesis of 65-9

Compound 65-9 (367 mg, 0.557 mmoL), bis(p-nitrophenyl) carbonate (224.57 mg, 1.114 mmoL) and pyridine (0.090 mL, 1.114 mmoL) were dissolved in 5mL of THF to stir for 16 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 380mg of a final product. MS(+)Na(+)=846.4.

Synthesis of 65-10

Compound 65-9 (350 mg, 0.425 mmoL), DMAP (5.19 mg, 0.043 mmoL) and exatecan mesylate (248.50 mg, 0.468 mmoL) were dissolved in 2 mL of anhydrous DMF to react for 2 hours at room temperature. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 350mg of a final product. MS(+)1120.6.

Synthesis of 65-11

Compound 65-10 (350 mg, 0.312 mmoL) was dissolved in 2 mL of anhydrous DMF, adding pyridine (0.05mL) to stir for 2 hours at room temperature. After concentration under reduced pressure, 380mg of a crude product was obtained and directly used for the next reaction. MS(+)=898.41.

Synthesis of 65-12

Compound 65-11 (127.34 mg, 0.306 mmoL) was dissolved in 3 mL of anhydrous DMF, adding HATU (116.28 mg, 0.306 mmoL) to stir for 5 minutes at room temperature, then adding Mal-amido-PEG4-acid (250 mg, 0.278 mmoL) and DIEA (3 mL) successively to stir for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 185mg of a final product. MS (+)=1296.6.

Synthesis of 65-13

Compound 65-12 (40 mg, 0.031 mmoL) was dissolved in 3mL of DCM, adding 0.6mL of TFA to stir for 2 hour at room temperature. LCMS showed that the reaction was performed completely. After concentration under reduced pressure, 37 mg of a final product was obtained. MS+Na(+)=1218.6.

Synthesis of LD-65

Quaternary ammonium sulfonate B (37 mg, 0.031 mmol, CAS#: 1349808-80-5, provided by Shanghai Haoyuan Pharmaceutical Co., Ltd., for synthetic method, referring to WO2011146595A2) was dissolved in 2 mL of anhydrous DMF, adding HATU (12.94 mg, 0.034 mmoL) to stir for 5 minutes at room temperature, then adding Compound 65-13 (11.75 mg, 0.046 mmoL) and DIEA (0.008 mL, 0.046 mmoL) successively to stir continuously for 2 hours at room temperature. LCMS showed that the reaction was performed completely. Reversed phase preparation was performed (mobile phase, containing 10 mM $NH_4OAc$) to obtain 5.2mg of white solid.

LCMS: m/z (ES+) (M+H)+ = 1432.6, Rt = 1.285 min
1H NMR (400 MHz, DMSO) δ 10.2-9.9 (m,1H), 8.19 (t, J = 7.2 Hz, 1H), 8.12 - 8.00 (m, 2H), 7.90 - 7.85 (m, 1H), 7.78 (d, J = 10.8 Hz, 1H), 7.39 - 7.30 (m, 3H), 7.00 (s, 2H), 6.52 (s, 1H), 5.44 (d, J = 4.0 Hz, 2H), 5.29 (s, 4H), 5.12 (d, J = 10.8 Hz, 2H), 4.64-4.70 (m, 2H), 4.39 - 4.34 (m, 1H), 4.22 - 4.17 (m, 1H), 3.48-3.47 (m, 16H), 3.04-2.86 (m, 14H), 2.39 - 2.30 (m, 11H), 2.03 - 1.85 (m, 10H), 1.29-1.30 (m, 3H), 0.897-0.817 (m, 9H).

**Example 8 (Preparation of LD-66)**

The synthetic scheme of LD-66 is as follows:

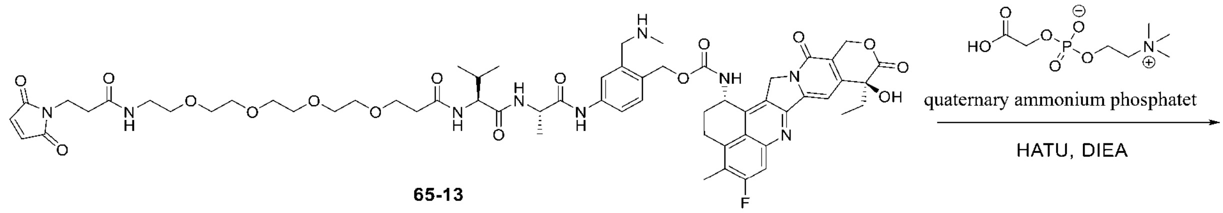


65-13

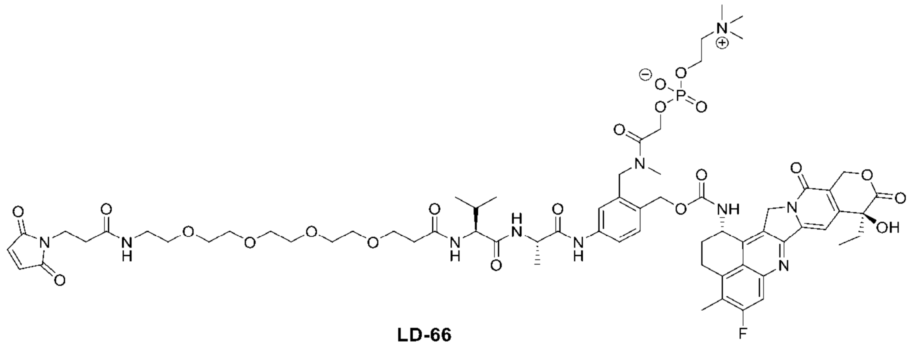

LD-66

Synthesis of LD-66

Quaternary ammonium phosphate (8.93 mg, 0.046 mmol, Cas#: 853798-56-8, provided by Shanghai Haoyuan Pharmaceutical Co., Ltd., for synthetic method, referring to WO2005114193) was dissolved in 2 mL of anhydrous DMF, adding DIEA (0.008 mL, 0.046 mmoL) and HATU (12.94 mg, 0.034 mmoL) to stir for 5 minutes at room temperature, then adding Compound 65-13 (37 mg, 0.031 mmoL) to stir for 2 hours. LCMS showed that the reaction was performed completely. Reversed phase preparation (formic acid) was performed to obtain 11.5 mg of white solid.

LCMS: m/z (ES+) (M+H)+ = 1420.6, Rt = 1.246 min

1H NMR (400 MHz, DMSO) $\delta$ 10.11 (s, 1H), 8.23-8.17 (m,1H), 8.07 - 7.87 (m, 2H), 7.79 - 7.69 (m, 2H), 7.45 - 7.13 (m, 4H), 7.00 (s, 2H), 6.51 (s, 1H), 5.47 - 5.06 (m, 8H), 4.83 (dd, J = 45.6, 17.8 Hz, 1H), 4.59 (d, J = 7.6 Hz, 1H), 4.48 (d, J = 8.4 Hz, 1H), 4.37 (d, J = 6.8 Hz, 1H), 4.21 - 4.09 (m, 4H), 3.49-3.48 (m, 16H), 3.19 - 3.10 (m, 12H), 2.96 (s, 5H), 2.39 - 2.32 (m, 8H), 1.99 - 1.83 (m, 4H), 1.30 (d, J = 6.8 Hz, 3H), 0.90-0.82 (m, 9H).

**Example 9 (Preparation of LD-71)**

The synthetic scheme of LD-71 is as follows:

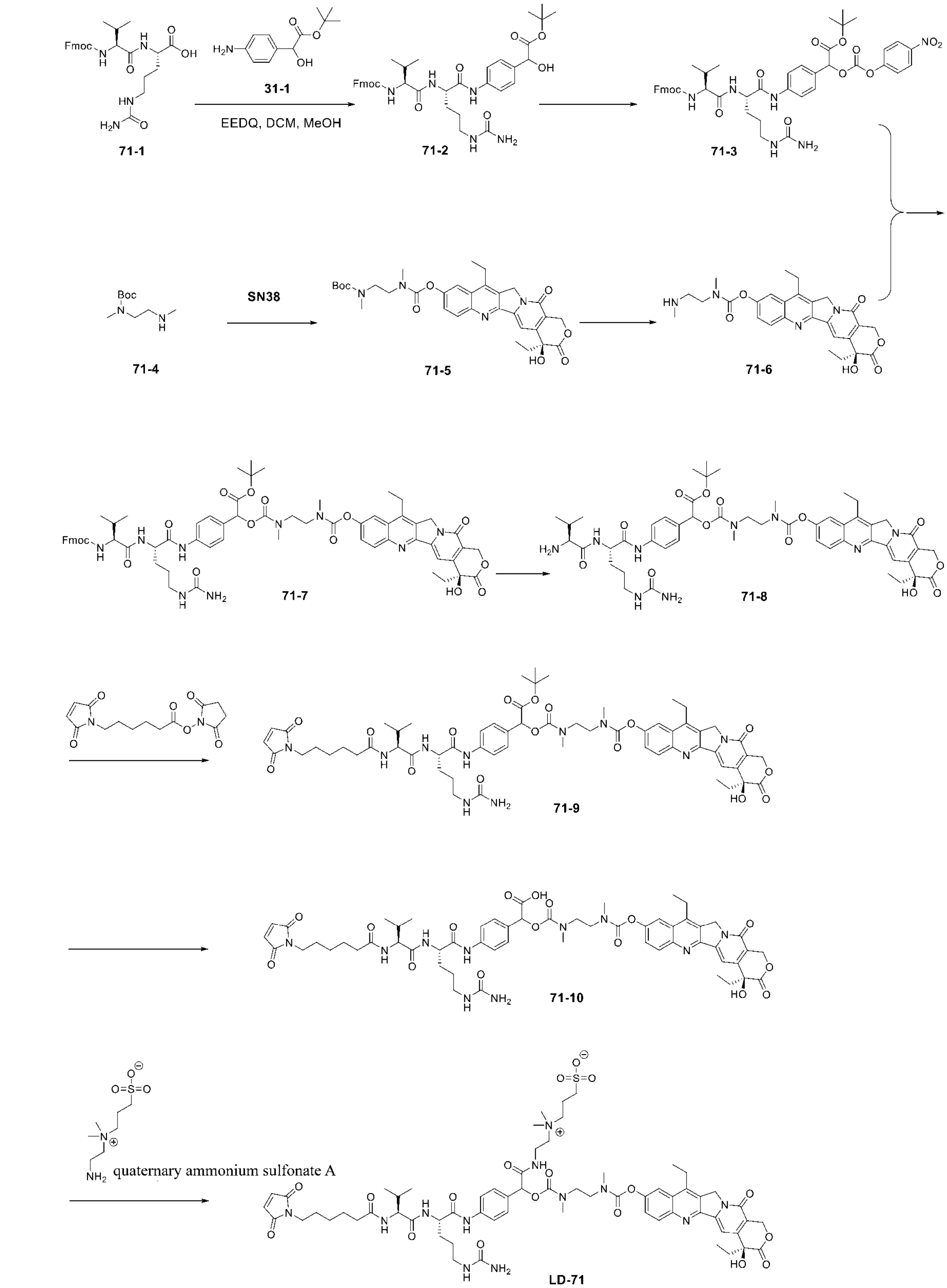


71-1 71-2 71-3 71-4 71-5 71-6 71-7 71-8 71-9 71-10 LD-71

Synthesis of 71-2

Compound 31-1 (240mg, 1.075 mmoL, the synthesis method refer to Example 1) and Compound 71-1 (267mg, 0.538 mmoL, provided by Shanghai Haoyuan Pharmaceutical Co., Ltd., for synthetic method, referring to WO2022058548A1) were dissolved in 3 mL of anhydrous DCM and 1.5mL of methanol, adding EEDQ (266mg, 1.075

mmol) to stir for 3 hours at room temperature. A solid product was precipitated. LCMS detection showed that the reaction was performed completely. After filtering the reaction solution, the filter cake was washed with DCM for three times, drying under reduced pressure to obtain 260mg of a final product. MS(+)=702.4.

Synthesis of 71-3

Compound 71-2 (240mg, 0.342 mmoL), bis(p-nitrophenyl) carbonate (312.09 mg, 1.026mmol) and DIEA (0.170 mL, 1.026 mmol) were dissolved in 5 mL of anhydrous DMF to stir for 16 hours at room temperature. LCMS detection showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 5% to 10%) to obtain 220mg of a final product. MS(+)=867.4.

Synthesis of 71-5

Triphosgene (136.1mg, 0.459 mmol) was dissolved in 4 mL of anhydrous DCM under protection of nitrogen at room temperature, adding triethylamine (0.128mL, 0.917mol) and 71-4 (172.6mg, 0.917mol) dropwise under ice bath to stir for 3 hours to react at room temperature, then adding pyridine (2mL), SN38 (120mg, 0.306 mmoL) and DMAP (7.47mg, 0.061mol) to react for 16 hours at 50°C. LCMS detection showed that the reaction was performed completely. After concentration and spin-drying, beating with methyl tert-butyl ether, 127mg of a final product was obtained. MS(+)=607.4.

Synthesis of 71-6

Compound 71-5 (140 mg, 0.231 mmoL) was dissolved in 3 mL of anhydrous DCM, adding 0.6mL of TFA to stir for 2 hours at room temperature. LCMS showed that the reaction was performed completely. After concentration under reduced pressure, 116.9mg of a final product was obtained. MS(+)=507.2.

Synthesis of 71-7

Compound 71-6 (116.9 mg, 0.231 mmoL) and Compound 71-3 (200 mg, 0.231mmoL) were dissolved in 2mL of DMSO and 1mL of DMF, adding HOBT (31.17 mg, 0.231 mmoL) and DIEA (59.64mg, 0.461 mmoL) successively, heating up to 40°C to stir for 3 hours, then adding saturated saline solution into the reaction solution to obtain a crude product. Purification was performed by using normal phase silica gel column (MeOH/DCM, 0% to 10%) to obtain 200mg of a final product. MS(+)=1234.6.

Synthesis of 71-8

100mg of crude product Compound 71-7 was dissolved in 2 mL of anhydrous DMF, adding pyridine (0.05mL) to stir for 2 hours at room temperature. After concentration under reduced pressure, beating with methyl tert-butyl ether and filtering, 100mg of a crude product was obtained and directly used for the next reaction. MS (+)1012.6.

Synthesis of 71-9

100mg of crude product Compound 71-8 and 6-maleimidohexanoic acid N-hydroxysuccinimide ester (43.9mg, 0.142 mmoL) were dissolved in 2 mL of anhydrous DMF, adding DIEA to stir for 2 hours at room temperature. After adding water into the reaction solution and extracting with ethyl acetate for three times, the organic phases were combined, washing with saturated saline solution, drying over anhydrous sodium sulfate, then filtering and concentrating to obtain a crude product. Purification was performed by using thin-layer silicone plate (MeOH/DCM, 0% to 10%) to obtain 60mg of a final product. MS(+)=1205.6.

Synthesis of 71-10

Compound 71-9 (60 mg, 0.05 mmoL) was dissolved in 3mL of DCM, adding 0.3mL TFA to stir for 2 hours at room temperature. LCMS showed that the reaction was performed completely. After concentration under reduced pressure, 57mg of a final product was obtained. MS(+)=1149.6.

Synthesis of LD-71

Compound 71-10 (57 mg, 0.050 mmoL) was dissolved in 2 mL of anhydrous DMF, adding HATU (20.76 mg, 0.055 mmol) to stir for 5 minutes at room temperature, then adding quaternary ammonium sulfonate A (15.64 mg, 0.074 mmol, CAS#: 78276-19-4, provided by Shanghai Haoyuan Pharmaceutical Co., Ltd., for synthetic method, referring to WO2011146595A2) and DIEA (0.025 mL, 0.149 mmoL) successively to stir continuously for 2 hours at room temperature. LCMS showed that the reaction was performed completely. Reversed phase preparation was performed (mobile phase, containing 0.1% formic acid) to obtain 24.0mg of white solid.

LCMS: m/z(ES+)(M+H)+=1341.6, Rt=1.210 min

1H NMR (400 MHz, DMSO) δ 10.04-9.96 (m, 1H), 8.45 (s, 1H), 8.185-8.085 (m, 2H), 7.968-7.782 (m, 3H), 7.63 - 7.32 (m, 9H), 6.99 (s, 2H), 6.53 (s, 1H), 5.99 (s, 1H), 5.70 (d, J = 20.6 Hz, 1H), 5.43-5.41 (m, 5H), 5.35 (s, 3H), 4.35-4.25 (m, 1H), 4.19-4.17 (m, 1H), 3.07-2.91 (m, 20H), 1.97 - 1.83 (m, 8H), 1.52 - 1.42 (m, 6H), 1.32 - 1.15 (m, 9H), 0.90-0.82 (m, 9H).

**Example 10 (Preparation of LD-72)**

The synthetic scheme of LD-72 is as follows:

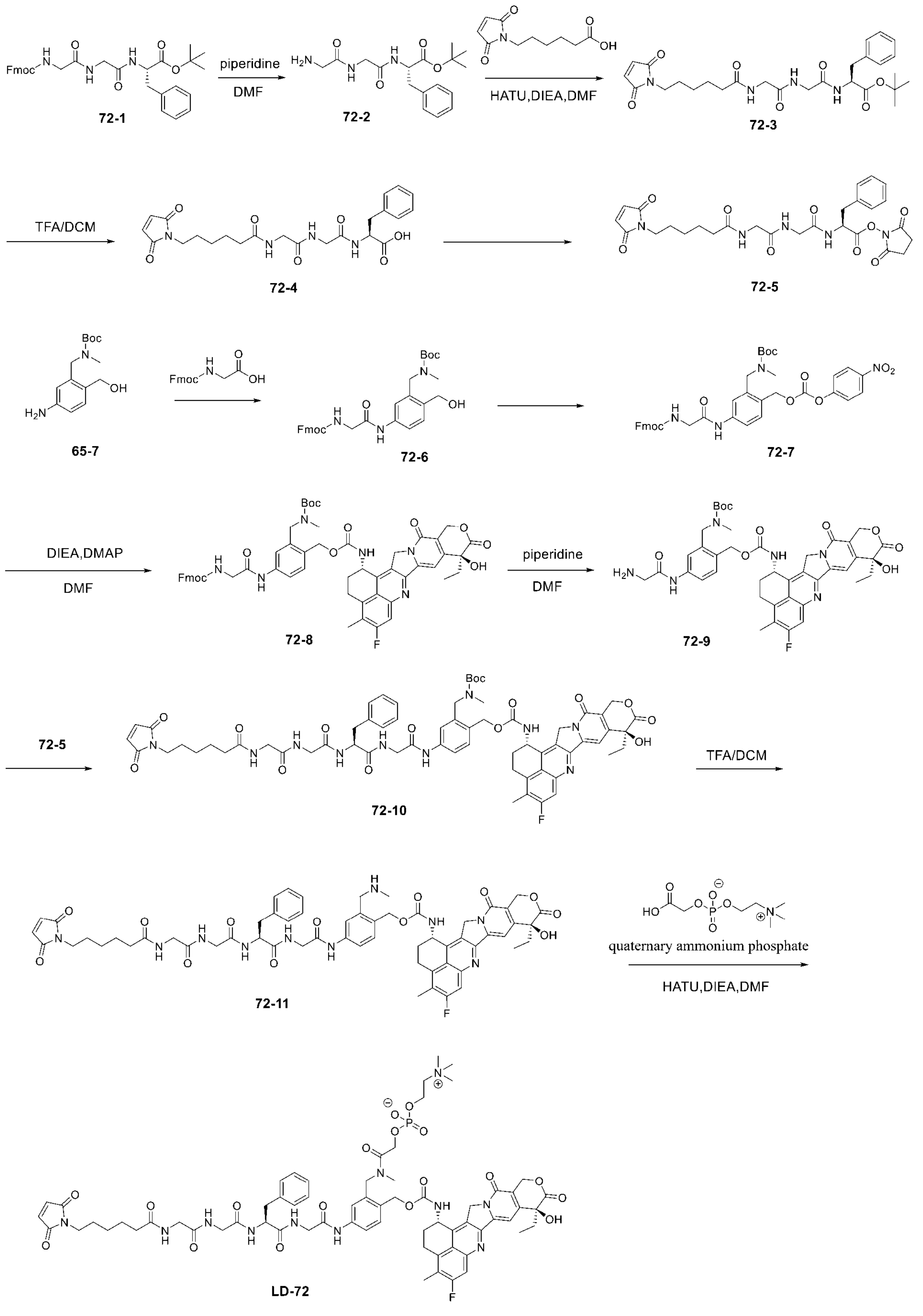


72-1 72-2 72-3

72-4 72-5

65-7 72-6 72-7

72-8 72-9

72-10

72-11

LD-72

Synthesis of 72-2

Compound 72-1 (600 mg, 1.076 mmoL) was dissolved in 4 mL of anhydrous DMF, adding 0.1mL of piperidine to stir for 1 hour at room temperature. After distillation under reduced pressure and beating with petroleum ether, 350mg of crude product was obtained. MS+Na(+)=357.9.

Synthesis of 72-3

Compound 72-2 (360 mg, 1.073 mmoL) and 6-maleimidohexanoic acid (340 mg, 1.610 mmoL) were dissolved in 10 mL of anhydrous DMF, adding DIEA (0.532 mL, 3.219 mmoL) and HATU (817 mg, 2.150 mmoL) to stir for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 400mg of final product. MS(+)=529.6.

Synthesis of 72-4

Compound 72-3 (90mg, 0.071 mmoL) was dissolved in 3 mL of anhydrous DCM, adding TFA (0.8mL) into the reaction solution to stir for 18 hours at room temperature. 80mg of crude product was obtained and directly used for the next reaction. MS(+)=473.5.

Synthesis of 72-5

Compound 72-4 (150 mg, 0.317 mmoL) was dissolved in 2mL of anhydrous DCM, adding HOSu (40.2 mg, 0.349 mmoL) and EDCI (67.2 mg, 0.351 mmoL) to stir for 2 hours at room temperature. After distillation under reduced pressure, 160mg of crude product was obtained and directly used for the next reaction. MS(+)=569.8.

Synthesis of 72-6

Compound 65-7 (700 mg, 2.628 mmoL) and Fmoc-glycine (1.56 g, 5.25mmoL) were dissolved in 20mL of anhydrous THF, adding EEDQ (780 mg, 3.158 mmoL) to stir for 18 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 5%) to obtain 1000mg of final product. MS+Na(+)=567.8.

Synthesis of 72-7

Compound 72-6 (290 mg, 0.531 mmoL) was dissolved in 6 mL of anhydrous THF, adding p-nitrophenyl chloroformate (129 mg, 0.640 mmol) and pyridine (63 mg, 0.797mmoL) successively to react for 3 hours at room temperature. After adding water into the reation solution and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 5%) to obtain 300mg of a final product. MS+Na(+)=732.7.

Synthesis of 72-8

Compound 72-7 (340 mg, 0.478 mmol) and exatecan mesylate (254 mg, 0.478mmoL) were dissolved in 8 mL of anhydrous DMF, adding DMAP (8mg, 0.025 mmoL) and DIEA (0.237 mL, 1.434 mmol) successively to react for 12 hours at room temperature. After adding water into the reaction solution and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 380mg of final product. MS(+)=1006.7.

Synthesis of 72-9

Compound 72-8 (300 mg, 0.298 mmol) was dissolved in 3 mL of anhydrous DMF, adding 0.06 mL of piperidine to stir for 1 hour at room temperature. After distillation under reduced pressure and beating with petroleum ether, 230mg of

crude product was obtained. MS(+)=785.8.

Synthesis of 72-10

Compound 72-9 (180 mg, 0.316 mmoL) and Compound 72-5 (207 mg, 0.264mmoL) were dissolved in 3 mL of anhydrous DMF, adding DIEA to stir for 2 hours at room temperature. After adding water into the reaction solution and extracting with ethyl acetate for three times, the organic phases were combined, washing with saturated saline solution, drying over anhydrous sodium sulfate, then filtering and concentrating to obtain a crude product. Purification was performed by using thin-layer silicone plate (MeOH/DCM, 0% to 10%) to obtain 100mg of final product. MS(+)=1240.8.

Synthesis of 72-11

Compound 71-10 (90mg, 0.071 mmoL) was dissolved in 3 mL of anhydrous DCM, adding TFA (0.3mL) into the reaction solution to stir for 1 hour at room temperature. 36mg of crude product was obtained and directly used for the next reaction. MS(+)=1138.6.

Synthesis of LD-72

Compound 71-11 (36mg, crude product) and quaternary ammonium phosphate (11.4 mg, 0.047 mmol, Cas#: 853798-56-8, provided by Shanghai Haoyuan Pharmaceutical Co., Ltd., for synthetic method, referring to WO2005114193) were dissolved in 3 mL of anhydrous DMF, adding DIEA (0.015 mL, 0.093 mmoL) and HATU (24 mg, 0.032 mmoL) to stir for 3 hours at room temperature. LCMS showed that the reaction was performed completely. Reversed phase preparation was performed (mobile phase, containing 10 mM $NH_4OAc$) to obtain 15mg of white solid product.

LCMS:m/z(ES+)(M+H)+=1361.5, Rt=1.114 min

1H NMR (400 MHz, DMSO) δ 10.01 (d, J = 12 Hz, 1H), 9.04 (d, J = 8.0 Hz, 1H), 8.40 (s, 2H), 8.27 - 8.07 (m, 3H), 7.79 - 7.60 (m, 2H), 7.48 - 7.11 (m, 9H), 6.99 (s, 2H), 6.52 (s, 1H), 5.57 - 5.00 (m, 8H), 4.94 - 4.37 (m, 5H), 4.25 - 4.09 (m, 2H), 3.99 - 3.48 (m, 10H), 3.08 (d, J = 13.6 Hz, 7H), 2.94 (d, J = 13.2 Hz, 9H), 2.40 - 2.33 (m, 3H), 2.19 - 2.07 (m, 4H), 1.91 - 1.81 (m, 2H), 1.52 - 1.41 (m, 4H), 1.33 - 1.00 (m, 3H), 0.88 (t, J = 7.2 Hz, 3H).

Example 11 (Preparation of LD-73)

The synthetic scheme of LD-73 is as follows:

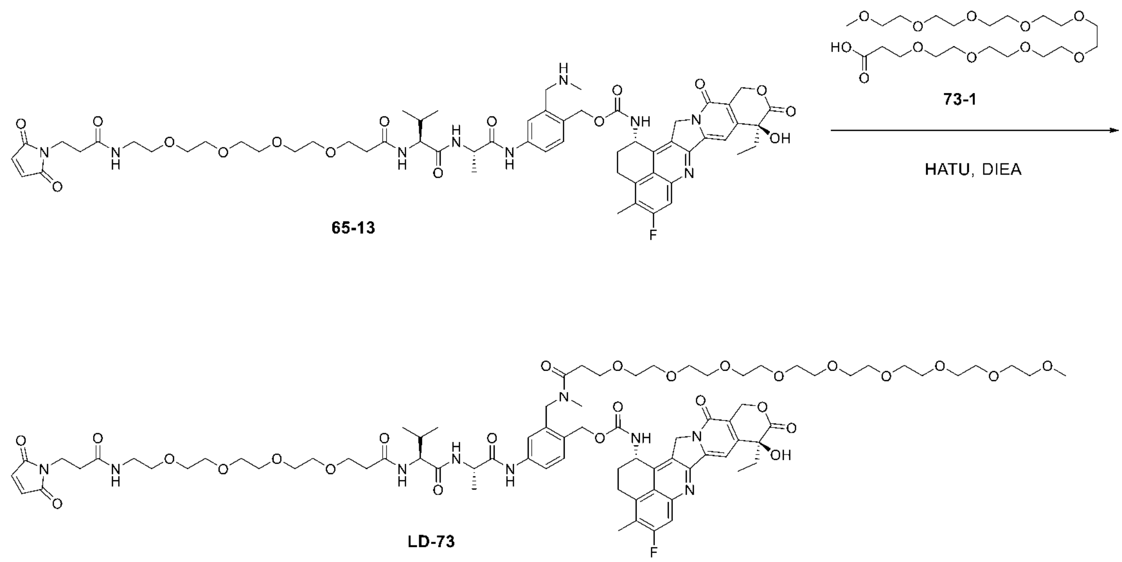


73-1

65-13

LD-73

Synthesis of LD-73

Compound 73-1 (25.19 mg, 0.055 mmoL) and HATU (20.98 mg, 0.055mmoL) were dissolved in 2mL of anhydrous DMF to stir for 5 minutes at room temperature, then adding Compound 65-13 (60 mg, 0.050 mmoL) and DIEA (0.008 mL, 0.075 mmoL) successively to react for 2 hours. LCMS detection showed that the reaction was performed

completely. Reversed phase preparation was directly performed (mobile phase, containing 0.1% formic acid) on the reaction system to obtain 19mg of white solid product.

LCMS: m/z (ES+) (M+H)+ = 1634.8, Rt = 1.392 min

1H NMR (400 MHz, DMSO) δ 9.96 (d, J = 40.0 Hz, 1H), 8.17 (d, J = 6.8 Hz, 1H), 8.00 (s, 1H), 7.78 (d, J = 10.8 Hz, 2H), 7.62 (s, 1H), 7.38 - 7.26 (m, 4H), 6.99 (s, 2H), 6.52 (s, 1H), 5.44 (s, 3H), 5.29 (s, 5H), 5.13-5.10 (m, 3H), 4.67 (s, 1H), 4.58 (s, 1H), 4.36 (d, J = 7.2 Hz, 1H), 4.22-4.19 (m, 1H), 3.66 - 3.55 (m, 10H), 3.52-3.40 (m, 50H), 3.23 (s, 6H), 3.18-3.10 (m, 5H), 1.24 (s, 3H), 0.89-0.81 (m, 9H).

**Example 12 (Preparation of LD-74)**

The synthetic scheme of LD-74 is as follows:

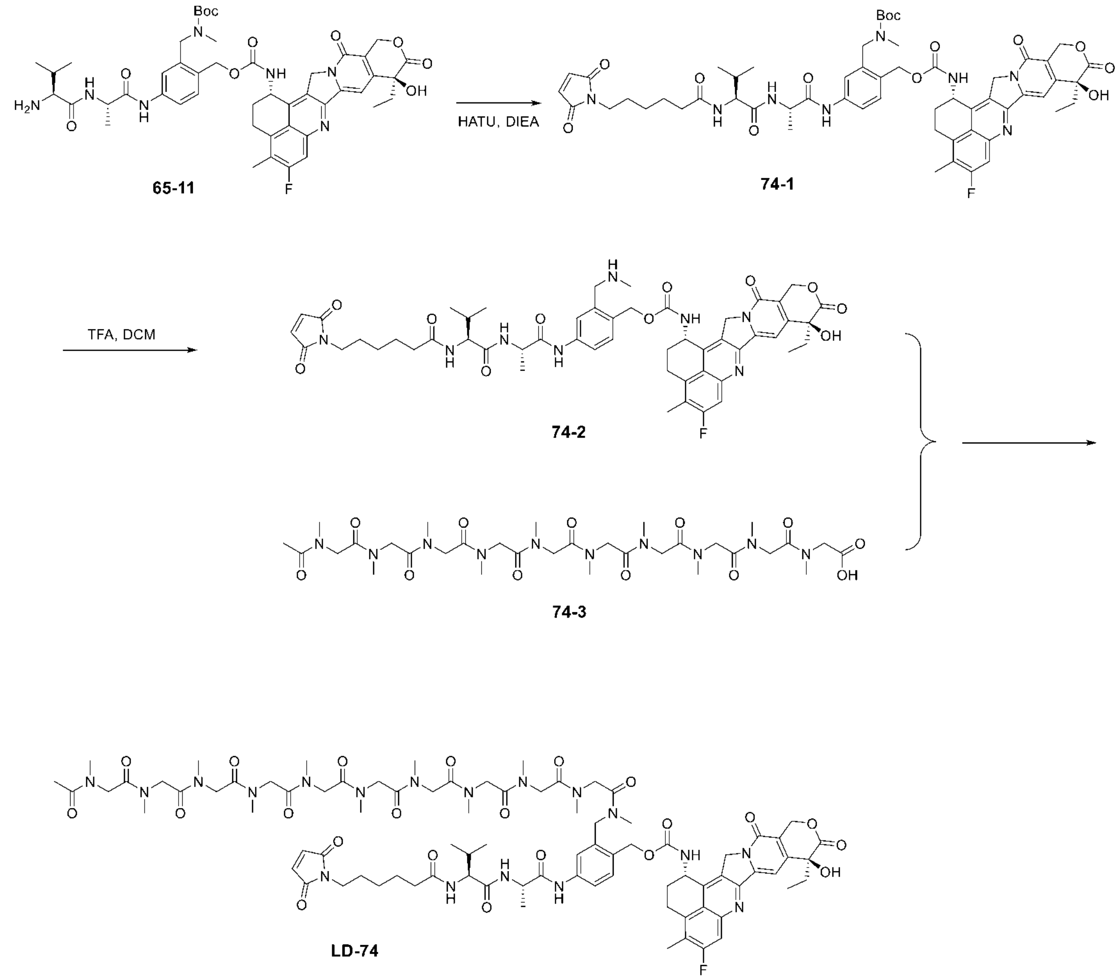


65-11, 74-1, 74-2, 74-3, LD-74

Synthesis of 74-1

6-maleimidohexanoic acid (18.59 mg, 0.088 mmoL) was dissolved in 1 mL of anhydrous DMF, adding HATU ((33.46 mg, 0.088 mmoL) to stir for 5 minutes at room temperature, adding Compound 65-11 (72.11 mg, 0.080 mmol) and DIEA (0.020 mL) successively to stir continuously for 2 hours at room temperature. LCMS showed that the reaction was performed completely. After adding water and extracting with ethyl acetate for three times, the organic phases were combined, washing with saturated saline solution, drying over anhydrous sodium sulfate, then filtering and concentrating to obtain a crude product. Purification was performed by using normal phase silica gel column (MeOH/DCM, 0% to 10%) to obtain 80mg of final product. MS (+)1091.6.

Synthesis of 74-2

Compound 74-1 (65 mg, 0.073 mmol) was dissolved in 3 mL of anhydrous DCM, adding 0.6mL of TFA to stir for 2

hours at room temperature. LCMS showed that the reaction was performed completely. After concentration under reduced pressure, 65mg of final product was obtained. MS (+)=991.4.

Synthesis of LD-74

Compound 74-3 (20.00 mg, 0.026 mmoL, purchased from Shanghai Haoyuan Pharmaceutical Co., Ltd., for synthetic method, referring to WO2022228493) was dissolved in 1 mL of anhydrous DMF, adding HATU (10.85 mg, 0.029 mmol) to stir for 5 minutes at room temperature, adding Compound 74-2 (25.71 mg, 0.026mmoL) and DIEA (0.008 mL, 0.046 mmol) successively to stir continuously for 2 hours at room temperature. LCMS showed that the reaction was performed completely. Reversed phase preparation was performed (mobile phase, containing 10 mM $NH_4OAc$) on the reaction solution to obtain 9.5mg of white solid.

LCMS: m/z (ES+) (M+H)+ = 1743.9, Rt = 1.325 min

1H NMR (400 MHz, DMSO) δ 7.79 (d, J = 10.9 Hz, 2H), 7.31 (s, 1H), 6.99 (s, 2H), 6.52 (s, 1H), 5.45 (s, 2H), 5.28 (s, 3H), 5.12 (s, 2H), 4.33-4.04 (m, 26H), 2.98 - 2.66 (m, 45H), 1.90 (m, 7H), 1.49-1.47 (m, 5H), 1.31 - 1.15 (m, 7H), 0.89-0.81 (m, 9H).

**Example 13 (Preparation of LD-88)**

The synthetic scheme of LD-88 is as follows:

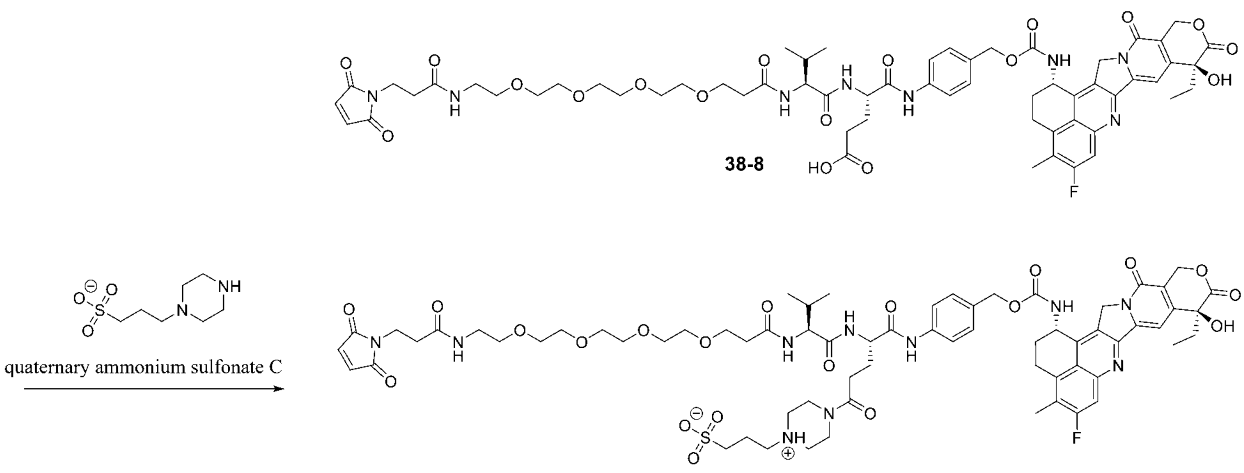


38-8

LD-88

Synthesis of LD-88

Compound 38-8 (50mg, 0.041mmol, the synthetic method refer to Example 3) and quaternary ammonium sulfonate C (16.34mg, 0.078mmol, CN115521998, Shanghai Haoyuan Pharmaceutical Co., Ltd. ) were dissolved in 2 mL of anhydrous DMF, adding DIEA(0.010 mL, 0.062 mmol) and HATU(17.27mg, 0.045 mmol) to stir for 2 hours at room temperature. LCMS showed that the reaction was performed completely. Reversed phase preparation was directly performed (trifluoroacetic acid) on the reaction solution to obtain LD-88 as light yellow solid (10.0 mg, 17.28% yield).

LCMS: m/z (ES+) (M+H)+ = 1402.6, Rt = 1.303 min

1H NMR (400 MHz, DMSO) δ 10.06 (s, 1H), 9.95 (s, 1H), 8.19 (d, J = 7.6 Hz, 1H), 8.10-8.00 (m, 2H), 7.91 (d, J = 8 Hz, 1H), 7.79 (d, J = 10.8 Hz, 1H), 7.61 (d, J = 8.4 Hz, 2H), 7.38 (d, J = 8.4 Hz, 2H), 7.32 (s, 1H), 7.00 (s, 2H), 6.53 (s, 1H), 5.45 (s, 2H), 5.29 (s, 3H), 5.09 (s, 2H), 4.45-4.41 (m, 2H), 4.20-3.99 (m, 2H), 3.62-3.57 (m, 4H), 3.51-3.47 (m, 14H), 3.19-3.13 (m, 5H), 2.67 (dt, J = 9.2, 4.4 Hz, 4H), 2.39 (s, 5H), 2.36-2.31 (m, 5H), 2.19 (s, 3H), 2.06-1.82 (m, 10H), 0.90-0.84 (m, 9H).

**Example 14 (Preparation of LD-89)**

The synthetic scheme of LD-89 is as follows:

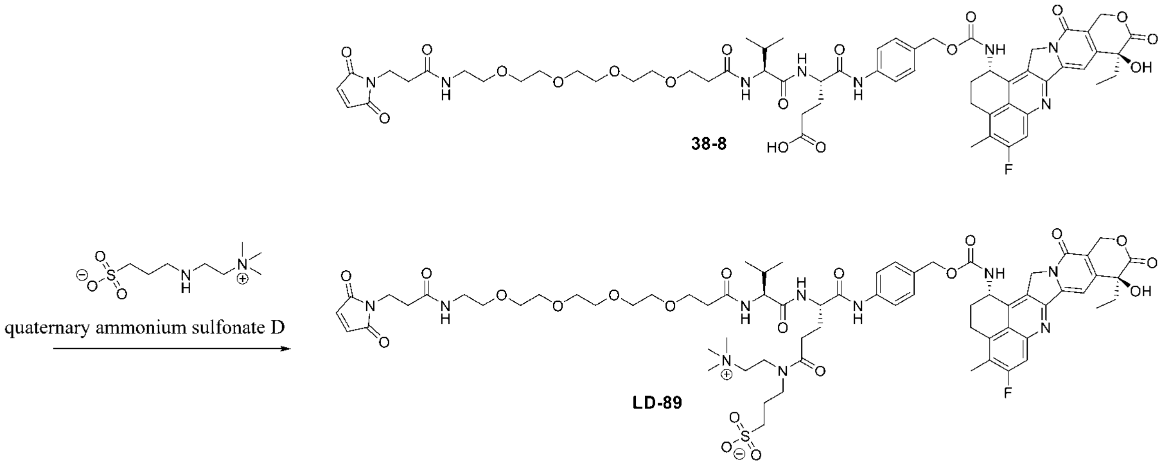


38-8

LD-89

Synthesis of LD-89

Compound 38-8 (50mg, 0.041mmol, the synthetic method refer to Example 3) and quaternary ammonium sulfonate D (17.58mg, 0.078mmol, Bioconjugate Chem. 2022, 33,718-725, Shanghai Haoyuan Pharmaceutical Co., Ltd.) were dissolved in 2 mL of anhydrous DMF, adding DIEA (0.010 mL, 0.062 mmol) and HATU (17.27mg, 0.045mmol) to stir for 2 hours at room temperature. LCMS showed that the reaction was performed completely. Reversed phase preparation (formic acid) was directly performed on the reaction solution to obtain 11.5 mg of LD-89 as white solid (5.4 mg, 10.06% yield).

LCMS: m/z (ES+) (M+H)+ = 1418.6, Rt = 1.288 min

1H NMR (400 MHz, DMSO) δ 9.86 (s, 1H), 8.11 (d, J = 7.2 Hz, 1H), 8.01 - 7.89 (m, 3H), 7.71 (d, J = 10.8 Hz, 1H), 7.55 (d, J = 8.4 Hz, 2H), 7.30 (d, J = 8.4 Hz, 2H), 7.24 (s, 1H), 6.93 (s, 2H), 6.45 (s, 1H), 5.38 (s, 2H), 5.22 (d, J = 4.0 Hz, 3H), 5.01 (s, 2H), 4.35 - 4.30 (m, 1H), 4.15-4.12 (m, 1H), 3.52 (s, 8H), 3.42 (d, J = 3.6 Hz, 17H), 3.25 (s, 5H), 3.09 - 2.99 (m, 14H), 2.31 (s, 4H), 2.26 (d, J = 7.2 Hz, 3H), 2.16-2.07 (m, 2H), 1.88-1.82 (m, 8H), 0.82-0.76 (m, 9H).

**Example 15 (Preparation of LD-94)**

The synthetic scheme of LD-94 is as follows:

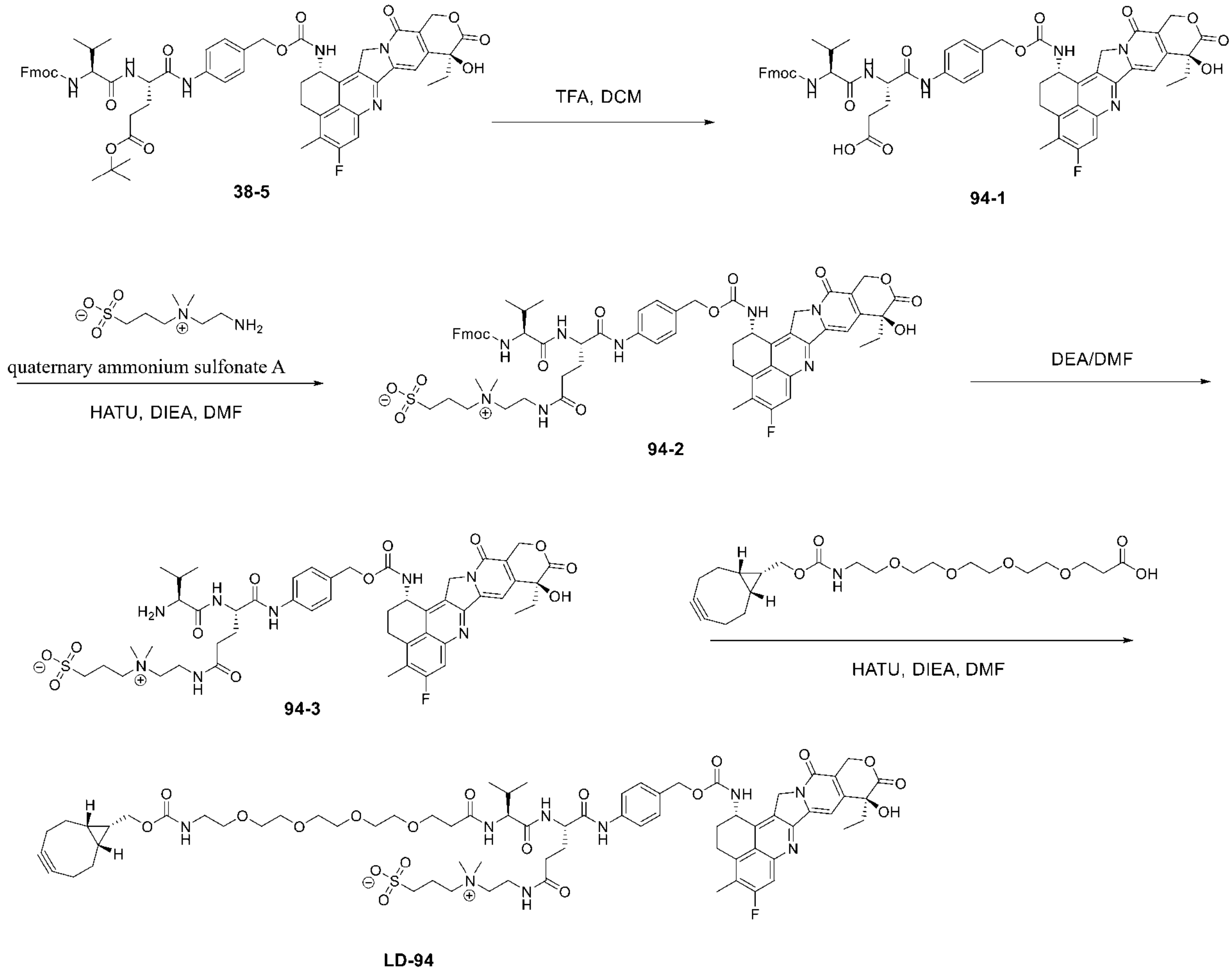


38-5 94-1 94-2 94-3 LD-94

Synthesis of Compound 94-1

Compound 38-5 (450 mg, 0.412 mmol, the synthetic method refer to Example 3) was added in dichloromethane (5 mL), adding trifluoroacetic acid (1 mL) dropwise to this mixture system to stir for 2 hours at room temperature. The mixture system was concentrated to dryness to obtain a crude product, which was used for the the next step. *m*/*z* (ES+) (M+1)=1035.4

Synthesis of Compound 94-2

Compound 94-1 (400 mg, 0.386 mmol), quaternary ammonium sulfonate A (121.90 mg, 0.580 mmol) and DIEA (74.91 mg, 0.580 mmol) were dissolved in DMF (4 mL) to stir this mixture system for 5 minutes at room temperature, adding HATU (161.63 mg, 0.425 mmol) into the reaction system for 4 hours at room temperature. Purification was performed by reverse column (water: acetonitrile, 0% to 70%) to obtain 170 mg of final product. *m*/*z* (ES+) (M+1) =1227.6.

Synthesis of Compound 94-3

Compound 94-2 (70 mg, 0.057 mmol) was dissolved in DMF (1 mL), adding diethylamine (14.30 mg, 0.196 mmol) dropwise into this mixture system to stir for 2 hours at room temperature. The mixture system was concentrated to dryness, beating with petroleum ether and filtering, then the filter cake was dried and directly used for the the next step. *m*/*z* (ES+) (M+1)=1005.6.

Synthesis of LD-94

Compound 94 (55 mg, 0.055 mmol), BCN-PEG4-acid (Cas#: 1881221-47-1, 24.16 mg, 0.055 mmol) and DIEA (0.011 mL, 0.067 mmol) were dissolved in DMF (2 mL) to stir for 5 minutes at room temperature, then adding HATU (18.73 mg, 0.049 mmol) to stir the mixture system for 2 hours at room temperature. A product LD-94 (6 mg, yield: 7.68%) was

obtained by Prep-HPLC ($NH_4HCO_3$ system).

LCMS: *m/z* (ES+) $(M+H)^+$= 1427.6, Rt = 1.721 min.

$^1H$ NMR (400 MHz, DMSO) δ 9.90 (s, 1H), 8.19 (s, 2H), 8.09 - 8.05 (m, 1H), 8.00 - 7.96 (m, 1H), 7.79 (d, *J* = 11.2 Hz, 1H), 7.63 (d, *J* = 7.6 Hz, 2H), 7.37 (d, *J* = 8.2 Hz, 2H), 7.31 (s, 1H), 6.53 (s, 1H), 5.45 (s, 1H), 5.29 (s, 3H), 5.08 (s, 2H), 4.19 - 4.15 (m, 1H), 4.02 (d, *J* = 8.6 Hz, 2H), 3.62 - 3.58 (m, 2H), 3.50 - 3.47 (m, 16H), 3.10 (s, 2H), 3.04 - 3.02 (m, 7H), 2.69 - 2.66 (m, 4H), 2.40 - 2.36 (m, 5H), 2.34 - 2.32 (m, 4H), 2.22 - 2.15(m, 9H), 1.99 (s, 4H), 1.92 - 1.86 (m, 3H), 1.55 - 1.46 (m, 2H), 1.23 (s, 2H), 0.90 - 0.83 (m, 14H).

**Example 16 (Preparation of LD-98)**

The synthetic scheme of LD-98 is as follows:

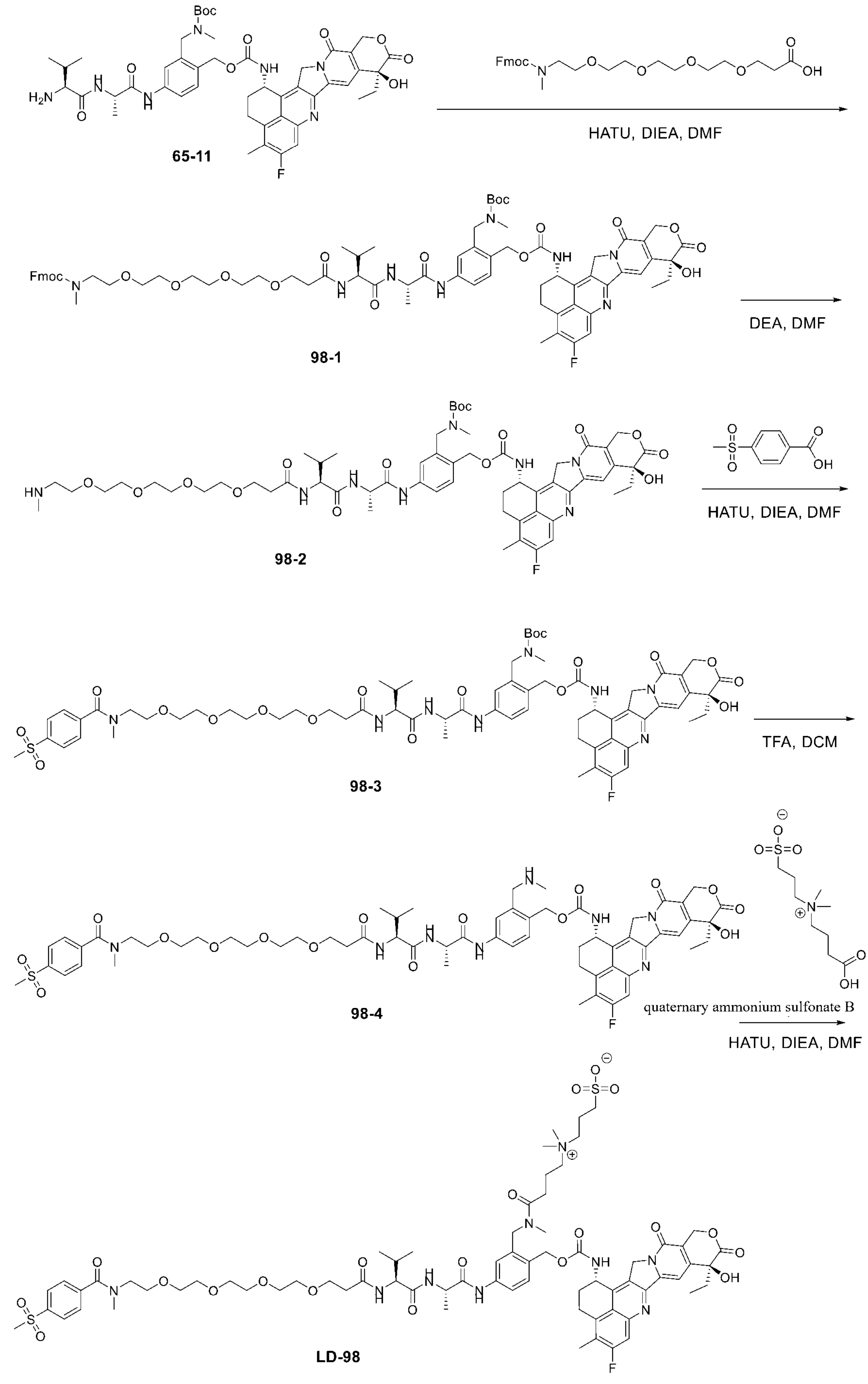


65-11

98-1

98-2

98-3

98-4

LD-98

Synthesis of Compound 98-1

Compound 65-11 (120 mg, 0.134 mmol, the synthetic method refer to Example 5) and Fmoc-NMe-PEG4-C2-Acid (Cas#: 2170240-98-7, 67.0 mg, 0.134 mmol) were dissolved in DMF (2 mL), adding HATU (55.9 mg, 0.147 mmol, 1.1 eq) and N,N-diisopropylethylamine (26 mg, 0.200 mmol, 1.5 eq) successively to stir for 2 hours at room temperature. The reaction solution was concentrated under reduced pressure, and the residue was purified by silica gel column chromatography (DCM: MeOH, 0-10% of MeOH) to obtain 100 mg of a final product. m/z (ES+) (M+1)=1381.6.

Synthesis of Compound 98-2

Compound 98-1 (100 mg, 0.072 mmol) was dissolved in DMF (2 mL), adding diethylamine (15.88 mg, 0.217 mmol) to stir for 2 hours at room temperature. The mixture was concentrated under reduced pressure to obtain 100 mg of a crude product. m/z (ES+) (M+1 ) =1159.6.

Synthesis of Compound 98-3

Compound 98-2 (80 mg, 0.069 mmol) and 4-methylsulfonyl benzoic acid (13.95 mg, 0.069 mmol) were dissolved in DMF (2 mL), adding HATU (28.86 mg, 0.076 mmol) and DIPEA (13.38 mg, 0.104 mmol) successively to stir for 2 hours at room temperature. The mixture was concentrated under reduced pressure, and the residue was purified by reverse phase chromatography column (C18, 20-60% acetonitrile-$H_2O$ solution) to obtain 26 mg of yellow solid. *m*/*z* (ES+) (M+1) =1343.49.

Synthesis of Compound 98-4

Compound 98-3 (25 mg, 0.019 mmol) was dissolved in DCM (2 mL), adding TFA (0.4 mL) to stir the reaction solution for 2 hours at room temperature. The mixture was concentrated under reduced pressure to obtain a crude product Compound 18 (23 mg), which was directly used for the next reaction. m/z (ES+) (M+1)=1243.4.

Synthesis of LD-98

Compound 98-4 (23 mg, 0.018 mmol) and quaternary ammonium sulfonate B (7.03 mg, 0.028 mmol) were dissolved in DMF (1 mL), adding successively HATU (7.74 mg, 0.020 mmol) and DIPEA (3.59 mg, 0.028 mmol) to stir the reaction solution for 2 hours at room temperature. LD-98 (6 mg, yield: 22.2%) was obtained from this reaction system by pre-HPLC (TFA).

LCMS: *m*/*z* (ES+) $(M+H)^+$= 1479.6, Rt = 1.340 min.

$^1$H NMR (400 MHz, DMSO) δ 9.97 (d, *J* = 40.2 Hz, 1H), 9.14 (d, *J* = 12.0 Hz, 2H), 8.19 (t, *J* = 7.4 Hz, 1H), 8.06 (d, *J* = 9.4 Hz, 1H), 7.85 (s, 1H), 7.78 (d, *J* = 11.2 Hz, 1H), 7.64 (dd, *J* = 49.2, 8.4 Hz, 1H), 7.33 (d, *J*= 12.4 Hz, 2H), 6.52 (s, 1H), 5.44 (d, *J*= 4.0 Hz, 2H), 5.30 (s, 3H), 5.12 (d, *J* = 10.8 Hz, 2H), 4.64 (d, *J* = 26.4 Hz, 2H), 4.36 (dd, *J* = 13.6, 6.8 Hz, 1H), 4.23 - 4.14 (m, 1H), 3.65 (s, 1H), 3.59 - 3.54 (m, 3H), 3.51 - 3.46 (m, 15H), 3.43 (s, 4H), 3.03 (d, *J* = 9.2 Hz, 7H), 2.98 (s, 3H), 2.92 (s, 2H), 2.86 (s, 1H), 2.68 - 2.67 (m, 1H), 2.38 (s, 5H), 2.33 (dd, *J* = 3.6, 1.8 Hz, 2H), 2.20 (s, 2H), 1.98 - 1.83 (m, 8H), 1.31 - 1.23 (m, 4H), 0.89 - 0.81 (m, 10H).

**Example 17** (Preparation of LD-99)

The synthetic scheme of LD-99 is as follows:

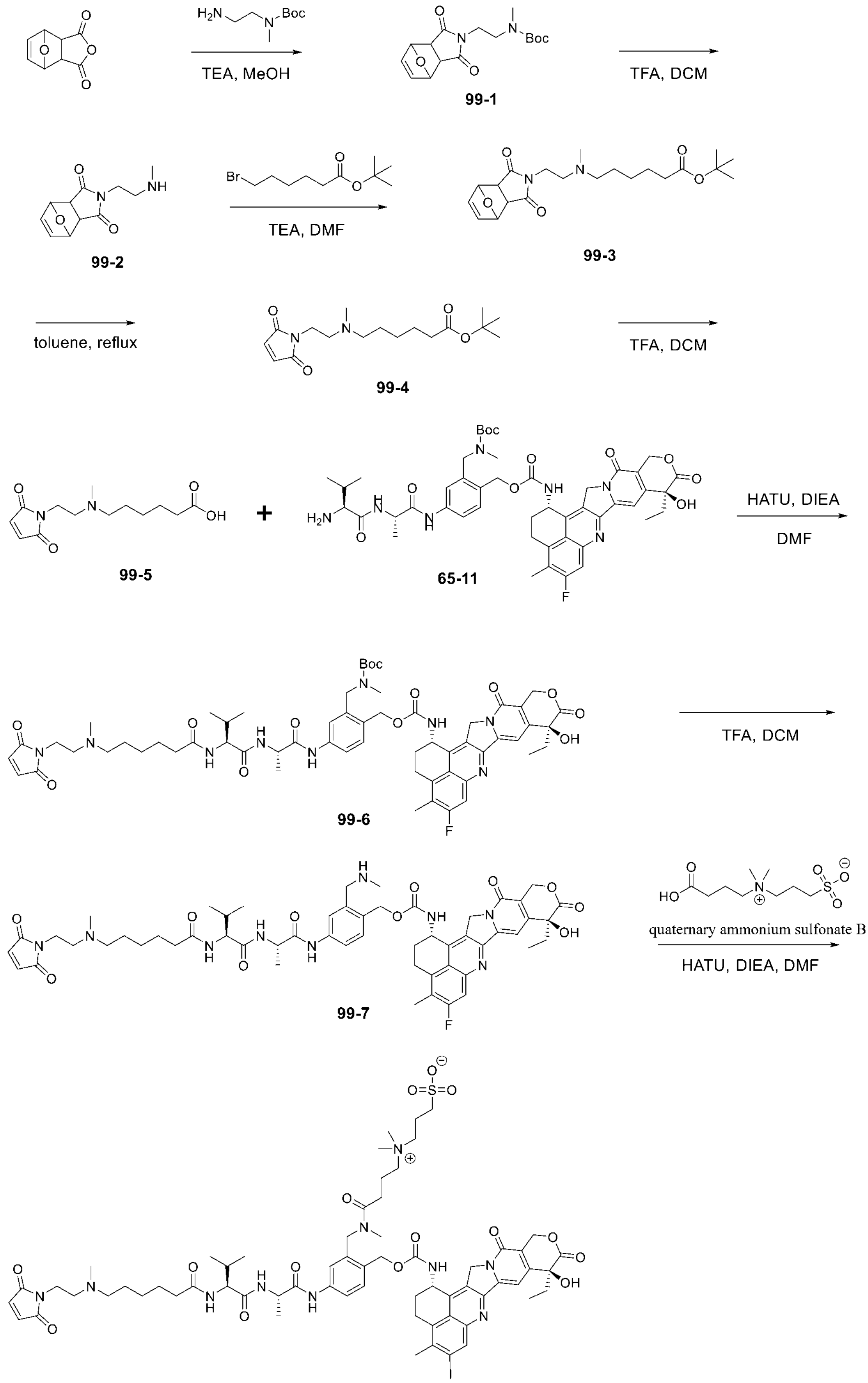


99-1

99-2

99-3

99-4

99-5

65-11

99-6

99-7

LD-99

Synthesis of Compound 99-1

Exo-3,6-epoxy-1,2,3,6-tetrahydrophthalic anhydride (CAS#: 6118-51-0, 2g, 12.04 mmol, 1.0 eq) and N-Boc-N-methylethylenediamine (CAS#: 121492-06-6, 2.52 g, 14.46 mmol, 1.2 eq) were dissolved in methanol (60 mL), adding triethylamine (2.43 g, 24.1 mmol, 2.0 eq) at room temperature, then heating the reaction solution up to 65°C to react for 48 hours. After concentration of the reaction solution, it was diluted with ethyl acetate (60 mL), drying with saturated saline solution and anhydrous sodium sulfate, then filtering and concentrating. 3g of white solid was obtained by column purification (petroleum ether: ethyl acetate =1:1). m/z(ES+)$(M+H)^+$=323.3.

Synthesis of Compound 99-2

Compound 99-1 (2.3 g, 7.135 mmol) was dissolved in dichloromethane (20 mL), adding trifluoroacetic acid (4 mL) under ice bath, and warming up the reaction solution to room temperature to react for 1 hour. Aftre concentration of the reaction solution, 1.5 g of crude product was obtained. m/z(ES+)$(M+H)^+$=223.2.

Synthesis of Compound 99-3

Compound 99-2 (1.58 g, 7.11 mmol, 1.0 eq) and tert-butyl 6-bromohexanoate (2.14 g, 8.52 mmol, 1.2 eq) were dissolved in DMF (10 mL) to react for 72 hours at room temperature. After concentration of the reaction solution, 2.5g of target product was obtained by column purification (petroleum ether: ethyl acetate =3:1). m/z(ES+)$(M+Na)^+$=393.2.

Synthesis of Compound 99-4

Compound 99-3 (1.5 g, 3.822 mmol) was dissolved in toluene (15 mL), heating up to 110°C to reat for 18 hours. After concentration of the reaction solution, 1 g of target product as a yellow oily substance was obtained. m/z (ES+) $(M+H)^+$ = 325.2.

Synthesis of Compound 99-5

Compound 99-4 (100 mg, 0.308 mmol) was dissolved in dichloromethane (2 mL), adding trifluoroacetic acid (0.7 mL) to stir for 1 hour at room temperature. After concentration of the reaction solution, 82 mg of crude product as a yellow oily substance was obtained.
m/z(ES+)$(M+H)^+$=269.2.

Synthesis of Compound 99-6

Compound 65-11 (88 mg, 0.098 mmol, 1.0 eq) and Compound 99-5 (78.8 mg, 0.294 mmol, 3.0 eq) were dissolved in DMF (3 mL), adding HATU (112 mg, 0.294 mmol, 3.0 eq) and diisopropylethylamine (0.081 mL, 0.489 mmol, 5.0 eq) successively to react for 2 hours at room temperature. After filtration of the reaction solution, 80 mg of yellow solid was obtained by reverse column purification. m/z(ES+)$(M+H)^+$=1147.7.

Synthesis of Compound 99-7

Compound 99-6 (80 mg, 0.070 mmol) was dissolved in dichloromethane (3 mL), adding trifluoroacetic acid (0.3 mL) dropwise to stir for 1 hour at room temperature. After concentration of the reaction solution, 73 mg of crude product as a yellow oily substance was obtained. m/z(ES+)$(M+H)^+$=1047.6.

Synthesis of LD-99

Compound 99-7 (73 mg, 0.070 mmol, 1.0 eq) and quaternary ammonium sulfonate B (35 mg, 0.14 mmol, 2.0 eq) were dissolved in DMF (2 mL), adding HATU (53 mg, 0.14 mmol, 2.0 eq) and N,N-diisopropylethylamine (0.058 mL, 0.35 mmol, 5.0 eq) successively to stir the reaction solution for 1 hour at room temperature. After fitering the reaction solution, 9.2 mg of white solid was obtained by purification.

LCMS: m/z(ES+)$(M+H)^+$=1284.6, Rt=1.217 min
$^1$H NMR (400 MHz, DMSO) δ 10.01 (d, *J* = 38.5 Hz, 1H), 9.24 (s, 1H), 8.26 - 8.16 (m, 1H), 8.13 - 8.01 (m, 1H), 7.80 (t, *J* = 9.2 Hz, 2H), 7.64 (dd, *J* = 47.3, 8.1 Hz, 1H), 7.41 - 7.31 (m, 3H), 7.17 (s, 1H), 7.10 (d, *J* = 4.1 Hz, 2H), 6.54 (s, 1H), 5.44 (d, *J* = 4.0 Hz, 2H), 5.29 (s, 3H), 5.13 (d, *J* = 8.2 Hz, 2H), 4.64 (d, *J* = 27.5 Hz, 2H), 4.36 (d, *J* = 7.5 Hz, 1H), 4.22 - 4.16 (m, 1H), 3.77 (s, 2H), 3.22 (s, 2H), 3.05 (s, 5H), 2.99 (d, *J* = 3.8 Hz, 4H), 2.94 (s, 2H), 2.87 (s, 2H), 2.81 (s, 4H), 2.36 (d, *J = 18.7* Hz, 5H), 2.19

(dd, $J$ = 13.7, 7.2 Hz, 5H), 2.06 - 1.84 (m, 9H), 1.53 (s, 5H), 1.34 - 1.21 (m, 7H), 0.89 - 0.83 (m, 9H).

**Experimental Example 18 (Preparation of Compound of Formula (2))**

Conjugation Method 1 (complete reduction, the ratio of drug to antibody is 8)

The antibody was transferred to a pH 7.4 phosphate buffer by using an ultrafiltration centrifuge tube, adding TCEP with 10-15 times the molar number of the antibody to react at 37°C for 2 hours to open the disulfide bonds between the chains of the antibody. After the reduction was completed, the antibody was transferred to a pH 7.4 phosphate buffer by using an ultrafiltration centrifuge tube to remove excess TCEP, adding the linker-drug (compound of Formula (1), Compound MC-GGFG-Exatecan, Compound MC-GGFG-Dxd, and Compound ELC122 prepared in Examples 1-5, and 7-17) with 12-20 times the molar number of the antibody respectively into the antibody to react for 1 hour at room temperature. After the reaction was completed, cysteine with 2 times the molar number of the linker-drug was added to quench the reaction, the reaction solution was transferred to a pH 5.5 acetic acid buffer by using ultrafiltration centrifuge tube, then filtering the solution by 0.22 micron sterilization filter to obtain the antibody-drug conjugate sample for later use.

Conjugation Method 2 (partial reduction, the ratio of drug to antibody is 4)

The antibody was transferred to a pH 7.4 phosphate buffer by using an ultrafiltration centrifuge tube, adding TCEP with 2.2-2.3 times the molar number of the antibody to react at 37°C for 2 hours to open the disulfide bonds between the chains of the antibody. After the reduction was completed, the linker-drug (compound of Formula (1) and Compound MC-VC-PAB-MMAE prepared in Example 6) with 6-8 times the molar number of the antibody was added into the antibody to react for 1 hour at room temperature. After the reaction was completed, cysteine with 2 times the molar number of the linker-drug was added to quench the reaction, and the reaction solution was transferred to a pH 5.5 acetic acid buffer by using ultrafiltration centrifuge tube, then filtering the solution by 0.22 micron sterilization filter to obtain the antibody-drug conjugate sample for later use.

**The Sequences of the Conjugated Antibodies:**

Heavy chain amino acid sequence of anti-Claudin18.2 humanized monoclonal antibody 9D1, SEQ ID NO: 1 is as follows:

QIQLVQSGAEVKKPGASVKISCKASGYSFTDYHMNWVRQAPGKGLEWIGNIDPYYGSPTYNHKFKG
RVTLTVDTSTSTAYMELSSLRSEDTAVYYCANYGRGNSFPYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSKLTVDKSRW
QQGNVFSCSVMHEALHNHYTQKSLSLSPG

Light chain amino acid sequence of anti-Claudin18.2 humanized monoclonal antibody 9D1, SEQ ID NO:2 is as follows:

DIVMTQSPSSLAVSLGERATINCKSSQSLLNSGNQRNYLTWYQQKPGQPPKLLIYWASTRESGVPDRF
SGSGSGTDFTLTISSLQAEDVAVYYCQNDYSFPFTFGQGTKLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQGLSSP
VTKSFNRGEC

Heavy chain amino acid sequence of anti-human folate receptor (FRa) humanized monoclonal antibody (Farletuzumab antibody), SEQ ID NO: 3 is as follows:

EVQLVESGGGVVQPGRSLRLSCSASGFTFSGYGLSWVRQAPGKGLEWVAMISSGGSYTYYADSVKG RFAISRDNAKNTLFLQMDSLRPEDTGVYFCARHGDDPAWFAYWGQGTPVTVSSASTKGPSVFPLAPSSKS TSGGTAALGCLVKDYFPEPVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSDGSFFLYSKLTVDKS RWQQGNVFSCSVMHEALHNHYTQKSLSLSPGK

Light chain amino acid sequence of anti-human folate receptor (FRa) humanized monoclonal antibody (Farletuzumab antibody), SEQ ID NO: 4 is as follows:

DIQLTQSPSSLSASVGDRVTITCSVSSSISSNNLHWYQQKPGKAPKPWIYGTSNLASGVPSRFSGSGSG

TDYTFTISSLQPEDIATYYCQQWSSYPYMYTFGQGTKVEIKRTVAAPSVFIFPPSDEQLKSGTASVVCLLNN FYPREAKVQWKVDNALQSGNSQESVTEQDSKDSTYSLSSTLTLSKADYEKHKVYACEVTHQGLSSPVTK SFNRGEC

Chromatographic Analysis of the Antibody-drug Conjugates

Size exclusion chromatography (SEC) analysis column is Tskgel G3000SWXL (TOSOH, 0008541); mobile phase is 0.1 M PB, pH7.0, 0.1M NaCl; isocratic elution, flow rate: 1 mL/min; column temperature: 25°C. Detection wavelength is 280 nm and 366 nm, respectively. Hydrophobic interaction chromatography (HIC) analysis column is Butyl-NPR 4.6×100 mm (TOSOH, 0042168); mobile phase A is 50 mM PB, pH7.0, 1.5 M $(NH_4)_2SO_4$; mobile phase B is 50 mM PB, pH7.0, 20% acetonitrile. Separation gradient: changing from 0%B to 100%B in 2-20 min, column temperature: 30°C, flow rate: 0.6 mL/min. Detection wavelength is 280 nm and 366 nm, respectively. The results are shown in Table 1 and Fig. 1-Fig. 6 (Fig. 1 comprises Fig. 1A and Fig. 1B; Fig. 2 comprises Fig. 2A and Fig. 2B; Fig. 3 comprises Fig. 3A, Fig. 3B, Fig. 3C, Fig. 3D, Fig. 3E, Fig. 3F, Fig. 3G and Fig. 3H; Fig. 4 comprises Fig. 4A and Fig. 4B; Fig. 5 comprises Fig. 5A and Fig. 5B; and Fig. 6 comprises Fig. 6A, Fig. 6B, Fig. 6C, Fig. 6D, Fig. 6E, Fig. 6F, Fig. 6G and Fig. 6H).

Table 1: results of the size exclusion chromatography (SEC) and hydrophobic interaction chromatography (HIC) in each of the Experimental Examples and Comparative Examples

| Experimental Examples | Compounds of Fomula 1 | Antibodies | Conjugation method | DAR | Conjugated drugs | SEC% | HIC retention time (min) |
|---|---|---|---|---|---|---|---|
| 1 | LD-12 | 9D1 | 1 | 8 | Exatecan | 98.40 | 15.489 |
| 2 | LD-27 | 9D1 | 1 | 8 | Exatecan | 89.22 | 15.107 |
| 3a | LD-31-P1 | 9D1 | 1 | 8 | Exatecan | 99.07 | 13.290 |
| 3b | LD-31-P2 | 9D1 | 1 | 8 | Exatecan | 99.13 | 13.404 |
| 4 | LD-37 | 9D1 | 1 | 8 | Exatecan | 97.50 | 14.069 |
| 5a | LD-38 | 9D1 | 1 | 8 | Exatecan | 98.02 | 13.574 |
| 5b | LD-38 | Farletuzumab | 1 | 8 | Exatecan | 96.84 | 13.359 |
| 5c | LD-38 | IgG 1-ISO | 1 | 8 | Exatecan | 98.24 | 13.457 |
| 6 | LD-57 | 9D1 | 2 | 4 | MMAE | 99.34 | 16.066 |
| 7 | LD-65 | 9D1 | 1 | 8 | Exatecan | 99.28 | 13.455 |
| 8 | LD-66 | 9D1 | 1 | 8 | Exatecan | 99.43 | 13.167 |
| 9 | LD-71 | 9D1 | 1 | 8 | SN38 | 99.39 | 12.928 |
| 10 | LD-72 | 9D1 | 1 | 8 | Exatecan | 98.90 | 13.126 |
| 11 | LD-73 | 9D1 | 1 | 8 | Exatecan | 99.53 | 16.002 |
| 12 | LD-74 | 9D1 | 1 | 8 | Exatecan | 99.53 | 13.583 |

(continued)

| Experimental Examples | Compounds of Fomula 1 | Antibodies | Conjugation method | DAR | Conjugated drugs | SEC% | HIC retention time (min) |
|---|---|---|---|---|---|---|---|
| 13 | LD-88 | 9D1 | 1 | 1 | Exatecan | 100.00 | 14.166 |
| 14 | LD-89 | 9D1 | 1 | 1 | Exatecan | 98.00 | 13.392 |
| 15 | LD-98 | 9D1 | 1 | 8 | Exatecan | 98.49 | 13.228 |
| 16 | LD-99 | 9D1 | 1 | 8 | Exatecan | 98.79 | 13.121 |
| Comparative Example 1 | MC-GGFG-Exatec an | 9D1 | 1 | 8 | Exatecan | 98.72 | 15.435 |
| Comparative Example 2 | MC-VC-PAB-MM AE | 9D1 | 2 | 4 | MMAE | 99.55 | 16.992 |
| Comparative Example 3 | ELC122 | 9D1 | 1 | 8 | SN38 | 100 | 16.742 |
| Comparative Example 4 | MC-GGFG-Dxd (Deruxtecan) | 9D1 | 1 | 8 | Dxd | 99.23 | 15.120 |

Conclusion: it can be seen from Table 1 and Fig. 1-Fig. 6, anti-Claudin18.2 humanized monoclonal antibody 9D1 (WO2021160155) was used as a pattern antibody to conjugate with the designed linker-drug compound and control linker-drug compound for preparing an antibody-drug conjugate. Linker-drug compounds MC-GGFG-Exatecan (Shanhai Haoyuan Biomedica, Cas#HY-114233), MC-VC-PAB-MMAE (Shanhai Haoyuan Biomedica, Cas#646502-53-6) and MC-GGFG-Dxd (Deruxtecan) (Shanhai Haoyuan Biomedica, Cas#HY-13631E) are commercial available; LD-74 was synthesized by the synthetic method of WO2022228493; ELC122 was synthesized by the synthetic method of WO2021225892.

The size exclusion chromatography (SEC) analysis results of the resulting 9D1 antibody-drug conjugate showed that the SEC purity of the antibody-drug conjugate is greater than 97%, no matter whether the conjugated drug is Exatecan, SN38, or MMAE. The purity of the antibody-drug conjugate of the control linker LD-27 is only 89.22% (Table 1).

The hydrophobic interaction chromatography (HIC) analysis results of 9D1 antibody-drug conjugate show that the inner salt linker of the present invention designed for toxin MMAE, SN38 and Exatecan has a significantly shorter HIC retention time than the control linker (excluding LD-74)-9D1 antibody-drug conjugate. The retention time of the main peak of the antibody-drug conjugate of inner salt linker-MMAE (it refers to an antibody-drug conjugate of LD-57) is about 1min shorter than the control linker-MMAE(MC-VC-PAB-MMAE) antibody-drug conjugate (it refers to the antibody-drug conjugate of Comparative Example 2) (a ratio of antibody to drug is 4) (16.07 vs 16.99), and for the ingredient with a higher ratio of antibody to drug, the retention time is shortened more significantly (Table 1 , Fig. 4). The retention time of the inner salt linker-SN38 antibody-drug conjugate (an antibody-drug conjugate of LD-71) is about 4min shorter than retention time of the control linker-SN38 (ELC122, WO2021225892) antibody-drug conjugate (it refers to the antibody-drug conjugate of Comparative Example 3) (12.93 vs 16.74, Table 1, Fig. 5). The HIC retention time of the inner salt linker-Exatecan antibody-drug conjugates of the present application (the antibody-drug conjugates of LD-31, LD-37, LD-38, LD-65, LD-66, LD-72, LD-88, LD-89, LD-98 and LD-99) is 13.12min to 14.17min, significantly shorter than the retention time of the control linker-Exatecan (LD-12, LD-27, MC-GGFG-Exatecan) and MC-GGFG-Dxd antibody-drug conjugate which do not have a hydrophilic side chain, and also significantly shorter than the retention time (16.00 min) of LD-73 antibody-drug conjugate having a polyethylene glycol (PEG 9) hydrophilic side chain, and comparable to the retention time (13.58 min) of LD-74 antibody-drug conjugate having a polysarcosine hydrophilic side chain (Table 1 , Fig. 6). An interesting finding is that, with respect to inner salt linker-Exatecan, for those inner salts having the same structure, the shorter of the distance from the inner salt on the linker to Exatecan toxin, the shorter is the HIC retention time of the corresponding antibody-drug conjugate. For example, with respect to sulfonate inner salt linker-Exatecan, for LD-37 (sulfonate inner salt located at Formula (1-1) Z position, far from Exatecan), the HIC retention time of the corresponding antibody-drug conjugate is 14.07 min; for LD-38 (sulfonate inner salt located at Formula (1-1-1) Z position), LD-65 (sulfonate inner salt located at Formula Z' position) and LD-31(P2)(sulfonate inner salt located at Formula Z" position), the retention times of the corresponding antibody-drug conjugates 13.57, 13.46 and 13.40 min, respectively, which is significantly shorter than the retention time (14.07 min) of the antibody-drug conjugate corresponding to LD-37 (Table 1 , Fig. 6).

Hydrophobic interaction chromatography showed the hydrophobicity characteristics of the antibody-drug

conjugates, and such hydrophobicity is caused by the hydrophobicity of the conjugated drug compound. If an antibody-drug conjugate has strong hydrophobicity, its retention time on the hydrophobic interaction chromatography will be longer, then the antibody-drug conjugate has a poor water solubility and stability, and it may also be easily cleared in the body. The super strong water solubility of the inner salt linker may greatly mask the hydrophobicity characteristics of the drug compound, improve the water solubility and stability, and shorten the retention time on the hydrophobic interaction chromatography.

Additionally, conjugates of the inner salt linker-Exatecan (LD-38) with anti-human folate receptor (FRa) humanized monoclonal antibody Farletuzumab, and IgG1 isotype control (Shanghai Biointron, cas#B117901) monoclonal antibody were prepared, with SEC purity >96%, and HIC retention time of about 13 min (Table 1), which is comparable to that of the corresponding 9D1 antibody-drug conjugate.

The 9D1 antibody-drug conjugates (i.e., the compound represented by Formula (2)) in the following Experimental Examples were prepared from Experimental Example 18.

**Experimental Example 19 (a ratio of drug to antibody (DAR))**

Sample Treatment:

9D1-LD-38 prepared in Experimental Example 5a was diluted to 1mg/mL with 0.1M Tris-HCl, and 5μL of 0.5M DTT was added into per 100μg of 9D1-LD-38 to incubate in water bath at 37°C for 30min, then adding 1%(v/v) of 10% TFA to terminate the reaction.

LC-MS parameters:

Mobile phase A: 0.10% TFA and 0.02% FA aqueous solution; mobile phase B: 0.08% FA and 0.02% TFA acetonitrile solution. Separation gradient: mobile phase B is changed from 20% to 32% in 2-4min, changed from 32% to 45% in 4-12min; flow rate: 0.4mL/min. Chromatographic column is BioResolveTM RP mAb Polyphenyl Column (2.7μm, 2.1×150 mm), column temperature: 70°C.

Mass spectrum is Themo Q Exactive Plus, Full Scan is performed by positive ion mode; spray voltage: 3.8 kV; scanning range: 500-3500 m/z.

RP-HPLC parameters:

Mobile phase A: 0.1% TFA aqueous solution; mobile phase B: 0.1%TFA acetonitrile solution. Separation gradient: mobile phase B is changed from 30% to 50% in 5-25min; flow rate: 0.6 mL/min. Chromatographic column is Proteomix RP-1000 (5μm, 4.6×100 mm); column temperature: 60°C.

The results are shown in Table 2 and Figs. 7-9.

Table 2: The theoretical molecular weights and detected molecular weights of the main ingredients of 9D1-LD-38 LC/MS

| Ingredients | Theoretical molecular weight (Da) | Detected molecular weight (Da) |
|---|---|---|
| LC+1*LD-38 | 25617.9 | 25617.6 |
| HC+3*LD-38(G0F) | 54610.6<br>50418.6 | 54614.1 |
| HC+3*LD-38(G1F) | 54772.8 | 54776.3 |
| HC+3*LD-38(G2F) | 54934.9 | 54939.3 |
| HC+3*LD-38(G0) | 54464.5 | 54466.2 |
| HC+3*LD-38(Man5) | 54382.4 | 54385.1 |

Conclusion: it can be seen from Table 2 and Figs. 7-9, since cysteine was used to conjugate in 9D1-LD-38, it underwent pre-treatment to remain glycosylation in a reduced state. The results show that, the light chain and the heavy chain each have one main component. After mass spectrometry molecular weight identification, the main conjugation form of the light chain is one LD-38 molecule, and the main conjugation form of the heavy chain is three LD-38 molecules. The deconvolution results of molecular weight are shown in Figs 7-8, which are consistent with the theoretical molecular weights. In addition, partial ring opening of LD-38 molecule and the presence of variants with different conjugation

numbers of heavy chains were also found. The DAR value of 9D1-LD-38 was calculated to be 7.8 by normalization method based on the area of the liquid chromatogram (Fig. 9), which is comparable to the theoretic ratio of conjugated drug to antibody.

**Experimental Example 20 (in vitro tumor cell killing activity)**

1. Human Claudin18.2 positive MC38-hClaudin18.2 cell line

9D1 antibody-drug conjugates prepared in the previous Experimental Examples and Comparative Examples were used for performing in vitro killing test on MC38 cell lines stably expressing human Claudin18.2. MC38-hClaudin18.2 cells were inoculated into a 96-well cell culture plate at 3000 cells/well, adding gradient diluted 9D1 ADCs next day to culture for 48-72 hours. The fluorescence values of each experimental well were detected by AlamarBlue reagent (ThermoFisher, item number DAL1100), and the cell viability was calculated.

MC38-hClaudin18.2 cells were cultured in RPMI 1640 medium containing 10% FBS, supplemented with 1 μg/mL puromycin to subject to adherent culture at 37°C in 5% $CO_2$ incubator. When the cell confluence was greater than 80%, the cells were passaged at a ratio of 1:10 to 1:20 every 3 days.

MC38-hClaudin18.2 cells were digested by a digestive solution of recombinant enzyme (TrypLETM Express Enzyme). After counting, an appropriate amount of cells were taken to resuspend with RPMI 1640+10% FBS. The cell density was adjusted to $3\times10^4$ cells/mL, inoculating into 96-well cell culture plate at 100μL/well. The cells were cultured overnight at 37°C in 5% $CO_2$ incubator. The next day, the test drug was diluted 10 times in a series with a total of 7-8 concentration points, simultaneously setting a blank control with a sample concentration of 0. The diluted sample was added to 96-well cell culture plate containing cells at 100μL/well, then incubating at 37°C in 5% $CO_2$ incubator for 48-72 hours. 20μL of AlamarBlue detection reagent was added to each well, incubating at 37°C for 4-6 hours, the fluorescence signal intensity was detected on a multifunctional enzyme-linked immunosorbent assay (ELISA) instrument with an excitation wavelength of 560 nm and an emission wavelength of 590 nm.

The cell viability of the blank control was set to 100%, and the cell viability (%) of the experimental well was calculated by the following formula:

Cell viability (%) = (fluorescence value of experimental well/fluorescence value of blank control well) × 100%

The detected average cell viability was takedn as the y-axis, a sigmoid dose response (Variable Slope) method (GraphPad Prism software, GraphPad Software, San Diego, California) was used for nonlinear regression to obtain the inhibitory effect curves of cell proliferation, and the corresponding IC50 values (half maximum inhibition concentration) were calculated. The results are shown in Table 3 and Fig. 10 (Figs. 10A, 10B, 10C, 10D, and 10E).

Table 3: Half effective concentration (IC50) of in vitro cell killing of 9D1 antibody-drug conjugates and the control antibody-drug conjugates on MC38-hClaudin18.2 cells

| Tested samples | IC50 values (nM) |
|---|---|
| 9D1-GGFG-Exatecan | 11.03 |
| 9D1-LD-12 | 0.7993 |
| 9D1-LD-31-P1 | 4.353 |
| 9D 1-LD-31-P2 | 2.909 |
| 9D1-LD-37 | 0.802 |
| 9D1-LD-38 | 1.362 |
| 9D1-LD-65 | 1.610 |
| 9D 1-LD-66 | 1.094 |
| 9D1-LD-72 | 1.085 |
| 9D1-LD-73 | 1.402 |
| 9D1-LD-74 | 2.222 |
| Isotype IgG1-LD-12 | NA |
| Isotype IgG1-LD-38 | NA |

(continued)

| Tested samples | IC50 values (nM) |
|---|---|
| Exatecan | 4.101 |
| 9D1-vc-PAB-MMAE | 0.407 |
| 9D1-LD-57 | 0.530 |
| MMAE | 2.039 |
| Isotype IgG1-vc-PAB-MMAE | 41.36 |

Conclusion: it can be seen from Table 3 and Fig. 10, with respect to Exatecan toxin, compared to the antibody-drug conjugates of IgG1 isotype control antibodies (Isotype IgG1-LD-12, Isotype IgG1-LD-38), the 9D1 antibody-drug conjugates all show specific killing activity against MC38-hClaudin18.2, with IC50 values of 0.7-11.03 nM. Particularly, 9D1-GGFG Exatecan has the worst pharmacological activity, with IC50 of 11.03 nM. The IC50 values of the 9D1 antibody-drug conjugates having hydrophilic side chain of the inner salt are 0.8-4.4 nM. Except for 9D1-LD-31-P1, the activity is significantly stronger than that of Exatecan small molecule toxins (IC50 value, 4.10 nM).

With respect to MMAE toxin, the antibody-drug conjugates of IgG1 isotype control antibody (Isotype IgG1-VC-PAB-MMAE) show an IC50 value of 41.3 nM in MC38-hClaudin18.2, while the 9D1 antibody-drug conjugates (9D1 vc-PAB MMAE and 9D1 LD-57) show specific killing, with IC50 values of 0.41 nM and 0.53 nM, respectively.

2. Human Claudin18.2 positive NUGC4-hClaudin18.2 cell line

NUGC4 (NUGC4-hClaudin18.2) cells of human gastric cancer cell line stably expressing human Claudin18.2 were cultured in RPMI 1640 medium containing 10% FBS, supplemented with 1 μg/mL puromycin to subject to adherent culture at 37°C in 5% $CO_2$ incubator. When the cell confluence was greater than 80%, the cells were passaged at a ratio of 1:3 to 1:10 every 3-4 days.

NUGC4-hClaudin18.2 cells were digested by a digestive solution of recombinant enzyme (TrypLETM Express Enzyme). After counting, an appropriate amount of cells were taken to resuspend with RPMI 1640+10% FBS. The cell density was adjusted to $3\times10^4$ cells/mL, inoculating into 96-well cell culture plate at 100μL/well. The cells were cultured overnight at 37°C in 5% $CO_2$ incubator. The next day, 9D1 ADC and small molecular toxin were diluted 5 times in a series from 1000 nM (final concentration, 500 nM) with a total of 7-8 concentration points, simultaneously setting a negative control with a sample concentration of 0, and a blank control containing only medium without cells. The diluted sample was added to 96-well cell culture plate containing cells at 100μL/well (two duplicated wells), then incubating at 37°C in 5% $CO_2$ incubator for 120 hours. 20μL of CCK-8 detection reagent (Dongren Chemical, CAS# CK04) was added to each well, incubating at 37°C for 3-4 hours. The OD values of each well in the ELISA plate were measured at a wavelength of 450 nm and a reference wavelength of 630 nm on a multifunctional enzyme-linked immunosorbent assay (ELISA) reader, for each well, OD value=OD450 nm - OD630 nm. The cell viability of the negative control was set to 100%, and the cell viability (%) of the experimental wells was calculated according to the following formula.

Cell viability (%) = (experimental well OD value - blank well OD value)/(negative control well OD value - blank control well OD value) × 100%

Nonlinear regression was performed by sigmoid dose response (Variable Slope) method (GraphPad Prism software, GraphPad Software, San Diego, California), calculating the corresponding IC50 values (half maximum inhibitory concentration). The results are shown in Table 4.

Table 4: Half effective concentration of in vitro cell killing (IC50) of D1 antibody-drug conjugate and the control antibody-drug conjugate on NUGC4-hClaudin18.2 cells

| Test samples | IC50 values (nM) |
|---|---|
| 9D 1-GGFG-Exatecan | 0.184 |
| 9D1-LD-12 | 0.580 |
| 9D1-LD-38 | 0.728 |
| 9D1-LD-65 | 0.701 |
| 9D1-LD-66 | 0.996 |

(continued)

| Test samples | IC50 values (nM) |
|---|---|
| 9D1-LD-72 | 0.553 |
| 9D1-LD-73 | 1.273 |
| 9D1-LD-74 | 1.155 |
| Isotype IgGl-LD-38 | 141.3 |
| Exatecan | 2.013 |
| 9D1-vc-PAB-MMAE | 0.064 |
| 9D1-LD-57 | 0.053 |
| MMAE | 0.0019 |

Conclusion: it can be seen from Table 4, with respect to Exatecan toxin, compared to the antibody-drug conjugate of IgG1 isotype control antibody (Isotype IgG1-LD-38, IC50 141.3 nM), the 9D1 antibody-drug conjugates all show specific killing activity against NUGC4-hClaudin18.2, with IC50 values of 0.18-1.27 nM. The IC50 values (0.55~1.00 nM) of the 9D1 antibody-drug conjugates having hydrophilic side chain of the inner salt are superior to those of the antibody-drug conjugates having hydrophilic side chain of polyethylene glycol (PEG, 9D1-LD-73, IC50 1.27 nM) and polysarcosine (PSAR, 9D1-LD-74, IC50 1.16 nM).

With respect to MMAE toxin, the IC50 value of 9D1-LD-57 antibody-drug conjugate having hydrophilic side chain of the inner salt and the IC50 value of the control antibody-drug conjugate 9D1-vc-PAB-MMAE are comparable, and they are 0.05 nM and 0.06 nM, respectively.

3. Human Claudin18.2 positive HEK293-hClaudin18.2 cell line

HEK293-hClaudin18.2 cells were cultured in DMEM medium containing 10% FBS, supplemented with 250 μg/mL G418 to subject to adherent culture at 37°C in 5% $CO_2$ incubator. When the cell confluence was greater than 80%, the cells were passaged at a ratio of 1: 20 to 1: 40 every 3 days.

HEK293-hClaudin18.2 cells were digested by a digestive solution of recombinant enzyme (TrypLETM Express Enzyme). After counting, an appropriate amount of cells were taken to resuspend with DMEM+10%FBS. The cell density was adjusted to $2\times10^4$ cells/mL, inoculating into 96-well cell culture plate at 100μL/well. The cells were cultured overnight at 37°C in 5% $CO_2$ incubator. The next day, 9D1 ADC and small molecular toxin were diluted 10 times in a series from 1000 nM (final concentration, 500 nM) with a total of 7-8 concentration points, simultaneously setting a negative control with a sample concentration of 0, and a blank control containing only same volume (200 μL/well) of medium without cells. The diluted sample was added to 96-well cell culture plate containing cells at 100μL/well (two duplicated wells), then incubating at 37°C in 5% $CO_2$ incubator for 72 hours. The cell viability was detected by the AlamarBlue detection method mentioned above, then processing data to calculate the IC50 values, and the results are shown in Table 5 below.

Table 5: Half effective concentration of in vitro cell killing (IC50) of the 9D1 antibody-drug conjugate and the control antibody-drug conjugate on HEK293-hClaudin18.2 cells

| Test samples | IC50 values (nM) |
|---|---|
| 9D1-LD-38 | 0.090 |
| 9D1-LD-65 | 0.086 |
| 9D 1-LD-66 | 0.996 |
| Isotype IgG1-LD-38 | 688.2 |
| Exatecan | 0.508 |
| 9D1-LD-71 | 0.397 |
| 9D1-ELC122 | 0.628 |
| Isotype IgG1-ELC122 | NA |
| Note: NA indicates not applicable | |

Conclusion: it can be seen from Table 5, with respect to Exatecan toxin, compared to the antibody-drug conjugate of IgG1 isotype control antibody (Isotype IgG1-LD-38, IC50 688.2 nM), the 9D1 antibody-drug conjugates having hydrophilic side chain of the inner salt all showe specific killing activity against HEK293-hClaudin18.2, with IC50 values of 0.09-1.00 nM, and IC50 value of small molecular Exatecan is 0.51 nM.

With respect to SN38 toxin, compared to the antibody-drug conjugate of IgG1 isotype control antibody (Isotype IgG1-ELC122, no killing), 9D1 antibody-drug conjugate showed specific killing activity against HEK293-hClaudin18.2. The killing of the 9D1 antibody-drug conjugate (9D1-LD-57) having hydrophilic side chain of the inner salt is a little stronger than that of the control (9D1-ELC122), their IC50 values are 0.40 and 0.63 nM, respectively.

**Experimental Example 21 (In vivo tumor therapeutic effect)**

1. Mouse xenograft tumor model of human Claudin18.2 positive MC38-hClaudin18.2 cell line

C57BL/6 mice, female, 5-6 week old, purchased from Shanghai Lingchang Biotech. Animals were kept in the laminar flow cabinet of the SPF animal room, with 5 animals per cage and 12 hours of alternating light exposure. Animals can freely drink and feed. The mouse colorectal cancer cell line MC38 Claudin18.2, which stably expresses human Claudin18.2, was cultured in RPMI 1640 medium containing 10% FBS (fetal bovine serum) to subject to adherent culture in T75 flasks at 37°C in 5% $CO_2$ incubator, and the cells were passaged every three days. MC38 h Claudin18.2 cells cells at logarithmic growth phase were collected, centrifuging at 1000 rpm for 5 minutes, and resuspending in PBS. $2\times10^6$ cells were mixed with matrix gel in a volume ratio of 1:1 to inoculate subcutaneously in C57BL/6 mice. When the tumor volume reaches 100-150 $mm^3$, the mice were grouped and administrated with the conjugates (5-6 mice/group). The tumor sizes were measured twice a week, then calculating the tumor volumes (TV, $mm^3$), and plotting tumor growth curves.

$$TV\ (mm^3)=(L\times W^2)/2;$$

L represents tumor length, and W represents tumor width

$$\text{Tumor inhibition rate (\%)}=(\text{TV control group}-\text{TV administration group})\times 100/\text{TV control group}$$

The days after the first administration was used as the x-axis, the tumor volume was used as the y-axis, and the GraphPad Prism software (GraphPad Software, San Diego, California) was used to plot tumor curves of mouse by XY mode. Two way ANOVA was used to analyze whether there were statistical differences between each experimental group and the vehicle control PBS group. *p<0.05, **p<0.01, ***p<0.001, and ****p<0.0001.

The therapeutic schemes of 9D1 antibody-drug conjugate conjugated to Exatecan on MC38-hClaudin18.2 mouse subcutaneous tumor model are listed in Table 6. The therapeutic effects on tumor and the corresponding tumor growth curves are shown in Table 6 and Fig. 11 (Fig. 11 comprises Fig. 11A, Fig. 11B and Fig. 11C).

Table 6: Grouping, administration regimens, and the tumor inhibition rates after drug treatment of the MC38-hClaudin18.2 mouse subcutaneous tumor model

| Grouping | Administration route | Administration dosage (mg/kg) | Administration times | Tumor inhibition rate (%) |
|---|---|---|---|---|
| **PBS control** | **i.p.** | **/** | **1** | **NA** |
| 9D1-ELC122 | i.p. | 10 | 1 | 39.82 |
| 9D1-GGFG-Exatecan | i.p. | 10 | 1 | 48.34 |
| 9D1-LD12 | i.p. | 10 | 1 | 48.63 |
| **PBS control** | **i.p.** | **/** | **1** | **NA** |
| 9D1-LD-31-P1 | i.p. | 10 | 1 | 27.8% |
| 9D 1-LD-31-P2 | i.p. | 10 | 1 | 80.0% |
| 9D1-LD-37 | i.p. | 10 | 1 | 37.4% |
| 9D1-LD-38 | i.p. | 10 | 1 | 80.6% |

(continued)

| Grouping | Administration route | Administration dosage (mg/kg) | Administration times | Tumor inhibition rate (%) |
|---|---|---|---|---|
| **PBS control** | **i.p.** | **10** | **Qw x 2** | **NA** |
| 9D1-GGFG-Dxd | i.p. | 10 | Qwx2 | 50.0% |
| 9D1-LD-38 | i.p. | 10 | Qwx2 | 98.0% |
| IgG1 Isotype-LD-38 | i.p. | 10 | Qwx2 | 27.2% |
| **PBS control** | **i.p.** | **/** | **1** | **NA** |
| 9D1-LD-65 | i.p. | 5 | 1 | 71.7% |
| 9D1-LD-66 | i.p. | 5 | 1 | 48.0% |
| 9D1-LD-72 | i.p. | 5 | 1 | 42.3% |
| 9D1-LD-73 | i.p. | 5 | 1 | 47.0% |
| 9D1-LD-74 | i.p. | 5 | 1 | 56.5% |
| 9D1-LD-38 | i.p. | 5 | 1 | 39.2% |
| IgG1 Isotype-LD-38 | i.p. | 5 | 1 | 5.5% |
| Note: i.p.: intraperitoneal injection, intraperitoneal administration; NA: not applicable. | | | | |

Conclusion: it can be seen from Table 6 and Fig. 11, under the condition of a single administration dosage of 10 mg/kg, growth of the tumor treated with the control antibody-drug conjugates (9D1-ELC122, 9D1-GGFG-Exatecan, and 9D1-LD-12) is reduced (Fig. 11A) by the end of the experiment, and their tumor inhibition rates (TGI) are 39.82%, 48.34% and 48.63%, respectively. The antibody-drug conjugates with a linker having inner salt group (9D1-LD-31-P2 and 9D1-LD-38) exhibits superior therapeutic effect on tumor (Fig. 11B), and their TGIs are 80.0% and 80.6%, respectively.

Under the conditions of 10 mg/kg, administrated once a week for a total of 2 doses, by the end of the experiment, the tumor inhibition rate of the control antibody-drug conjugate 9D1-GGFG-Dxd is 50.0%, while the tumor of 9D1-LD-38 is almost completely eliminated (tumor inhibition rate is 98.0%). The homologous control antibody-drug conjugate (IgG1 Isotype LD-38) only shows mild tumor inhibitory effect (Table 6).

In order to further compare and distinguish the tumor therapeutic effects of other 9D1 antibody-drug conjugates having inner salt groups, a low dose (5mg/kg) was chosen for drug efficacy experiments, and 9D1-LD-38 was set as the internal reference. Under the condition of low dose (5mg/kg) administration, the isotype control antibody-drug conjugate (IgG1 Isotype LD-38) does not show significant tumor therapeutic effect. The tumor therapeutic effect of 9D1-LD-38 is significant, with a TGI of 39.2%. The tumor therapeutic effect of other 9D1 antibody-drug conjugates with a linker having inner salt group (9D1-LD-65, 9D1-LD-66, and 9D1-LD-72) is better (Fig. 11C), with TGIs of 71.7%, 48.0% and 42.3%, respectively. Especially for 9D1-LD-65, the tumor inhibition effect is optimal, and the tumor growth inhibition curves (Fig. 11C) and TGI are even significantly better than the reported 9D1 antibody-drug conjugates with a linker having other water-soluble groups, such as the 9D1-LD-73 (TGI 47.0%) control having polyethylene glycol (PEG) water-soluble group and the 9D1-LD-74 (TGI 56.5%) control having polysarcosine (PSAR) water-soluble group.

In summary, the efficacy of 9D1 antibody-drug conjugates with a linker having inner salt water-soluble group (9D1-LD-31-P2, 9D1-LD-38, 9D1-LD-65, 9D1-LD-66, and 9D1-LD-72) is significantly better than that of classical antibody-drug conjugates with a linker having no water-soluble group, such as 9D1-ELC122, 9D1-GGFG Exatecan, and 9D1-LD12. Especially for 9D1-LD-65, 9D1-LD-66, and 9D1-LD-72, the tumor therapeutic effect is comparable to or better than that of 9D 1 antibody-drug conjugates with a linker having other types of water-soluble groups (9D1-LD-73 having PEG, and 9D1-LD-74 having PSAR); for example, 9D1-LD-65.

2. Mouse xenograft tumor model of human Claudin18.2 positive NUGC4-hClaudin18.2 cell line

The NUGC4 hClaudin 18.2 gastric cancer cell line, which stably expresses human density protein 18.2, was cultured in RPMI-1640 medium containing 10% fetal bovine serum, 100 U/ml penicillin, and 100 μg/mL streptomycin in 37°C, 5% $CO_2$ incubator. After the cells were fully grown every 4 to 5 days, they were subcultured in bottles. The tumor cells in the logarithmic growth phase were used for in vivo tumor inoculation.

NUGC4 hClaudin18.2 tumor cells were adjusted to a concentration of $5\times10^7$/mL with PBS: Matrix (1:1 volume ratio) and inoculated subcutaneously on the right rib of BALB/c nu/nu mice (female, 6-7 week old) at a dose of 100

μL/mouse, with a tumor cell inoculation volume of 5×10$^6$ cells/mouse. When the tumor volume reached about 150 mm$^3$, the mice were dosed in groups. The groups and administration regimens are shown in Table 7. The tumor sizes were measured twice a week, then calculating the tumor volumes (TV, mm$^3$), and plotting tumor growth curves.

$$TV\ (mm^3)=(L\times W^2)/2,$$

L represents tumor length, and W represents tumor width

The days after the first administration was used as the x-axis, the tumor volume was used as the y-axis, and the GraphPad Prism software (GraphPad Software, San Diego, California) was used to plot tumor curves of mouse by XY mode. The results are shown in Fig. 12.

Table 7: Grouping and administration regimens of NUGC4-hClaudin18.2 mouse subcutaneous tumor model

| Groups | Test drugs | Animal number | Dosage (mg/kg) | Administration route | Administration frequency |
|---|---|---|---|---|---|
| 1 | 9D1-LD-38 | 6 | 5 | i.p | QW x 3 |
| 2 | 9D1 unconjugated antibody | 6 | 5 | i.p | QW x 3 |
| 3 | 9D1-ELC-122 | 6 | 5 | i.p | QW x 3 |
| 4 | 9D1-GGFG-Exatecan | 6 | 5 | i.p | QW x 3 |
| 5 | Isotype-LD-38 | 6 | 5 | i.p | QW x 3 |
| 6 | PBS | 6 | 5 | i.p | QW x 3 |
| Note: i.p: intraperitoneal injection; QW X 3: administration one a week, for a total of three times. | | | | | |

Conclusion: it can be seen from Table 7 and Fig. 12 that, under the condition of, administrated once a week at 5 mg/kg with a total of three times, 9D1-LD-38 with a linker having an inner salt water-soluble group shows the best therapeutic effect, significantly better than 9D1-ECL-122 and 9D1-GGFG-Exatecan. After 24 days of treatment, the tumor basically disappeares in the 9D1-LD-38 treatment group, while the tumor growth is inhibited in the 9D1-GGFG-Exatecan treatment group, but no tumor reduction is observed. The 9D1-ECL-122 only shows a weak tumor inhibitory effect, which may be because the toxin of 9D1-ECL-122 is the less toxic SN38.

3. Mouse xenograft tumor model of folate receptor positive OVCAR-3 cells

OVCAR-3 human ovarian cancer tumor cells expressing folate receptor (FRa) were cultured in RPMI-1640 medium containing inactivated 20% fetal bovine serum, 100U/ml penicillin, and 100μg/mL streptomycin in 37°C, 5% $CO_2$ incubator. After the cells were fully grown, they were subcultured every 4-5 days in bottles, and the tumor cells in logarithmic growth phase were used for in vivo tumor inoculation.

OVCAR3 tumor cells were adjusted to a concentration of 1× 10$^8$/mL with PBS: Matrix (1:1 volume ratio) and inoculated subcutaneously into the right rib of M-NSG experimental mice at 100uL/mouse, with a tumor cell inoculation volume of 1×10$^7$/mouse.

When the tumor grew to a volume of about 150 mm$^3$, the mice were grouped for administration (5 mice/group), wherein control group (Control) was administrated with PBS, and the treatment group was administrated with an antibody-drug conjugate of anti-human folate receptor (FRa) humanized monoclonal antibody, Farletuzumab-LD-38, at a dosage of 5 mg/kg with single dosage administration. The tumor sizes were measured twice a week, then calculating the tumor volumes (TV, mm$^3$), and plotting tumor growth curves.

$$TV\ (mm^3)=(L\times W^2)/2,$$

L represents tumor length, and W represents tumor width

$$\text{Tumor inhibition rate (\%)}=(\text{TV control group}-\text{TV administration group})\times 100/\text{TV control group}$$

The days after the first administration was used as the x-axis, the tumor volume was used as the y-axis, and the

GraphPad Prism software (GraphPad Software, San Diego, California) was used to plot tumor curves of mouse by XY mode. Two way ANOVA was used to analyze whether there were statistical differences between each experimental group and the vehicle control PBS group. *p<0.05, **p<0.01, ***p<0.001, and ****p<0.0001. The results are shown in Fig. 13.

Conclusion: it can be seen from Fig. 13, one week after single administration at 5mg/kg, Farletuzumab-LD-38 antibody-drug conjugate shows tumor growth inhibition as compared to the control group, and subsequently shows tumor shrinkage. On day 24, the tumor inhibition rate (TGI) reachs 93.5%, which is significantly different from the control group.

4. Mouse xenograft tumor model of folate receptor positive IGROV1 cells

IGROV1 human ovarian cancer tumor cells expressing folate receptor (FRa) were cultured in DMEM medium containing inactivated 10% fetal bovine serum, 100U/ml penicillin, and 100μg/mL streptomycin in 37°C, 5% $CO_2$ incubator. After the cells were fully grown, they were subcultured every 3-4 days in bottles, and the tumor cells in logarithmic growth phase were used for in vivo tumor inoculation.

IGROV1 tumor cells were adjusted to a concentration of $1\times10^8$/mL with PBS and inoculated subcutaneously into the right rib of BALB/c nude experimental mice at 100uL/mouse, with a tumor cell inoculation volume of $1\times10^7$/mouse.

When the tumor grew to an average volume of about 150 $mm^3$, the mice were grouped for administration (The day of grouping was recorded as D0), and each model was evenly divided into 2 groups, 5 mice/group, wherein control group (Control) was administrated with PBS, and the treatment group was administrated with an antibody-drug conjugate of anti-human folate receptor (FRa) humanized monoclonal antibody, Farletuzumab-LD-38, at a dosage of 5 mg/kg, administrated once a week with a total of two times. The tumor sizes were measured twice a week, then calculating the tumor volumes (TV, $mm^3$), and plotting tumor growth curves.

$$TV\ (mm^3)=(L\times W^2)/2,$$

L represents tumor length, and W represents tumor width

$$\text{Tumor inhibition rate (\%)}=(\text{TV control group}-\text{TV administration group})\times 100/\text{TV control group}$$

The days after the first administration was used as the x-axis, the tumor volume was used as the y-axis, and the GraphPad Prism software (GraphPad Software, San Diego, California) was used to plot tumor curves of mouse by XY mode. Two way ANOVA was used to analyze whether there were statistical differences between each experimental group and the vehicle control PBS group. *p<0.05, **p<0.01, ***p<0.001, and ****p<0.0001.

The results are shown in Fig. 14, after administrated once a week, at 5mg/kg with a total of two times, the antibody-drug conjugate of Farletuzumab-LD-38 shows rapid shrinkage of the tumor as compared to the control group, with a tumor inhibition rate (TGI) of 100% on day 30, and maintains efficacy 50 days after administration.

**Experimental Example 22: Pharmacokinetics in Rats**

Three male SD rats were selected for the experiment and randomly divided into three groups. They were given a single intravenous injection of test samples including 9D1-LD-38 prepared in the Experimental Example and 9D1-GGFG-Exatecan prepared in the Comparative Example, each with a dose of 10 mg/kg. Before administration, and 5 minutes, 1 hour, 5 hours, 24 hours (D2), 48 hours (D3), 120 hours (D6), 168 hours (D8), 240 hours (D11), 336 hours (D15), and 504 hours (D22) after administration, whole blood was collected from animals in each test group, and serum and plasma were separated. ELISA method was used to analyze the concentrations of total Ab (antibody) and ADC (conjugated antibody) of each test sample in serum samples. The pharmacokinetic parameters of each animal were calculated by PK Solver non-compartmental analysis (NCA) model.

The detection method of total antibody and conjugated antibody: ELISA method was used to determine the blood drug concentration of total antibody and conjugated antibody. The specific method was as follows:

a) The anti-human IgG $(Fab)_2$ was diluted to 1 μg/mL with PBS, coating the 96-well ELISA plate at 100 μL/well, and leaving it overnight at 4°C.
b) The plate was washed for three times, blocking with 3% BSA-PBST for 2 hours at 300 μL/well at room temperature.
c) The plate was washed for three times, diluting the 9D1-LD-38/9D1-GGFG-Exatecan with rat blank serum at a ratio of 2 times, with the initial concentration of 1 μg/mL for a total of 7 concentrations. Additionally, and the rat blank serum was set as the "0" point. At the same time, the samples at different time points after administration were diluted with rat blank serum at appropriate multiples. All the above samples were further diluted 50 times (for 9D1-GGFG-Dxd) and

100 times (for 9D1-LD-38) with 1% BSA-PBST, then adding to the ELISA plate at 100μL/well to incubate for 2 hours at room temperature.

d) The plate was washed for 6 times.

Detection of the total antibody: HRP sheep anti-human IgGFC was diluted with 1% BSA-PBST at a ratio of 1:20000, adding to the ELISA plate at 100μL/well to incubate for 1 hour at room temperature.

Detection of ADC: the anti-exatecan antibody was diluted with 1% BSA-PBST to 0.5 μg/mL, then adding to the ELISA plate at 100μL/well to incubate for 1 hour at room temperature; washing the plate for 6 times, and HRP sheep anti-mouse IgG (H+L) was diluted with 1% BSA-PBST at a ratio of 1:20000, adding to the ELISA plate at 100μL/well to incubate for 1 hour at room temperature.

The plate was washed for 6 times, then adding TMB to the plate at 100μL/well, and color-rendering for about 10 minutes in dark at room temperature.

The termination solution was added at 50μL/well, and detection was performed on the ELISA plate (wavelength is 450/630 nm). The results are shown in Table 8 and Fig. 15.

Table 8: The pharmacokinetics parameters of 9D1-LD-38 and 9D1-GGFG-Exatecan in rats

| *PK parameters* | *Unit* | *9D1-LD-38* | | *9D1-GGFG-Exatecan* | |
|---|---|---|---|---|---|
| | | Total Ab | ADC | Total Ab | ADC |
| *t1/2* | h | 145.4 | 119.8 | 65.1 | 57.8 |
| *Cmax* | μg/mL | 482.51 | 428.64 | 613.84 | 533.28 |
| *AUC* | μg/mL*h | 37883.16 | 29359.05 | 34804.63 | 23434.00 |
| *Cl_obs* | mL/kg/h | 0.26 | 0.34 | 0.29 | 0.43 |
| *Vss_obs* | (mL/kg) | 40.3 | 39.2 | 23.8 | 29.7 |

Conclusion: it can be seen from Table 8 and Fig. 15, under the condition of single intravenous injection at 10 mg/kg to rats, the serum drug concentrations of the total antibody and the conjugated antibody (ADC) of 9D1-GGFG-Exatecan are decreased rapidly (Fig. 15), with a shorter half-life of 65.1h and 57.8h, respectively. The AUC ratio of the conjugated antibody to the total antibody is 0.67.

The decrease trend of serum drug concentrations for the total antibody and the conjugated antibody of 9D1-LD-38 is relatively slow (Fig. 15), with a half-life of 145.4h and 119.8h, respectively. The AUC ratio of the conjugated antibody to the total antibody is 0.78.

The half-life of the conjugated antibody 9D1-LD-38 is 2 times of that of the 9D1-GGFG-Exatecan; the AUC of the conjugated antibody 9D1-LD-38 is 1.3 times of the 9D1-GGFG-Exatecan. The AUC ratio of the conjugated antibody 9D1-LD-38 to the total antibody is greater than the AUC ratio of the 9D1-GGFG-Exatecan to the total antibody (0.78 vs 0.67), indicating that 9D1-LD-38 is more stable, and the small molecule is less likely to be detached.

In summary, the drug clearance of 9D1-LD-38 with a linker having inner salt water-soluble group is significantly weaker in rats than that of 9D1-GGFG-Exatecan with a linker without water-soluble group, and its in vivo stability is better, manifested by a significantly prolonged half-life of 9D1-LD-38, a significant increase in drug exposure, and a higher AUC ratio of the conjugated antibody to the total antibody.

**Experimental Example 23: In vitro cell killing effects of 9D1-LD-38, 9D1-LD-82, 9D1-LD-88 and 9D1-LD-89**

MC38-hClaudin18.2 cells were cultured in RPMI 1640 (Thermo, Cat#: 11875-085) medium containing 10% FBS (Thermo, Cat#: 10099-141C), supplemented with 1μg/mL puromycin (Thermo, Cat#: A11138-03) to subject to adherent culture at 37°C in 5% $CO_2$ incubator. When the cell confluence was greater than 80%, the cells were passaged at a ratio of 1:10 to 1:20 every 3 days.

MC38-hClaudin18.2 cells were digested by a digestive solution of recombinant enzyme (TrypLETM Express Enzyme, Themo, Cat#: 12605028). After counting, an appropriate amount of cells were taken and resuspended with RPMI 1640+10% FBS. The cell density was adjusted to $1.5\times10^4$ cells/mL, inoculating into 96-well cell culture plate at 100μL/well. The cells were cultured overnight at 37°C in 5% $CO_2$ incubator. The next day, 9D1-LD-38, 9D1-LD-82, 9D1-LD-88, and 9D1-LD-89 were respectively diluted 10 times in a series from a concentration of 500 nM with a total of 7-8 concentration points, simultaneously setting a negative control with a sample concentration of 0, and and a blank control with the same volume (200 μ L/well) of culture medium without cells. The diluted sample was added to 96-well cell culture plate containing cells at 100μL/well, then incubating at 37°C in 5% $CO_2$ incubator for 72 hours. 20μL of AlamarBlue

(Thermo, Cat:DAL1100) detection reagent was added to each well, incubating at 37°C for 6 hours, the fluorescence signal intensity was detected on a multifunctional ELISA instrument (Molecular Devices, M5) with an excitation wavelength of 560 nm and an emission wavelength of 590 nm. The cell viability of the negative control was set to 100%, then calculating the cell viability (%) of the experimental well according to the following formula:

Cell viability (%)=(OD value of experimental well - OD value of the blank well)/( OD value ofnegative control well - OD value of the blank control well)×100%

The logarithm of ADC concentration was used as the x-axis, and the cell viability was used as the y-axis, and the sigmoid dose response (Variable Slope) method (GraphPad Software, San Diego, California) was used for nonlinear regression to obtain the inhibitory effect curves of cell proliferation, as shown in Fig. 16.

Conclusion: it can be seen from Fig. 16, 9D1-LD-38, 9D1-LD-82, 9D1-LD-88 and 9D1-LD-89 each may inhibit the proliferation of MC38-hClaudin18.2 cells in a dose-dependent manner, the inhibition effects of 9D1-LD-38, 9D1-LD-88 and 9D1-LD-89 are coomparable (their IC50 values are 0.632, 1.458 and 0.757 nM, respectively), and 9D1-LD-82 is slightly weaker (IC50 value is 8.013 nM).

Experimental Example 24: Inducing B16F10-hClaudin18.2 cells to generate **immunogenic cell death**

The B16F10-hClaudin18.2 cells were resuscitated and cultured in a medium of DMEM (Thermo, cat#: 10569044) +10%FBS (Thermo, Cat#: 10099-141C) +0.5μg/mL Puromycin to subject to adherent culture at 37°C in 5% $CO_2$ incubator. When the cell confluence was greater than 80%, the cells were passaged at a ratio of 1:10 to 1:20 every 3-4 days.

When the cells grew to a confluence of about 80%, the cells were digested by a digestive solution of recombinant enzyme (TrypLETM Express Enzyme, Thermo, cat#: 12605028). Then the cells were resuspended with DMEM+10% FBS, and an appropriate volume was taken for counting. The cell density was adjusted to $1\times10^4$cells/mL, and inoculated into 96-well cell plate at 100μL/well to culture overnight at 37°C in 5% $CO_2$ incubator. The next day, the ADC drug was diluted with DMEM+10% FBS medium to a final concentration of 750nM, the small molecule toxin Exatecan was diluted to a final concentration of 10nM, and sample concentration of 0 was set as blank control. The diluted samples were respectively added to 96-well cell culture plate containing cells at 100μL/well to incubate at 37°C in 5% $CO_2$ incubator for 48 hours. After taking out the 96-well cell culture plate from the incubator, the cells were digested with Accutase (Thermo, cat#: 00-4555-56), washing once with 1% FBS PBS, then adding Anti-Calreticulin-Alexa Fluor 647 antibody (Abcam, cat#: AB196159, diluted with 1% FBS PBS in a ratio of 1:100) at 100μL/well to incubate in the dark for 30 minutes at room temperature. After washing twice with 1% FBS PBS, 2μl of PI dye (Thermo, cat#: 00-4555-56) was added to each sample to incubate in dark for 10 minutes at room temperature, then performing FACS (Bechman, CytoFLEX) detection on the instrument, and plotting curves as shown in Fig. 17 based on the ratio of Calreticulin (+)/PI (-) cells.

Conclusion: it can be seen from Fig. 17, when the concentration of ADC is 750nM, compared with control ADC, the ratio of Calreticulin(+)/PI(-) cells for 9D1-LD-38/9D1-GGFG-Dxd treatment groups are significantly raised, and the 9D1-LD-38 group is higher than the 9D1-GGFG-Dxd group; when the concentration of the small molecular drug load Dxd is 10nM, the ratio of Calreticulin(+)/PI(-) cells is significantly raised, and Exatecan (MCE, cat#: HY-13631A) >Dxd (MCE, cat#: HY-13631D). The results show that, compared to 9D1-GGFG-Dxd, 9D1-LD-38 can induce stronger immunogenic cell death, providing a theoretical basis for its combination with immune checkpoint inhibitors

**Experimental Example 25: Bystander killing on HEK293-hClaudin18.2/HEK293 cells**

AlarmaBlue detection method: hClaudin18.2 positive cells HEK293-hClaudin18.2 and negative cells HEK293 were resuscitated, and cultured in a medium of DMEM+10%FBS+250μg/mL G418 and DMEM+10%FBS respectively to subject to adherent culture at 37°C in 5% $CO_2$ incubator. When the cell confluence was greater than 80%, the cells were passaged at a ratio of 1:10 to 1:20 every 3-4 days.

When the cells grew to a confluence of about 80%, the negative cells and positive cells were digested by a digestive solution of recombinant enzyme (TrypLETM Express Enzyme). After counting, an appropriate amount of cell suspension was taken to centrifuge, then resuspending with DMEM+10% FBS. The cell density was adjusted to $5\times10^3$ cells/mL. Both positive cell group and negative cell group were inoculated into 96-well cell culture plate at 100μL/well. The mixed positive and negative cell groups were mixed well at 1:1 volume, then inoculating into 96-well cell culture plate at 200μL/well to culture overnight at 37°C in 5% $CO_2$ incubator. The next day, the ADC drug was diluted with DMEM+10% FBS medium to a final concentration of 0.5nM, and a sample concentration of 0 was set as blank control. As for the mixed group of positive and negative cells, 100μL of culture medium was discarded per well, then adding the diluted ADC drug to the 96-well cell culture plate containing cells at 100μL/well to incubate for 120 hours at 37°C in 5% $CO_2$ incubator. After

taking out the 96-well cell culture plate from the incubator, 20μL of AlamarBlue (Thermo, cat#: DAL1100) detection reagent was added to each well to incubate at 37°C for 4 hours, the fluorescence signal intensity was detected on a multifunctional enzyme-linked immunosorbent assay (ELISA) instrument with an excitation wavelength of 560 nm and an emission wavelength of 590 nm.

Cell viability of the blank control was set as 100%, calculating the cell viability (%) according to the following formula:

Cell viability (%) = (fluorescence value of experimental well /fluorescence value of blank control well) × 100%

FACS detection method: HEK293 cells were labelled, after counting the HEK293 cells in logarithmic growth phase, taking an appropriate amount of cell suspension to centrifuge, washing twice with PBS, then adjusting the density to $5\times10^6$ cells/mL, adding an appropriate volume of CFSE (eBioscience, 65-0850-84) to a final concentration of 0.5 nM and immediately mixing well to incubate for 10 minutes in the dark at room temperature, adding 4-5 times volume of pre-cooled DMEM+10% FBS to incubate on ice for 5 minutes to terminate the reaction. After washing twice with pre-cooled DMEM+10% FBS, the cell density was adjusted to $2\times10^4$ cells/mL with DMEM+10% FBS medium.

Co-incubation and detection: HEK293-hClaudin18.2 positive cells were digested by a digestive solution of recombinant enzyme (TrypLETM Express Enzyme). After counting, an appropriate amount of cell suspension was taken to centrifuge, then resuspending with DMEM+10% FBS. The cell density was adjusted to $2\times10^4$ cells/mL. CFSE labeled HEK293 cells and HEK293-hClaudin18.2 cells were mixed with equal volumes, then inoculating into 96-well cell culture plate at 100μL/well to culture overnight at 37°C in 5% $CO_2$ incubator. The next day, the ADC drug was diluted with DMEM+10% FBS medium to a final concentration of 0.5nM, and sample concentration of 0 was set as blank control. The diluted samples were respectively added to 96-well cell culture plate containing cells at 100μL/well to incubate for 72 hours at 37°C in 5% $CO_2$ incubator. The supernatant of the 96-well cell culture plate was discarded, after digesting the cells with a digestive solution of recombinant enzyme, washing once with 1% FBS PBS, resuspending each sample with 250μL 1% FBS PBS and counting, and calculating the total number of cells for each sample. 100μL of each sample was taken for FACS detection to determine the proportion of hClaudin18.2 positive and negative cells, then calculating the number of hClaudin18.2 positive and negative cells in each sample based on the total number of cells.

Conclusion: after the ADC drugs were co-cultured with HEK293 rexpectively, HEK293-hClaudin18.2 and HEK293+HEK293-hClaudin18.2 mixed culture cells were co-cultured for 5 days, and then the cell viability was detected by AlarmaBlue assay. The results are shown as Fig. 18. Fig. 18 comprises Fig. 18A and Fig. 18B, wherein Fig. 18A is the result by Alarma Blue assay, and Fig. 18B is the result by flow cytometry method. As shown in Fig. 18A, 4 ADC drugs have no killing effect on negative cells HEK293; as compared with control (Isotype IgG1) ADC, both 9D1-LD-38 and 9D1-GGFG-Dxd have killing effect on positive cells HEK293-hClaudin18, and they both have comparable killing effects; as compared with control (Isotype IgG1)ADC, both 9D1-LD-38 and 9D1-GGFG-Dxd have killing effect on positive and negative mixed cells, and the killing effect of 9D1-LD38 is stronger than that of 9D1-GGFG-Dxd. The above results indicate that, 9D1-LD-38 has stronger bystander killing than 9D1-GGFG-Dxd.

After the ADC drug was co-cultured with HEK293 (CFSE labelled)+HEK293-hClaudin18.2 mixed culture cells for 3 days, the total amount and ratio of positive to negative cells were measured by cell counters and flow cytometry, respectively. As shown in Fig. 18B, the isotype control (Isotype IgG1) ADC has no killing effect on both positive cells HEK293-hClaudin18.2 and negative cells HEK293, while both 9D1-LD-38 and 9D1-GGFG-Dxd have killing effect on HEK293-hClaudin18.2 and negative cells HEK293, and the killing effect of 9D1-LD38 on negative cells HEK293 is stronger than that of 9D1-GGFG-Dxd. The above results indicate that, 9D1-LD38 has stronger bystander killing than 9D1-GGFG-Dxd.

**Experimental Example 26: In vitro enzymatic release of free toxin molecules**

Preparation of activated buffer solution (10 mM L-cysteine (ACRO, cat#: 173600250), 15 mM EDTA, 0.1M His HCl, pH 5.5): 3.63g of solid cysteine was weighed, adding 3 mL of 0.1M His HCl (pH 5.5) solution for dissolution, and then adding 90μL of 0.5M EDTA solution to mix well.

Sample preparation: 1) 30μL of activated buffer solution was taken, adding 5μL of Cathepsin B enzyme solution (0.15mg/mL), Sino Biological, cat#: 10483-H08H) to mix well andplace in 37°C water bath for 15 minutes, adding 300μL of 0.1M His HCl (pH 5.5) solution and 36μL of 9D1-LD-38 (375μg) to stay in 37°C water bath overnight for confirmation of free small molecule digested by enzyme. 2) LD-38, LD-65, and Deruxtecan (MC-GGFG-Dxd) were diluted to 14 mg/mL with DMSO respectively; 10μL of the diluted linker-toxins mentioned above were taken, adding 20μL of activated buffer into each of them, then adding 250μL (LD-38), 244.5μL (LD-65), and 344.8μL (MC-GGFG-Dxd) of activated buffer respectively to mix well to stay in 37°C water bath for 15 minutes; 2.3μL of 0.4mg/mL Cathepsin B enzyme (Sino Biological, cat#: 10483-H08H) was taken, adding 950μL of activated buffer to stay in 37°C water bath for 15 minutes; 230μL of activated

linker-toxin solution was taken respectively, adding 230μL of activated enzyme solution (the molar ratio of linker-toxin to Cathepsin B is about 1200: 1) to stay in 37°C water bath for 0.5h, 2h, 4h, and 20h. At each detection time point, 50μL of enzyme digestion solution was taken, adding 2.5μL of 10% TFA solution to terminate the reaction for enzyme digestion kinetics detection. The results are shown in Fig. 19.

Negative control preparation: 30μL of activated buffer was taken to stay in 37°C water bath for 15 minutes, then adding 300μL of 0.1M His HCl (pH 5.5) solution and 36μL of 9D1-LD-38 (375μg) to stay in 37°C water bath overnight. The parameters of RP-HPLC (Proteomix RP-1000) are shown in Table A:

Table A

| | | | | |
|---|---|---|---|---|
| Injection volume | 20 μL/5μL (sample preparation 1); 10 μL (sample preparation 2) | Column temperature | | 40°C (sample preparation 1) 60°C (sample preparation 2) |
| Temperature of the sample room | 10°C | Detection wavelength | | 280/366 nm |
| Gradient | | | | |
| Time (min) | Flow rate (mL/min) | Mobile phase A (0.1%TFA) | Mobile phase B (0.1%TFA/ACN) | Curve |
| 0 | 0.6 | 90% | 10% | / |
| 2.5 | 0.6 | 90% | 10% | 6 |
| 12.5 (sample preparation 1) / 17.5 (sample preparation 2) | 0.6 | 50% (sample preparation 1) / 20% (sample preparation 2) | 50% (sample preparation 1) / 80% (sample preparation 2) | 6 |
| 15 (sample preparation 1) / 20 (sample preparation 2) | 0.6 | 5% | 95% | 1 |
| 20 (sample preparation 1) / 25 (sample preparation 2) | 0.6 | 90% | 10% | 1 |

Conclusion: it can be seen from Fig. 19, the toxin molecules released from 9D1-LD-38 after being cleaved by Cathepsin B enzyme is consistent with the peak time of the standard Exatecan, and no interference peak appeares in the control group (Control) without the addition of Cathepsin B enzyme. Therefore, it can be determined that Cathepsin B enzyme can effectively cleave the linker of 9D1-LD-38, and the cleaved release product is free Exatecan.

The enzymatic cleavage kinetics of LD-38, LD-65, and Deruxtecan (MC-GGFG-Dxd) are shown in Table 9. LD-38 can effectively release toxin molecules after being cleaved by Cathepsin B enzyme, and the cleavage is almost completed (95%) after 4 hours; LD-65 and MC-GGFG-Dxd are not sensitive to CathepsinB enzyme, after 20 hours of enzyme digestion, LD-65 only releases a slight amount of toxin (4%), while MC-GGFG-Dxd does not release any enzyme digestion products

Table 9: Cathepsin B enzyme digestion kinetics of LD-38, LD-65 and Deruxtecan (MC-GGFG-Dxd)

| Time (h) / Linker | 0 | 1h | 2h | 4h | 20h |
|---|---|---|---|---|---|
| LD-38 | 0% | 20% | 77% | 95% | 100% |
| LD-65 | 0% | 0% | 1% | 1% | 4% |
| Deruxtecan (MC-GGFG-Dxd) | 0% | 0% | 0% | 0% | 0% |

**Experimental Example 27: In vitro serum stability**

Sample incubation: 9D1-LD-38, 9D1-LD-65, 9D1-LD-73, 9D1-LD-74, and 9D1 Deuxtecan (9D1-GGFG-Dxd) (DAR value of which is 8, respectively) prepared from the aforementioned Experimental Examples were prepared with serum of human, cynomolgus monkey, and rat (mouse serum was added for preparation of 9D1-LD-38 and 9D1-LD-65) respectively, with a final concentration of 300μg/mL, 200μL/tube, three duplicated wells, and negative control (0.5% BSA-PBS) and standard were set. All samples were incubated in 37°C incubator for 0, 1 day, 3 days, 7 days, 14 days, and 21 days, followed by the determination of free toxin Exatecan.

Sample processing: 10.0μL of standard curve sample, quality control sample, test sample, double blank sample (10.0μL matrix sample), and zero concentration sample (10.0μL matrix sample) were taken respectively to add into strip tubes, then adding 25.0μL of internal standard working solution (the concentration of Exatecan-D5 was 3.00ng/mL, double blank samples were added with 25.0μL of internal standard diluent); adding 90.0μL of the mixture of methanol and acetonitrile (1:1, v/v) to each well to shake at 2500 rpm for 5 minutes; centrifuging at 4°C and 3800g for 5 minutes. 80.0μL of the supernatant was taken to dry with nitrogen at 40°C; adding 100μL of 0.1% FA in 15% MeOH to each well to mix well, and analyzing by LC-MS injection.

Sample analysis: under liquid chromatography (Nexera, Shimadzu) conditions, mobile phase A: 0.1% aqueous solution of formic acid; mobile phase B: 0.1% acetonitrile solution. Chromatographic column, ACE Excel 3μm 300-Å, 50 x 2.1 mm; flow rate: 0.6 mL/min; column temperature: 40°C; mass spectrometry analysis (Triple Quad 7500, AB/Science); data collection (Analyst, Version 1.6.2, Applied Biosystems /Sciex).

Data processing: the toxin content in ADC was calculated by formula $C_{toxin}$ = (molecular weight of the toxin * DAR)/ molecular weight of ADC * 300μg/mL, and the toxin release rate in serum = toxin content in serum/$C_{toxin}$. The curves were plotted by Graph pad Prism9 with incubation time as the x-axis and release rate as the y-axis.

Conclusion: in the study of in vitro serum stability, the curves on the change of toxin Exatecan release rate vs. time of are shown in Fig. 20. The toxin release of ADC increases with the prolongation of incubation time. Fig. 20 comprises Figs. 20A, 20B, 20C, 20D, 20E, and 20F. The toxin release rates of 9D1-LD-38 (Fig. 20A) in the serum of human, cynomolgus monkey, and rat after incubation at 37°C for 21 days are only 2.8%, 0.9%, and 3.3%, respectively (the peak release in rat serum is about 7 days after incubation). The Dxd release rates of 9D1-Deuxtecan (9D1-GGFG-Dxd) (Fig. 20B) after 21 days of incubation under the same conditions are 8.5% (human serum), 4.6% (cynomolgus monkey serum), and 5.3% (rat serum), respectively, which are equivalent to 3 times, 5 times, and 1.5 times the toxin releases of 9D1-LD-38 in the serum of corresponding species. The serum stability of 9D1-LD-65 (Fig. 20C), 9D1-LD-73 (Fig. 20D) and 9D1-LD74 (Fig. 20E) in human, cynomolgus monkey, and rat is similar to that of 9D1-LD-38. The highest release rate in human serum is around 3%, and the highest toxin release rate in cynomolgus monkey and rat serum is below 1.5%. The toxin release of 9D1-LD-38 in mouse serum is slightly higher than that of 9D1-LD-65 (Fig. 20F), but the difference is not significant, with the maximum release rates of both not exceeding 2% after 21 days.

**Experimental Example 28: Dynamic changes of ADC DAR values in rats**

Three male SD rats were selected and randomly divided into three groups. The test samples (i.e., 9D1-LD-38, 9D1-LD-65 and 9D1-Deruxtecan (9D1-GGFG-Dxd) prepared in the previous Experimental Examples, each with a DAR value of 8) were administered intravenously in a single dose at a dose of 10 mg/kg. Whole blood was collected from animals in each test group before administration, and 5 minutes, 1 hour, 5 hours, 24 hours (D2), 48 hours (D3), 120 hours (D6), 168 hours (D8), 240 hours (D11), 336 hours (D15), and 504 hours (D22) afteradministration; and the serum and plasma were collected separately to store at -60°C.

Method for detecting total antibody in serum: 10.0μL of standard curve sample, quality control sample, test sample, double blank sample (10.0μL matrix sample) and zero concentration sample (10.0μL matrix sample) were taken respectively to add to the strip tubes; adding 100μL of precipitant (isopropanol solution of 1% trichloroacetic acid) to each tube to shake at 2000 rpm for 5 minutes, centrifuging at 10°C and 500g for 5 minutes, then removing the supernatant; adding 100μL of ammonium bicarbonate solution (100mM, pH=8.0) to each tube to vortex and mix well to resuspend the precipitate, centrifuging at 10°C and 1000g for 5 minutes, then removing the supernatant; adding 50.0μL ammonium bicarbonate solution (100mM, pH=8.0) to each tube to vortex and mix well; adding 10.0μL of Sigma trypsin solution (Sigma, cat#: Y0002311) (5 mg/mL) to each tube, centrifuging at 3500 rpm for 30 seconds, vortexing at 1000 rpm for 3 minutes, then incubating for 1.5 hours in a shaking incubator at 60 °C, 550 rpm. After incubation, 20.0μL of 10% FA in ACN (termination agent) was added to each tube. For zero concentration sample, standard curve sample, quality control sample, and test sample, 10.0μL of internal standard working solution (TTPP-IS, human Fc universal peptide segment, 5000ng/ml) was added, respectively. For double blank sample, 10.0μL of internal standard preparation solvent (ammonium bicarbonate solution (100mM, pH=8.0)) was added. The above samples were centrifuged at 3800g for 10 seconds and vortexed at 1200 rmp for 2 minutes; centrifugijng at 3800g for 5 minutes, aking 50.0μL of supernatant, adding 50.0μL of water to each tube to mix well, and analyzing by LC-MS injection.

Method for detecting the conjugated toxin in serum: 10.0μL of standard curve sample, quality control sample, test sample, double blank sample (10.0μL matrix sample) and zero concentration sample (10.0μL matrix sample) was taken respectively to add to the strip tubes; adding 90μL of precipitant (1% trichloroacetic solution in isopropanol) to each tube to shake at 2000 rpm for 5 minutes, centrifuging at 10°C and 500g for 5 minutes, then removing the supernatant; adding 60μL of ammonium bicarbonate solution (100mM, pH=6.0) to each tube to vortex and mix well to resuspend the precipitate, centrifuging at 10°C and 1000g for 5 minutes, then removing the supernatant; adding 60.0μL ammonium bicarbonate solution (100mM, pH=8.0) to each tube to vortex and mix well; adding 60.0μL of papain (Adamas life, cat#: 88915D, 5 mg/mL) to each tube, centrifuging at 3500 rpm for 30 seconds, vortexing at 1000 rpm for 3 minutes, then incubating for 2 hours in a shaking incubator at 40°C, 550 rpm. After incubation, 20.0μL of 10% FA in ACN (termination agent) was added to each tube. For zero concentration sample, standard curve sample, quality control sample, and test sample, 10.0μL of internal standard working solution (TTPP-IS, human Fc universal peptide segment, 5000ng/ml) was added, respectively. For double blank sample, 10.0μL of internal standard preparation solvent (ammonium bicarbonate solution (100mM, pH=8.0)) was added. The above samples were centrifuged at 3800g for 10 seconds and vortexed at 1200 rmp for 2 minutes; them centrifugijng at 3800g for 5 minutes, and taking 50.0μL of supernatant, adding 50.0μL of water to each tube to mix well, and analyzing by LC-MS injection.

LC/MS method was used to determine the concentrations of total Ab (antibody) and conjugated toxin for each test sample in the serum samples. The DAR values of the conjugated antibody at various time points for an animal were calculated by the following formula: DAR= concentration of the conjugated toxin (mmol/L)/ concentration of the total antibody (mmol/L).

Conclusion: the curves for DAR values of ADC vs. time in serum of the animals in each test sample group are shown in Fig. 21: under the condition of single intravenous injection to rats at a dose of 10 mg/kg, the DAR values of 9D1-LD-38, 9D1-LD-65, and 9D1 Deuxtecan (9D1-GGFG-Dxd) are gradually decreased over time. The DAR value curves of 9D1-LD-65 and 9D1-LD-38 are almost consistent, and can be maintained at a high level of DAR values. At 48 hours after administration, the DAR value is maintained at about 7, and by day 21 it remains at about 3-4. The DAR values of 9D1-Deuxtecan (9D1-GGFG-Dxd) show a rapid decline trend. At 48 hours after administration, the DAR value decreases from about 7.5 in the initial administration stage to 5.5, and by day 21 it decreases to about 1.6. The above results indicate that after in vivo administration, compared to 9D1-Deuxtecan (9D1-GGFG-Dxd), 9D1-LD-38 and 9D1-LD-65 are more stable and exhibit higher DAR values.

### Experimental Example 29: In vivo tissue distribution in tumor bearing mice

Modeling and administration for tumor bearing mouse: male C57BL/6 mice (6 week old) were subcutaneously inoculated with MC38-hClaudin18.2 cells (1 x $10^6$ cells in 100μL PBS) on the ventral side. When the tumor volumes of the mice grew to about 300 $mm^3$, the mice were divided into groups to be administered via tail vein injection of 9D1-LD-38, 9D1-LD-65, 9D1-Deuxtecan (9D1-GGFG-Dxd) prepared in the previous Experimental Examples, and PBS, each of them had a DAR valu of 8 and a dose of 10mg/kg, respectively.

Sample collection: blood and tumor tissue were collected at 24 hours, 72 hours, and 168 hours after administration, with 3 mice sampled at each time point. Whole blood was collected from the orbit of the tumor bearing mice to put into an anticoagulant tube containing EDTA-K2 anticoagulant, centrifuging within 30 minutes to obtain plasma. Mice were euthanized by $CO_2$ suffocation method, then collecting the tumors. Plasma and tumor tissues were stored at -80°C.

Determination of free toxins in plasma: 10.0μL of standard curve sample, quality control sample, test sample, double blank sample (10.0μL matrix sample) and zero concentration sample (10.0μL matrix sample) were taken respectively to add to the strip tubes; adding 25.0μL of internal standard working solution (the concentration of Exatecan-D5 is 3.00 ng/mL, and 25.0μL of internal standard diluent is added to the double blank sample); adding 90.0μL of a mixture of methanol and acetonitrile (1:1, v/v) to each tube to shake at 2500 rpm for 5 minutes; centrifuging at 4°C and 3800 g for 5 minutes. 80.0μL of supernatant was taken to dry with nitrogen at 40°C, addin 100μL of 0.1% FA in 15% MeOH to each tube to mix well, and analyzing by LC-MS injection.

Determination of total toxins in blood (conjugated toxin+free toxin): 10.0μL of standard curve sample, quality control sample, test sample, double blank sample (10.0μL matrix sample) and zero concentration sample (10.0μL matrix sample) were taken respectively to add to the strip tubes; adding 10.0μL of papain (Adamas life, cat#: 88915D, 5 mg/ml) to each tube to centrifuge at 3500 rpm for 1 minute; the samples were incubated for 6-8 hours in a shaking incubator at 40 °C and 550rpm; add 1.00μL of 1% FA in H2O to each tube to centrifuge at 3500 rpm for 1 minute, incubating at 40 °C and 550 rpm for another 30 minutes. For zero concentration sample, standard curve sample, quality control sample, and test sample, 10.0μL of internal standard working solution (Exatecan-D5, 1000ng/ml) was added; and for double blank sample, 10.0μL of internal standard preparation solvent was added; then centrifuging the above samples at 3800 g for 10 seconds and vortexing at 1200 rmp for 2 minutes; 100μL of pre-cooled ACN was added to each tube to shake at 2500 rpm for 5 minutes, then centrifuging at 3500 g for 5 minutes. 30.0μL of supernatant was taken, adding 90.0μL of 0.5% FA in $H_2O$ to each tube, vortexing for 3 minutes, and analyzing by LCMS injection.

Determination of free toxin in tumor tissue: homogenization treatment of tumor tissue (administration group and blank PBS group): the tumor tissue was weighed, adding 10 times the volume of 50% methanol aqueous solution to perform homogenization treatment. The homogenization of blank PBS group was used as the matrix for preparing the standard curve and quality control samples. 10.0μL of standard curve sample, quality control sample, test sample, double blank sample (10.0μL matrix sample) and zero concentration sample (10.0μL matrix sample) were taken respectively to add to the strip tubes; then adding 25.0μL of internal standard working solution (the concentration of Exatecan-D5 is 3.00 ng/mL, and 25.0μL of internal standard diluent was added to the double blank sample); adding 90.0μL of a misture of methanol and acetonitrile (1:1, v/v) to each tube to shake at 2500 rpm for 5 minutes; centrifuging at 4 °C and 3800 g for 5 minutes; 80.0μL of supernatant was taken to dry with nitrogen at 40 °C; 100μL of 0.1% FA in 15% MeOH was added to each tube, vortexing for 3 minutes, and analyzing by LCMS injection.

Determination of total toxins (conjugated toxin+free toxin) in tumor tissue: 10.0μL of standard curve sample, quality control sample, test sample, double blank sample (10.0μL matrix sample) and zero concentration sample (10.0μL matrix sample) were taken respectively to add to the strip tubes; adding 10.0μL of papain (Adamas life, cat#: 88915D 5 mg/ml) to each tube to centrifuge at 3500 rpm for 1 minute; incubating for 6-8 hours in shaking incubator at 40°C and 550rpm; then adding 1.00μL of 1% FA in $H_2O$ to each tube to centrifuge at 3500 rpm for 1 minute, incubating for another 30 minutes at 40°C and 550 rpm. For zero concentration sample, standard curve sample, quality control sample, and test sample, 10.0μL of internal standard working solution (Exatecan-D5, 1000ng/ml) was added. For double blank sample, 10.0μL of internal standard preparation solvent was added; then centrifuging the above samples at 3800g for 10 seconds and vortexing at 1200 rmp for 2 minutes; 100μL of pre-cooled ACN was added to each tube to shake at 2500 rpm for 5 minutes; centrifuging 3500 g for 5 minutes; 30.0μL of supernatant was taken, adding 90.0μL of 0.5% FA in $H_2O$ to each tube, vortexing for 3 minutes, and analyzing by LCMS injection.

Sample analysis: under liquid chromatography (Nexera, Shimadzu) conditions, mobile phase A: 0.1% aqueous solution of formic acid; mobile phase B: 0.1% acetonitrile solution. Chromatographic column, ACE Excel 3μm 300 Å, 50 x 2.1 mm; flow rate: 0.6 mL/min; column temperature: 40°C; mass spectrometry analysis (Triple Quad 7500, AB/Science); data collection (Analyst, Version 1.6.2, Applied Biosystems / Sciex). GraphPad Prism9 software was used to input the time points of sample collection and corresponding toxin contents to plot toxin content curves in plasma and tumor tissue. The results are shown in Figs. 22 and 23; Fig. 22 comprises Figs. 22A and 22B, wherein Fig. 22A shows the total toxin (conjugated toxin + free toxin) in the plasma of tumor bearing mice, and Fig. 22B shows the content of free toxins in the plasma of tumor bearing mice. Fig. 23 comprises Figs. 23A and 23B, wherein Fig. 23A shows the total toxins (conjugated toxin + free toxin) in the tissues of tumor bearing mice, and Fig. 23B shows the content of free toxin in the tissues of tumor bearing mice.

Conclusion: it can be seen from Fig. 22, the trend of changes in free and total toxins in the plasma of animals in each test group is basically consistent, reaching the highest at 24 hours and then slowly decreasing thereafter. The total toxin contents (Fig. 22A) of the 9D1-LD-38 and 9D1-LD-65 groups are basically the same, with a content of 3000-4000 ng/mL at 24 hours and a decrease of approximately 70% -80% after 7 days. The total toxin content in the plasma of 9D1-Deuxtecan (9D1-GGFG-Dxd) at each time point is only about half (1/2) of that of 9D1-LD-38 and 9D1 LD-65. The contents and change trends of free toxins in the plasma of animals in the 9D1-LD-38, 9D1-LD-65, and 9D1-Deuxtecan (9D1-GGFG-Dxd) groups are also basically consistent (Fig. 22B), with the highest level at 24 hours and a slow decrease thereafter. Compared to the total toxin contents in plasma, the free toxin contents are extremely low, and their concentrations at 24 hours are lower than 0.4 ng/mL, accounting for 0.01%-0.02% of the total toxin content, and much lower than the IC50 value for in vitro target cell killing (3 ng/mL). It indicates that the toxins of the three ADC drugs are less prone to be detached in plasma and are relatively stable.

It can be seen from Fig. 23, at 24 hours after administration, the total toxin level in tumor tissues of each treatment group is the highest (Fig. 23A), and after 168 hours it rapidly decreases to about 1/3 of the content at 24hours. Particularly, the enrichment levels of 9D1-LD38 and 9D1-LD-65 in tumor tissues are significantly higher than those in 9D1-Deuxtecan (9D1-GGFG-Dxd). At 24 hours after administration, the level of free toxin in tumor tissues of each treatment group is the highest (Fig. 23B), gradually decreasing from 72 hours and then maintaining until 168 hours. The levels of free toxins in tumor tissues of 9D1-LD-38 and 9D1-LD-65 are significantly higher than those of 9D1-Deuxtecan (9D1-GGFG-Dxd) (2-5 times) throughout the entire time period, wherein that of 9D1-LD-38 is the optimal. The results of the total toxin (conjugated toxin+free toxin) and free toxin content in the tumor tissue of the tumor bearing mice mentioned above indicate that, 9D1-LD-38 and 9D1-LD-65 have better osmotic enrichment in the tumor tissue, and the released levels of free toxins are also higher. Therefore, compared to 9D1-Deuxtecan (9D1-GGFG-Dxd), 9D1-LD-38 and 9D1-LD-65 can produce better tumor treatment effects.

**Experimental Example 30: Pharmacodynamics of p-gp mediated drug-resistant tumor bearing mouse model**

Modeling and administration of tumor bearing mice: BALB/c Nude mice (female, 5-6 week old) were subcutaneously inoculated with MC38 hClaudin18.2 cells ($1\times10^6$ cells in 100 PBS) on the ventral side. When the tumor volume

increased to about 120 $mm^3$, the mice were randomly divided into groups and administered according to Table 10. The tumor and body weight were measured twice a week to calculate the tumor volumes (TV, $mm^3$) and plot the tumor growth curves. TV ($mm^3$)=($L\times W^2$)/2, wherein L represents tumor length, and W represents tumor width. GraphPad Prism software (GraphPad Software, San Diego, California) and XY mode were selected to plot the tumor growth curves and weight curves of mice, and the tumor volumes and weights were represented as mean ± standard error. The results are shown in Fig. 24, which comprises Figs. 24A and 24B, wherein Fig. 24A shows the mouse tumor growth curves, and Fig. 24B shows the mouse body weight curves.

Table 10: Grouping and administration regimen of p-gp mediated drug-resistant tumor bearing mouse model

| Groups | Animal number | Administrated drugs | Dosage (mg/kg) | Administration route | Administration period |
|---|---|---|---|---|---|
| *1* | 5 | 9D1-LD-38 | 10 | i.p. | QW×2 doses |
| *2* | 5 | 9D1-Deruxtecan | 10 | i.p. | QW×2 doses |
| | | (9D1-GGFG-Dxd) | | | |
| *3* | 5 | Tariquidar | 10 | p.o. | QD×10 doses (5on+2off/-cycle,×2) |
| *4* | 5 | 9D1-VC-MMAE | 10+15 | i.p. | QW×2 doses |
| *5* | 5 | 9D1-LD-38 + Tariquidar | 10+15 | i.p.+p.o. | QW×2 doses + QD×10 doses (5on+2off/cycle, ×2) |
| *6* | 5 | 9D1-Deruxtecan (9D1-GGFG-Dxd) +Tariquidar | 10+15 | i.p.+ p.o. | QW×2 doses + QD×10 doses (5on+2off/cycle, ×2) |
| *7* | 5 | 9D1-vc-PAB-MMAE+ Tariquidar | 10+15 | i.p.+ p.o. | QW×2 doses + QD×10 doses (5on+2off/cycle, ×2) |
| *8* | 5 | PBS | -- | i.p.+ p.o. | QW×2 doses + QD×10 doses (5on+2off/cycle, ×2) |
| Note: i.p., intraperitoneal injection, intraperitoneal administration; p.o., Peros, oral administration; QW, once a week; QD, once a day; 5on+2off/cycle , 7 days as one period, 5 days for administration and 2 days for rest; 9D1-LD-38 and 9D1-Deruxtecan (9D1-GGFG-Dxd), DAR=8; 9D1-vd-PAB-MMAE, DAR=4; Tariquidar, p-gp inhibitor (Cat#: HY-10550/CS-0722; Lot: 242595). | | | | | |

Conclusion: P-glycoprotein (P-gp) transmembrane transporter protein leads to chemotherapy resistance through active drug efflux, which is one of the main reasons for treatment failure of chemotherapy. The mouse MC38 rectal cancer model has high expression of p-gp, and is insensitive to many chemotherapy drugs. The tumor therapeutic effect of ADC drug monotherapy or combination with p-gp inhibitor Tariquidar on MC38-hClaudin18.2 tumor bearing mouse model is shown in Fig. 24A. Tariquidar monotherapy has no tumor inhibitory effect compared to PBS control group; the tumor growth in the 9D1-vc-PAB-MMAE group was rapid, and the tumor growth curve showes a weak inhibitory effect as compared to the PBS control group (the tumor volumes are about 2600 $mm^3$ and 1900 $mm^3$ on day 17 after administration, respectively). The combination of 9D1-vc-PAB-MMAE and Tariquidar can greatly inhibit tumor growth, leading to tumor shrinkage to almost disappearance; and 9D1-Deruxtecan (9D1-GGFG-Dxd) can significantly inhibit tumor growth. The tumor inhibitory effect of 9D1-Deruxtecan (9D1-GGFG-Dxd)+Tariquidar is slightly enhanced as compared to 9D1-Deruxtecan (9D1-GGFG-Dxd) monotherapy, but none of them can cause tumor shrinkage (the tumor volumes are about 1000 $mm^3$ and 660 $mm^3$ on day 17 after administration, respectively). Both 9D1-LD-38 monotherapy and 9D1-LD-38+Tariquidar can strongly inhibit tumor growth and cause tumor shrinkage to almost disappear. The weight of the mice remains within the normal fluctuation range after administration, and no treatment induced weight loss was observed (Fig. 24B). The above results indicate that, compared to 9D1-vc-PAB-MMAE and 9D1-Deuxtecan (9D1-GGFG-Dxd), 9D1-LD-38 can resist drug-resistance in tumor caused by p-gp, and single drug administration can produce good tumor treatment effects.

**Experimental Example 31: The anti-tumor effect of ICI combined administration on tumor bearing mice**

Modeling and administration of tumor bearing mice: 4T1-hClaudin18.2 cells were cultured in 1640 complete medium (supplemented with 10% FBS, and 2μg/mL puromycin, Gibco,Cat#: A1113803) to subject to adherent culture in square bottle at 37°C in 5% $CO_2$ incubator. When the cell confluence reached about 80%, the cells were digested with

TRYPLE EXPRESS trypsin (Gibco, Cat#: 12605028) and then 2.5×$10^7$ cells were collected by centrifugation, washing once with PBS; after discarding the supernatant, the cells were resuspended with PBS containing 50% Matrigel (Corning, Cat#: 326234), with a total volume of 5 ml. The cells were inoculated into the skin of the right side of the abdomen of BALB/c female mice (female, 5-6 week old) at 100μL cell suspension/mouse. When the tumors grew to a volume of about 150 $mm^3$, the mice were randomly divided into groups according to Table 11 for administration. The sizes of the tumors were measured twice a week by vernier caliper. TV ($mm^3$)=(L×$W^2$)/2, wherein L represents tumor length, and W represents tumor width. GraphPad Prism software (GraphPad Software, San Diego, California) and XY mode were selected to plot the mouse tumor growth curves, and the tumor volumes were represented as the mean ± standard error.

Table 11: Grouping and administration regimen of ICI combined administration in tumor bearing mouse model

| Groups | Animal number | Drugs | Administration dosage (mg/kg) | Administration route | Administration frequency | Administration times |
|---|---|---|---|---|---|---|
| G1 | 5 | 9D1-LD-38 | 3 | i.p. | QW | 2 |
| G2 | 5 | αPD-1 | 10 | i.p. | BIW | 4 |
| G3 | 5 | 9D1-LD-38+αPD-1 | 3+10 | i.p. | QW+BIW | 2+4 |
| G4 | 5 | PBS | - | i.p. | QW | 2 |
| Note: i.p., intraperitoneal injection, intraperitoneal administration; QW, administrated once a week; BIW, administrated twice a week; αPD-1, anti-mouse PD-1 antibody (InVivoMAb, Cat#: BE0146). | | | | | | |

Conclusion: immune checkpoint inhibitors (ICI) drugs have become one of the cornerstones of tumor treatment. The combined therapeutic effect of 9D1-LD-38 with anti-mouse PD1 drugαPD-1 in a 4T1-hClaudin18.2 tumor bearing mouse model of immune healthy BALB/c mice is shown in Fig. 25. Compared with the PBS control group, both 9D1-LD38 monotherapy and αPD-1 monotherapy can produce partial tumor growth inhibition (the tumor volumes on day 14 after administration areabout 850 $mm^3$, 480 $mm^3$, and 426 $mm^3$, respectively). The combined administration of 9D1-LD38+αPD-1 can greatly inhibit tumor growth and even produce significant tumor regression (the tumor volume on day 10 after administration is about 70 $mm^3$).

**Experimental Example 32: The anti-tumor effect of bystander killing on tumor bearing mice**

Modeling and administration of tumor bearing mice: MC38 hClaudin18.2 cells and MC38-luc (luciferase) cells were cultured in DMEM medium containing inactivated 10% fetal bovine serum (Excel Bio, cat#: 12A222) and 1 x penicillin/streptomycin (Solarbio, cat#: 20221231) respectively at 37°C in 5% $CO_2$ incubator. Tumor cells in logarithmic growth phase were used for in vivo tumor inoculation. MC38-hClaudin18.2 cells were mixed with MC38 luc cells (in a ratio of 1:1), then resuspending with PBS, adjusting the cell concentration to 5 × $10^6$/ml, and inoculating subcutaneously on the right side of the abdomen of BALB/c node mice at 100 μL/mouse. When the tumors grew to about 120$mm^3$, the mice were divided into 4 groups and administered (the first administration day was recorded as PG-D0), with 5 mice in each group. The specific administration regimens are shown in the table below:

Table 12: Grouping and administration regimen of bystander killing in tumor bearing mouse model

| Groups | Animal number | Treatment | Dosage (mg/kg)* | Administration route | Administration frequency |
|---|---|---|---|---|---|
| G1 | 5 | 9D1-LD-38 | 10 | i.V. | QW×2 |
| G2 | 5 | ISO-LD-38 | 10 | i.v. | QW×2 |
| G3 | 5 | 9D1-Deruxtecan (9D1-GGFG-Dxd) | 10 | i.v. | QW×2 |
| G4 | 5 | Control (PBS) | - | i.v. | QW×2 |
| Note: *, the administration volume is 10 μl/g body weight;<br>i.v.: intravenous injection; QW × 2: administrated once a week for a total of 2 times. | | | | | |

The tumor volumes were measured 2-3 times a week by a vernier caliper, measuring the length diameter and short diameter of a tumor. The formula for calculating the volume is: volume (TV) = 0.5 x length diameter x short diameter x

short diameter. By intraperitoneal injection of fluorescein substrate (15mg/ml, 10μl/g) into mice, and anesthesia with isoflurane, the luminescent signals were collected by small animal live imaging device (IVIS Lumina Series III, PerkinElmer).

Conclusion: bystander killing is the ability of ADC drugs to release toxins after being internalized by target positive tumor cells, wherein the toxins may kill adjacent target negative tumor cells through transmembrane action. Bystander killing is considered one of the reasons why ADC can produce good therapeutic effects against tumors with heterogeneous target expression. This Experimental Example generated a tumor bearing mouse model with heterogeneous target expression by co-inoculating target positive (MC38-hClaudin18.2) cells and target negative (MC38-luc) cells on mice, and validated the bystander killing of the test ADC drugs. The results are shown in Fig. 26, which comprises Figs. 26A and 26B. Fig. 26A shows the change of tumor volumes in mice after administration, and Fig. 26B shows the bioluminescence signal of target negative tumor cells in tumor bearing mice on day 3 and day 7 day after administration. It can be seen from Fig. 26A that, compared to the PBS control group (Contral), ISO(Isotype)-LD-38 group grows faster and only produce weaker tumor inhibitory effects (on day 16 after tumor inoculation, the tumor volumes are about 2700 $mm^3$ and 1700 $mm^3$, respectively); 9D1-Deuxtecan (9D1-GGFG-Dxd) can partially inhibit tumor growth (on day 16 after tumor inoculation, the tumor volume was about 1000 $mm^3$), but the tumor volume continues to increase; 9D1-LD-38 can greatly inhibit tumor growth, and on day 16, the tumor is almost completely cleared, indicating that 9D1-LD-38 can not only kill target positive MC38-hClaudin18.2 cells, but also target negative MC38-luc cells. The bioluminescence signals (Fig. 26B) of target negative tumor cells in tumor bearing mice collected on day 3 (PG-3, 3rd day after inoculation) and day 7 (PG-7, 12th day after inoculation) after grouping clearly show that, compared to the control group and the ISO-LD38 and 9D1-Deuxtecan (9D1-GGFG-Dxd) treatment groups, 9D1-LD38 is able to effectively kill target negative tumor cells and cause significant reduction or disappearance of the bioluminescence signals.

Although the embodiments of the present application have been described in conjunction with the accompanying drawings, the present application is not limited to the above specific embodiments and application field. The above specific embodiments are only illustrative and guiding, and not restrictive. Under the inspiration of this specification and without departing from the scope of the claims of this application, a person skilled in the art can also make many variants, all of which fall in the protection scope of this application.

## Claims

1. A compound represented by Formula (1), or a tautomer, mesomer, raceme, enantiomer or diastereomer thereof, or mixture thereof, or a pharmaceutically acceptable salt, prodrug or solvate thereof, wherein Formula (1) has the following structural formula:

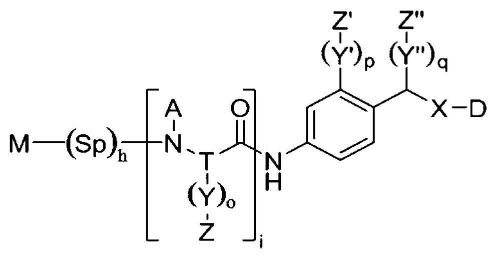

Formula (1);

M is a spacer;
Sp is $-L_a-Q_a-$;
A is selected from absent, hydrogen or alkyl;
T represents a straight or branched ($C_1$-$C_8$) trivalent alkyl group;
Y, Y' and Y" are each independently $-L_b-Q_b-$; $L_a$ and $L_b$ are each independently selected from absent, alkylene, alkenylene, alkynylene, alicyclylene, heteroalicyclylene, arylene, heteroarylene or polyethylene glycol group, wherein the alkylene, alkenylene, alkynylene, alicyclylene, heteroalicyclylene, arylene, heteroarylene or polyethylene glycol group is optionally substituted by a substituent;
$Q_a$, and $Q_b$ are each independently selected from absent, -C(=O)NR-, -C(=O)O-, -OC(=O)NR-, $-S(=O)_2NR-$, -NRC(=O)-, -OC(=O)-, -NRC(=O)O-, $-NRS(=O)_2-$, -NRC(=O)NR-, -NHC-(=NH)NH-, -C(=O)-, -OC(=O)O-, -O-, -NR-, -S-, $S(=O)_2-$, S(=O)-, or -Se-, wherein R is selected from hydrogen or optionally substituted alkyl;
Z is selected from absent, hydrogen, optionally substituted alkyl or a sulfonate inner salt;
Z', and Z" are each independently selected from absent, hydrogen, optionally substituted alkyl, a sulfonate inner salt or a phosphate inner salt;

at least one of Z, Z' and Z" is a sulfonate inner salt or a phosphate inner salt;
X comprises a self-immolative unit or is absent;
D comprises a drug molecule;
i is an integer equal to or greater than 1;
h is an integer from 1 to 6; and
o, p and q are each independently an integer from 0 to 5.

**2.** The compound, or a tautomer, mesomer, raceme, enantiomer or diastereomer thereof, or mixture thereof, or a pharmaceutically acceptable salt, prodrug or solvate thereof according to claim 1, wherein Formula (1) is Formula (1-1):

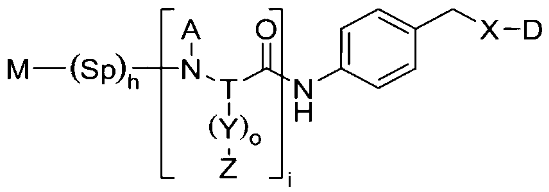

Formula (1-1),

wherein M, Sp, A, T, Y, h, o, i, X, D, and Z are defined as in claim 1; and
at least one of the i Zs is a sulfonate inner salt.

**3.** The compound, or a tautomer, mesomer, raceme, enantiomer or diastereomer thereof, or mixture thereof, or a pharmaceutically acceptable salt, prodrug or solvate thereof according to claim 2, wherein the Formula (1-1) is Formula (1-1-1):

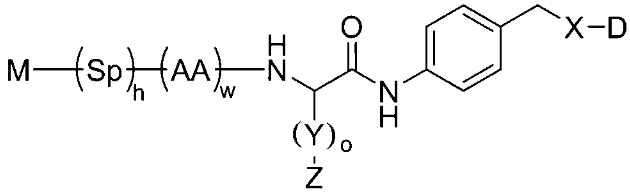

· Formula (1-1-1),

wherein AA is an amino acid residue, and W is an integer equal to or greater than 1.

**4.** The compound, or a tautomer, mesomer, raceme, enantiomer or diastereomer thereof, or mixture thereof, or a pharmaceutically acceptable salt, prodrug or solvate thereof according to claim 1, wherein Formula (1) is Formula (1-2):

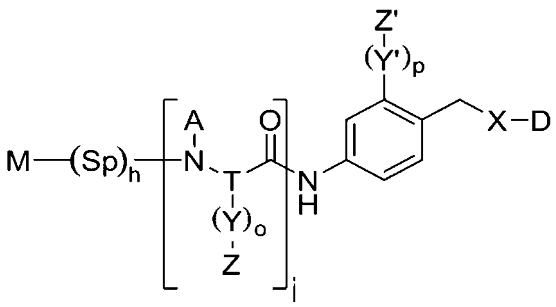

Formula (1-2),

wherein M, Sp, A, T, Y, Z, o, p, i, h, X, Y', and D are defined as in claim 1; and Z' is a sulfonate inner salt or a phosphate inner salt.

**5.** The compound, or a tautomer, mesomer, raceme, enantiomer or diastereomer thereof, or mixture thereof, or a pharmaceutically acceptable salt, prodrug or solvate thereof according to claim 4, wherein Formula (1) is Formula (1-2-1):

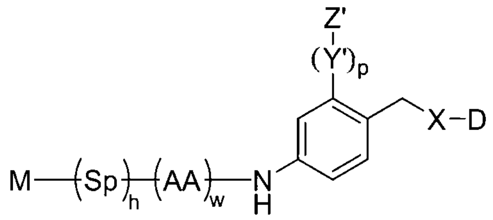

Formula (1-2-1),

wherein AA is an amino acid residue, and W is an integer equal to or greater than 1.

**6.** The compound, or a tautomer, mesomer, raceme, enantiomer or diastereomer thereof, or mixture thereof, or a pharmaceutically acceptable salt, prodrug or solvate thereof according to claim 1, wherein Formula (1) is Formula (1-3):

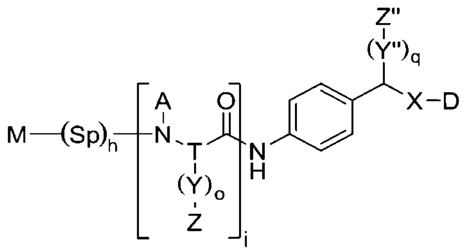

Formula (1-3),

wherein M, Sp, A, Y, T, Z, o, i, h, q, X, Y", and D are defined as in claim 1; and Z" is a sulfonate inner salt or a phosphate inner salt.

**7.** The compound, or a tautomer, mesomer, raceme, enantiomer or diastereomer thereof, or mixture thereof, or a pharmaceutically acceptable salt, prodrug or solvate thereof according to claim 6, wherein Formula (1) is Formula (1-3-1):

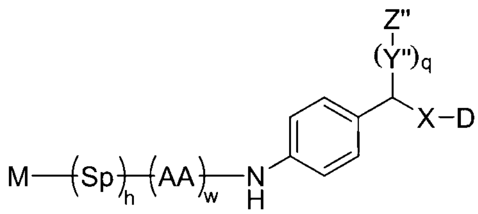

Formula (1-3-1),

wherein AA is an amino acid residue, and W is an integer equal to or greater than 1.

**8.** The compound, or a tautomer, mesomer, raceme, enantiomer or diastereomer thereof, or mixture thereof, or a pharmaceutically acceptable salt, prodrug or solvate thereof according to any one of claims 1-7, wherein when A and Z are absent, N, Y and T form a ring to have a structural formula of:

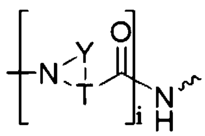

,

preferably

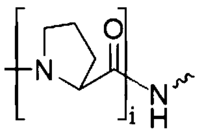.

**9.** The compound, or a tautomer, mesomer, raceme, enantiomer or diastereomer thereof, or mixture thereof, or a pharmaceutically acceptable salt, prodrug or solvate thereof according to any one of claims 3, 5 and 7, wherein the amino acid(s) is one or more selected from the group consisting of: alanine, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, and valine.

**10.** The compound, or a tautomer, mesomer, raceme, enantiomer or diastereomer thereof, or mixture thereof, or a pharmaceutically acceptable salt, prodrug or solvate thereof according to any one of claims 3, 5 and 7, wherein the amino acid(s) is one or more selected from the group consisting of: valine, glutamic acid, citrulline, lysine, alanine, phenylalanine, arginine, glutamine, asparagine, and glycine.

**11.** The compound, or a tautomer, mesomer, raceme, enantiomer or diastereomer thereof, or mixture thereof, or a pharmaceutically acceptable salt, prodrug or solvate thereof according to any one of claims 3, 5 and 7, wherein the amino acid(s) is one or more selected from the group consisting of: valine, citrulline, alanine, phenylalanine, glutamine, and glycine.

**12.** The compound, or a tautomer, mesomer, raceme, enantiomer or diastereomer thereof, or mixture thereof, or a pharmaceutically acceptable salt, prodrug or solvate thereof according to any one of claims 1-11, wherein M is selected from $N_3$, SH, $ONH_2$, $NH_2$, $CO_2H$, (alkyl)-CO-, HCO-, $BrCH_2$-, $ICH_2$-,

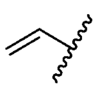, 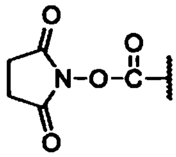 、 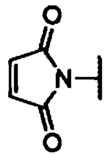, 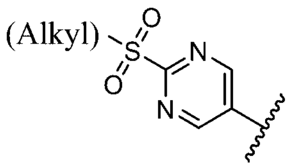, 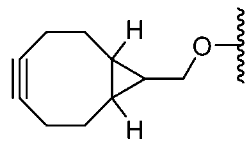

or

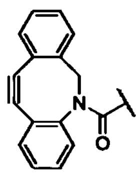.

**13.** The compound, or a tautomer, mesomer, raceme, enantiomer or diastereomer thereof, or mixture thereof, or a pharmaceutically acceptable salt, prodrug or solvate thereof according to any one of claims 1-12, wherein M is

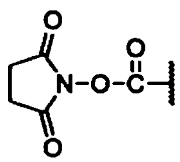 、 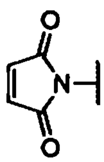, 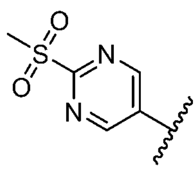 or 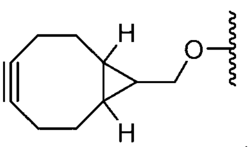.

**14.** The compound, or a tautomer, mesomer, raceme, enantiomer or diastereomer thereof, or mixture thereof, or a pharmaceutically acceptable salt, prodrug or solvate thereof according to any one of claims 1-13, wherein M is

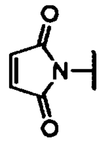.

**15.** The compound, or a tautomer, mesomer, raceme, enantiomer or diastereomer thereof, or mixture thereof, or a pharmaceutically acceptable salt, prodrug or solvate thereof according to any one of claims 1-14, wherein A is hydrogen, methyl, ethyl or propyl.

**16.** The compound, or a tautomer, mesomer, raceme, enantiomer or diastereomer thereof, or mixture thereof, or a pharmaceutically acceptable salt, prodrug or solvate thereof according to any one of claims 1-15, wherein A is hydrogen.

**17.** The compound, or a tautomer, mesomer, raceme, enantiomer or diastereomer thereof, or mixture thereof, or a pharmaceutically acceptable salt, prodrug or solvate thereof according to any one of claims 1-16, wherein the T is any one selected from the group consisting of:

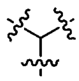, 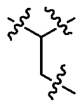, 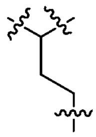, and 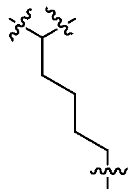.

**18.** The compound, or a tautomer, mesomer, raceme, enantiomer or diastereomer thereof, or mixture thereof, or a pharmaceutically acceptable salt, prodrug or solvate thereof according to any one of claims 1-17, wherein the T is

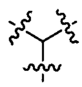.

**19.** The compound, or a tautomer, mesomer, raceme, enantiomer or diastereomer thereof, or mixture thereof, or a pharmaceutically acceptable salt, prodrug or solvate thereof according to any one of claims 1-18, wherein $L_a$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**20.** The compound, or a tautomer, mesomer, raceme, enantiomer or diastereomer thereof, or mixture thereof, or a pharmaceutically acceptable salt, prodrug or solvate thereof according to any one of claims 1-19, wherein $L_a$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**21.** The compound, or a tautomer, mesomer, raceme, enantiomer or diastereomer thereof, or mixture thereof, or a pharmaceutically acceptable salt, prodrug or solvate thereof according to any one of claims 1-20, wherein $L_a$ is one, two, three or more selected from the group consisting of: $C_1$-$C_6$ linear or branched alkylene, $C_2$-$C_6$ linear or branched alkenylene, or $C_2$-$C_6$ linear or branched alkynylene, and polyethylene glycol group; and wherein the alkylene, alkenylene, or alkynylene is optionally substituted by a substituent.

**22.** The compound, or a tautomer, mesomer, raceme, enantiomer or diastereomer thereof, or mixture thereof, or a pharmaceutically acceptable salt, prodrug or solvate thereof according to any one of claims 1-21, wherein $L_a$ is one, two, three or more selected from the group consisting of: $C_1$-$C_6$ linear or branched alkylene, and polyethylene glycol group; and wherein the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$; and n is an integer equal to or greater than 1.

**23.** The compound, or a tautomer, mesomer, raceme, enantiomer or diastereomer thereof, or mixture thereof, or a pharmaceutically acceptable salt, prodrug or solvate thereof according to any one of claims 1-22, wherein $L_a$ is one, two, three or more selected from the group consisting of: ethylidene, linear propylidene, linear butylidene, linear pentylidene, and $-(CH_2CH_2O)_nCH_2CH_2-$; and wherein n is 2, 3, 4, 5, 6, 7 or 8.

**24.** The compound, or a tautomer, mesomer, raceme, enantiomer or diastereomer thereof, or mixture thereof, or a pharmaceutically acceptable salt, prodrug or solvate thereof according to any one of claims 1-23, wherein $L_a$ is ethylidene, and/or $-(CH_2CH_2O)_4CH_2CH_2-$ or pentylidene.

**25.** The compound, or a tautomer, mesomer, raceme, enantiomer or diastereomer thereof, or mixture thereof, or a pharmaceutically acceptable salt, prodrug or solvate thereof according to any one of claims 1-24, wherein $L_b$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, or heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**26.** The compound, or a tautomer, mesomer, raceme, enantiomer or diastereomer thereof, or mixture thereof, or a pharmaceutically acceptable salt, prodrug or solvate thereof according to any one of claims 1-25, wherein $L_b$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**27.** The compound, or a tautomer, mesomer, raceme, enantiomer or diastereomer thereof, or mixture thereof, or a pharmaceutically acceptable salt, prodrug or solvate thereof according to any one of claims 1-26, wherein $L_b$ is selected from $C_1$-$C_6$ linear or branched alkylene, $C_2$-$C_6$ linear or branched alkenylene, or $C_2$-$C_6$ linear or branched alkynylene, or polyethylene glycol group; the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; and the alkylene, alkenylene, or alkynylene is optionally substituted by a substituent.

**28.** The compound, or a tautomer, mesomer, raceme, enantiomer or diastereomer thereof, or mixture thereof, or a pharmaceutically acceptable salt, prodrug or solvate thereof according to any one of claims 1-27, wherein $L_b$ is one, two, three or more selected from the group consisting of: $C_1$-$C_6$ linear or branched alkylene, and $C_6$-$C_{10}$ arylene.

**29.** The compound, or a tautomer, mesomer, raceme, enantiomer or diastereomer thereof, or mixture thereof, or a pharmaceutically acceptable salt, prodrug or solvate thereof according to any one of claims 1-28, wherein $L_b$ is one, two, three or more selected from the group consisting of: methylene, ethylene, n-propylidene, isopropylidene, n-butylidene, isobutylidene, sec-butylidene, tert-butylidene, and benzylidene.

**30.** The compound, or a tautomer, mesomer, raceme, enantiomer or diastereomer thereof, or mixture thereof, or a pharmaceutically acceptable salt, prodrug or solvate thereof according to any one of claims 1-29, wherein
$Q_a$ is one, two, three or more selected from the group consisting of: -C(=O)NR-, -NR-, -C(=O)O-, -OC(=O)NR-, -NRC(=O)-, -OC(=O)-, -NRC(=O)O-, -NRC(=O)NR-, NHC-(=NH)NH-, -C(=O)-, and -OC(=O)O-; and wherein R is selected from hydrogen, or optionally substituted $C_1$-$C_6$ linear or branched alkyl.

**31.** The compound, or a tautomer, mesomer, raceme, enantiomer or diastereomer thereof, or mixture thereof, or a pharmaceutically acceptable salt, prodrug or solvate thereof according to claim 30, wherein $Q_a$ is one, two, three or more selected from the group consisting of: -C(=O)-, -NR-, -C(=O)NR-, -NRC(=O)NR-, -OC(=O)O-, -OC(=O)NR-, -NRC(=O)- and -OC(=O)-; and wherein R is selected from hydrogen, or optionally substituted $C_1$-$C_6$ linear or branched alkyl.

**32.** The compound, or a tautomer, mesomer, raceme, enantiomer or diastereomer thereof, or mixture thereof, or a pharmaceutically acceptable salt, prodrug or solvate thereof according to claim 30, wherein $Q_a$ is one, two, three or more selected from the group consisting of: -C(=O)-, -NR-, -NRC(=O)-, and -(C=O)NR-; and wherein R is selected from hydrogen, or optionally substituted $C_1$-$C_6$ linear or branched alkyl.

**33.** The compound, or a tautomer, mesomer, raceme, enantiomer or diastereomer thereof, or mixture thereof, or a pharmaceutically acceptable salt, prodrug or solvate thereof according to claim 30, wherein $Q_a$ is -C(=O)-, -N($CH_3$)-, -NHC(=O)-, -C(=O)-NH-, or -C(=O)-N($CH_3$)-.

**34.** The compound, or a tautomer, mesomer, raceme, enantiomer or diastereomer thereof, or mixture thereof, or a pharmaceutically acceptable salt, prodrug or solvate thereof according to any one of claims 1-33, wherein $Q_b$ is one, two, three or more selected from the group consisting of: absent, -C(=O)NR-, -C(=O)O-, -OC(=O)NR-, -NRC(=O)-, -OC(=O)-, -NRC(=O)O-, -NRC(=O)NR-, NHC-(=NH)NH-, -C(=O)-, and -OC(=O)O-; and wherein R is selected from hydrogen, or optionally substituted $C_1$-$C_6$ linear or branched alkyl.

**35.** The compound, or a tautomer, mesomer, raceme, enantiomer or diastereomer thereof, or mixture thereof, or a pharmaceutically acceptable salt, prodrug or solvate thereof according to claim 34, wherein $Q_b$ is one, two, three or more selected from the group consisting of: absent, -C(=O)-, -C(=O)NR-, -OC(=O)NR-, -NRC(=O)-, -NRC(=O)O-, and -NRC(=O)NR-; and wherein R is selected from hydrogen, or optionally substituted $C_1$-$C_6$ linear or branched alkyl.

**36.** The compound, or a tautomer, mesomer, raceme, enantiomer or diastereomer thereof, or mixture thereof, or a pharmaceutically acceptable salt, prodrug or solvate thereof according to claim 34, wherein $Q_b$ is one, two, three or more selected from the group consisting of: absent, -C(=O)-, -NRC(=O)-, -NRC(=O)NR-, and -C(=O)NR-; and wherein R is hydrogen or methyl.

**37.** The compound, or a tautomer, mesomer, raceme, enantiomer or diastereomer thereof, or mixture thereof, or a pharmaceutically acceptable salt, prodrug or solvate thereof according to any one of claims 1-36, wherein X is selected from:

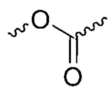 , 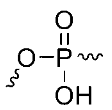 , 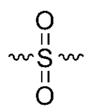 , 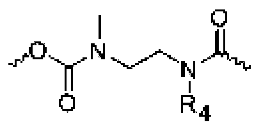 ,

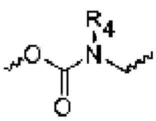 , 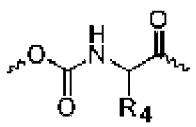 , 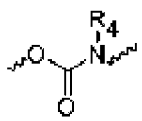 or 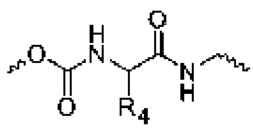 ;

the wavy line at right side of X is connected to D;

$R_4$ is selected from hydrogen, $C_1$-$C_6$ linear or branched alkyl, -$(CH_2CH_2O)_mR_2$, -$CH_2CH_2NR_2R_3$, or -$CH_2CH_2SO_2R_2$;

$R_2$, and $R_3$ are each independently selected from hydrogen, or $C_1$-$C_6$ linear or branched alkyl; and m is an integer greater than 1.

**38.** The compound, or a tautomer, mesomer, raceme, enantiomer or diastereomer thereof, or mixture thereof, or a pharmaceutically acceptable salt, prodrug or solvate thereof according to claim 37, wherein

X is

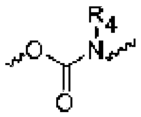, 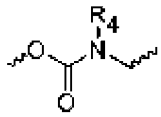 or 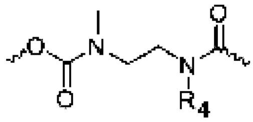;

and

$R_4$ is hydrogen, methyl, $-CH_2CH_2OCH_2CH_2OH$, $-CH_2CH_2NMe_2$, or $-CH_2CH_2SO_2Me$.

**39.** The compound, or a tautomer, mesomer, raceme, enantiomer or diastereomer thereof, or mixture thereof, or a pharmaceutically acceptable salt, prodrug or solvate thereof according to claim 37, wherein
X is

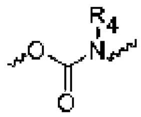 or 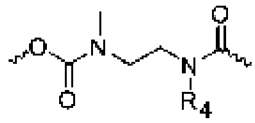,

and $R_4$ is hydrogen or methyl.

**40.** The compound, or a tautomer, mesomer, raceme, enantiomer or diastereomer thereof, or mixture thereof, or a pharmaceutically acceptable salt, prodrug or solvate thereof according to any one of claims 1-39, wherein the sulfonate inner salt is

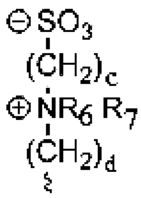, 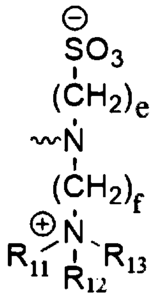 or 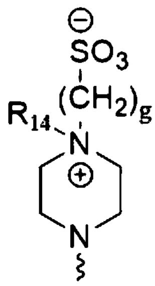;

the wavy line is connected to Y, Y' or Y";
wherein c, d, e, f, and g are each independently an integer selected from 1, 2, 3, 4, 5 or 6; and
$R_6$, $R_7$, $R_{11}$, $R_{12}$, $R_{13}$, and $R_{14}$ are each independently selected from absent, hydrogen, or $C_1$-$C_6$ linear or branched alkyl.

**41.** The compound, or a tautomer, mesomer, raceme, enantiomer or diastereomer thereof, or mixture thereof, or a pharmaceutically acceptable salt, prodrug or solvate thereof according to claim 40, wherein the sulfonate inner salt is

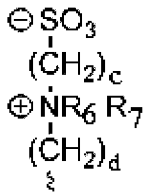, 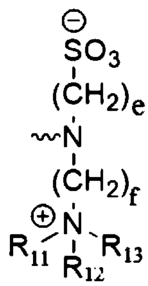 or 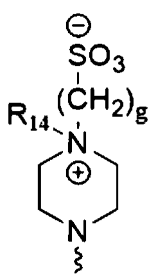;

wherein

$R_6$, $R_7$, $R_{11}$, $R_{12}$, $R_{13}$ and $R_{14}$ are each independently selected from absent, methyl or ethyl; and

c, d, e, f, and g are each independently 1, 2, or 3.

**42.** The compound, or a tautomer, mesomer, raceme, enantiomer or diastereomer thereof, or mixture thereof, or a pharmaceutically acceptable salt, prodrug or solvate thereof according to claim 40, wherein the sulfonate inner salt is any one selected from the group consisting of:

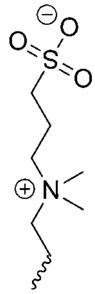 , 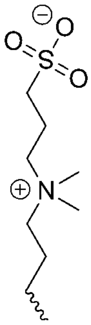 , 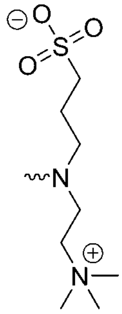 , and 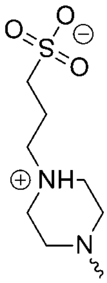 .

**43.** The compound, or a tautomer, mesomer, raceme, enantiomer or diastereomer thereof, or mixture thereof, or a pharmaceutically acceptable salt, prodrug or solvate thereof according to any one of claims 1-42, wherein the phosphate inner salt is

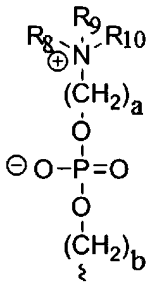 ,

wherein
a and b are each independently an integer selected from 1, 2, 3, 4, 5 or 6; and wherein $R_8$, $R_9$ and $R_{10}$ are each independently selected from hydrogen, or $C_1$-$C_6$ linear or branched alkyl.

**44.** The compound, or a tautomer, mesomer, raceme, enantiomer or diastereomer thereof, or mixture thereof, or a pharmaceutically acceptable salt, prodrug or solvate thereof according to claim 43, wherein the phosphate inner salt is

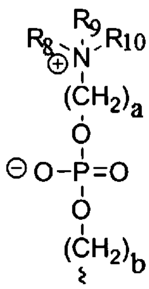 ,

wherein
$R_8$, $R_9$, and $R_{10}$ are each independently methyl or ethyl; a is 1, 2, or 3; and b is 1, 2, or 3.

**45.** The compound, or a tautomer, mesomer, raceme, enantiomer or diastereomer thereof, or mixture thereof, or a pharmaceutically acceptable salt, prodrug or solvate thereof according to claim 43, wherein the phosphate inner salt is

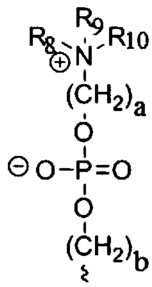

,

wherein

$R_8$, $R_9$, and $R_{10}$ are each independently methyl; a is 2; and b is 1.

**46.** The compound, or a tautomer, mesomer, raceme, enantiomer or diastereomer thereof, or mixture thereof, or a pharmaceutically acceptable salt, prodrug or solvate thereof according to any one of claims 1-45, wherein the D comprises a cytotoxin or immune agonist.

**47.** The compound, or a tautomer, mesomer, raceme, enantiomer or diastereomer thereof, or mixture thereof, or a pharmaceutically acceptable salt, prodrug or solvate thereof according to claim 46, wherein the cytotoxin is one or two selected from the group consisting of: a microtubulin inhibitor, a DNA synthesis inhibitor, a topoisomerase inhibitor, and a RNA polymerase-2 inhibitor.

**48.** The compound, or a tautomer, mesomer, raceme, enantiomer or diastereomer thereof, or mixture thereof, or a pharmaceutically acceptable salt, prodrug or solvate thereof according to claim 46, wherein the cytotoxin is one, two, or more selected from the group consisting of: auristatin, mertansine DM, calicheamicin, duocarmycin, pyrrolobenzodiazepine, camptothecin derivative, and $\alpha$-amanitin.

**49.** The compound, or a tautomer, mesomer, raceme, enantiomer or diastereomer thereof, or mixture thereof, or a pharmaceutically acceptable salt, prodrug or solvate thereof according to claim 46, wherein the immune agonist is selected from TLR7/8 agonist or STING agonist.

**50.** The compound, or a tautomer, mesomer, raceme, enantiomer or diastereomer thereof, or mixture thereof, or a pharmaceutically acceptable salt, prodrug or solvate thereof according to any one of claims 1-49, wherein i is 2, 3, 4, 5, 6, 7, or 8.

**51.** The compound, or a tautomer, mesomer, raceme, enantiomer or diastereomer thereof, or mixture thereof, or a pharmaceutically acceptable salt, prodrug or solvate thereof according to any one of claims 1-50, wherein h is 1, 2, 3, or 4.

**52.** The compound, or a tautomer, mesomer, raceme, enantiomer or diastereomer thereof, or mixture thereof, or a pharmaceutically acceptable salt, prodrug or solvate thereof according to any one of claims 1-51, wherein o, p, and q are each independently 0, 1, 2 or 3.

**53.** The compound, or a tautomer, mesomer, raceme, enantiomer or diastereomer thereof, or mixture thereof, or a pharmaceutically acceptable salt, prodrug or solvate thereof according to any one of claims 1-52, wherein $L_a$ is one, two, three or more selected from the group consisting of: $C_1$-$C_6$ linear or branched alkylene, and polyethylene glycol group;

wherein the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$; n is an integer equal to or greater than 1;

$Q_a$ is one, two, three or more selected from the group consisting of: -C(=O)-, -NR-, -NR(C=O)-, and -(C=O)NR-; and wherein

R is selected from hydrogen, or $C_1$-$C_6$ linear or branched alkyl.

**54.** The compound, or a tautomer, mesomer, raceme, enantiomer or diastereomer thereof, or mixture thereof, or a pharmaceutically acceptable salt, prodrug or solvate thereof according to claim 53, wherein $L_a$ is one, two, three or

more selected from the group consisting of: ethylidene, linear propylidene, linear butylidene, linear pentylidene, and $-(CH_2CH_2O)_nCH_2CH_2-$; wherein n is 2, 3, 4, 5, 6, 7 or 8; $Q_a$ is one, two, three or more selected from the group consisting of: -C(=O)-, -NR-, -NR(C=O)- and -(C=O)NR-; and wherein R is methyl and/or hydrogen.

**55.** The compound, or a tautomer, mesomer, raceme, enantiomer or diastereomer thereof, or mixture thereof, or a pharmaceutically acceptable salt, prodrug or solvate thereof according to claim 53, wherein $L_a$ is ethylidene and/or $-(CH_2CH_2O)_4CH_2CH_2-$ or pentylidene; and
$Q_a$ is -C(=O)-, $-N(CH_3)-$, -NH(C=O)-, -C(=O)NH- or $-C(=O)-N(CH_3)-$.

**56.** The compound, or a tautomer, mesomer, raceme, enantiomer or diastereomer thereof, or mixture thereof, or a pharmaceutically acceptable salt, prodrug or solvate thereof according to any one of claims 1-55, wherein $L_b$ is selected from $C_1$-$C_6$ linear or branched alkylene, $C_2$-$C_6$ linear or branched alkenylene, or $C_2$-$C_6$ linear or branched alkynylene, arylene with 6-10 carbon atoms in one or more rings, or polyethylene glycol group;

the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; the alkylene, alkenylene, or alkynylene is optionally substituted by a substituent;
$Q_b$ is one, two, three or more selected from the group consisting of: absent, -C(=O)NR-, -C(=O)O-, -OC(=O)NR-, -NRC(=O)-, -OC(=O)-, -NRC(=O)O-, $-NRS(=O)_2-$, -NRC(=O)NR-, -C(=O)-, and -OC(=O)O-; and wherein R is selected from hydrogen, or optionally substituted $C_1$-$C_6$ linear or branched alkyl.

**57.** The compound, or a tautomer, mesomer, raceme, enantiomer or diastereomer thereof, or mixture thereof, or a pharmaceutically acceptable salt, prodrug or solvate thereof according to claim 56, wherein $L_b$ is one, two, three or more selected from the group consisting of: $C_1$-$C_6$ linear or branched alkylene, and $C_6$-$C_{10}$ arylene;
$Q_b$ is one, two, three or more selected from the group consisting of: absent, -C(=O)NR-, -C(=O)-, -OC(=O)NR-, -NRC(=O)-, -NRC(=O)O-, and -NRC(=O)NR-; and wherein R is selected from hydrogen, or $C_1$-$C_6$ linear or branched alkyl.

**58.** The compound, or a tautomer, mesomer, raceme, enantiomer or diastereomer thereof, or mixture thereof, or a pharmaceutically acceptable salt, prodrug or solvate thereof according to claim 56, wherein $L_b$ is one, two, three or more selected from the group consisting of: methylene, ethylene, n-propylidene, isopropylidene, n-butylidene, isobutylidene, sec-butylidene, tert-butylidene, and benzylidene;
$Q_b$ is one, two, three or more selected from the group consisting of: absent, -C(=O)-, -NRC(=O)-, -NRC(=O)NR-, and -C(=O)NR-; and wherein R is hydrogen or methyl.

**59.** The compound, or a tautomer, mesomer, raceme, enantiomer or diastereomer thereof, or mixture thereof, or a pharmaceutically acceptable salt, prodrug or solvate thereof according to any one of claims 1-58, wherein the substituent is any one selected from the group consisting of: deuterium, tritium, halogen, amino, hydroxyl, cyano, nitro, optionally substituted alkyl, alkenyl, alkynyl, optionally substituted heterocyclyl, acyl, optionally substituted amino, optionally substituted aminoformyl, optionally substituted thioaminoformyl, optionally substituted sulfamine, optionally substituted hydroxyl, optionally substituted thioalkyl (SH), and optionally substituted silyl.

**60.** The compound, or a tautomer, mesomer, raceme, enantiomer or diastereomer thereof, or mixture thereof, or a pharmaceutically acceptable salt, prodrug or solvate thereof according to any one of claims 1-59, wherein the compound is any one selected from the group consisting of:

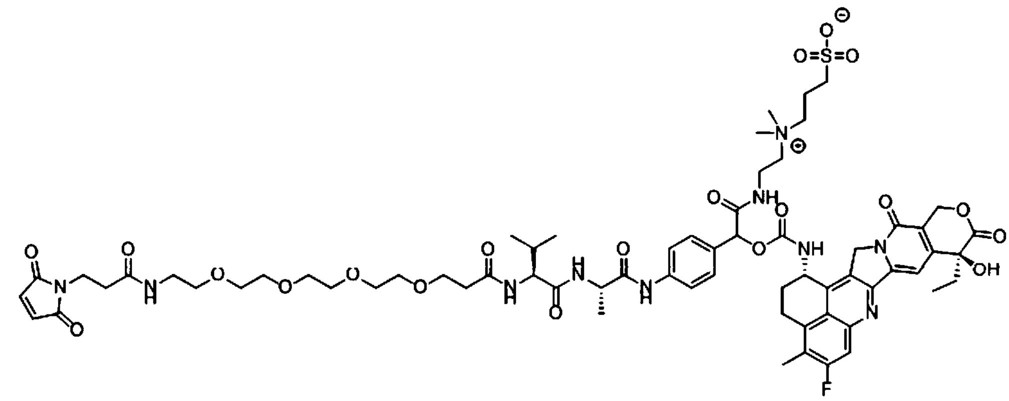

LD-31,

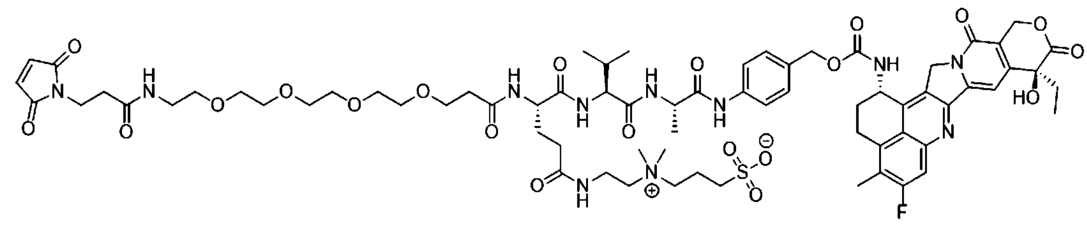

LD-37,

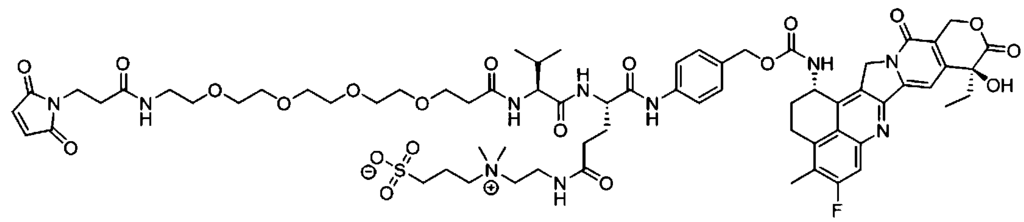

LD-38,

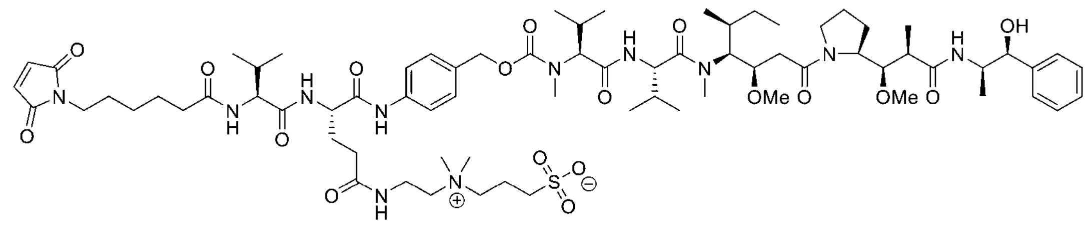

LD-57,

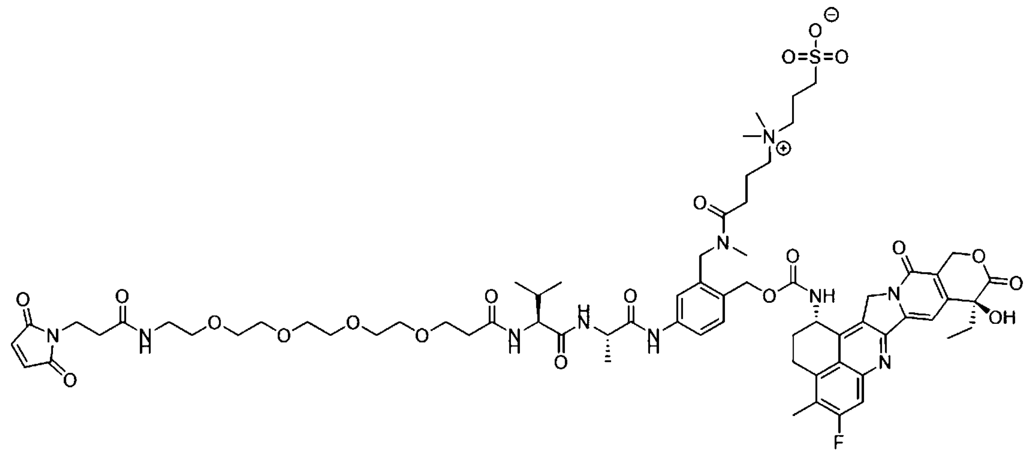

LD-65,

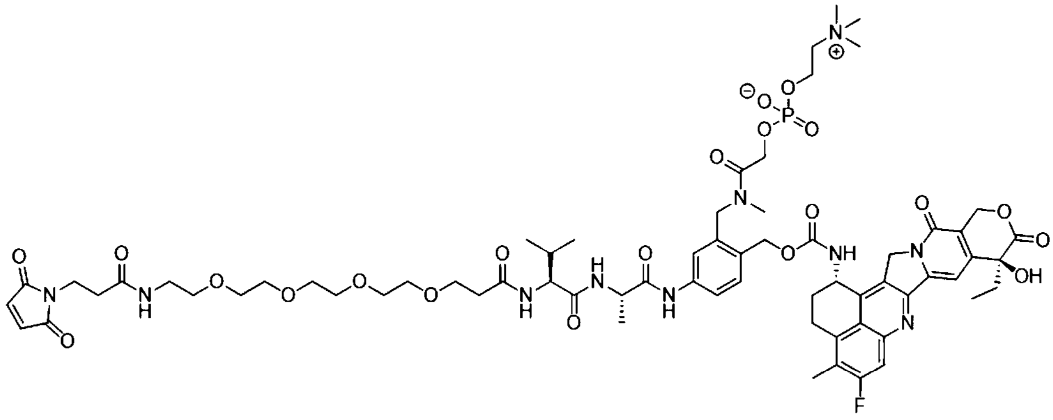

LD-66,

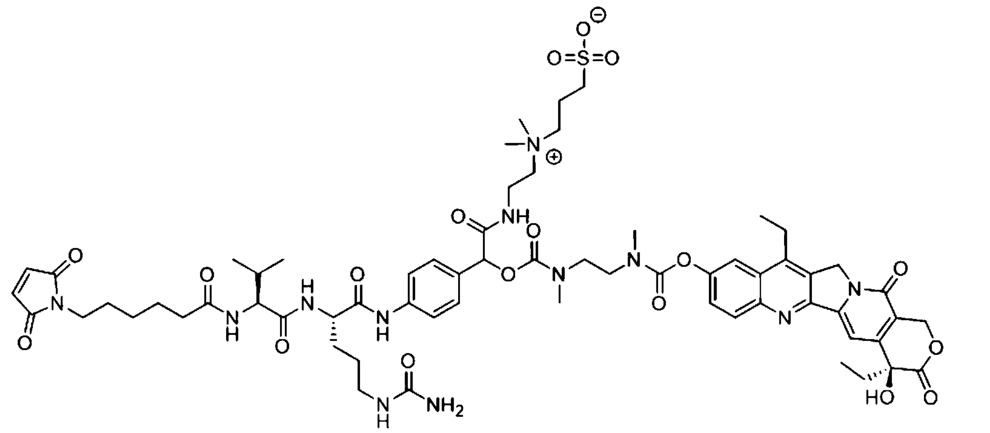

LD-71,

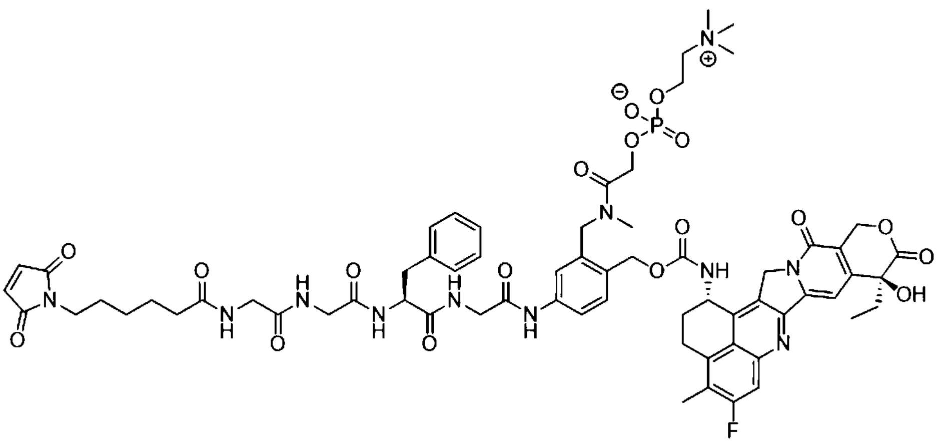

LD-72,

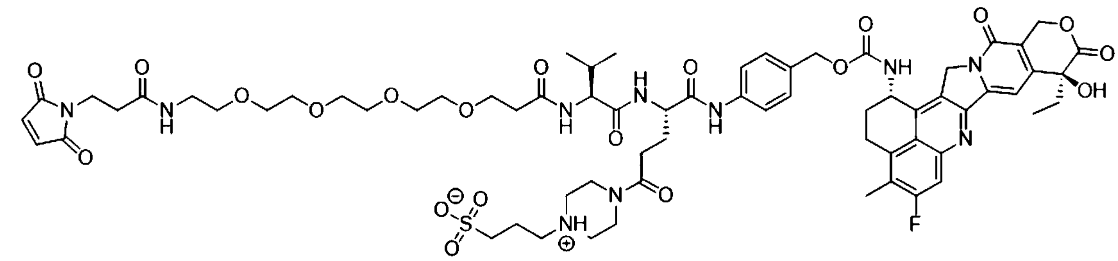

LD-88,

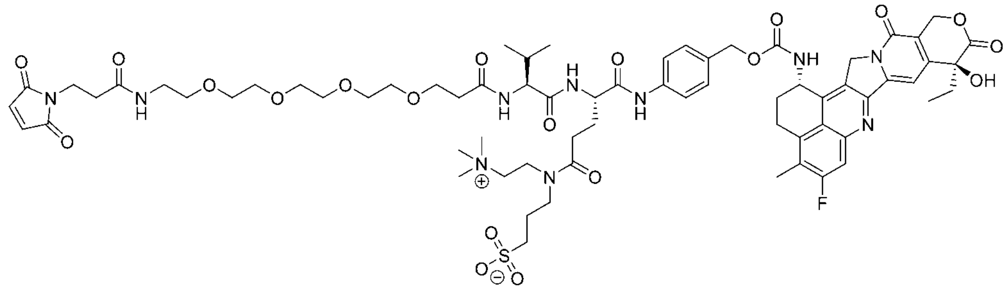

LD-89 ,

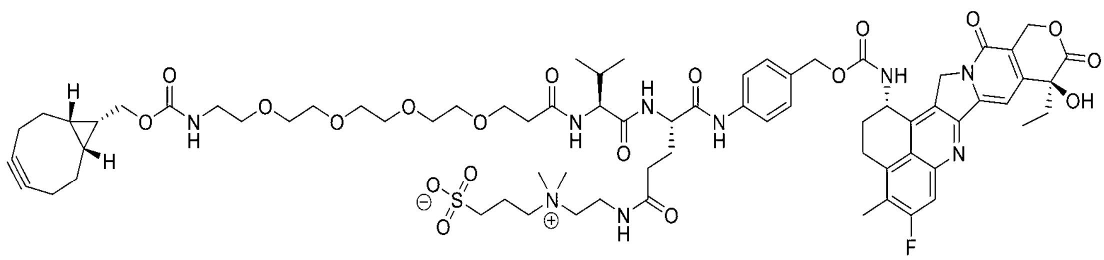

LD-94,

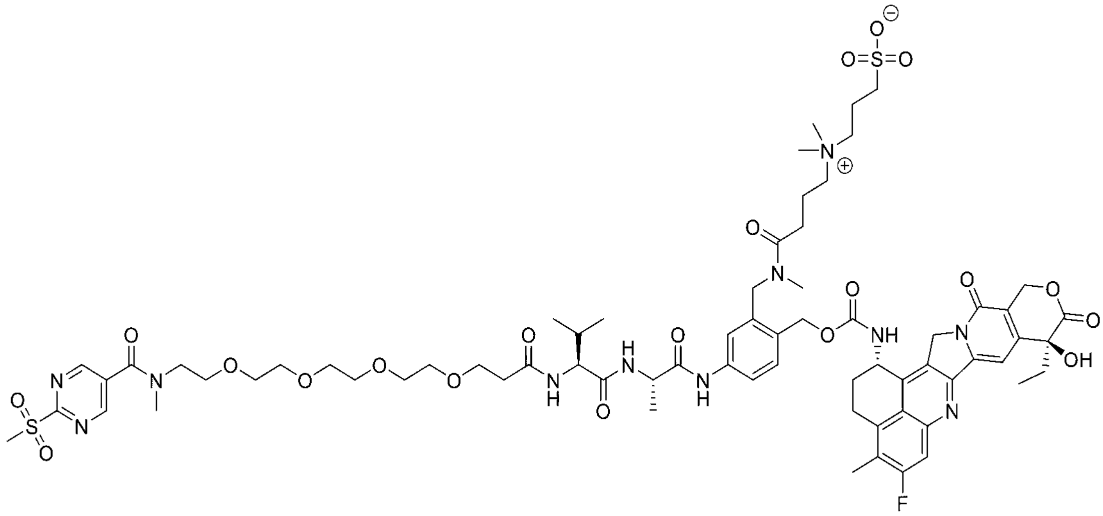

LD-98,

and

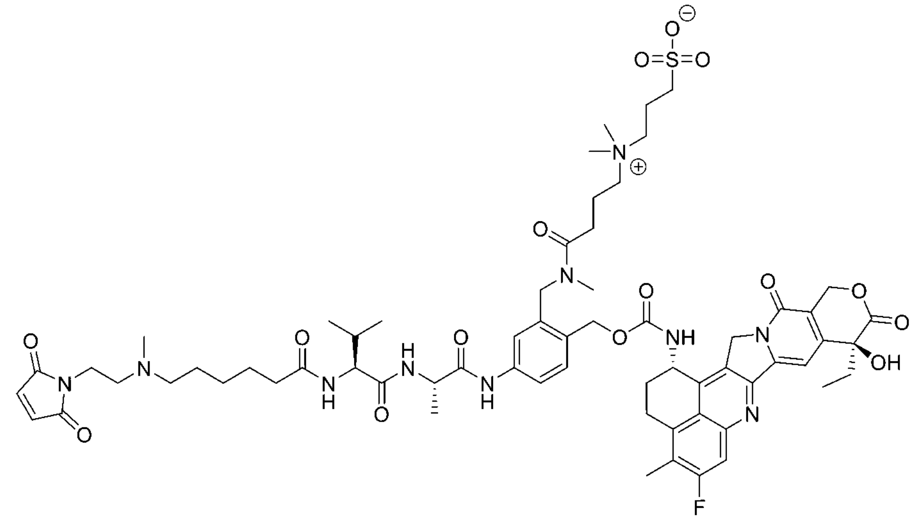

LD-99.

**61.** A compound represented by Formula (2), or a tautomer, mesomer, raceme, enantiomer or diastereomer thereof, or mixture thereof, or a pharmaceutically acceptable salt, prodrug or solvate thereof, wherein Formula (2) has the following structural formula:

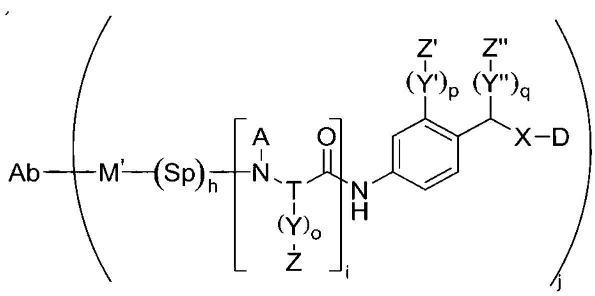

Formula (2):

Ab comprises an antibody or an antigen-binding fragment thereof;
M' is a spacer;
Sp is $-L_a-Q_a-$;
A is selected from absent, hydrogen or alkyl;
T represents a straight or branched ($C_1$-$C_8$) trivalent alkyl group;

Y, Y' and Y" are each independently $-L_b-Q_b-$;
$L_a$ and $L_b$ are each independently selected from absent, alkylene, alkenylene, alkynylene, alicyclylene, heteroalicyclylene, arylene, heteroarylene or polyethylene glycol group, wherein the alkylene, alkenylene, alkynylene, alicyclylene, heteroalicyclylene, arylene, heteroarylene or polyethylene glycol group is optionally substituted by a substituent;
$Q_a$, and $Q_b$ are each independently selected from absent, -C(=O)NR-, -C(=O)O-, -OC(=O)NR-, $-S(=O)_2NR-$, -NRC(=O)-, -OC(=O)-, -NRC(=O)O-, $-NRS(=O)_2-$, -NRC(=O)NR-, -NHC-(=NH)NH-, -C(=O)-, -OC(=O)O-, -O-, -NR-, -S-, $S(=O)_2-$, S(=O)-, or -Se-; wherein R is selected from hydrogen or optionally substituted alkyl;
Z is selected from absent, hydrogen, optionally substituted alkyl or a sulfonate inner salt;
Z' and Z" are each independently selected from absent, hydrogen, optionally substituted alkyl, a sulfonate inner salt or a phosphate inner salt;
at least one of Z, Z' and Z" is a sulfonate inner salt or a phosphate inner salt;
X comprises a self-immolative unit or is absent;
D comprises a drug molecule;
i is an integer equal to or greater than 1;
h is an integer from 1 to 6;
o, p, and q are each independently an integer selected from 0-5; and
j is an integer equal to or greater than 1.

**62.** The compound, or a tautomer, mesomer, raceme, enantiomer or diastereomer thereof, or mixture thereof, or a pharmaceutically acceptable salt, prodrug or solvate thereof according to claim 61, wherein the Formula (2) is Formula (2-1):

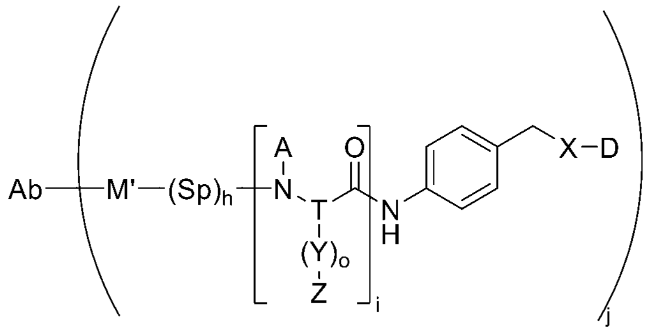

Formula (2-1)

wherein Ab, M', Sp, A, T, Y, h, o, i, j, X, D, and Z are defined as in claim 61; and
at least one of the i Zs is a sulfonate inner salt.

**63.** The compound, or a tautomer, mesomer, raceme, enantiomer or diastereomer thereof, or mixture thereof, or a pharmaceutically acceptable salt, prodrug or solvate thereof according to claim 62, wherein the Formula (2-1) is Formula (2-1-1):

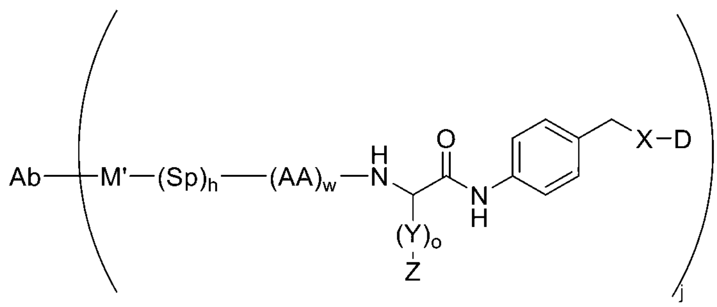

Formula (2-1-1)

wherein AA is an amino acid residue, and
W is an integer equal to or greater than 1.

**64.** The compound, or a tautomer, mesomer, raceme, enantiomer or diastereomer thereof, or mixture thereof, or a

pharmaceutically acceptable salt, prodrug or solvate thereof according to claim 61, wherein the Formula (2) is Formula (2-2):

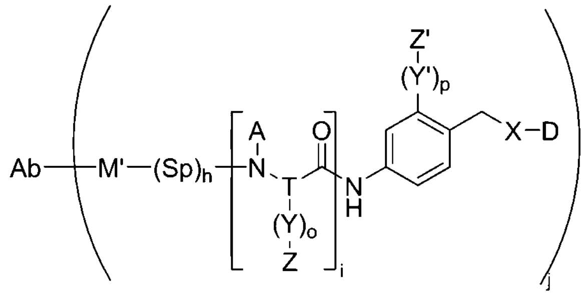

Formula (2-2)

wherein Ab, M', Sp, A, T, Y, Z, o, p, i, h, j, X, Y', and D are defined as in claim 61; and Z' is a sulfonate inner salt or a phosphate inner salt.

**65.** The compound, or a tautomer, mesomer, raceme, enantiomer or diastereomer thereof, or mixture thereof, or a pharmaceutically acceptable salt, prodrug or solvate thereof according to claim 64, wherein the Formula (2-2) is Formula (2-2-1):

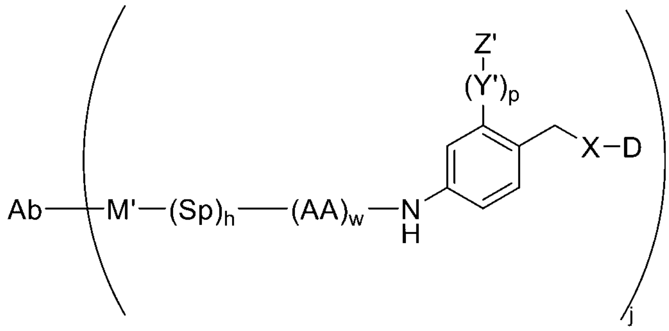

Formula (2-2-1)

wherein AA is an amino acid residue, and W is an integer equal to or greater than 1.

**66.** The compound, or a tautomer, mesomer, raceme, enantiomer or diastereomer thereof, or mixture thereof, or a pharmaceutically acceptable salt, prodrug or solvate thereof according to claim 61, wherein the Formula (2) is Formula (2-3):

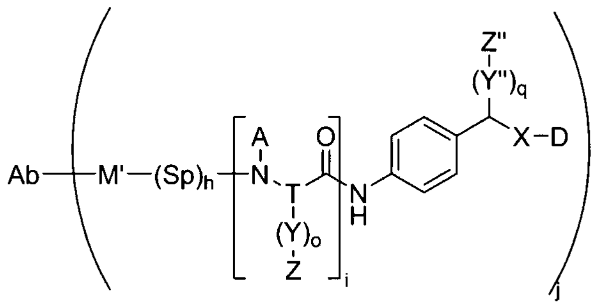

Formula (2-3)

wherein Ab, M', Sp, A, Y, T, Z, o, i, h, j, q, X, Y", and D are defined as in claim 61; and Z" is a sulfonate inner salt or a phosphate inner salt.

**67.** The compound, or a tautomer, mesomer, raceme, enantiomer or diastereomer thereof, or mixture thereof, or a pharmaceutically acceptable salt, prodrug or solvate thereof according to claim 66, wherein the Formula (2-3) is Formula (2-3-1):

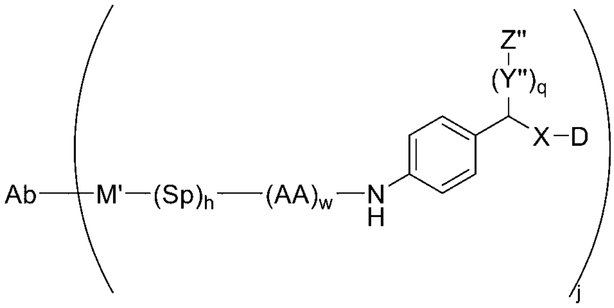

Formula (2-3-1)

wherein AA is an amino acid residue, and W is an integer equal to or greater than 1.

**68.** The compound, or a tautomer, mesomer, raceme, enantiomer or diastereomer thereof, or mixture thereof, or a pharmaceutically acceptable salt, prodrug or solvate thereof according to any one of claims 61-67, wherein when A and Z are absent, N, Y and T form a ring to have a structural formula of:

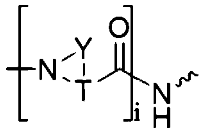

, preferably

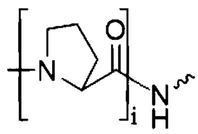

.

**69.** The compound, or a tautomer, mesomer, raceme, enantiomer or diastereomer thereof, or mixture thereof, or a pharmaceutically acceptable salt, prodrug or solvate thereof according to any one of claims 63, 65 or 67, wherein the amino acid(s) is one or more selected from the group consisting of: alanine, arginine, asparagine, aspartic acid, γ-carboxyglutamic acid, citrulline, cysteine, glutamic acid, glutamine, glycine, histidine, isoleucine, leucine, lysine, methionine, norleucine, norvaline, ornithine, phenylalanine, proline, serine, threonine, tryptophan, tyrosine, and valine.

**70.** The compound, or a tautomer, mesomer, raceme, enantiomer or diastereomer thereof, or mixture thereof, or a pharmaceutically acceptable salt, prodrug or solvate thereof according to any one of claims 63, 65 or 67, wherein the amino acid(s) is one or more selected from the group consisting of: valine, glutamic acid, citrulline, lysine, alanine, phenylalanine, arginine, glutamine, asparagine, and glycine.

**71.** The compound, or a tautomer, mesomer, raceme, enantiomer or diastereomer thereof, or mixture thereof, or a pharmaceutically acceptable salt, prodrug or solvate thereof according to any one of claims 63, 65 or 67, wherein the amino acid(s) is one or more selected from the group consisting of: valine, citrulline, alanine, phenylalanine, glutamine, and glycine.

**72.** The compound, or a tautomer, mesomer, raceme, enantiomer or diastereomer thereof, or mixture thereof, or a pharmaceutically acceptable salt, prodrug or solvate thereof according to any one of claims 61-71, wherein M' is any one selected from the group consisting of:

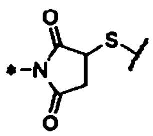 , 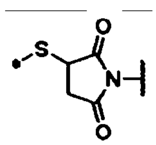 , 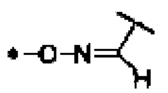 , 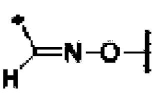 , 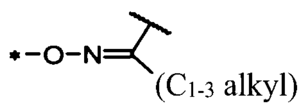 ,

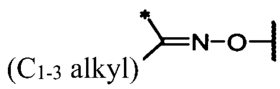, 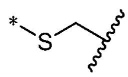, 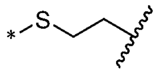, 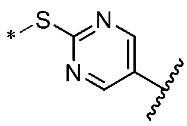, 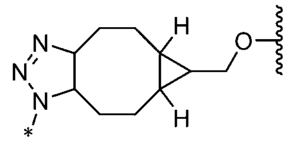,

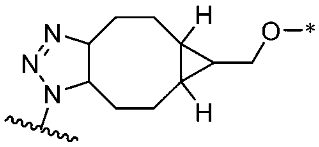, 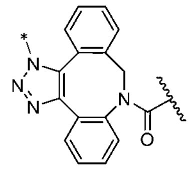, 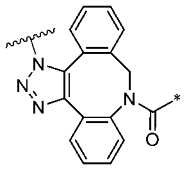, 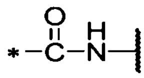 or 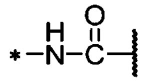

; and
* is connected to Ab.

**73.** The compound, or a tautomer, mesomer, raceme, enantiomer or diastereomer thereof, or mixture thereof, or a pharmaceutically acceptable salt, prodrug or solvate thereof according to any one of claims 61-72, wherein M' is any one selected from the group consisting of:

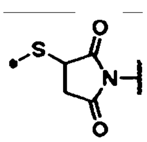, 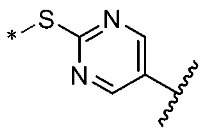, 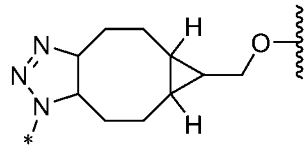, and 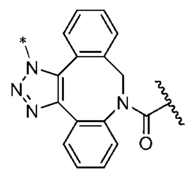.

**74.** The compound, or a tautomer, mesomer, raceme, enantiomer or diastereomer thereof, or mixture thereof, or a pharmaceutically acceptable salt, prodrug or solvate thereof according to any one of claims 61-73, wherein M' is

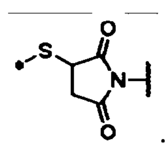.

**75.** The compound, or a tautomer, mesomer, raceme, enantiomer or diastereomer thereof, or mixture thereof, or a pharmaceutically acceptable salt, prodrug or solvate thereof according to any one of claims 61-74, wherein A is hydrogen, methyl, ethyl or propyl.

**76.** The compound, or a tautomer, mesomer, raceme, enantiomer or diastereomer thereof, or mixture thereof, or a pharmaceutically acceptable salt, prodrug or solvate thereof according to any one of claims 61-75, wherein A is hydrogen.

**77.** The compound, or a tautomer, mesomer, raceme, enantiomer or diastereomer thereof, or mixture thereof, or a pharmaceutically acceptable salt, prodrug or solvate thereof according to any one of claims 61-76, wherein the T is any one selected from the group consisting of:

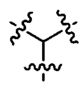, 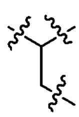, 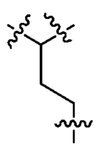, and 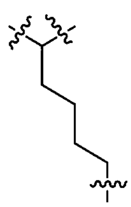.

**78.** The compound, or a tautomer, mesomer, raceme, enantiomer or diastereomer thereof, or mixture thereof, or a

pharmaceutically acceptable salt, prodrug or solvate thereof according to any one of claims 61-77, wherein the T is

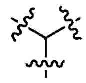.

**79.** The compound, or a tautomer, mesomer, raceme, enantiomer or diastereomer thereof, or mixture thereof, or a pharmaceutically acceptable salt, prodrug or solvate thereof according to any one of claims 61-78, wherein $L_a$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, heteroarylene with 6-18 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**80.** The compound, or a tautomer, mesomer, raceme, enantiomer or diastereomer thereof, or mixture thereof, or a pharmaceutically acceptable salt, prodrug or solvate thereof according to any one of claims 61-79, wherein $L_a$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**81.** The compound, or a tautomer, mesomer, raceme, enantiomer or diastereomer thereof, or mixture thereof, or a pharmaceutically acceptable salt, prodrug or solvate thereof according to any one of claims 61-80, wherein $L_a$ is one, two, three or more selected from the group consisting of: $C_1$-$C_6$ linear or branched alkylene, $C_2$-$C_6$ linear or branched alkenylene, $C_2$-$C_6$ linear or branched alkynylene, and polyethylene glycol group; and wherein the alkylene, alkenylene, or alkynylene is optionally substituted by a substituent.

**82.** The compound, or a tautomer, mesomer, raceme, enantiomer or diastereomer thereof, or mixture thereof, or a pharmaceutically acceptable salt, prodrug or solvate thereof according to any one of claims 61-81, wherein $L_a$ is one, two, three or more selected from the group consisting of: $C_1$-$C_6$ linear or branched alkylene, and polyethylene glycol group;
the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, and n is an integer equal to or greater than 1.

**83.** The compound, or a tautomer, mesomer, raceme, enantiomer or diastereomer thereof, or mixture thereof, or a pharmaceutically acceptable salt, prodrug or solvate thereof according to any one of claims 61-82, wherein $L_a$ is one, two, three or more selected from the group consisting of: ethylidene, linear propylidene, linear butylidene, linear pentylidene, and $-(CH_2CH_2O)_nCH_2CH_2-$; and wherein n is 2, 3, 4, 5, 6, 7 or 8.

**84.** The compound, or a tautomer, mesomer, raceme, enantiomer or diastereomer thereof, or mixture thereof, or a pharmaceutically acceptable salt, prodrug or solvate thereof according to any one of claims 61-83, wherein $L_a$ is ethylidene and/or $-(CH_2CH_2O)_4CH_2CH_2-$ or pentylidene.

**85.** The compound, or a tautomer, mesomer, raceme, enantiomer or diastereomer thereof, or mixture thereof, or a pharmaceutically acceptable salt, prodrug or solvate thereof according to any one of claims 61-84, wherein $L_b$ is one, two, three or more selected from the group consisting of: alkylene, alkenylene, alkynylene, saturated or unsaturated alicyclylene with 3-10 ring carbon atoms, saturated or unsaturated heteroalicyclylene with 3-10 ring carbon atoms and 1-4 heteroatoms, arylene with 6-18 carbon atoms in one or more rings, or heteroarylene with 6-18 carbon atoms and 1-4 heteroatoms in one or more rings, and polyethylene glycol group; and
wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**86.** The compound, or a tautomer, mesomer, raceme, enantiomer or diastereomer thereof, or mixture thereof, or a

pharmaceutically acceptable salt, prodrug or solvate thereof according to any one of claims 61-85, wherein $L_b$ is one, two, three or more selected from the group consisting of: $C_1$-$C_{10}$ linear or branched alkylene, $C_2$-$C_{12}$ linear or branched alkenylene, $C_2$-$C_{12}$ linear or branched alkynylene, saturated or unsaturated alicyclylene with 3-6 ring carbon atoms, saturated or unsaturated heteroalicyclylene with 3-6 ring carbon atoms and 1-4 heteroatoms, or arylene with 6-10 carbon atoms in one or more rings, heteroarylene with 6-10 carbon atoms and 1-4 heteroatoms in one or more rings, and polyethylene glycol group; and
wherein the alkylene, alkenylene, alkynylene, alicyclylene, heteroalicyclylene, arylene, or heteroarylene is optionally substituted by a substituent; and the heteroatom is selected from nitrogen, oxygen, or sulfur.

**87.** The compound, or a tautomer, mesomer, raceme, enantiomer or diastereomer thereof, or mixture thereof, or a pharmaceutically acceptable salt, prodrug or solvate thereof according to any one of claims 61-86, wherein $L_b$ is selected from $C_1$-$C_6$ linear or branched alkylene, $C_2$-$C_6$ linear or branched alkenylene, or $C_2$-$C_6$ linear or branched alkynylene, or polyethylene glycol group; and

wherein the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1, and
the alkylene, alkenylene, or alkynylene is optionally substituted by a substituent.

**88.** The compound, or a tautomer, mesomer, raceme, enantiomer or diastereomer thereof, or mixture thereof, or a pharmaceutically acceptable salt, prodrug or solvate thereof according to any one of claims 61-87, wherein $L_b$ is one, two, three or more selected from the group consisting of: $C_1$-$C_6$ linear or branched alkylene, and $C_6$-$C_{10}$ arylene.

**89.** The compound, or a tautomer, mesomer, raceme, enantiomer or diastereomer thereof, or mixture thereof, or a pharmaceutically acceptable salt, prodrug or solvate thereof according to any one of claims 61-88, wherein $L_b$ is one, two, three or more selected from the group consisting of: methylene, ethylene, n-propylidene, isopropylidene, n-butylidene, isobutylidene, sec-butylidene, tert-butylidene, and benzylidene.

**90.** The compound, or a tautomer, mesomer, raceme, enantiomer or diastereomer thereof, or mixture thereof, or a pharmaceutically acceptable salt, prodrug or solvate thereof according to any one of claims 61-89, wherein $Q_a$ is one, two, three or more selected from the group consisting of: -C(=O)NR-, -NR-, -C(=O)O-, -OC(=O)NR-, -NRC(=O)-, -OC(=O)-, -NRC(=O)O-, -NRC(=O)NR-, NHC-(=NH)NH-, -C(=O)-, -OC(=O)O-; and wherein R is selected from hydrogen, or optionally substituted $C_1$-$C_6$ linear or branched alkyl.

**91.** The compound, or a tautomer, mesomer, raceme, enantiomer or diastereomer thereof, or mixture thereof, or a pharmaceutically acceptable salt, prodrug or solvate thereof according to claim 90, wherein $Q_a$ is one, two, three or more selected from the group consisting of: -C(=O)-, -NR-, -C(=O)NR-, -NRC(=O)NR-, -OC(=O)O-, -OC(=O)NR-, -NRC(=O)-, and -OC(=O)-; and wherein
R is selected from hydrogen, or optionally substituted $C_1$-$C_6$ linear or branched alkyl.

**92.** The compound, or a tautomer, mesomer, raceme, enantiomer or diastereomer thereof, or mixture thereof, or a pharmaceutically acceptable salt, prodrug or solvate thereof according to claim 90, wherein $Q_a$ is one, two, three or more selected from the group consisting of: -C(=O)-, -NR-, -NRC(=O)-, and -(C=O)NR-; and wherein
R is selected from hydrogen, or optionally substituted $C_1$-$C_6$ linear or branched alkyl.

**93.** The compound, or a tautomer, mesomer, raceme, enantiomer or diastereomer thereof, or mixture thereof, or a pharmaceutically acceptable salt, prodrug or solvate thereof according to claim 90, wherein $Q_a$ is -C(=O)-, $-N(CH_3)-$, -NHC(=O)-, -C(=O)-NH- or $-C(=O)-N(CH_3)-$.

**94.** The compound, or a tautomer, mesomer, raceme, enantiomer or diastereomer thereof, or mixture thereof, or a pharmaceutically acceptable salt, prodrug or solvate thereof according to any one of claims 61-93, wherein $Q_b$ is one, two, three or more selected from the group consisting of: absent, -C(=O)NR-, -C(=O)O-, -OC(=O)NR-, -NRC(=O)-, -OC(=O)-, -NRC(=O)O-, -NRC(=O)NR-, NHC-(=NH)NH-, -C(=O)-, -OC(=O)O-; and wherein R is selected from hydrogen, or optionally substituted $C_1$-$C_6$ linear or branched alkyl.

**95.** The compound, or a tautomer, mesomer, raceme, enantiomer or diastereomer thereof, or mixture thereof, or a pharmaceutically acceptable salt, prodrug or solvate thereof according to claim 94, wherein $Q_b$ is one, two, three or more selected from the group consisting of: absent, -C(=O)-, -C(=O)NR-, -OC(=O)NR-, -NRC(=O)-, -NRC(=O)O-,

and -NRC(=O)NR-; and wherein R is selected from hydrogen, or optionally substituted $C_1$-$C_6$ linear or branched alkyl.

**96.** The compound, or a tautomer, mesomer, raceme, enantiomer or diastereomer thereof, or mixture thereof, or a pharmaceutically acceptable salt, prodrug or solvate thereof according to claim 94, wherein $Q_b$ is one, two, three or more selected from the group consisting of: absent, -C(=O)-, -NRC(=O)-, -NRC(=O)NR-, and -C(=O)NR-; and wherein R is hydrogen or methyl.

**97.** The compound, or a tautomer, mesomer, raceme, enantiomer or diastereomer thereof, or mixture thereof, or a pharmaceutically acceptable salt, prodrug or solvate thereof according to any one of claims 61-96, wherein X is selected from:

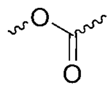 , 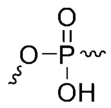 , 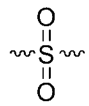 , 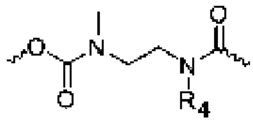 ,

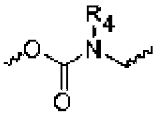 , 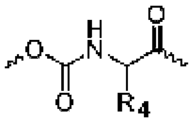 , 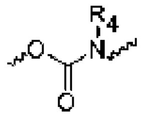 or 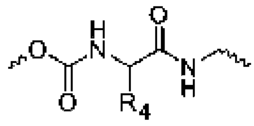 ;

the wavy line at right side of X is connected to D;
$R_4$ is selected from hydrogen, $C_1$-$C_6$ linear or branched alkyl, $-(CH_2CH_2O)_mR_2$, $-CH_2CH_2NR_2R_3$, or $-CH_2CH_2SO_2R_2$-;
$R_2$, and $R_3$ are each independently selected from hydrogen, or $C_1$-$C_6$ linear or branched alkyl; and m is an integer greater than 1.

**98.** The compound, or a tautomer, mesomer, raceme, enantiomer or diastereomer thereof, or mixture thereof, or a pharmaceutically acceptable salt, prodrug or solvate thereof according to claim 97, wherein

X is

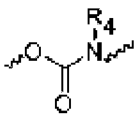 , 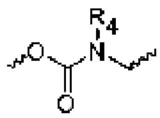 or 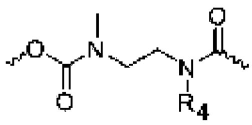 ;

and
$R_4$ is hydrogen, methyl, $-CH_2CH_2OCH_2CH_2OH$, $-CH_2CH_2NMe_2$, or $-CH_2CH_2SO_2Me$.

**99.** The compound, or a tautomer, mesomer, raceme, enantiomer or diastereomer thereof, or mixture thereof, or a pharmaceutically acceptable salt, prodrug or solvate thereof according to claim 97, wherein X is

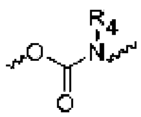 or 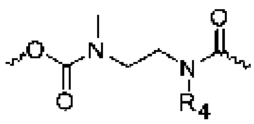

, and $R_4$ is hydrogen or methyl.

**100.**
The compound, or a tautomer, mesomer, raceme, enantiomer or diastereomer thereof, or mixture thereof, or a pharmaceutically acceptable salt, prodrug or solvate thereof according to any one of claims 61-99, wherein the sulfonate inner salt is

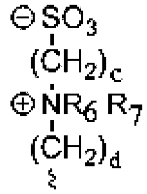 , 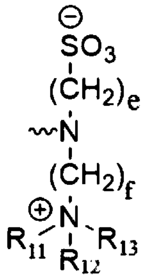 or 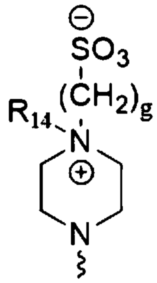 ,

the wavy line is connected to Y, Y' or Y";
wherein c, d, e, f, and g are each independently an integer selected from 1, 2, 3, 4, 5 or 6; and
$R_6$, $R_7$, $R_{11}$, $R_{12}$, $R_{13}$ and $R_{14}$ are each independently selected from absent, hydrogen, $C_1$-$C_6$ linear or branched alkyl.

**101.**
The compound, or a tautomer, mesomer, raceme, enantiomer or diastereomer thereof, or mixture thereof, or a pharmaceutically acceptable salt, prodrug or solvate thereof according to claim 100, wherein the sulfonate inner salt is

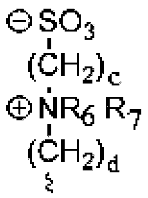 , 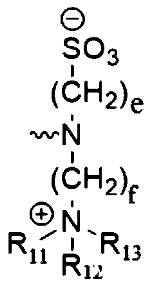 or 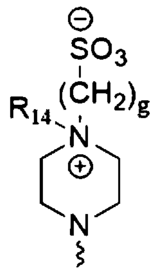 ,

wherein

$R_6$, $R_7$, $R_{11}$, $R_{12}$, $R_{13}$ and $R_{14}$ are each independently selected from absent, methyl or ethyl, and
c, d, e, f, and g are each independently 1, 2, or 3.

**102.**
The compound, or a tautomer, mesomer, raceme, enantiomer or diastereomer thereof, or mixture thereof, or a pharmaceutically acceptable salt, prodrug or solvate thereof according to claim 100, wherein the sulfonate inner salt is any one selected from the group consisting of:

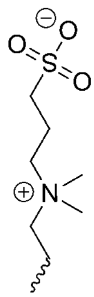 , 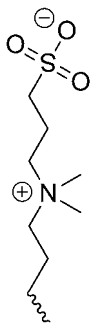 , 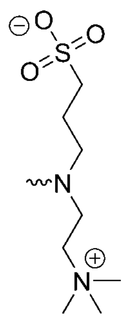 , and 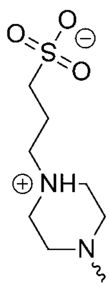 .

**103.**
The compound, or a tautomer, mesomer, raceme, enantiomer or diastereomer thereof, or mixture thereof, or a pharmaceutically acceptable salt, prodrug or solvate thereof according to any one of claims 61-102, wherein the phosphate inner salt is

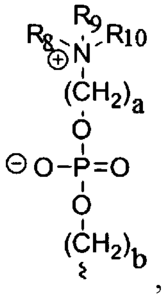

wherein
a and b are each independently an integer selected from 1, 2, 3, 4, 5 or 6; and $R_8$, $R_9$ and $R_{10}$ are each independently selected from hydrogen, or $C_1$-$C_6$ linear or branched alkyl.

**104.**
The compound, or a tautomer, mesomer, raceme, enantiomer or diastereomer thereof, or mixture thereof, or a pharmaceutically acceptable salt, prodrug or solvate thereof according to claim 103, wherein the phosphate inner salt is

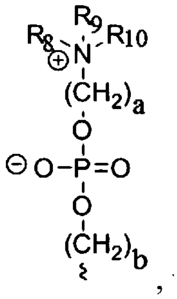

wherein
$R_8$, $R_9$, and $R_{10}$ are each independently methyl or ethyl; a is 1, 2, or 3; and b is 1, 2, or 3.

**105.**
The compound, or a tautomer, mesomer, raceme, enantiomer or diastereomer thereof, or mixture thereof, or a pharmaceutically acceptable salt, prodrug or solvate thereof according to claim 103, wherein the phosphate inner salt is

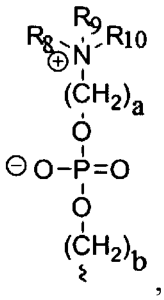

wherein
$R_8$, $R_9$, and $R_{10}$ are each independently methyl; a is 2; and b is 1.

**106.**
The compound, or a tautomer, mesomer, raceme, enantiomer or diastereomer thereof, or mixture thereof, or a pharmaceutically acceptable salt, prodrug or solvate thereof according to any one of claims 61-105, wherein the D comprises a cytotoxin or immune agonist.

**107.**
The compound, or a tautomer, mesomer, raceme, enantiomer or diastereomer thereof, or mixture thereof, or a

pharmaceutically acceptable salt, prodrug or solvate thereof according to claim 106, wherein the cytotoxin is one or two selected from the group consisting of: a microtubulin inhibitor, a DNA synthesis inhibitor, a topoisomerase inhibitor, and a RNA polymerase-2 inhibitor.

**108.**
The compound, or a tautomer, mesomer, raceme, enantiomer or diastereomer thereof, or mixture thereof, or a pharmaceutically acceptable salt, prodrug or solvate thereof according to claim 106, wherein the cytotoxin is one, two, or more selected from the group consisting of: auristatin, mertansine DM, calicheamicin, duocarmycin, pyrrolobenzodiazepine, camptothecin derivative, and $\alpha$-amanitin.

**109.**
The compound, or a tautomer, mesomer, raceme, enantiomer or diastereomer thereof, or mixture thereof, or a pharmaceutically acceptable salt, prodrug or solvate thereof according to claim 106, wherein the immune agonist is selected from TLR7/8 agonist or STING agonist.

**110.**
The compound, or a tautomer, mesomer, raceme, enantiomer or diastereomer thereof, or mixture thereof, or a pharmaceutically acceptable salt, prodrug or solvate thereof according to any one of claims 61-109, wherein i is 2, 3, 4, 5, 6, 7, or 8.

**111.** The compound, or a tautomer, mesomer, raceme, enantiomer or diastereomer thereof, or mixture thereof, or a pharmaceutically acceptable salt, prodrug or solvate thereof according to any one of claims 61-110, wherein h is 1, 2, 3, or 4.

**112.**
The compound, or a tautomer, mesomer, raceme, enantiomer or diastereomer thereof, or mixture thereof, or a pharmaceutically acceptable salt, prodrug or solvate thereof according to any one of claims 61-111, wherein o, p, and q are each independently 0, 1, 2 or 3.

**113.**
The compound, or a tautomer, mesomer, raceme, enantiomer or diastereomer thereof, or mixture thereof, or a pharmaceutically acceptable salt, prodrug or solvate thereof according to any one of claims 61-112, wherein $L_a$ is one, two, three or more selected from the group consisting of: $C_1$-$C_6$ linear or branched alkylene, and polyethylene glycol group;

the polyethylene glycol group is $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, or $-CH_2O(CH_2CH_2O)_nCH_2-$, n is an integer equal to or greater than 1;
$Q_a$ is one, two, three or more selected from the group consisting of: -C(=O)-, -NR(C=O)-, and -(C=O)NR-; and wherein
R is selected from hydrogen, or $C_1$-$C_6$ linear or branched alkyl.

**114.**
The compound, or a tautomer, mesomer, raceme, enantiomer or diastereomer thereof, or mixture thereof, or a pharmaceutically acceptable salt, prodrug or solvate thereof according to any one of claims 61-113, wherein $L_a$ is one, two, three or more selected from the group consisting of: ethylidene, linear propylidene, linear butylidene, linear pentylidene, and $-(CH_2CH_2O)_nCH_2CH_2-$; wherein n is 2, 3, 4, 5, 6, 7 or 8;
$Q_a$ is one, two, three or more selected from the group consisting of: -C(=O)-, -NR-, -NR(C=O)-, and -(C=O)NR-; and wherein R is methyl and/or hydrogen.

**115.**
The compound, or a tautomer, mesomer, raceme, enantiomer or diastereomer thereof, or mixture thereof, or a pharmaceutically acceptable salt, prodrug or solvate thereof according to claim 114, wherein $L_a$ is ethylidene and/or $-(CH_2CH_2O)_4CH_2CH_2-$ or pentylidene; and
$Q_a$ is -C(=O)-, $-N(CH_3)-$, -NH(C=O)-, -C(=O)NH- or $-C(=O)-N(CH_3)-$.

**116.**
The compound, or a tautomer, mesomer, raceme, enantiomer or diastereomer thereof, or mixture thereof, or a pharmaceutically acceptable salt, prodrug or solvate thereof according to any one of claims 61-115, wherein $L_b$ is

selected from $C_1$-$C_6$ linear or branched alkylene, $C_2$-$C_6$ linear or branched alkenylene, or $C_2$-$C_6$ linear or branched alkynylene, arylene with 6-10 carbon atoms in one or more rings, or polyethylene glycol group; and wherein

the polyethylene glycol group is one, two, three or more selected from the group consisting of: $-(CH_2CH_2O)_n-$, $-O(CH_2CH_2O)_n-$, $-(CH_2CH_2O)_nCH_2-$, $-(CH_2CH_2O)_nCH_2CH_2-$, $-CH_2O(CH_2CH_2O)_n-$, and $-CH_2O(CH_2CH_2O)_nCH_2-$; n is an integer equal to or greater than 1; the alkylene, alkenylene, or alkynylene is optionally substituted by a substituent;

$Q_b$ is one, two, three or more selected from the group consisting of: absent, -C(=O)NR-, -C(=O)O-, -OC(=O)NR-, -NRC(=O)-, -OC(=O)-, -NRC(=O)O-, $-NRS(=O)_2-$, -NRC(=O)NR-, -C(=O)-, and -OC(=O)O-; and R is selected from hydrogen, or optionally substituted $C_1$-$C_6$ linear or branched alkyl.

**117.**

The compound, or a tautomer, mesomer, raceme, enantiomer or diastereomer thereof, or mixture thereof, or a pharmaceutically acceptable salt, prodrug or solvate thereof according to claim 116, wherein $L_b$ is one, two, three or more selected from the group consisting of: $C_1$-$C_6$ linear or branched alkylene, and $C_6$-$C_{10}$ arylene;
$Q_b$ is one, two, three or more selected from the group consisting of: absent, -C(=O)-, -C(=O)NR-, -OC(=O)NR-, -NRC(=O)-, -NRC(=O)O-, and -NRC(=O)NR-; and R is selected from hydrogen, or optionally substituted $C_1$-$C_6$ linear or branched alkyl.

**118.**

The compound, or a tautomer, mesomer, raceme, enantiomer or diastereomer thereof, or mixture thereof, or a pharmaceutically acceptable salt, prodrug or solvate thereof according to claim 116, wherein $L_b$ is one, two, three or more selected from the group consisting of: methylene, ethylene, n-propylidene, isopropylidene, n-butylidene, isobutylidene, sec-butylidene, tert-butylidene, and benzylidene;
$Q_b$ is one, two, three or more selected from the group consisting of: absent, -C(=O)-, -NRC(=O)-, -NRC(=O)NR-, and -C(=O)NR-; and R is hydrogen or methyl.

**119.**

The compound, or a tautomer, mesomer, raceme, enantiomer or diastereomer thereof, or mixture thereof, or a pharmaceutically acceptable salt, prodrug or solvate thereof according to any one of claims 61-118, wherein the substituent is any one selected from the group consisting of: deuterium, tritium, halogen, amino, hydroxyl, cyano, nitro, optionally substituted alkyl, alkenyl, alkynyl, optionally substituted heterocyclyl, acyl, optionally substituted amino, optionally substituted aminoformyl, optionally substituted thioaminoformyl, optionally substituted sulfamine, optionally substituted hydroxyls, optionally substituted thioalkyl (SH), and optionally substituted silyl.

**120.**

The compound, or a tautomer, mesomer, raceme, enantiomer or diastereomer thereof, or mixture thereof, or a pharmaceutically acceptable salt, prodrug or solvate thereof according to any one of claims 61-119, wherein j is an integer from 1 to 20.

**121.**

Thecompound, or a tautomer, mesomer, raceme, enantiomer or diastereomer thereof, or mixture thereof, or a pharmaceutically acceptable salt, prodrug or solvate thereof according to any one of claims 61-120, wherein the compound is any one selected from the group consisting of:

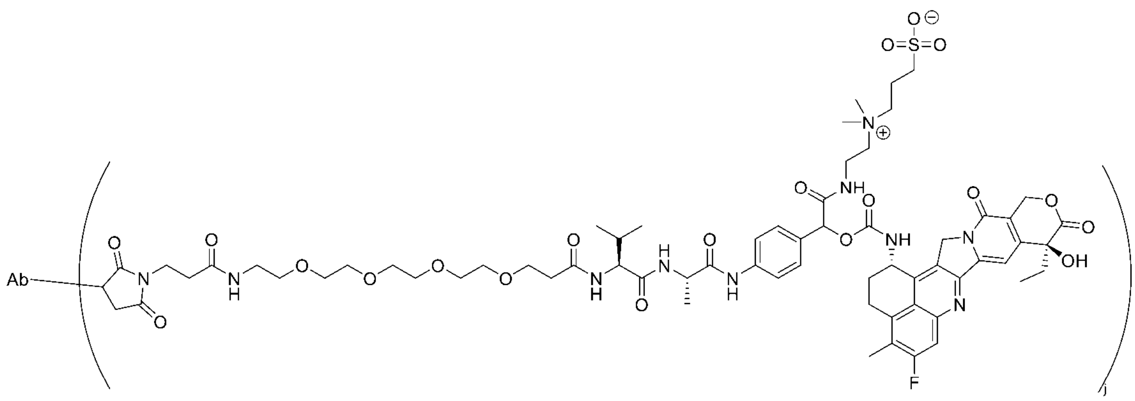
,

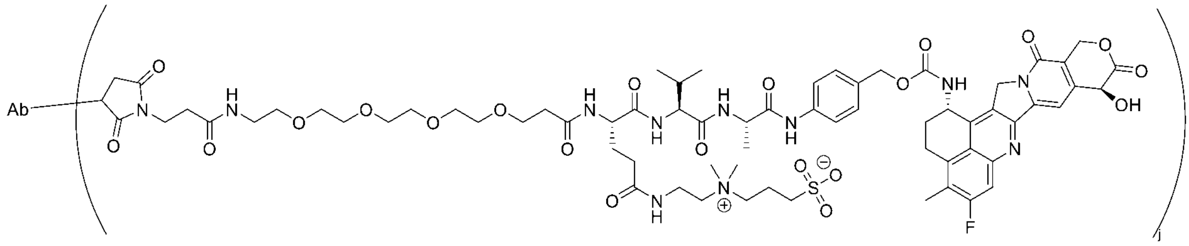

,

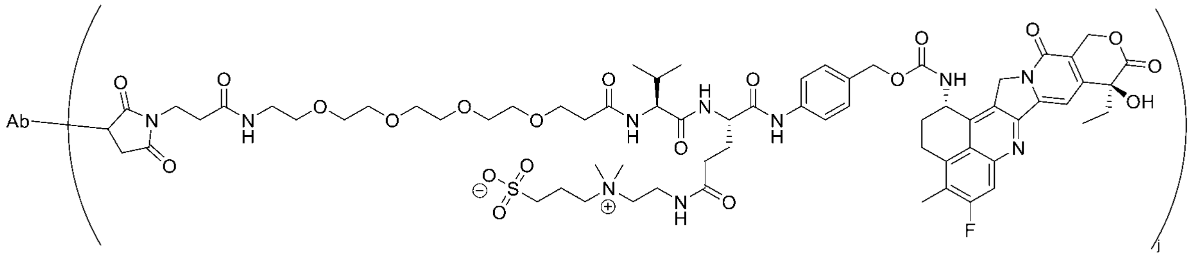

,

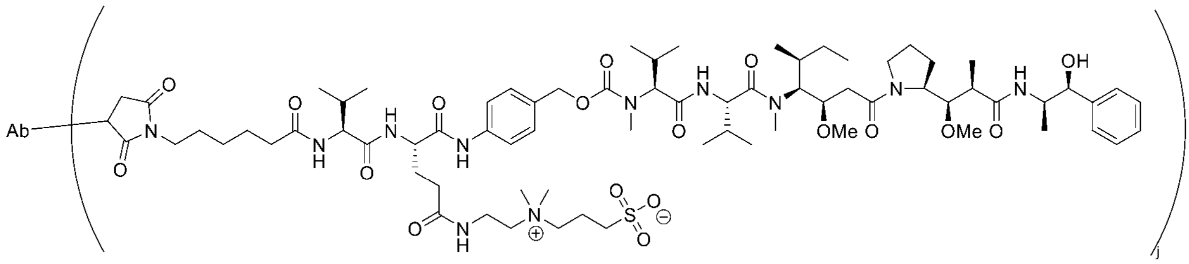

,

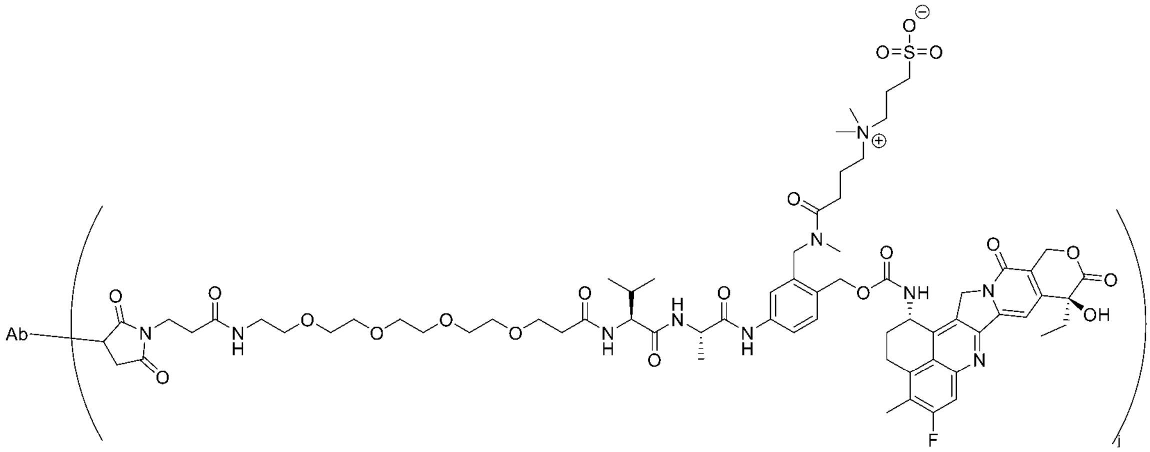

,

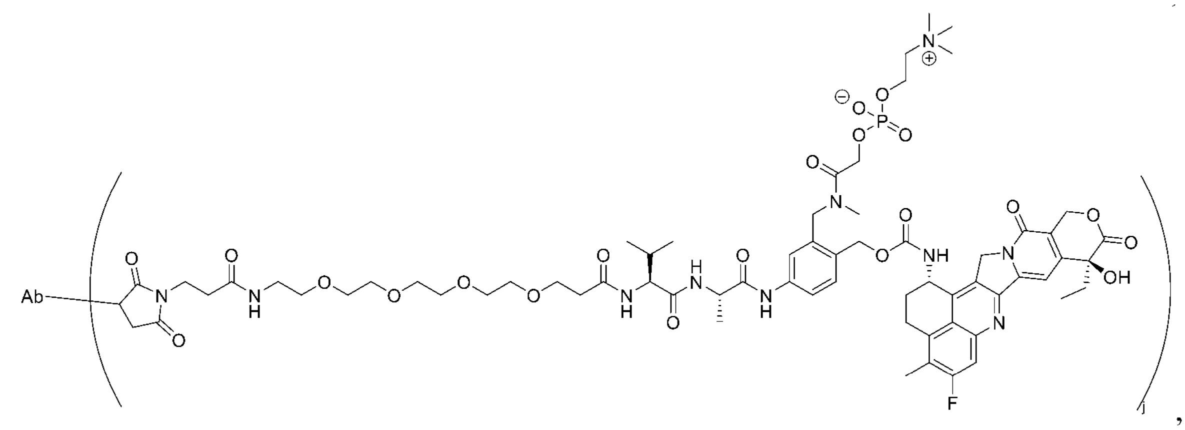

,

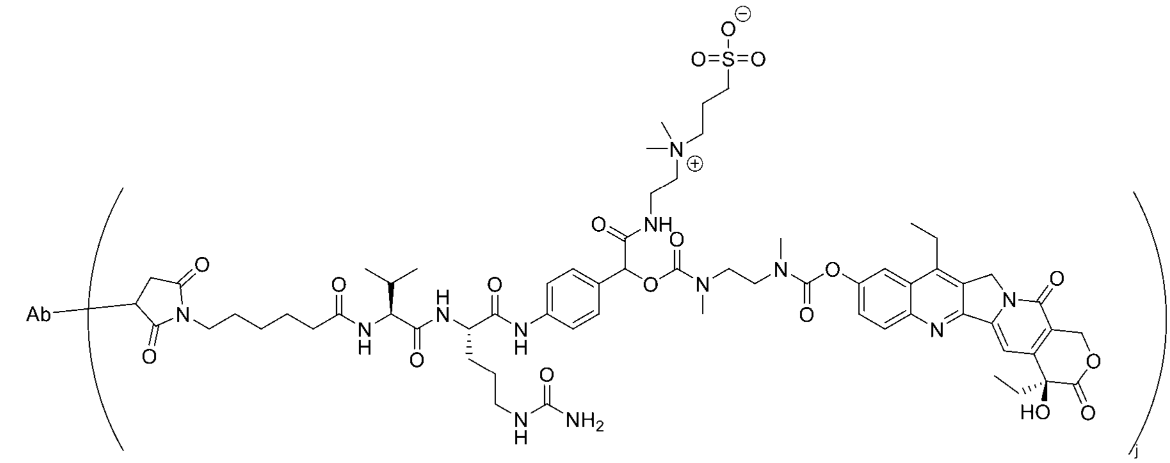

,

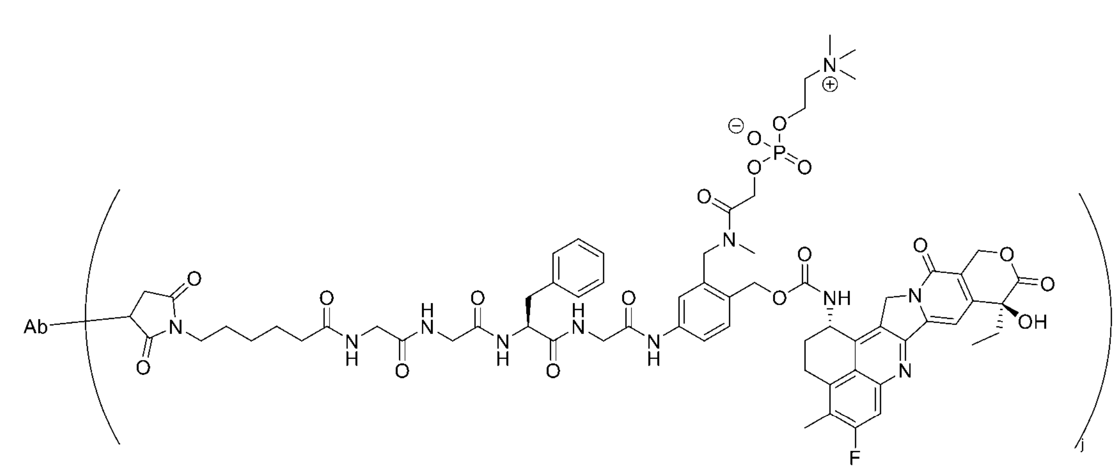

,

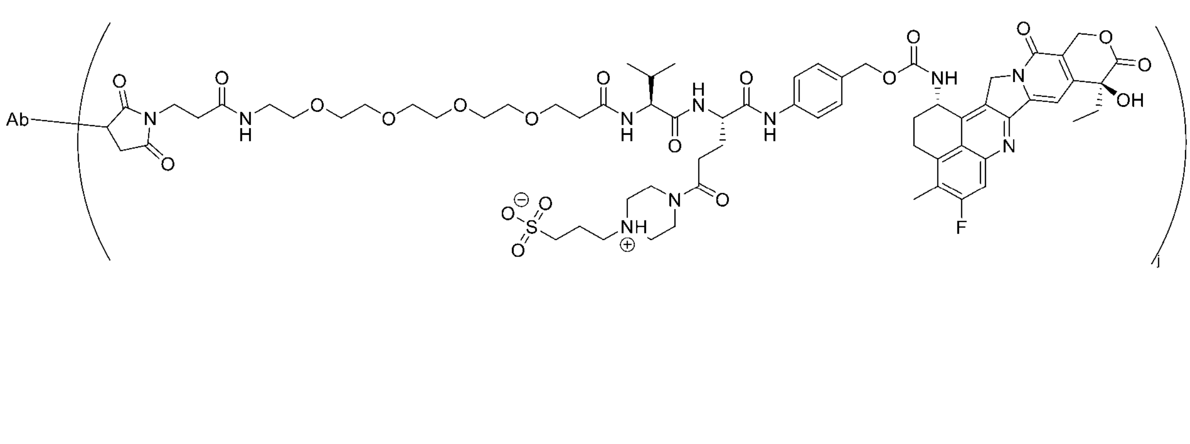

,

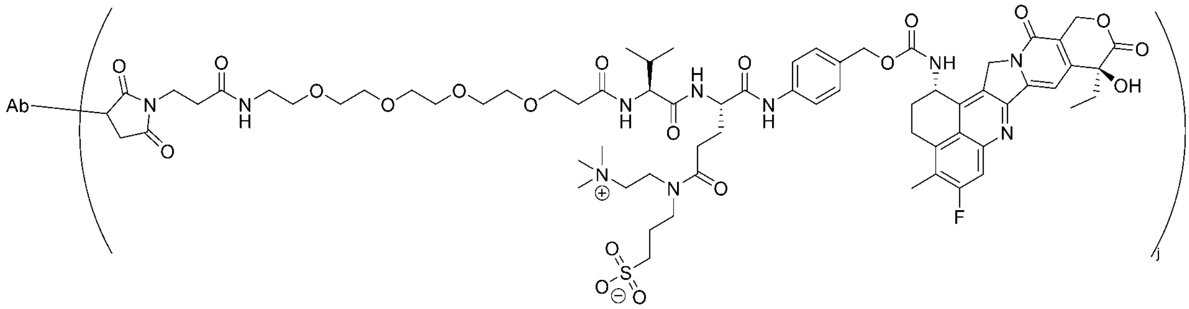

,

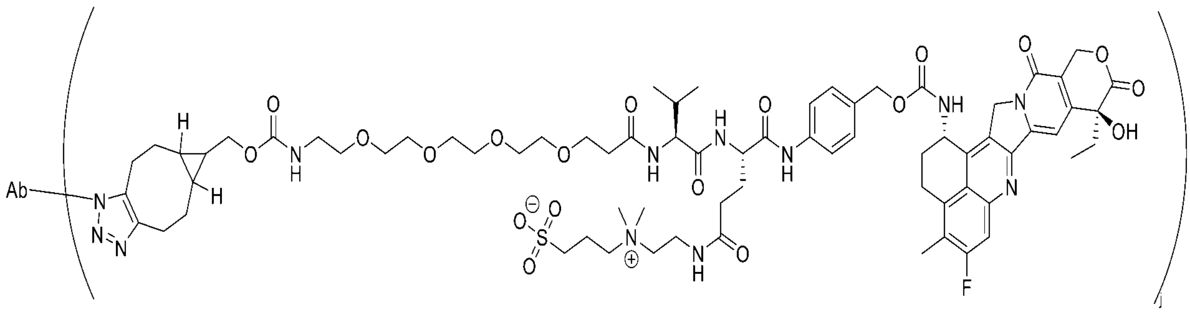

,

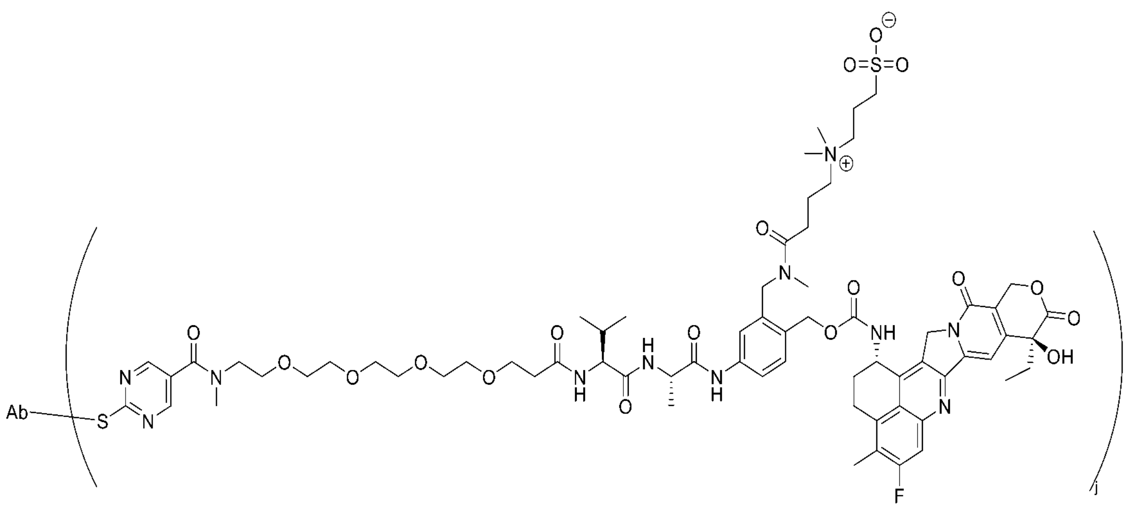

,

and

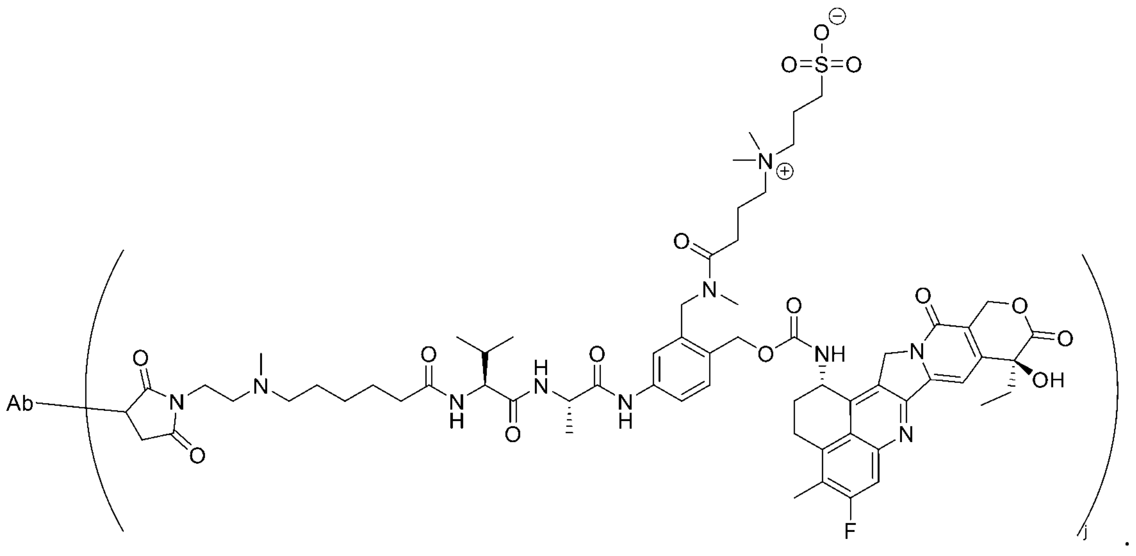

**122.**
The compound, or a tautomer, mesomer, raceme, enantiomer or diastereomer thereof, or mixture thereof, or a pharmaceutically acceptable salt, prodrug or solvate thereof according to any one of claims 61-121, wherein the antibody is selected from the group consisting of: a mouse-derived antibody, a chimeric antibody, a humanized antibody, and a fully human antibody.

**123.**
The compound, or a tautomer, mesomer, raceme, enantiomer or diastereomer thereof, or mixture thereof, or a pharmaceutically acceptable salt, prodrug or solvate thereof according to any one of claims 61-122, wherein the antibody comprises a monoclonal antibody.

**124.**
The compound, or a tautomer, mesomer, raceme, enantiomer or diastereomer thereof, or mixture thereof, or a pharmaceutically acceptable salt, prodrug or solvate thereof according to any one of claims 61-123, wherein the antibody comprises a bispecific antibody.

**125.**
The compound, or a tautomer, mesomer, raceme, enantiomer or diastereomer thereof, or mixture thereof, or a pharmaceutically acceptable salt, prodrug or solvate thereof according to any one of claims 61-124, wherein the antigen-binding fragment is selected from the group consisting of: Fab, Fab', Fv fragment, $F(ab')_2$, $F(ab)_2$, scFv, di-scFv and VHH.

**126.**
The compound, or a tautomer, mesomer, raceme, enantiomer or diastereomer thereof, or mixture thereof, or a pharmaceutically acceptable salt, prodrug or solvate thereof according to any one of claims 61-125, wherein the Ab is selected from the group consisting of: an anti-Claudin18.2 antibody, an anti-human folate receptor antibody, an anti-HER2 antibody, an anti-HER3 antibody, an anti-TROP2 antibody, an anti-B7-H3 antibody, an anti-GPC20 antibody, an anti-CDH6 antibody, an anti-EGFR antibody, an anti-EGFRvIII antibody, an anti-AXL antibody, an anti-Nectin-4 antibody, an anti-Tissue factor antibody, an anti-TIM-1 antibody, an anti-PSMA antibody, an anti-EpCAM antibody, an anti-MUC1 antibody, an anti-STEAP1 antibody, an anti-GPNMB antibody, an anti-FGF2 antibody, an anti-FOLR1 antibody, an anti-c-MET antibody, an anti-GFR antibody, an anti-AGS-16, an anti-Guanylyl cyclase C antibody, an anti-Mesothelin antibody, an anti-SLC44A4 antibody, an anti-EphA2 antibody, an anti-AGS-5 antibody, an anti-GPC-3 antibody, an anti-c-KIT antibody, an anti-ROR1 antibody, an anti-PD-L1 antibody, an anti-CD27L antibody, an anti-5T4 antibody, an anti-Mucin 16 antibody, an anti-NaPi2b antibody, an anti-STEAP antibody, an anti-SLITRK6 antibody, an anti-ETBR antibody, an anti-BCMA antibody, an anti-CEACAM5 antibody, an anti-SC-16 antibody, an anti-SLC39A6 antibody, an anti-Delta-like protein3 antibody, an anti-Claudin18.2 antibody, an anti-CD19 antibody, an anti-CD20 antibody, an anti-CD22 antibody, an anti-CD30 antibody, an anti-CD33 antibody, an anti-CD37 antibody, an anti-CD45 antibody, an anti-CD56 antibody, an anti-CD66e antibody, an anti-CD70 antibody, an anti-CD73 antibody, an anti-CD74 antibody, an anti-CD79b antibody, an anti-CD138 antibody, an anti-CD147 antibody, an anti-CD166 antibody, an anti-CD223 antibody, an anti-MUC16 antibody,

an anti-MSLN antibody, an anti-ENPP3 antibody, an anti-SLTRK6 antibody, an anti-FGFR antibody, an anti-LIV-1 antibody, an anti-Lewis Y antibody, an anti-av-integrin antibody, an anti-ASCT2 antibody, an anti-C4.4a antibody, an anti-CA-IX antibody, an anti-CD324 antibody, an anti-CD352 antibody, an anti-CD44v6 antibody, an anti-CD48a antibody, an anti-CLL-1 antibody, an anti-Cripto antibody, an anti-CS1 antibody, an anti-DPEP3 antibody, an anti-Ephrin-A2 antibody, an anti-Ephrin-A4 antibody, an anti-ETBR antibody, an anti-FGFR2 antibody, an anti-FGFR3 antibody, an anti-FLT3 antibody, an anti-GD3 antibody, an anti-GloboH antibody, an anti-GPC3 antibody, an anti-LAMP-1 antibody, an anti-LRRC15 antibody, an anti-Ly6E antibody, an anti-MFI2 antibody, an anti-NOTCH3 antibody, an anti-p-cadherin antibody, an anti-PRLR antibody and an anti-RNF43 antibody, and antigen-binding fragments of the above antibodies.

**127.**
The compound, or a tautomer, mesomer, raceme, enantiomer or diastereomer thereof, or mixture thereof, or a pharmaceutically acceptable salt, prodrug or solvate thereof according to any one of claims 61-126, wherein the heavy chain HCDR1, HCDR2 and HCDR3, and light chain LCDR1, LCDR2 and LCDR3 of Ab comprise the heavy chain HCDR1, HCDR2 and HCDR3, and light chain LCDR1, LCDR2 and LCDR3 of the antibody, respectively.

**128.**
The compound, or a tautomer, mesomer, raceme, enantiomer or diastereomer thereof, or mixture thereof, or a pharmaceutically acceptable salt, prodrug or solvate thereof according to any one of claims 61-127, wherein the heavy chain variable region (VH) and light chain variable region (VL) of Ab comprise the heavy chain variable region (VH) and light chain variable region (VL) of the antibody, respectively.

**129.**
The compound, or a tautomer, mesomer, raceme, enantiomer or diastereomer thereof, or mixture thereof, or a pharmaceutically acceptable salt, prodrug or solvate thereof according to any one of claims 61-128, wherein the heavy chain and light chain of the Ab comprise the heavy chain and light chain of the antibody, respectively.

**130.**
The compound, or a tautomer, mesomer, raceme, enantiomer or diastereomer thereof, or mixture thereof, or a pharmaceutically acceptable salt, prodrug or solvate thereof according to any one of claims 61-129, wherein the Ab is an anti-Claudin18.2 antibody or an antigen-binding fragment thereof.

**131.**
The compound, or a tautomer, mesomer, raceme, enantiomer or diastereomer thereof, or mixture thereof, or a pharmaceutically acceptable salt, prodrug or solvate thereof according to any one of claims 61-130, wherein the Ab is an anti-human folate receptor antibody or an antigen-binding fragment thereof.

**132.**
A pharmaceutical composition, comprising the compound, or a tautomer, mesomer, raceme, enantiomer or diastereomer thereof, or mixture thereof, or a pharmaceutically acceptable salt, prodrug or solvate thereof according to any one of claims 1-131.

**133.**
Use of the compound, or a tautomer, mesomer, raceme, enantiomer or diastereomer thereof, or mixture thereof, or a pharmaceutically acceptable salt, prodrug or solvate thereof according to any one of claims 1-132 in the preparation of a medicament for treating and/or preventing a tumor.

**134.**
The use according to claim 133, wherein the tumor is a tumor associated with a target selected from the group consisting of: Claudin18.2, human folate receptor, HER2, HER3, TROP2, B7-H3, GPC20, CDH6, EGFR, EGFRvIII, AXL, Nectin-4, Tissue factor, TIM-1, PSMA, EpCAM, MUC1, STEAP1, GPNMB, FGF2, FOLR1, c-MET, GFR, AGS-16, Guanylyl cyclase C, Mesothelin, SLC44A4, EphA2, AGS-5, GPC-3, c-KIT, ROR1, PD-L1, CD27L, 5T4, Mucin 16, NaPi2b, STEAP, SLITRK6, ETBR, BCMA, CEACAM5, SC-16, SLC39A6, Delta-like protein3, Claudin18.2, CD19, CD20, CD22, CD30, CD33, CD37, CD45, CD56, CD66e, CD70, CD73, CD74, CD79b, CD138, CD147, CD166, CD223, MUC16, MSLN, ENPP3, SLTRK6, FGFR, LIV-1, Lewis Y, av-integrin, ASCT2, C4.4a, CA-IX, CD324, CD352, CD44v6, CD48a, CLL-1, Cripto, CS1, DPEP3, Ephrin-A2, Ephrin-A4, ETBR, FGFR2, FGFR3, FLT3, GD3, Globo H, GPC3, LAMP-1, LRRC15, Ly6E, MFI2, NOTCH3, p-cadherin, PRLR and RNF43.

**135.**

The use according to claim 133, wherein the tumor associated with the targets comprises a tumor having high expression of the targets and/or a tumor with positive targets.

**136.**
The use according to claim 133, wherein the tumor is selected from the group consisting of: a solid tumor, a hematological tumor, and metastatic, refractory or recurrent lesion of cancer.

**137.**
The use according to claim 133, wherein the tumor is selected from the group consisting of:
esophageal cancer, gastrointestinal cancer, pancreatic cancer, thyroid cancer, colorectal cancer, kidney cancer, lung cancer (such as non-small cell lung cancer), liver cancer, stomach cancer, gastric adenocarcinoma, gastroesophageal junction (GEJ) adenocarcinoma, head and neck cancer, bladder cancer, breast cancer, uterine cancer, cervical cancer, ovarian cancer, prostatic cancer, testicular cancer, germ cell cancer, bone cancer, skin cancer, thymus cancer, bile duct cancer, gallbladder cancer, melanoma, mesothelioma, lymphoma, myeloma (such as multiple myeloma), sarcoma, glioblastoma and leukemia.

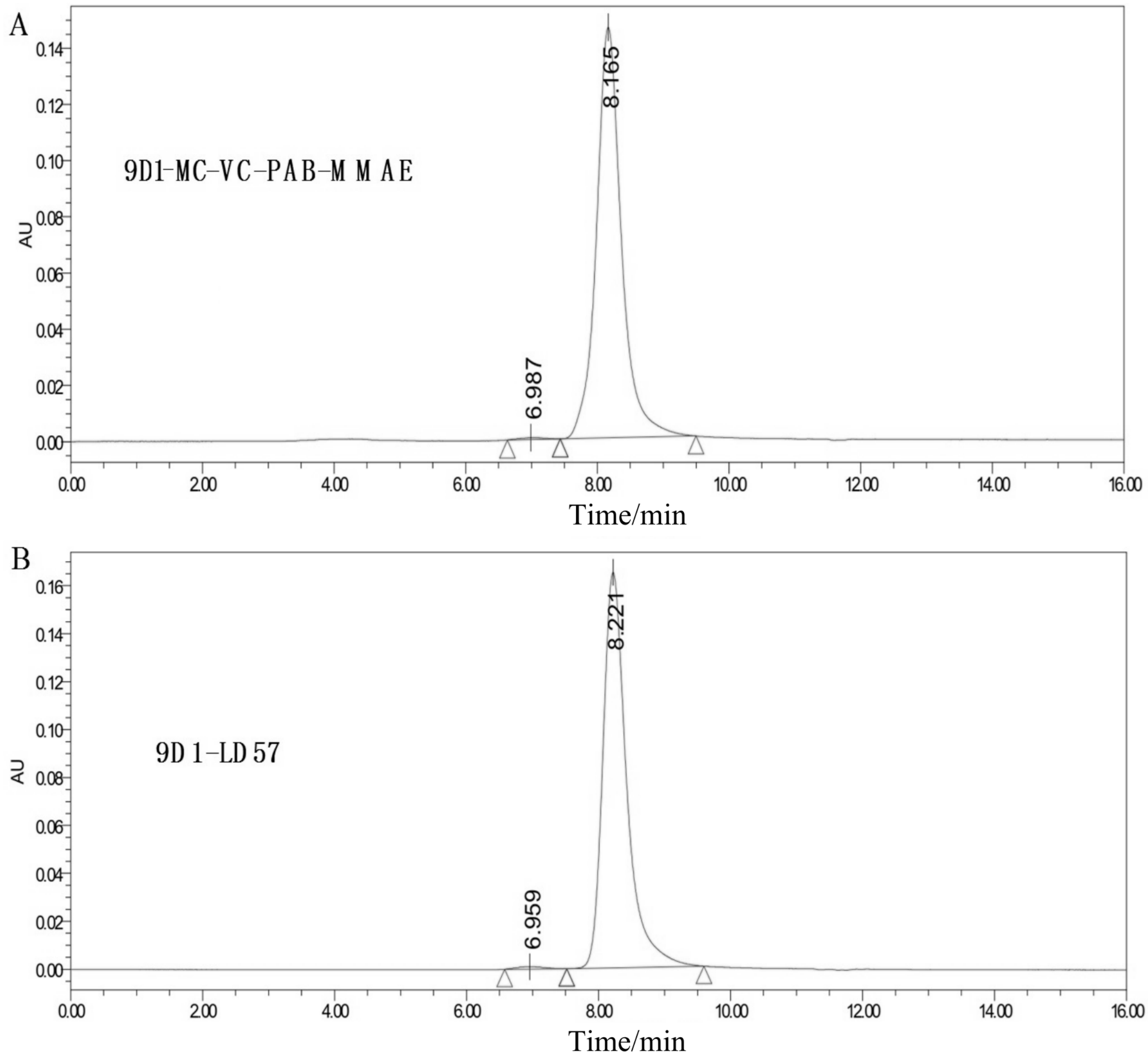
A
9D1-MC-VC-PAB-MMAE
AU
8.165
6.987
Time/min
B
9D1-LD57
AU
8.221
6.959
Time/min

Fig. 1

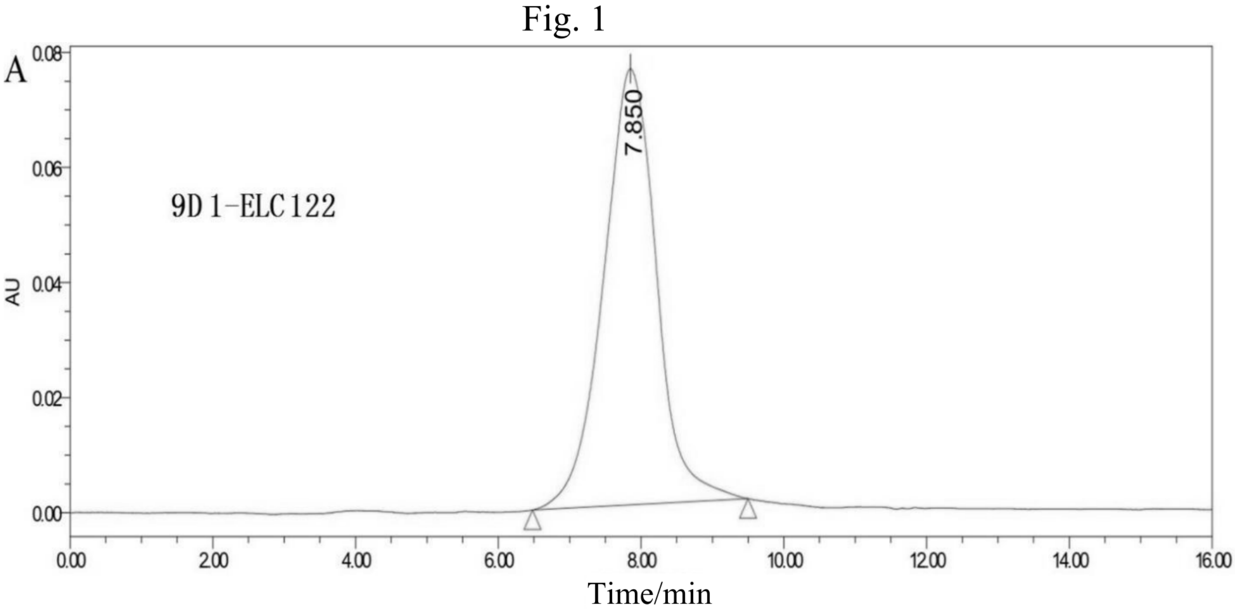
A
9D1-ELC122
AU
7.850
Time/min

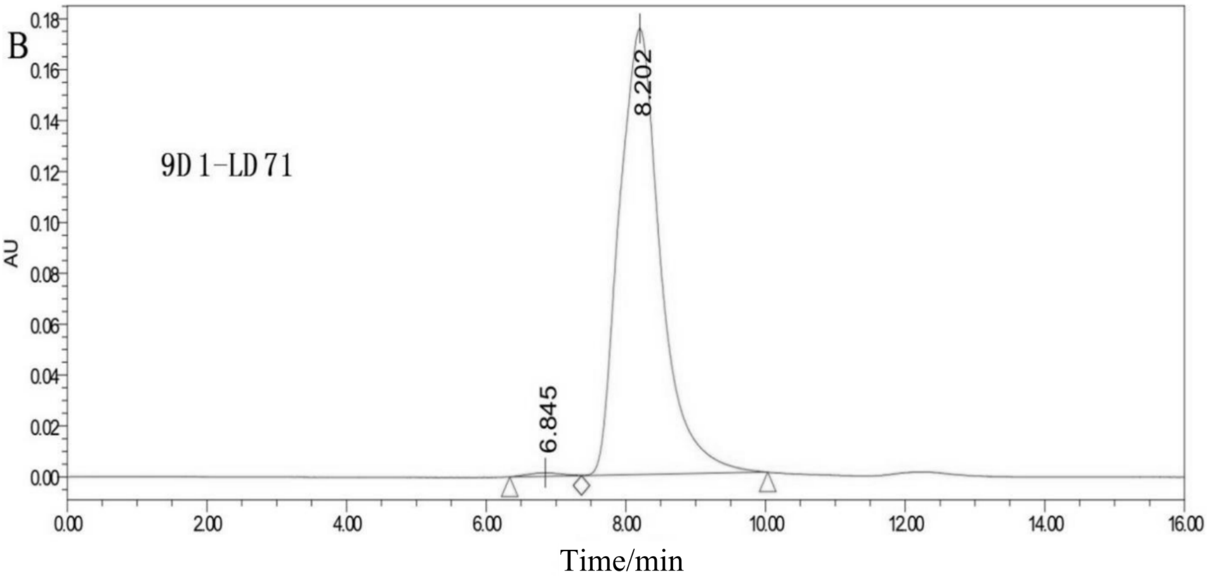
B
9D1-LD71
8.202
6.845
AU
Time/min

Fig. 2

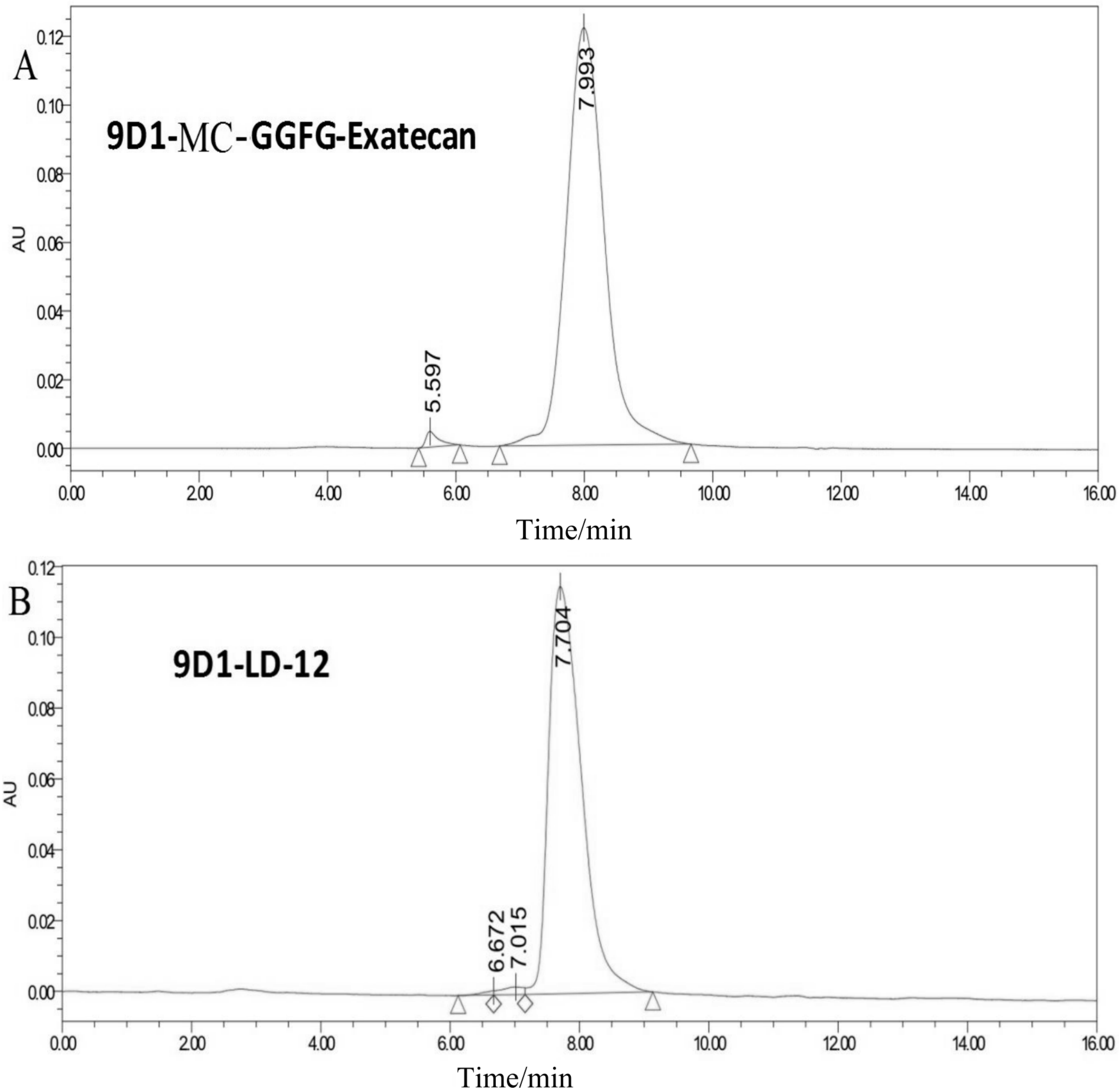
A
9D1-MC-GGFG-Exatecan
7.993
5.597
AU
Time/min
B
9D1-LD-12
7.704
6.672
7.015
AU
Time/min

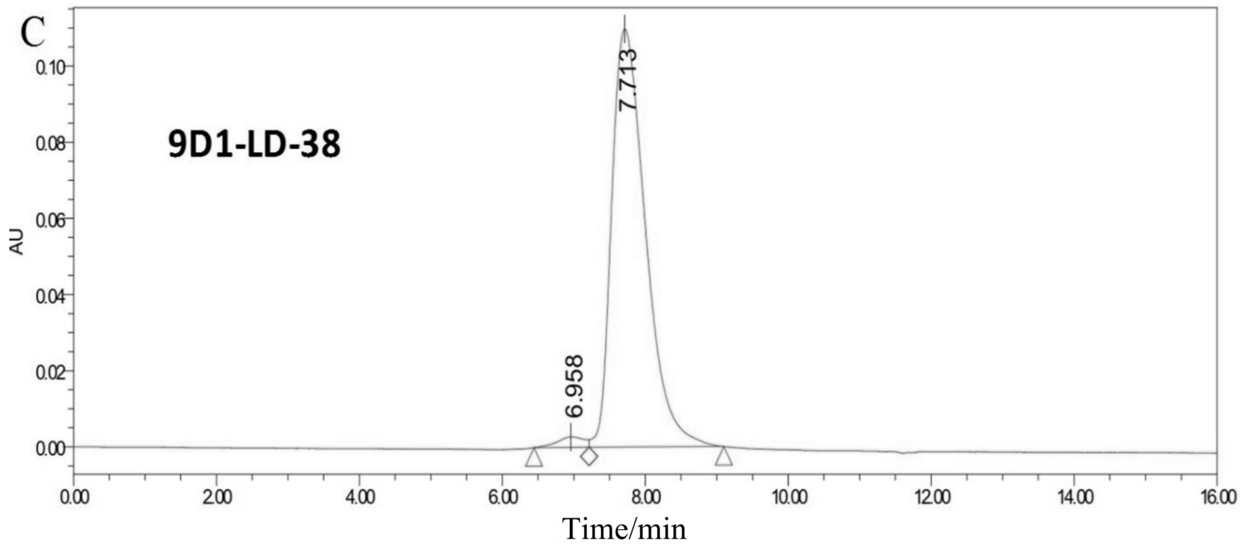
C
9D1-LD-38
7.713
6.958
AU
0.00
0.02
0.04
0.06
0.08
0.10
0.00
2.00
4.00
6.00
8.00
10.00
12.00
14.00
16.00
Time/min

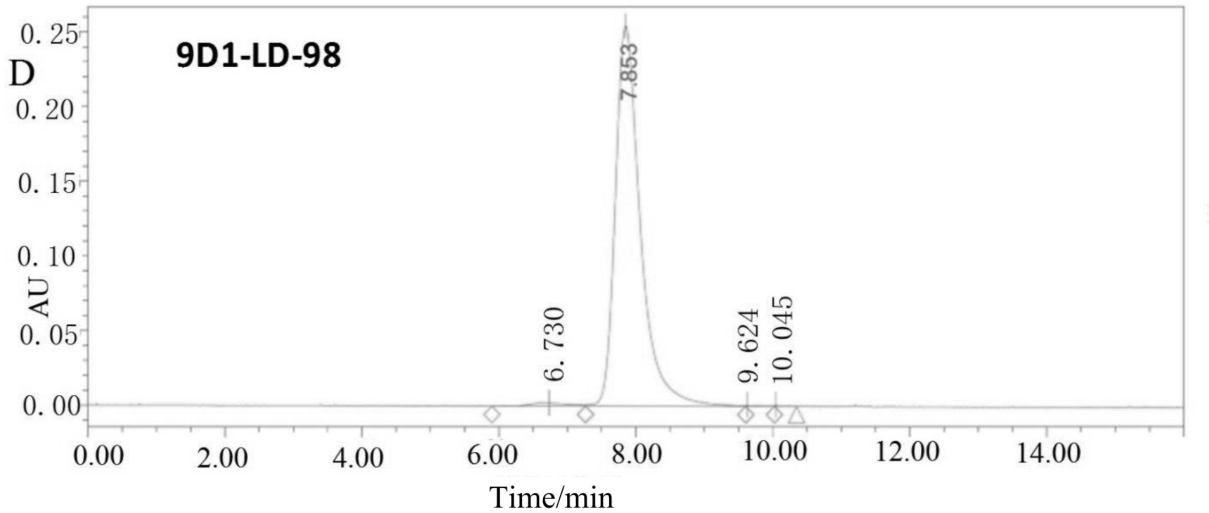
D
9D1-LD-98
7.853
6. 730
9. 624
10. 045
AU
0. 00
0. 05
0. 10
0. 15
0. 20
0. 25
0.00
2.00
4.00
6.00
8.00
10.00
12.00
14.00
Time/min

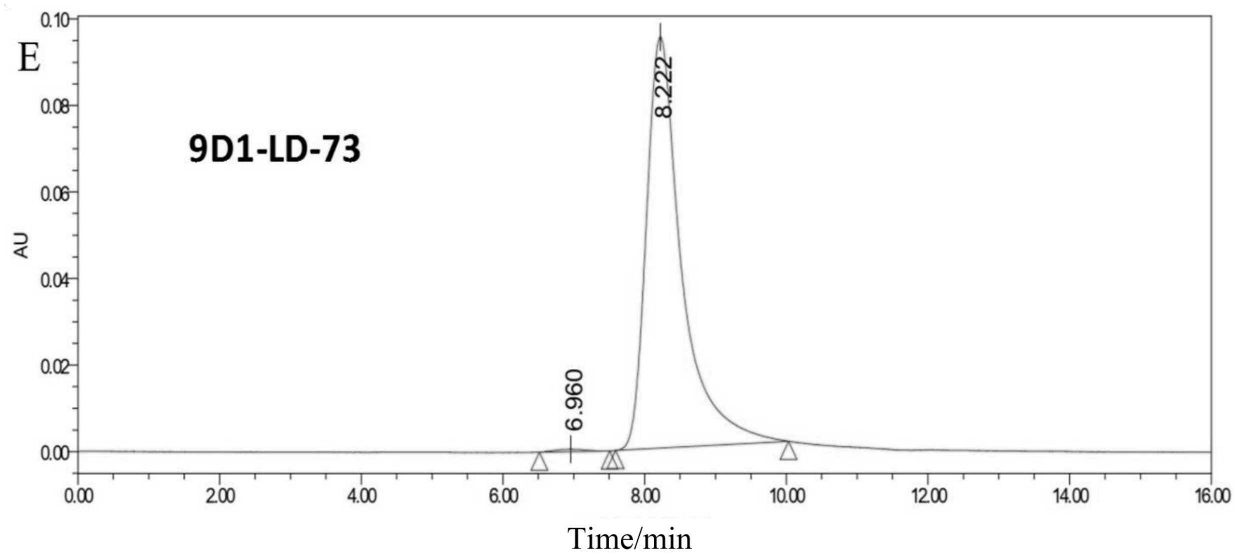
E
9D1-LD-73
8.222
6.960
AU
0.00
0.02
0.04
0.06
0.08
0.10
0.00
2.00
4.00
6.00
8.00
10.00
12.00
14.00
16.00
Time/min

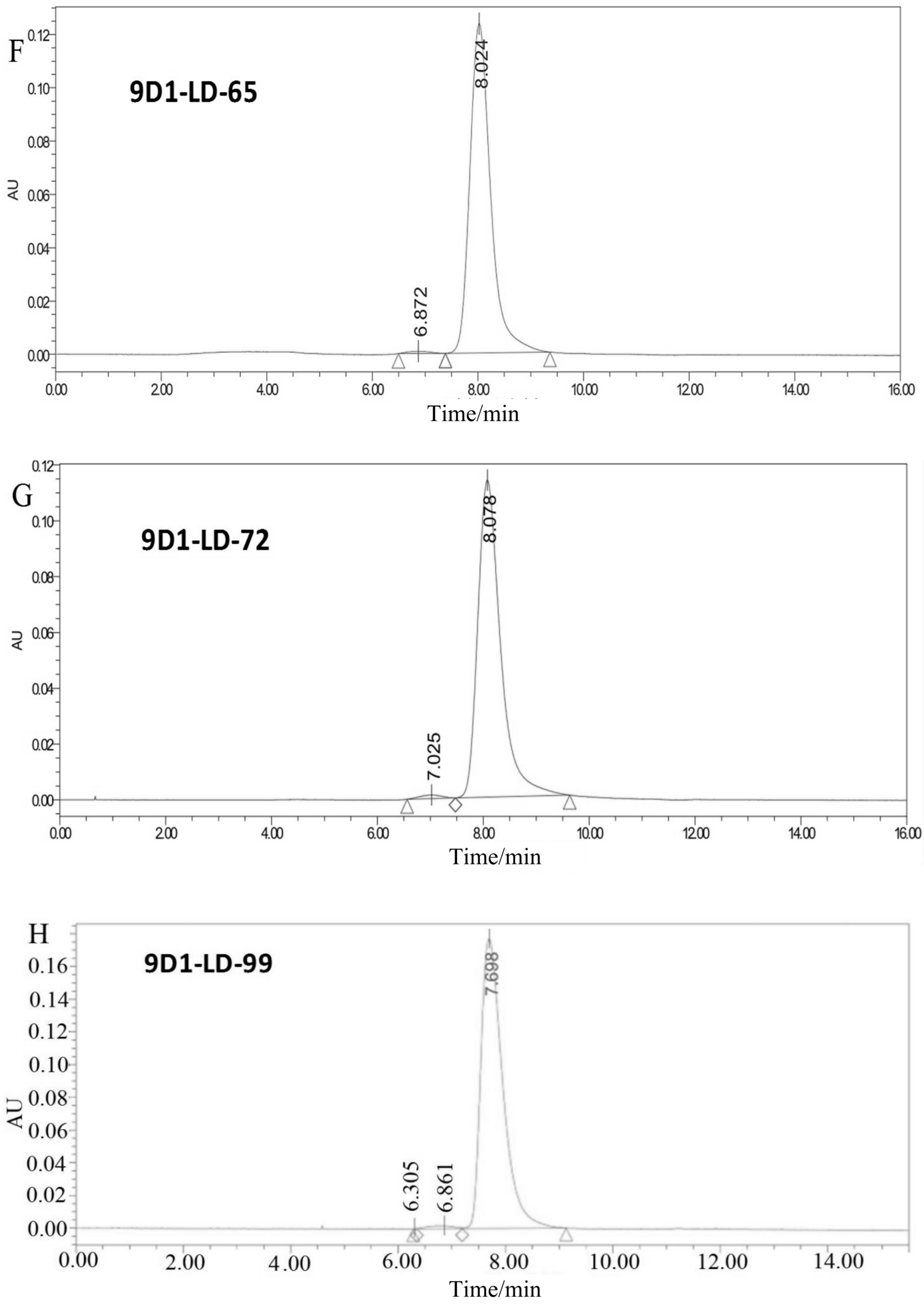
F
9D1-LD-65
8.024
6.872
AU
Time/min
G
9D1-LD-72
8.078
7.025
AU
Time/min
H
9D1-LD-99
7.698
6.305
6.861
AU
Time/min

Fig. 3

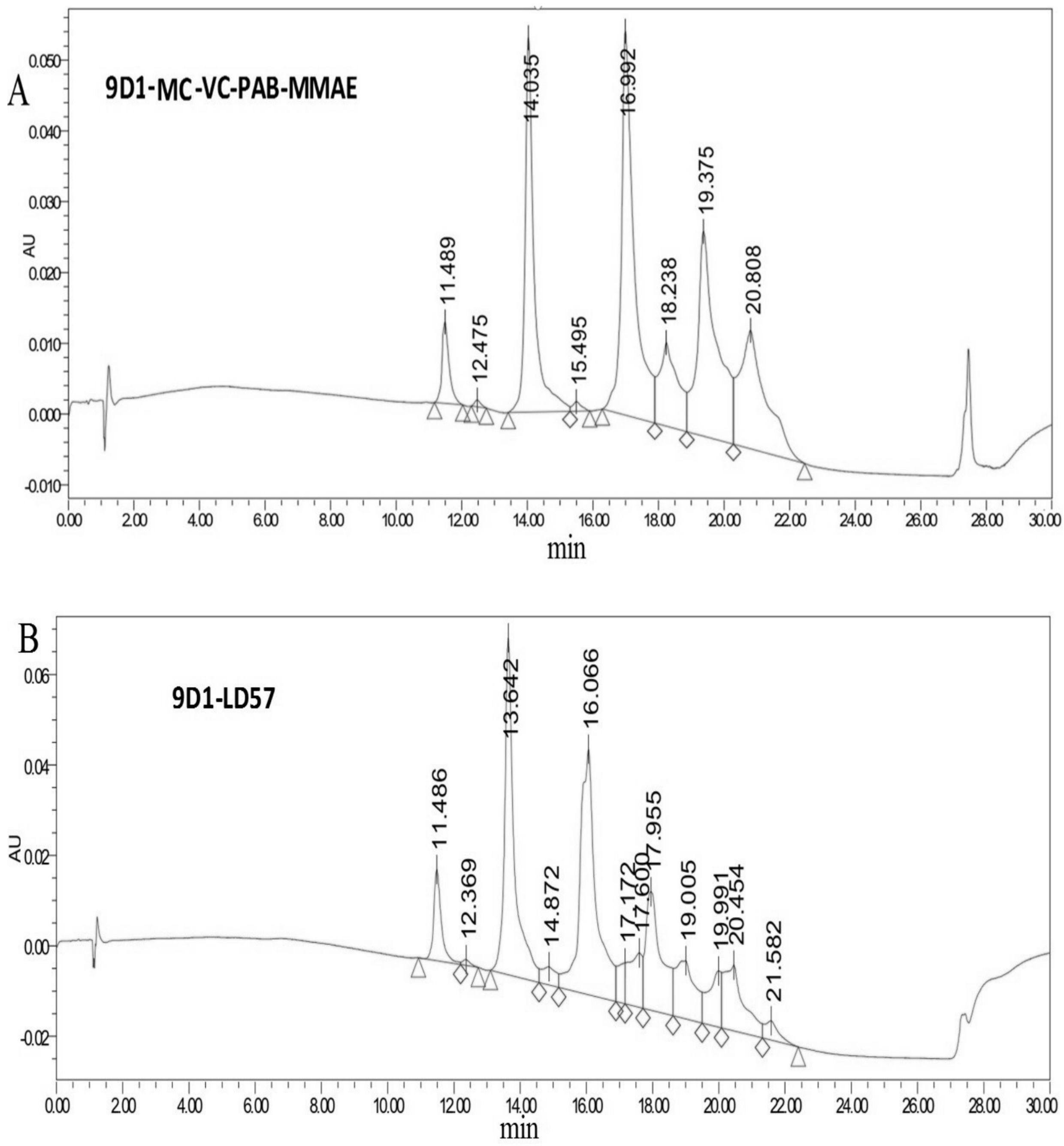
A
9D1-MC-VC-PAB-MMAE
AU
min
11.489
12.475
14.035
15.495
16.992
18.238
19.375
20.808
B
9D1-LD57
11.486
12.369
13.642
14.872
16.066
17.172
17.600
17.955
19.005
19.991
20.454
21.582

Fig. 4

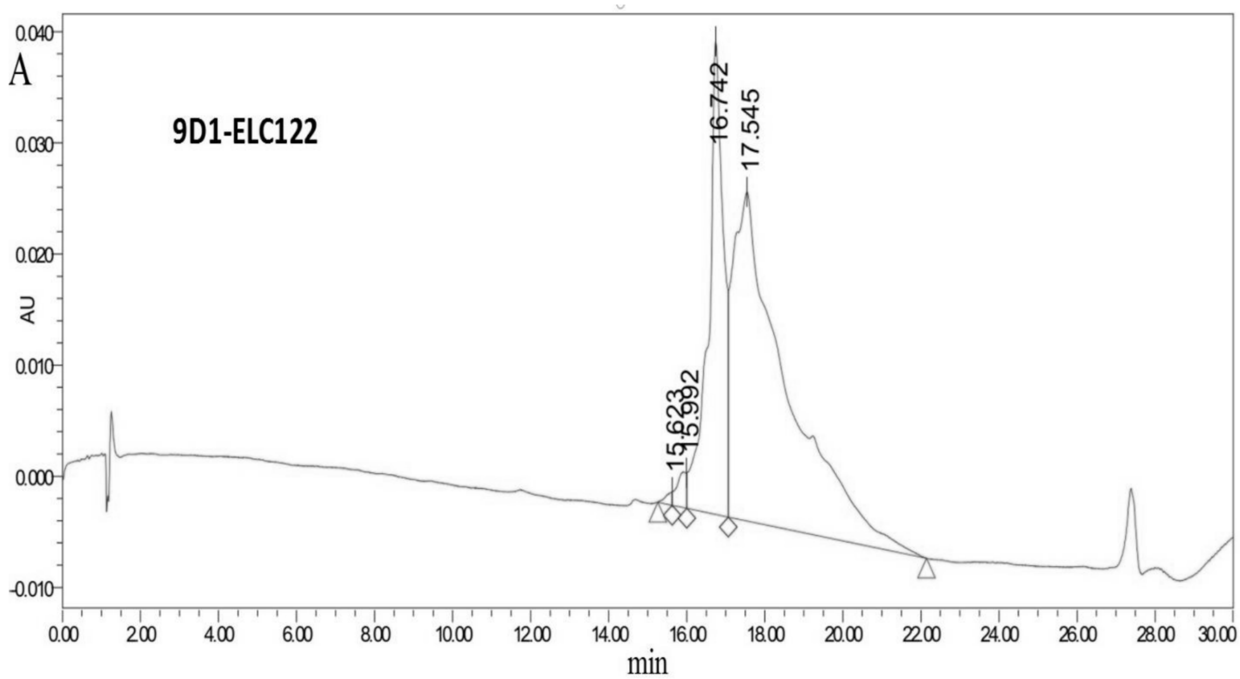
A
9D1-ELC122
0.040
0.030
0.020
0.010
0.000
-0.010
AU
16.742
17.545
15.623
15.992
0.00
2.00
4.00
6.00
8.00
10.00
12.00
14.00
16.00
18.00
20.00
22.00
24.00
26.00
28.00
30.00
min

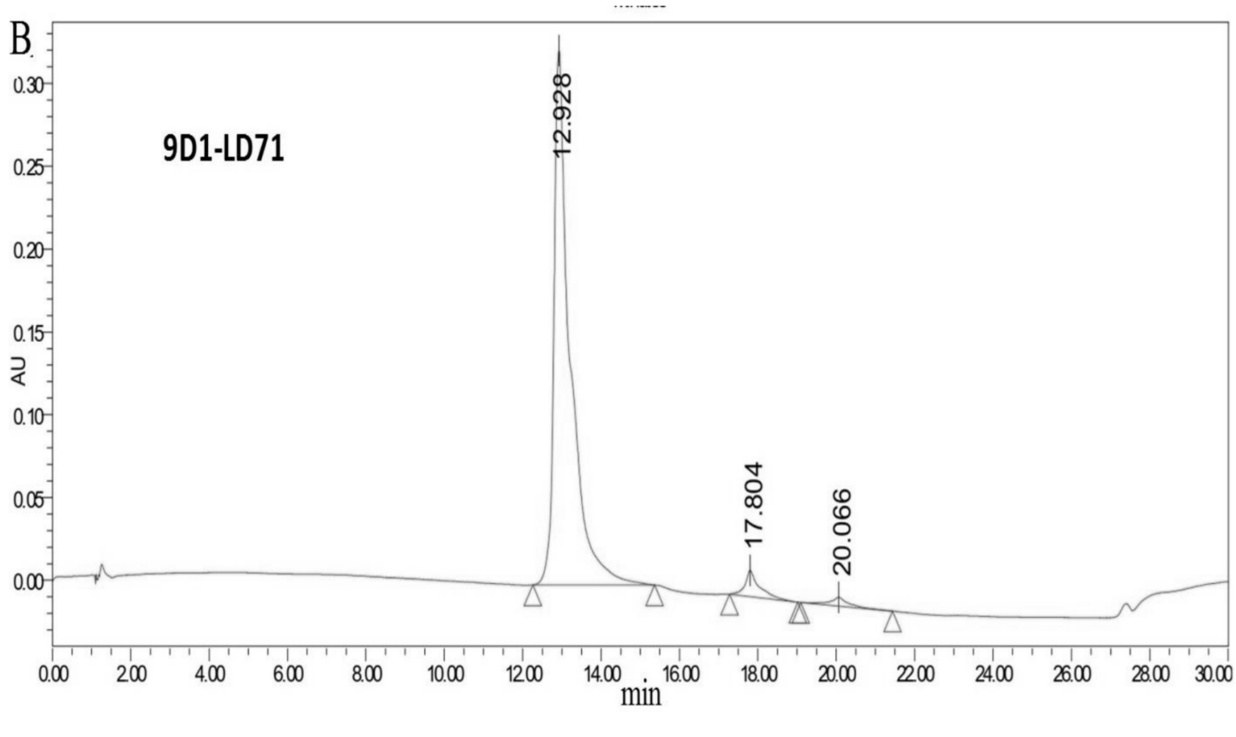
B
9D1-LD71
0.30
0.25
0.20
0.15
0.10
0.05
0.00
AU
12.928
17.804
20.066
0.00
2.00
4.00
6.00
8.00
10.00
12.00
14.00
16.00
18.00
20.00
22.00
24.00
26.00
28.00
30.00
min

Fig. 5

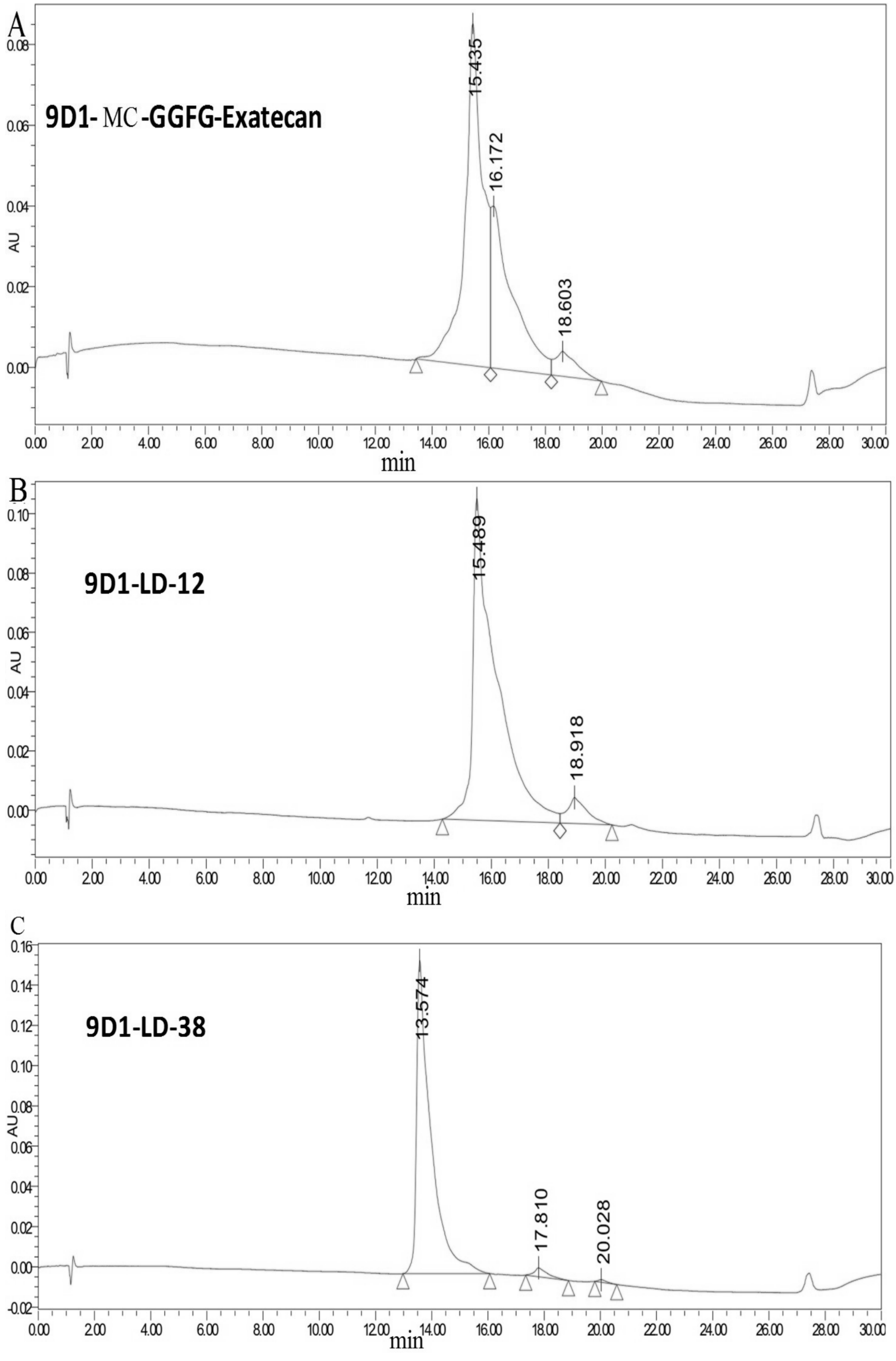
A
9D1- MC -GGFG-Exatecan
15.435
16.172
18.603
AU
min
B
9D1-LD-12
15.489
18.918
AU
min
C
9D1-LD-38
13.574
17.810
20.028
AU
min

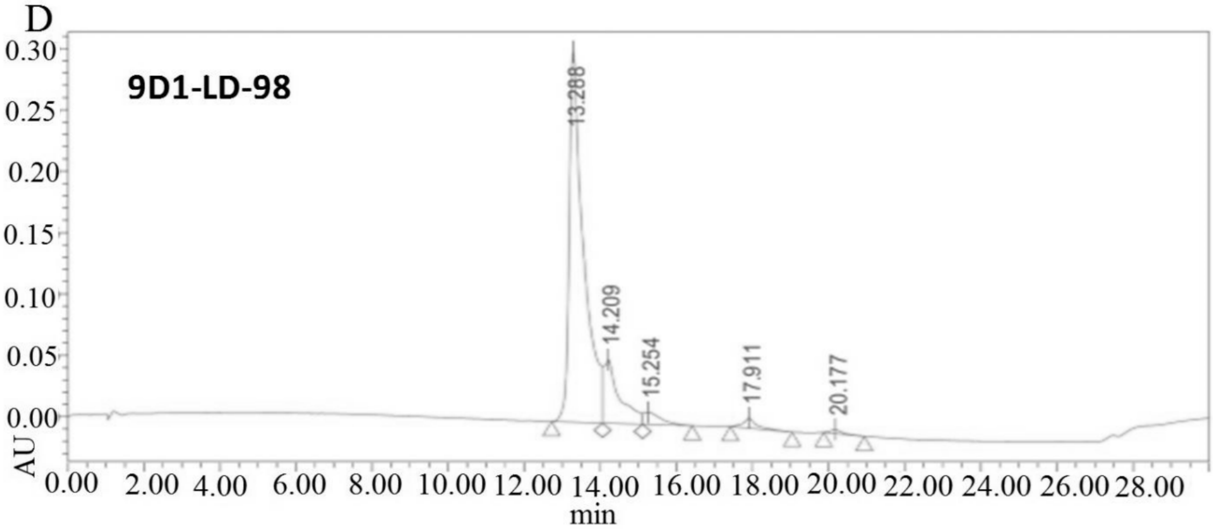
D
9D1-LD-98
13.288
14.209
15.254
17.911
20.177
0.30
0.25
0.20
0.15
0.10
0.05
0.00
AU
0.00 2.00 4.00 6.00 8.00 10.00 12.00 14.00 16.00 18.00 20.00 22.00 24.00 26.00 28.00
min

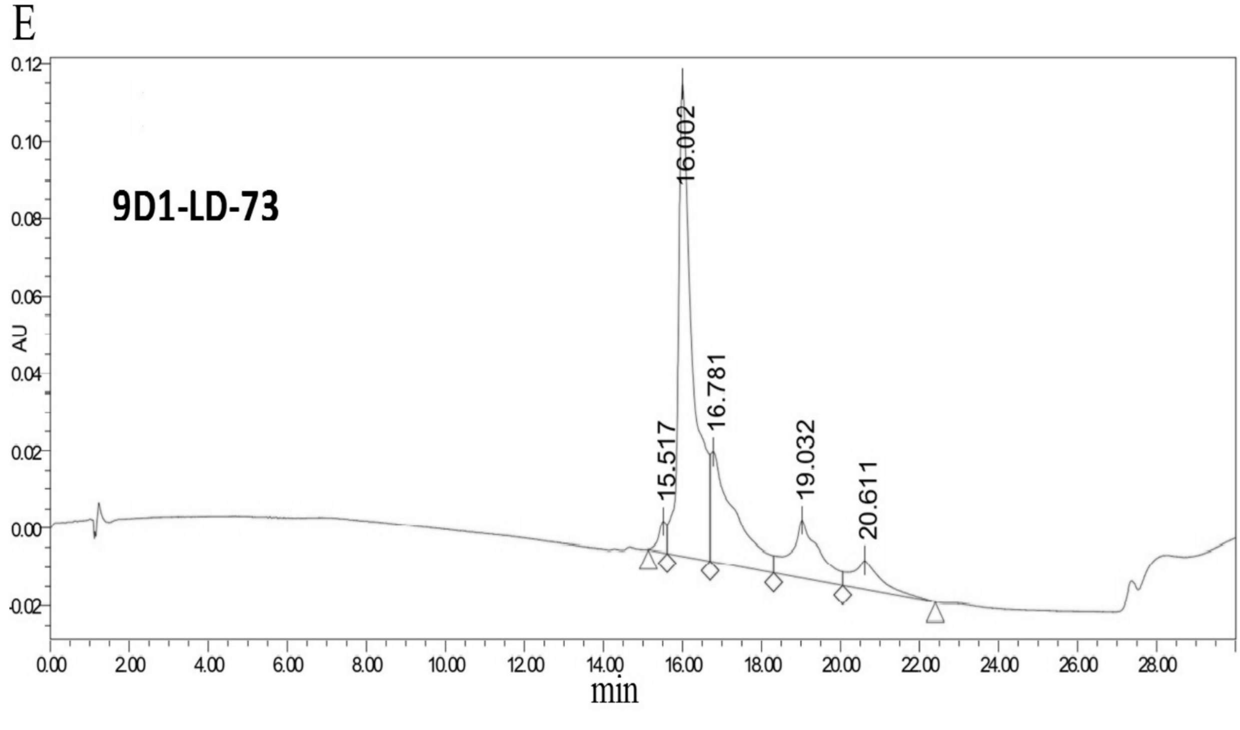
E
9D1-LD-73
16.002
15.517
16.781
19.032
20.611
0.12
0.10
0.08
0.06
0.04
0.02
0.00
-0.02
AU
0.00 2.00 4.00 6.00 8.00 10.00 12.00 14.00 16.00 18.00 20.00 22.00 24.00 26.00 28.00
min

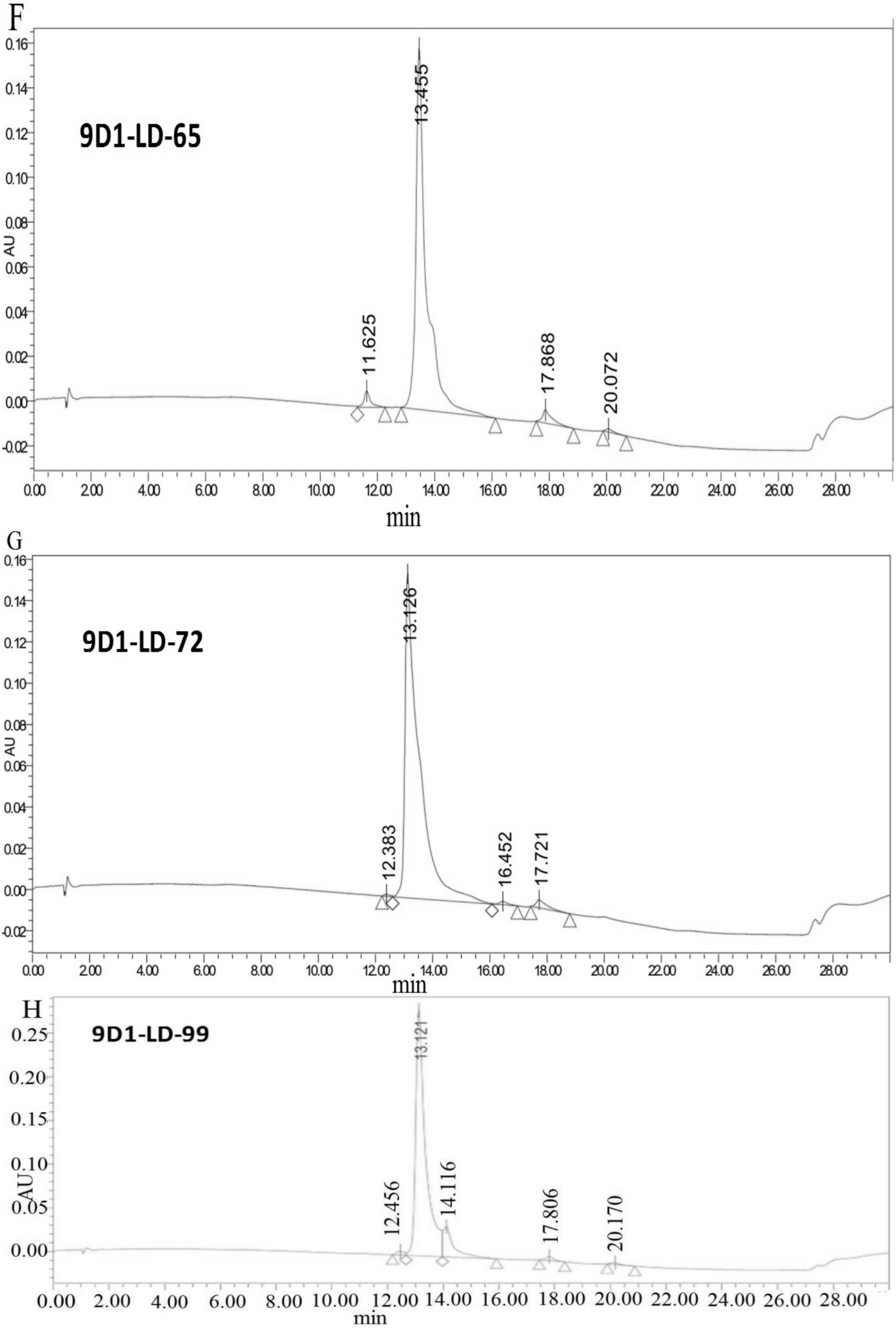
F
9D1-LD-65
13.455
11.625
17.868
20.072
AU
min
G
9D1-LD-72
13.126
12.383
16.452
17.721
AU
min
H
9D1-LD-99
13.121
12.456
14.116
17.806
20.170
AU
min

Fig. 6

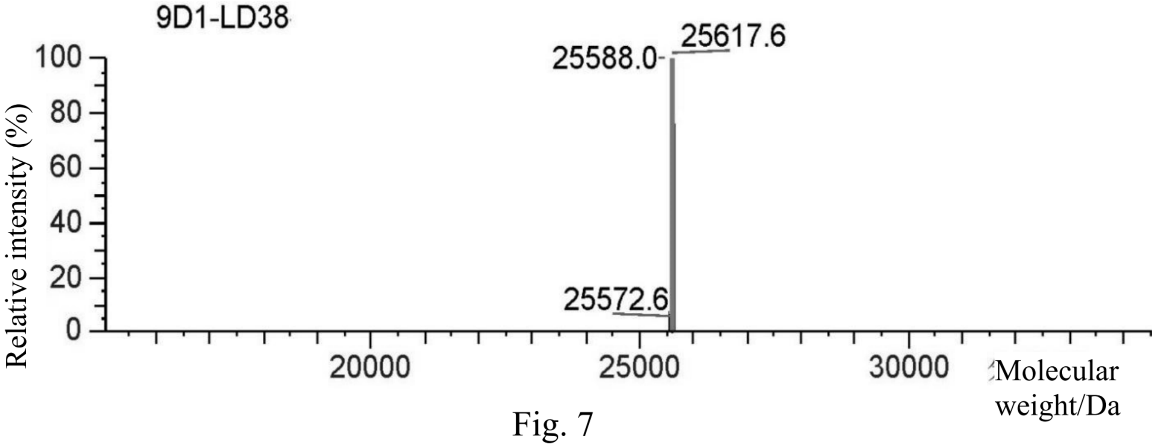


Fig. 7

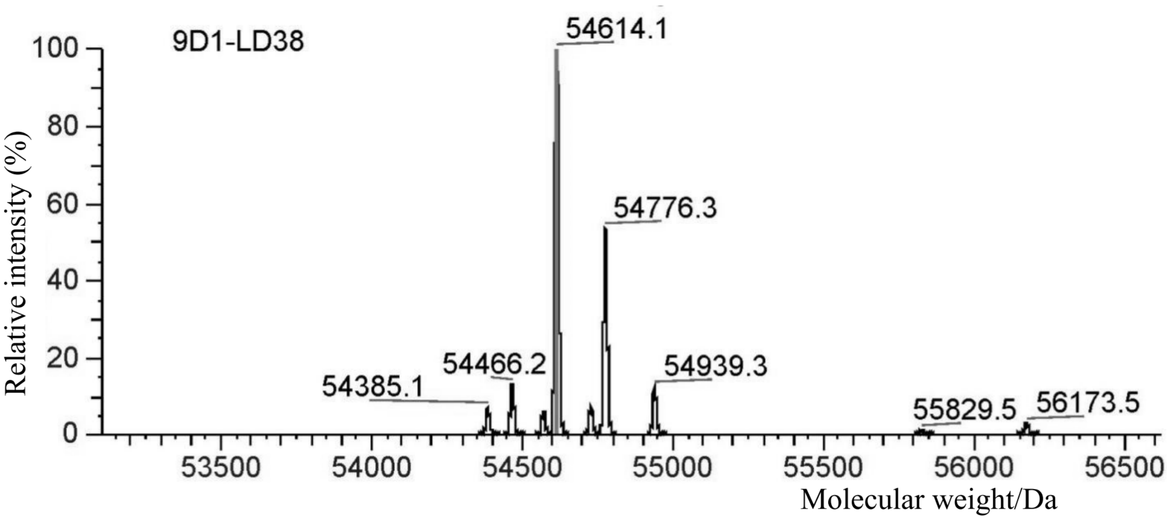


Fig. 8

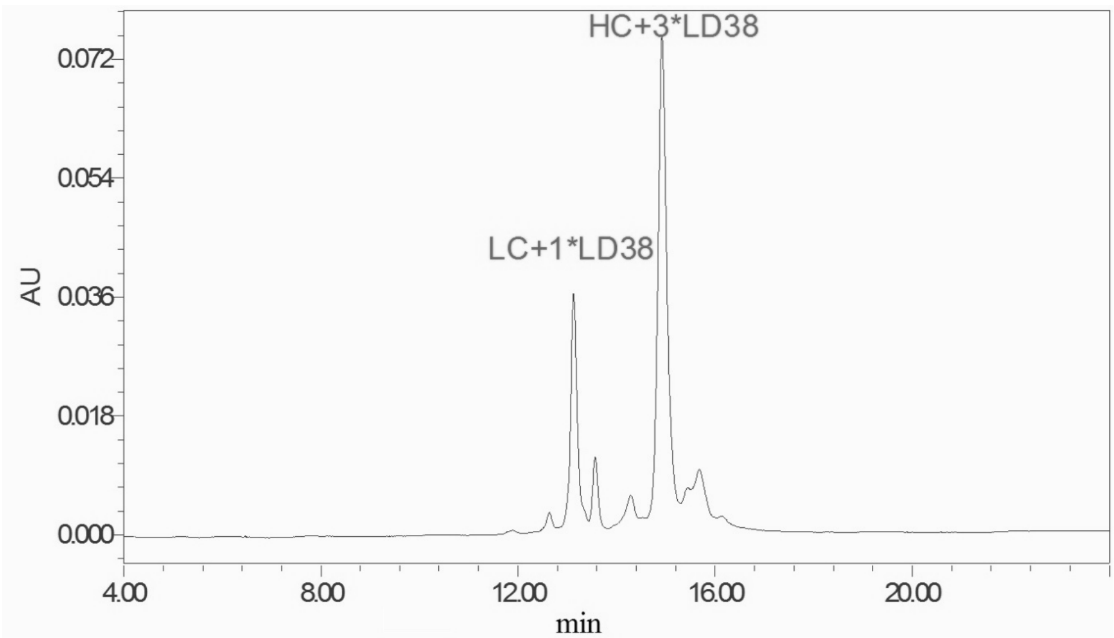


Fig. 9

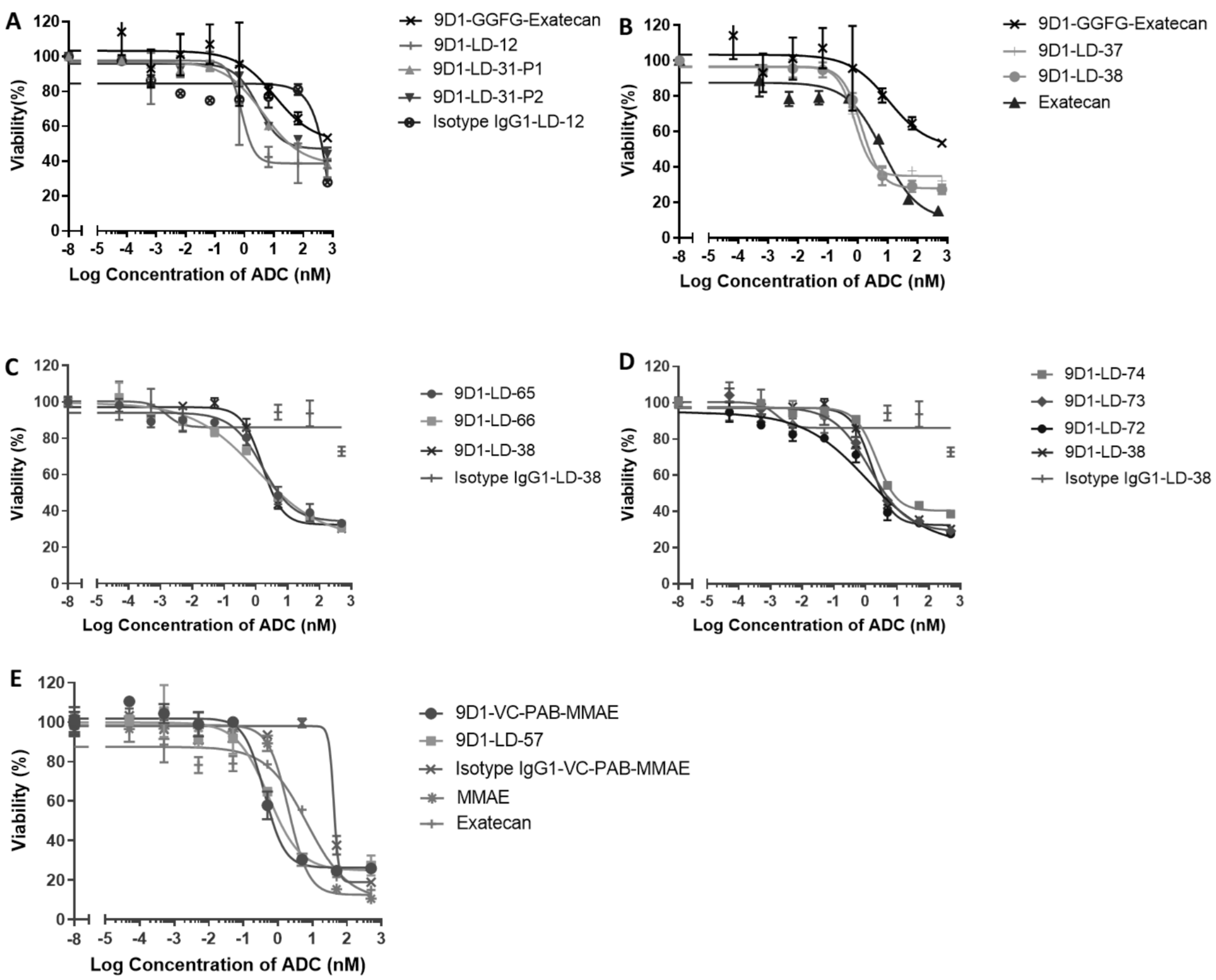
A
B
C
D
E
Viability(%)
Viability (%)
Log Concentration of ADC (nM)
9D1-GGFG-Exatecan
9D1-LD-12
9D1-LD-31-P1
9D1-LD-31-P2
Isotype IgG1-LD-12
9D1-LD-37
9D1-LD-38
Exatecan
9D1-LD-65
9D1-LD-66
Isotype IgG1-LD-38
9D1-LD-74
9D1-LD-73
9D1-LD-72
9D1-VC-PAB-MMAE
9D1-LD-57
Isotype IgG1-VC-PAB-MMAE
MMAE

Fig. 10

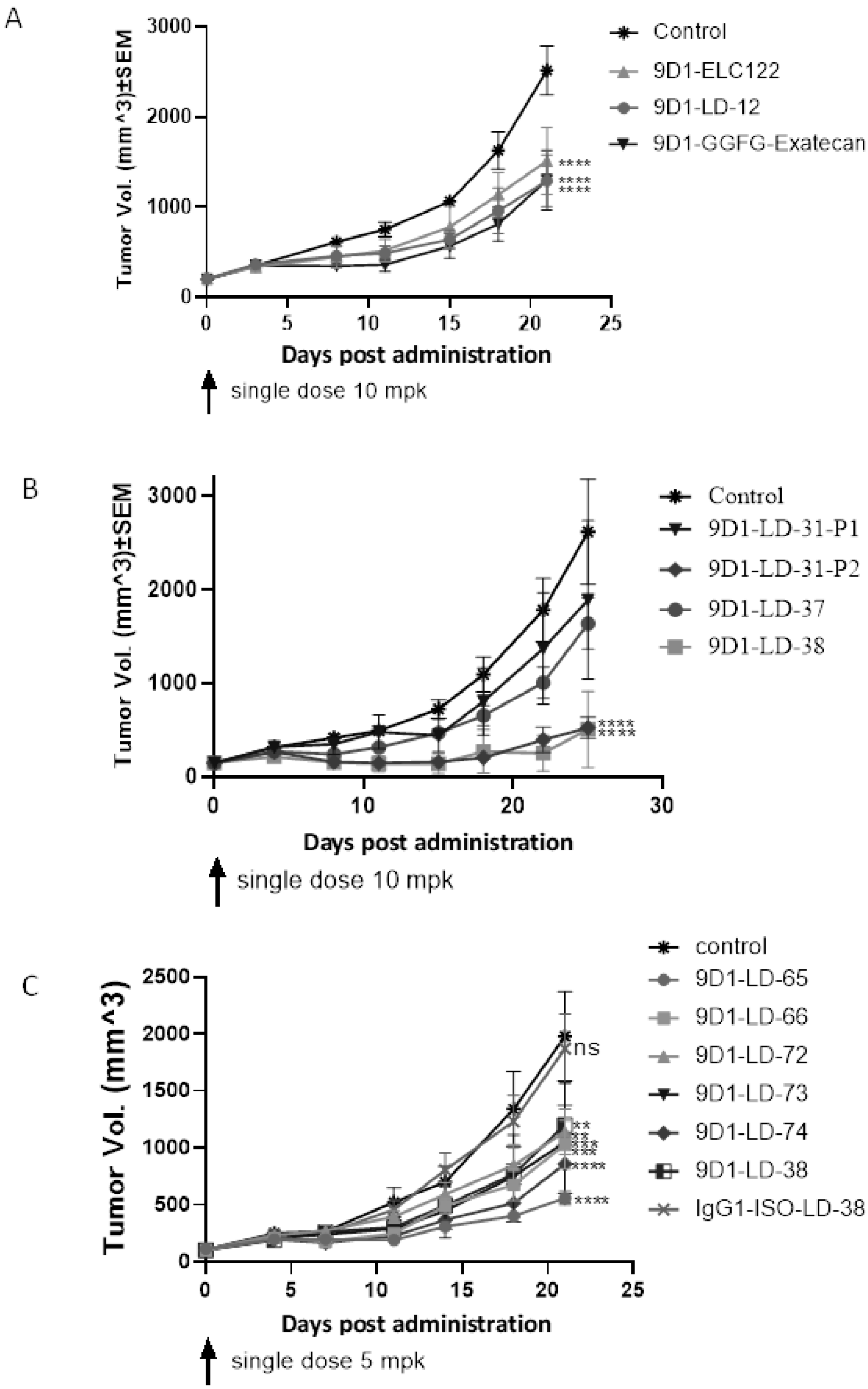
A
Tumor Vol. (mm^3)±SEM
3000
2000
1000
0
0
5
10
15
20
25
Days post administration
single dose 10 mpk
Control
9D1-ELC122
9D1-LD-12
9D1-GGFG-Exatecan
****
****
****
B
Tumor Vol. (mm^3)±SEM
3000
2000
1000
0
0
10
20
30
Days post administration
single dose 10 mpk
Control
9D1-LD-31-P1
9D1-LD-31-P2
9D1-LD-37
9D1-LD-38
****
****
C
Tumor Vol. (mm^3)
2500
2000
1500
1000
500
0
0
5
10
15
20
25
Days post administration
single dose 5 mpk
control
9D1-LD-65
9D1-LD-66
9D1-LD-72
9D1-LD-73
9D1-LD-74
9D1-LD-38
IgG1-ISO-LD-38
ns
****
****

Fig. 11

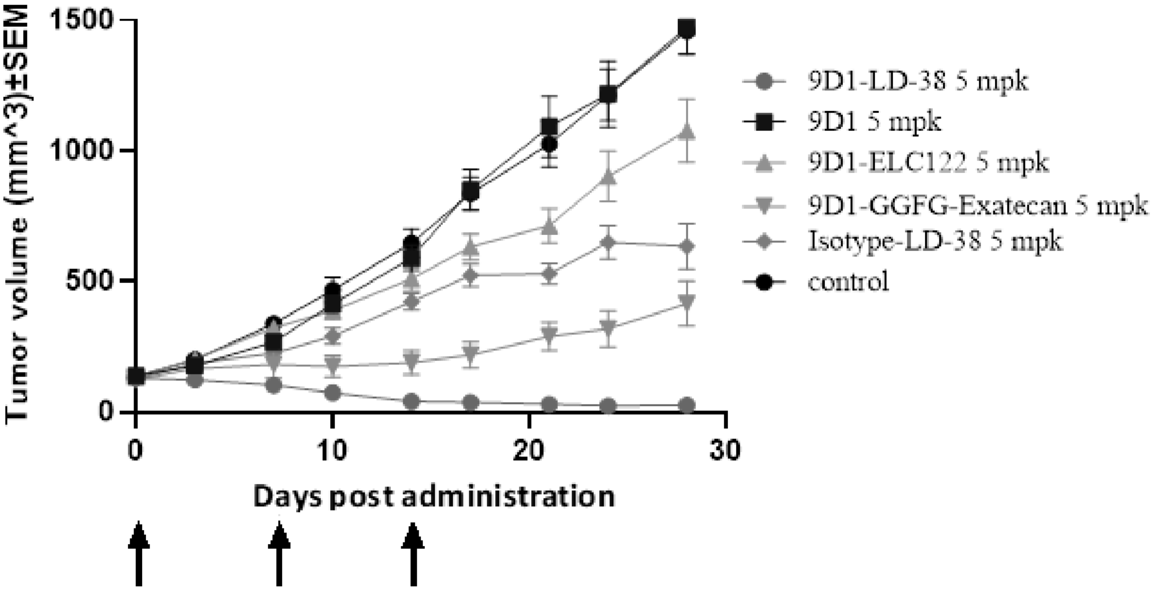
Tumor volume (mm^3)±SEM
1500
1000
500
0
0
10
20
30
Days post administration
9D1-LD-38 5 mpk
9D1 5 mpk
9D1-ELC122 5 mpk
9D1-GGFG-Exatecan 5 mpk
Isotype-LD-38 5 mpk
control

Fig. 12

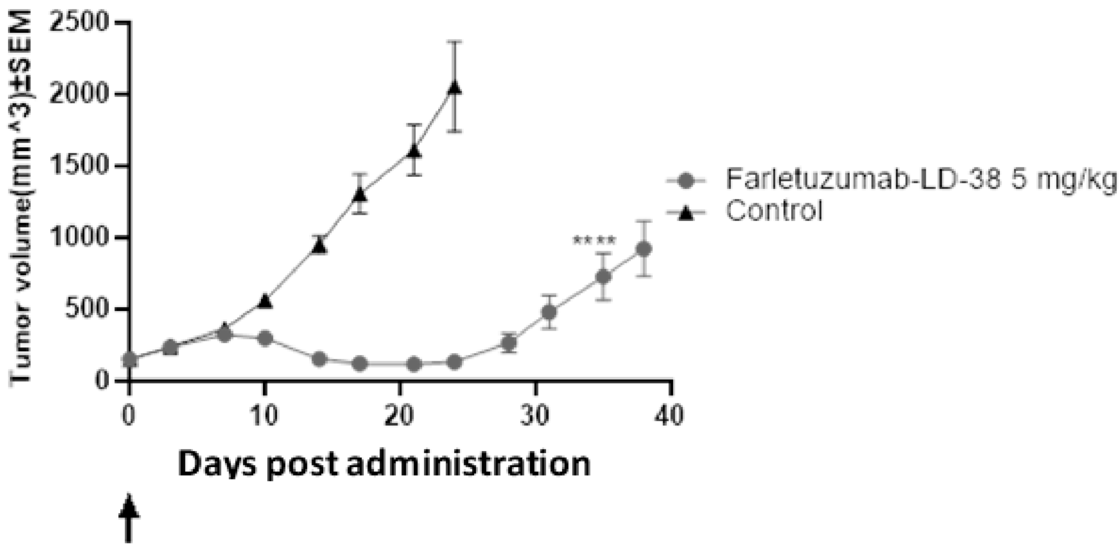
Tumor volume(mm^3)±SEM
2500
2000
1500
1000
500
0
0
10
20
30
40
Days post administration
Farletuzumab-LD-38 5 mg/kg
Control
****

Fig. 13

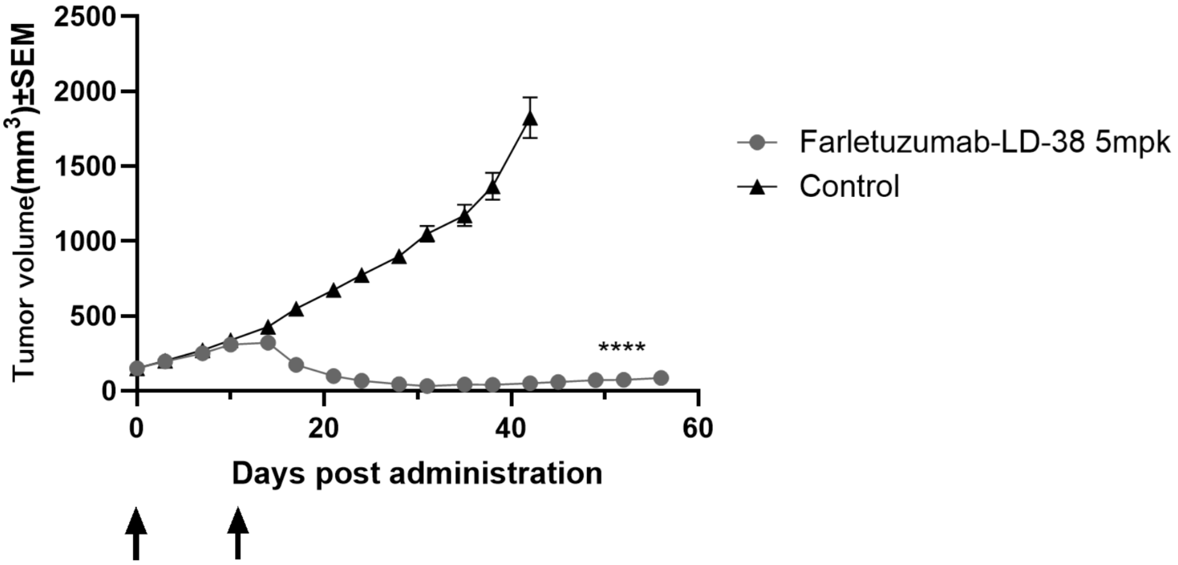
Tumor volume(mm3)±SEM
2500
2000
1500
1000
500
0
0
20
40
60
Days post administration
****
Farletuzumab-LD-38 5mpk
Control

Fig. 14

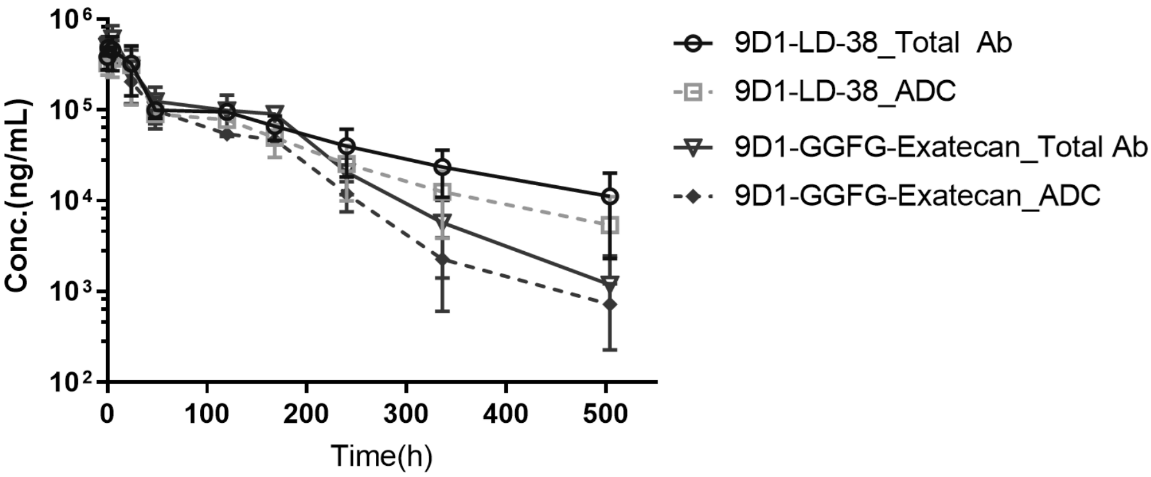
Conc.(ng/mL)
10⁶
10⁵
10⁴
10³
10²
0
100
200
300
400
500
Time(h)
9D1-LD-38_Total Ab
9D1-LD-38_ADC
9D1-GGFG-Exatecan_Total Ab
9D1-GGFG-Exatecan_ADC

Fig. 15

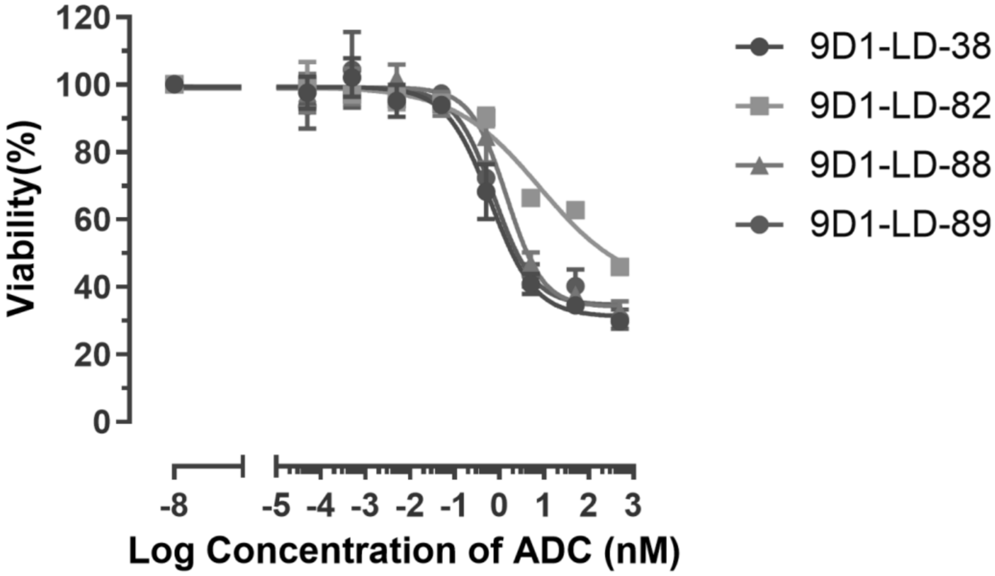
120
100
80
60
40
20
0
Viability(%)
-8
-5 -4 -3 -2 -1 0 1 2 3
Log Concentration of ADC (nM)
9D1-LD-38
9D1-LD-82
9D1-LD-88
9D1-LD-89

Fig. 16

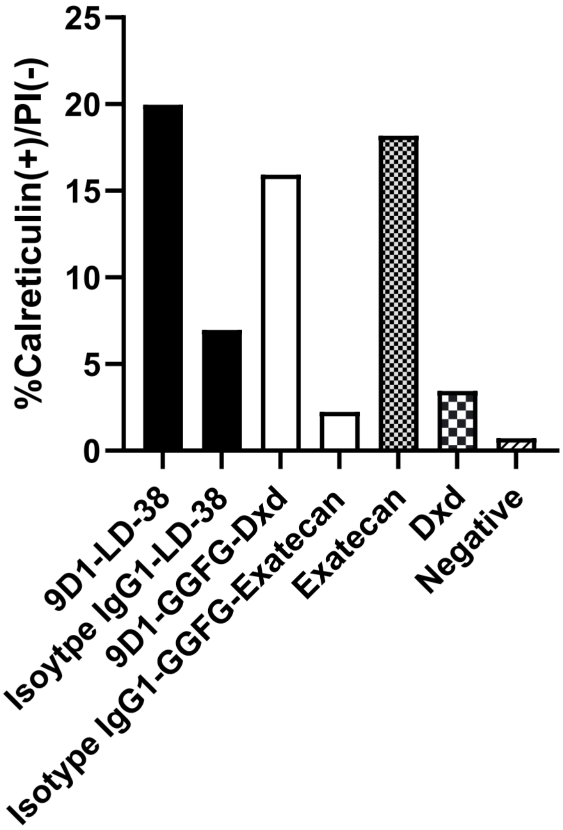
25
20
15
10
5
0
%Calreticulin(+)/PI(-)
9D1-LD-38
Isoytpe IgG1-LD-38
9D1-GGFG-Dxd
Isotype IgG1-GGFG-Exatecan
Exatecan
Dxd
Negative

Fig. 17

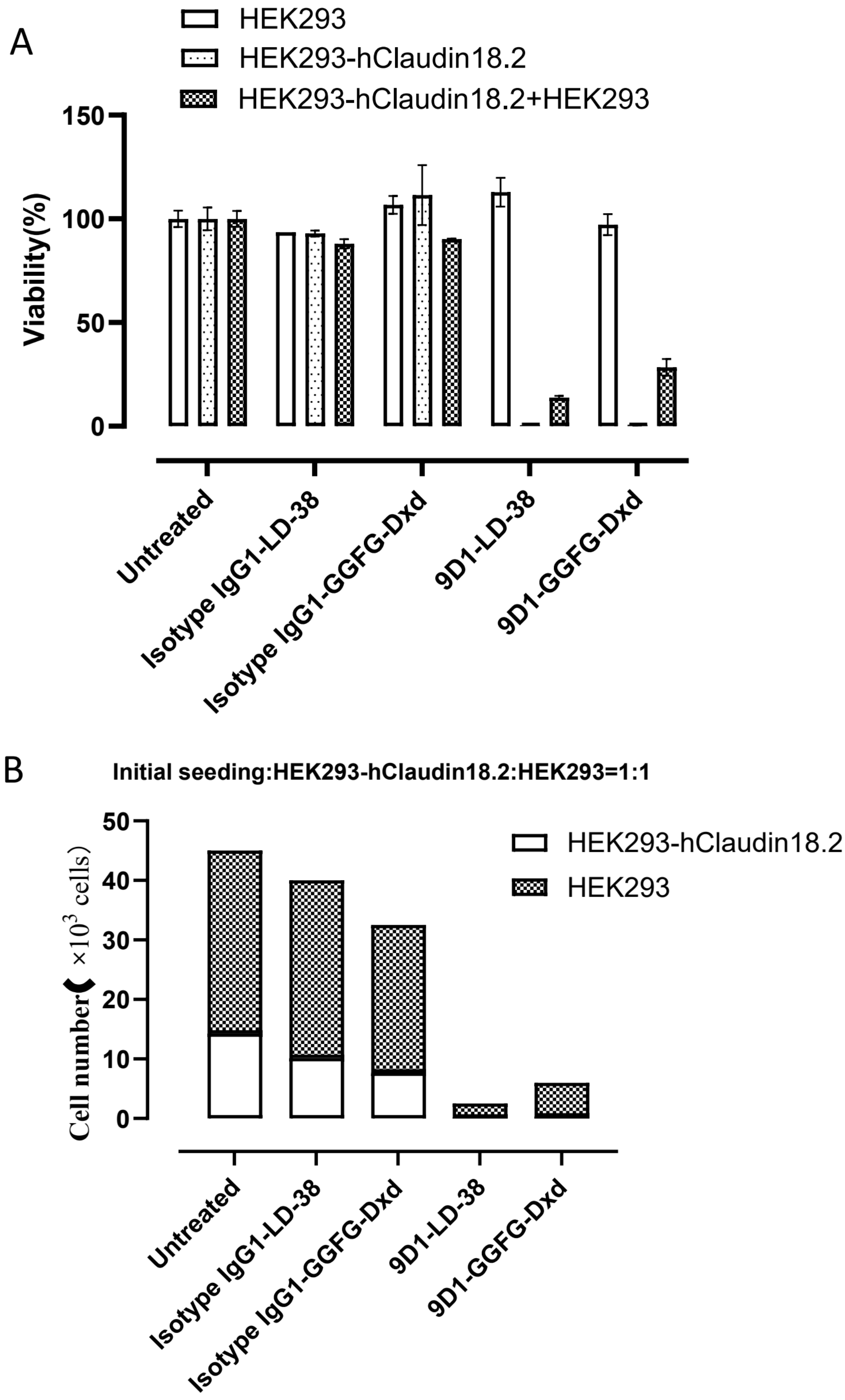
A
HEK293
HEK293-hClaudin18.2
HEK293-hClaudin18.2+HEK293
Viability(%)
150
100
50
0
Untreated
Isotype IgG1-LD-38
Isotype IgG1-GGFG-Dxd
9D1-LD-38
9D1-GGFG-Dxd
B
Initial seeding:HEK293-hClaudin18.2:HEK293=1:1
Cell number（×10³ cells)
50
40
30
20
10
0
HEK293-hClaudin18.2
HEK293
Untreated
Isotype IgG1-LD-38
Isotype IgG1-GGFG-Dxd
9D1-LD-38
9D1-GGFG-Dxd

Fig. 18

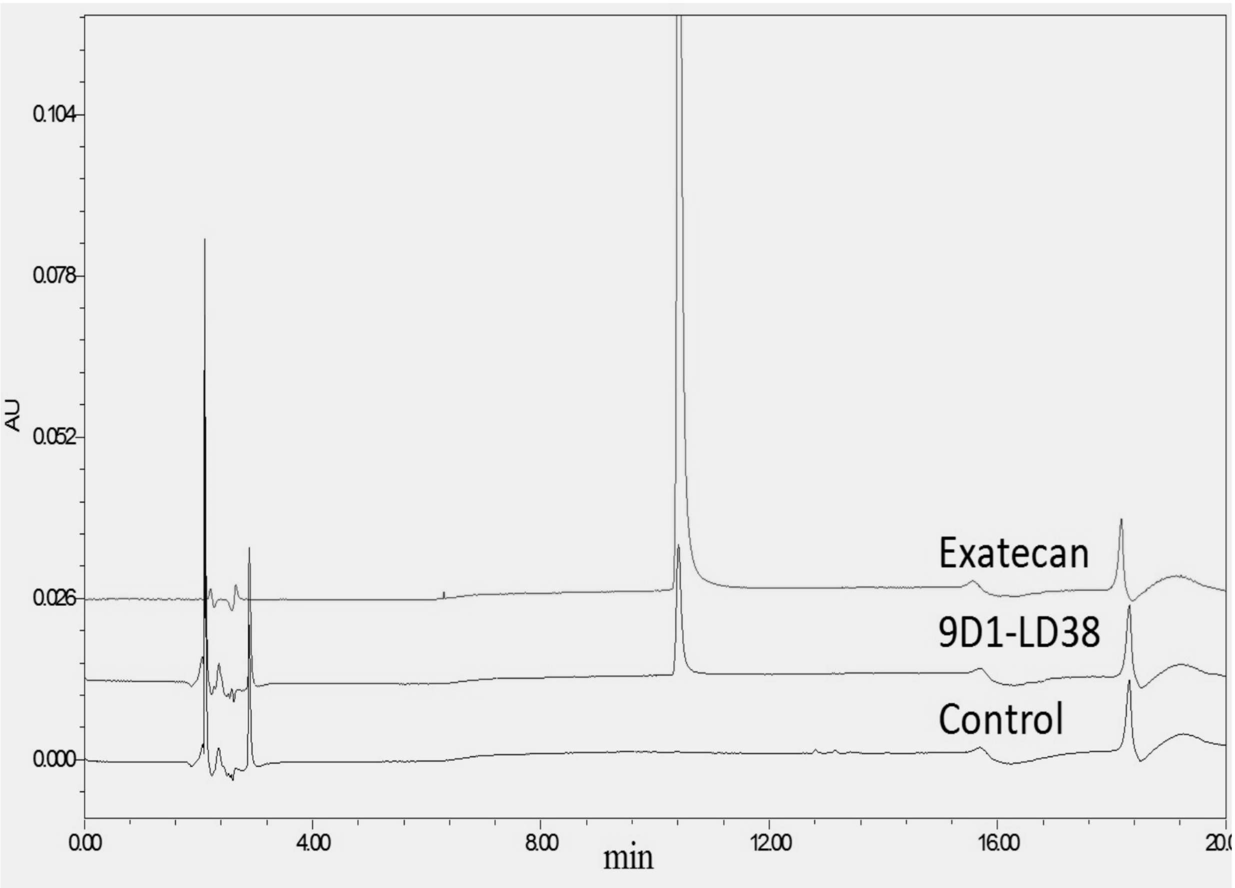
AU
0.104
0.078
0.052
0.026
0.000
0.00
4.00
8.00
12.00
16.00
min
Exatecan
9D1-LD38
Control

Fig. 19

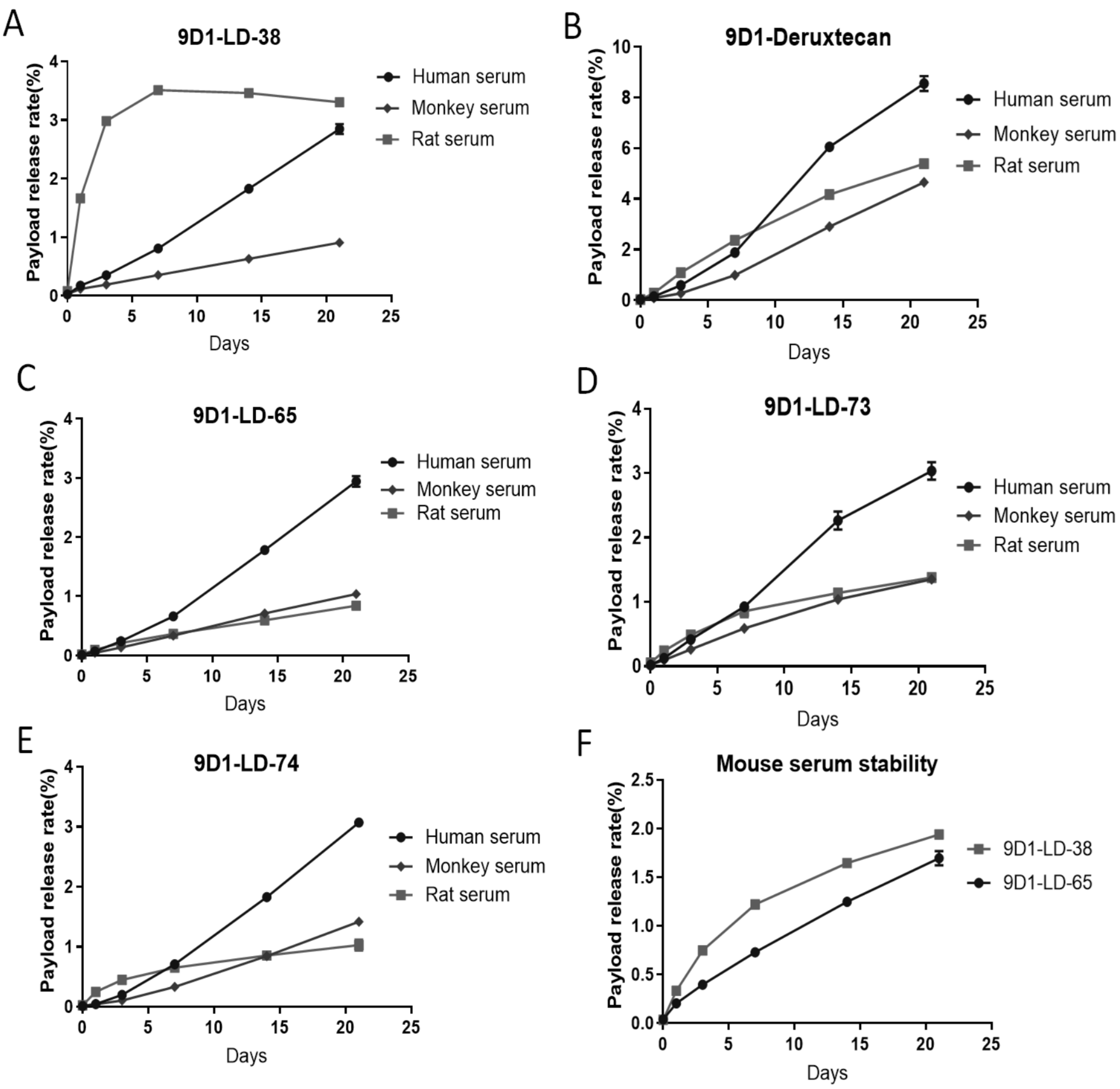
A
9D1-LD-38
Payload release rate(%)
Days
Human serum
Monkey serum
Rat serum
B
9D1-Deruxtecan
Payload release rate(%)
Days
Human serum
Monkey serum
Rat serum
C
9D1-LD-65
Payload release rate(%)
Days
Human serum
Monkey serum
Rat serum
D
9D1-LD-73
Payload release rate(%)
Days
Human serum
Monkey serum
Rat serum
E
9D1-LD-74
Payload release rate(%)
Days
Human serum
Monkey serum
Rat serum
F
Mouse serum stability
Payload release rate(%)
Days
9D1-LD-38
9D1-LD-65

Fig. 20

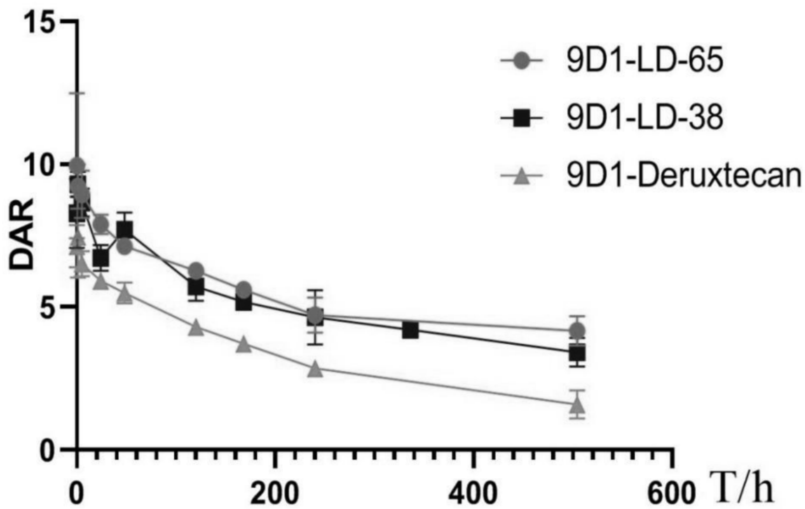
DAR
T/h
9D1-LD-65
9D1-LD-38
9D1-Deruxtecan

Fig. 21

A

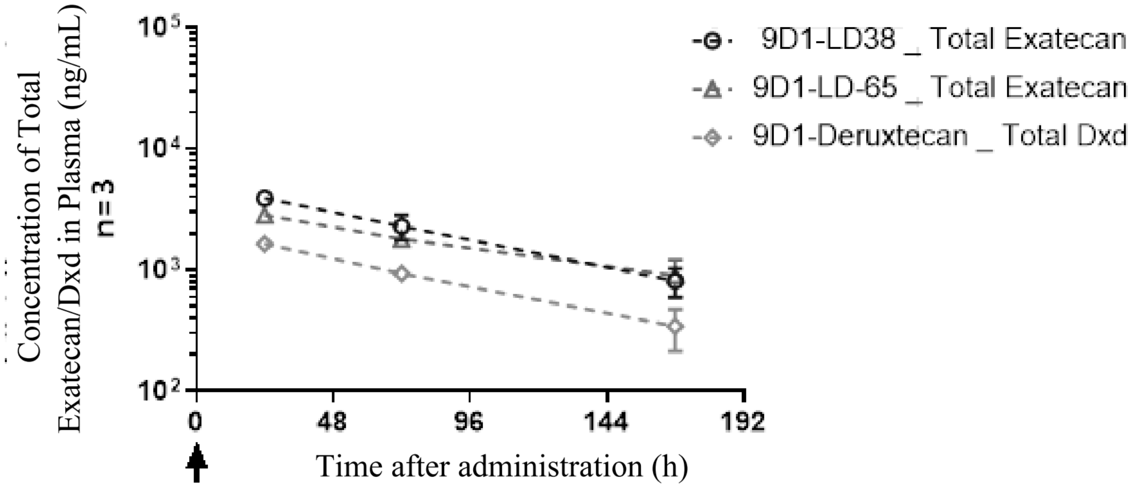
Concentration of Total Exatecan/Dxd in Plasma (ng/mL)
n=3
10^5
10^4
10^3
10^2
0
48
96
144
192
Time after administration (h)
9D1-LD38 _ Total Exatecan
9D1-LD-65 _ Total Exatecan
9D1-Deruxtecan _ Total Dxd

B

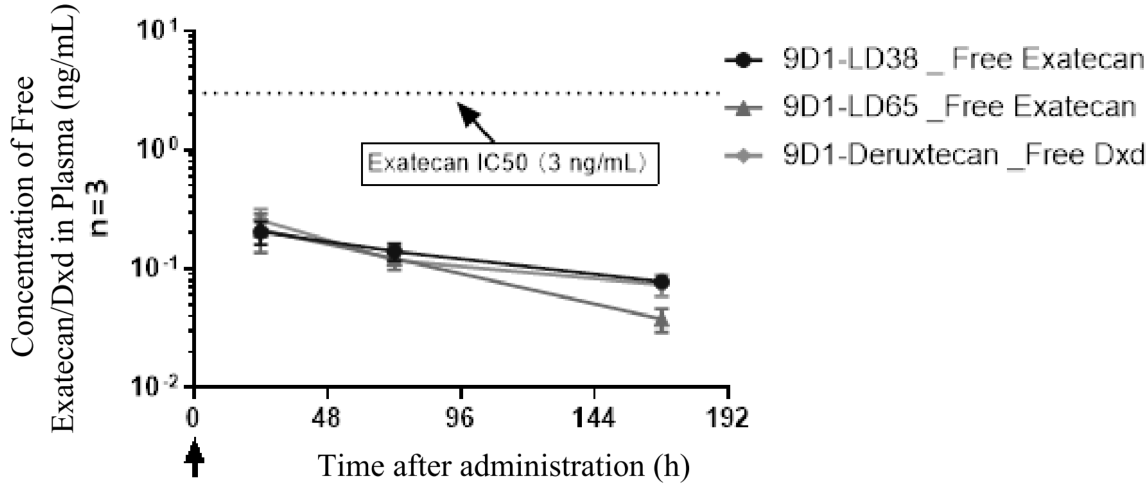
Concentration of Free Exatecan/Dxd in Plasma (ng/mL)
n=3
10^1
10^0
10^-1
10^-2
0
48
96
144
192
Time after administration (h)
Exatecan IC50 (3 ng/mL)
9D1-LD38 _ Free Exatecan
9D1-LD65 _Free Exatecan
9D1-Deruxtecan _Free Dxd

Fig. 22

A

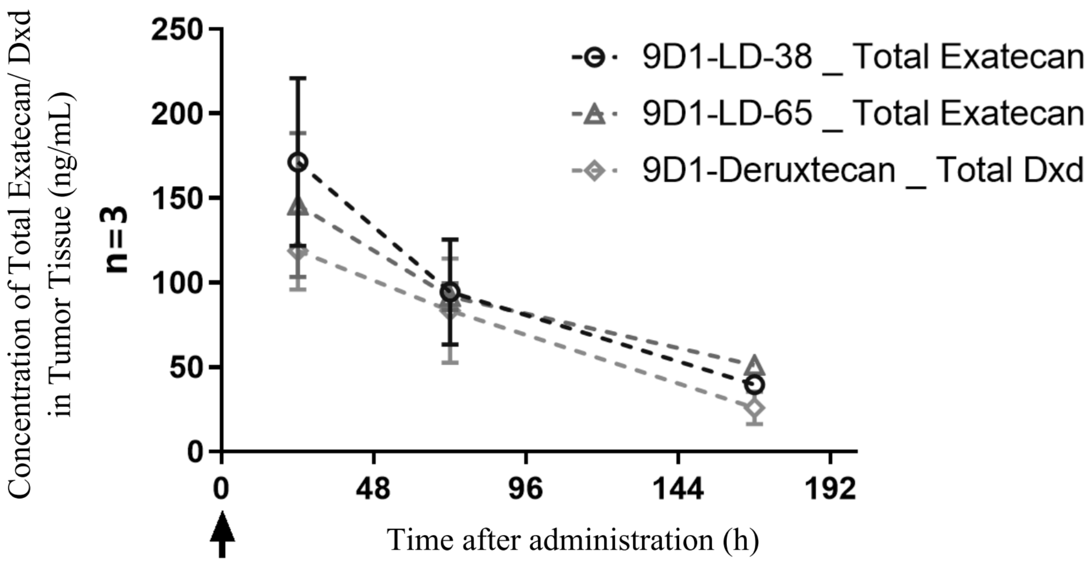
Concentration of Total Exatecan/ Dxd in Tumor Tissue (ng/mL)
n=3
250
200
150
100
50
0
0
48
96
144
192
Time after administration (h)
9D1-LD-38 _ Total Exatecan
9D1-LD-65 _ Total Exatecan
9D1-Deruxtecan _ Total Dxd

B

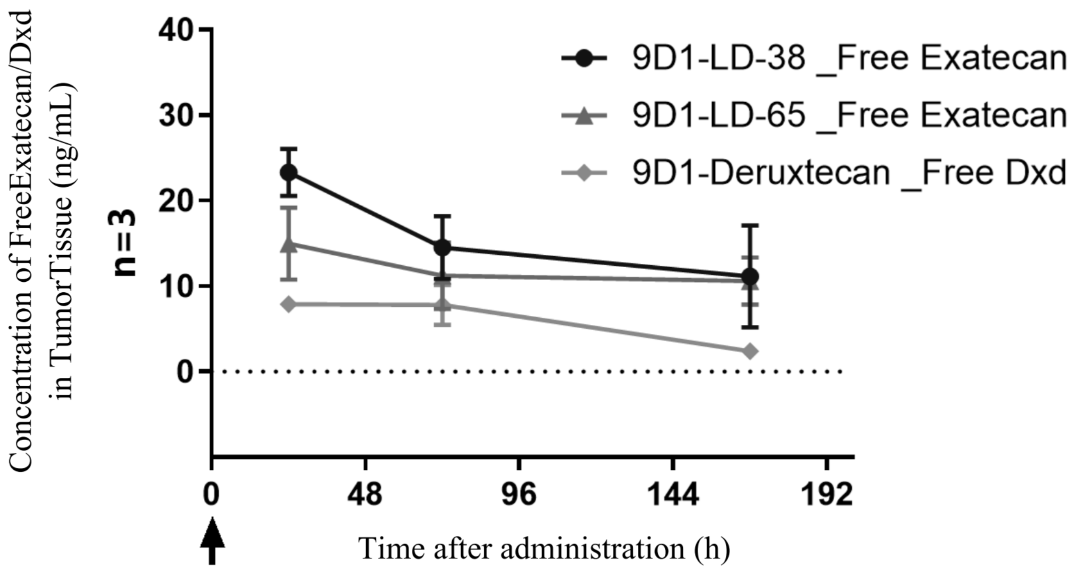
Concentration of FreeExatecan/Dxd in TumorTissue (ng/mL)
n=3
40
30
20
10
0
0
48
96
144
192
Time after administration (h)
9D1-LD-38 _Free Exatecan
9D1-LD-65 _Free Exatecan
9D1-Deruxtecan _Free Dxd

Fig. 23

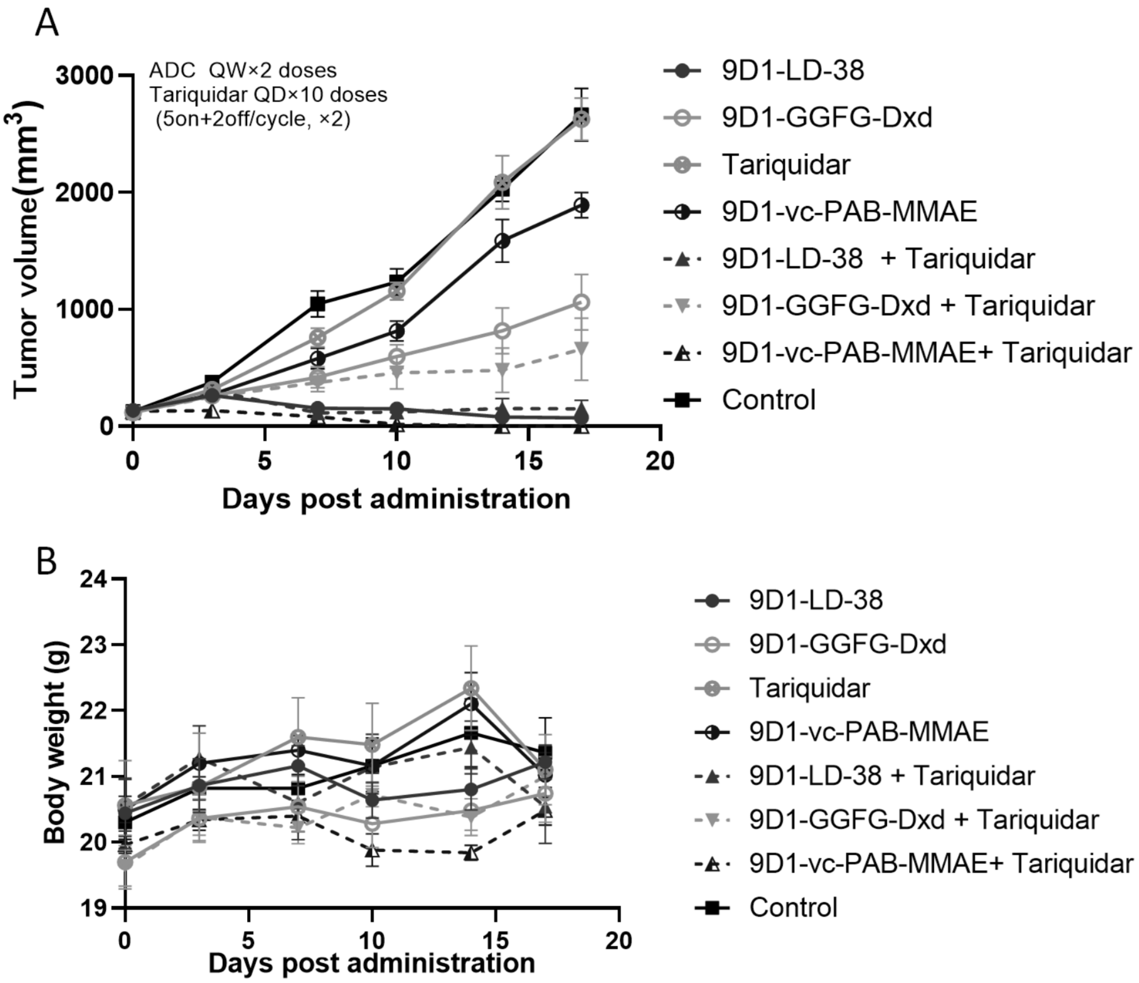
A
ADC QW×2 doses
Tariquidar QD×10 doses
(5on+2off/cycle, ×2)
Tumor volume(mm3)
3000
2000
1000
0
0 5 10 15 20
Days post administration
9D1-LD-38
9D1-GGFG-Dxd
Tariquidar
9D1-vc-PAB-MMAE
9D1-LD-38 + Tariquidar
9D1-GGFG-Dxd + Tariquidar
9D1-vc-PAB-MMAE+ Tariquidar
Control
B
Body weight (g)
24
23
22
21
20
19
0 5 10 15 20
Days post administration
9D1-LD-38
9D1-GGFG-Dxd
Tariquidar
9D1-vc-PAB-MMAE
9D1-LD-38 + Tariquidar
9D1-GGFG-Dxd + Tariquidar
9D1-vc-PAB-MMAE+ Tariquidar
Control

Fig. 24

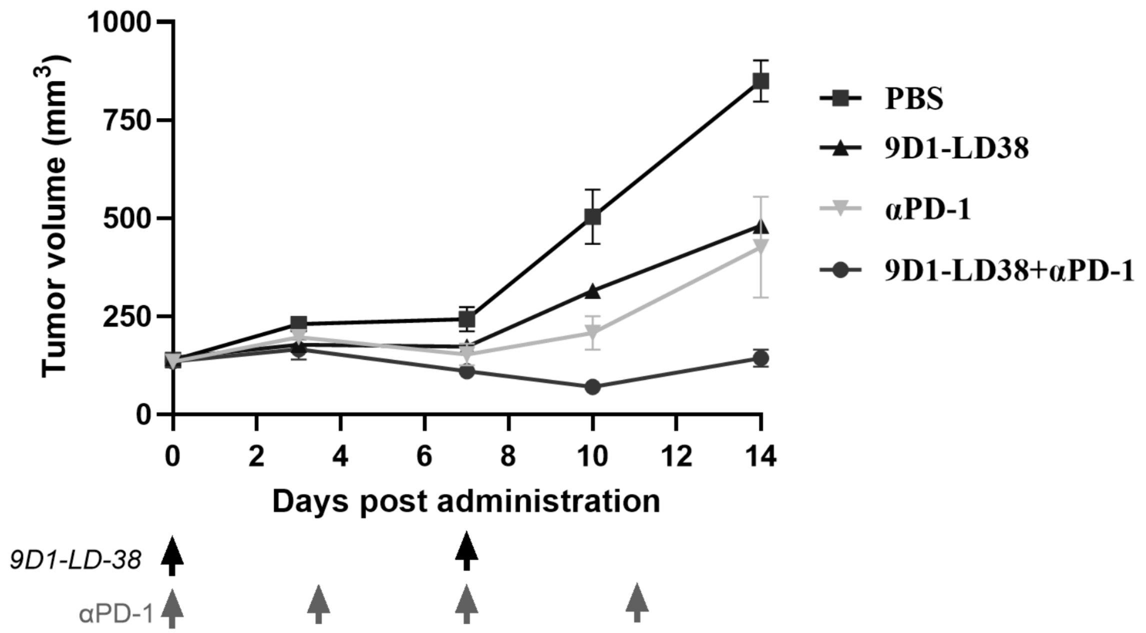
Tumor volume (mm3)
1000
750
500
250
0
0 2 4 6 8 10 12 14
Days post administration
PBS
9D1-LD38
αPD-1
9D1-LD38+αPD-1
9D1-LD-38
αPD-1

Fig. 25

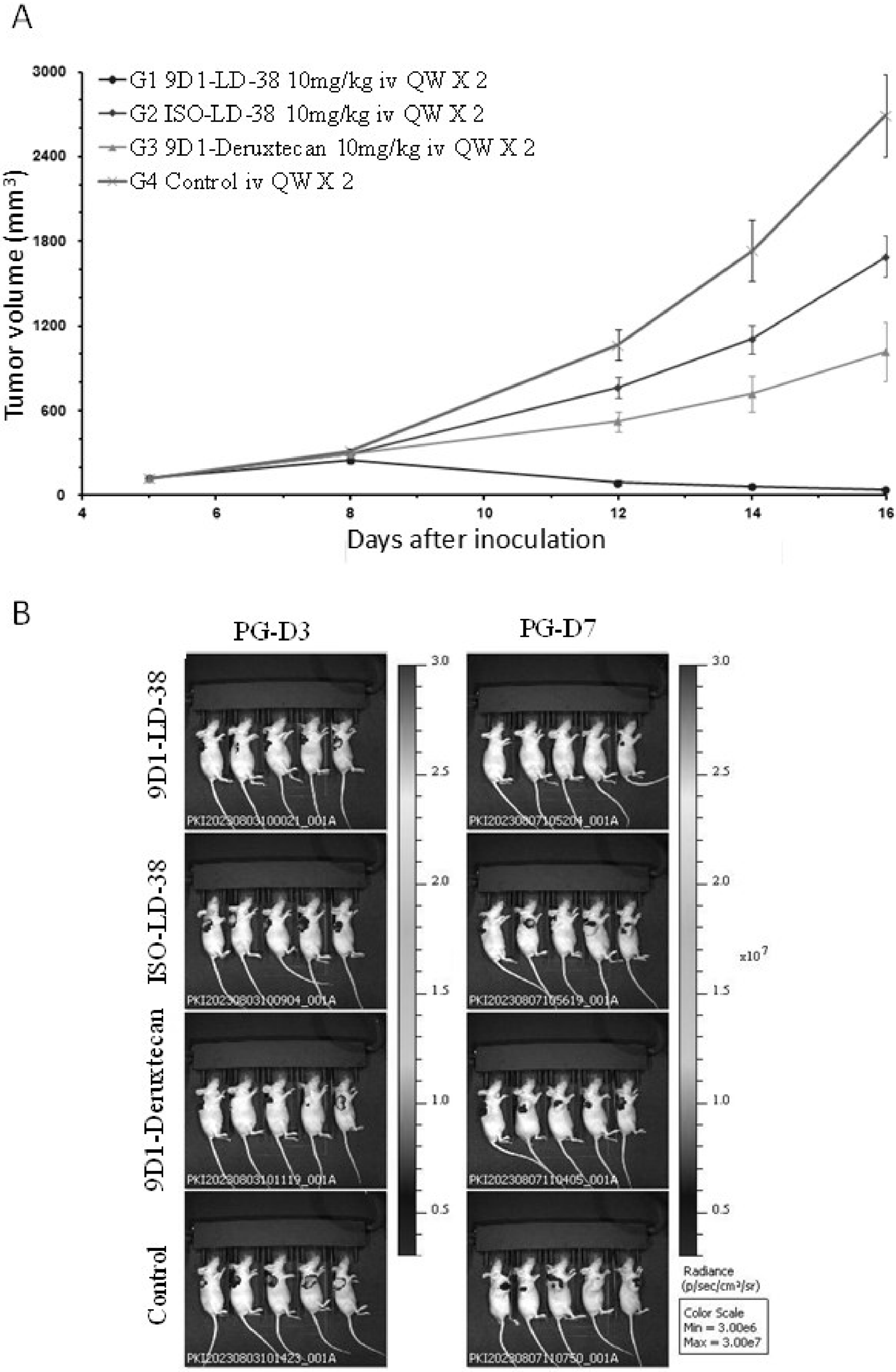
A
G1 9D1-LD-38 10mg/kg iv QW X 2
G2 ISO-LD-38 10mg/kg iv QW X 2
G3 9D1-Deruxtecan 10mg/kg iv QW X 2
G4 Control iv QW X 2
Tumor volume (mm3)
Days after inoculation
B
PG-D3
PG-D7
9D1-LD-38
ISO-LD-38
9D1-Deruxtecan
Control
x10^7
Radiance (p/sec/cm²/sr)
Color Scale
Min = 3.00e6
Max = 3.00e7

Fig. 26

## INTERNATIONAL SEARCH REPORT

International application No.
**PCT/CN2024/103480**

**A. CLASSIFICATION OF SUBJECT MATTER**

A61K 47/68(2017.01)i; A61K 47/65(2017.01)i; C07D491/22(2006.01)i; C07D493/22(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07D, A61P

Documentation searched other than minimum documentation to the extent that such documents are included in the fields searched

Electronic data base consulted during the international search (name of data base and, where practicable, search terms used)

WPABSC; CNTXT; CNABS; WPABS; DWPI; ENTXTC; VCN; Web of Science; CNKI; 万方, WANFANG; STNext: 磺酸内盐, 磷酸内盐, 季铵盐, 两性离子, 磺酸季铵盐, 磷酸季铵盐, 磺基甜菜碱, Sulfobetaine, 磷酸酯甜菜碱, Phosphobetaine, inner salt, zwitterions, ADC, 药物抗体偶联, 缀合物, 连接子, linker, sulfonic, quaternary, CAS: 78276-19-4, 1349808-80-5, 853798-56-8

**C. DOCUMENTS CONSIDERED TO BE RELEVANT**

| Category* | Citation of document, with indication, where appropriate, of the relevant passages | Relevant to claim No. |
|---|---|---|
| X | WO 2022199237 A1 (LEVENA SUZHOU BIOPHARMA CO., LTD.) 29 September 2022 (2022-09-29)<br>description, page 36 lines 1-4, and page 70 lines 1-9 | 1-137 |
| X | CN 113816990 A (LEVENA SUZHOU BIOPHARMA CO., LTD.) 21 December 2021 (2021-12-21)<br>description, page 35 paragraph 271, and page 61 paragraphs 0509-0600 | 1-137 |
| Y | WO 2022228493 A1 (SHANGHAI HUILIAN BIO-PHARM CO., LTD) 03 November 2022 (2022-11-03)<br>description, page 3 paragraph 1-page 20 paragraph 1 | 1-137 |
| Y | WO 2011146595 A2 (SIEMENS HEALTHCARE DIAGNOSTICS INC. et al.) 24 November 2011 (2011-11-24)<br>description, paragraphs 0001-0005, and claims 1 and 56 | 1-137 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 September 2024** | **08 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/103480**

**C. DOCUMENTS CONSIDERED TO BE RELEVANT**

| Category* | Citation of document, with indication, where appropriate, of the relevant passages | Relevant to claim No. |
|---|---|---|
| Y | WO 2021160155 A1 (SHANGHAI ESCUGEN BIOTECHNOLOGY CO., LTD.) 19 August 2021 (2021-08-19)<br>description, page 2 line 9-page 5 line 26 | 122-131, 133-137 |
| Y | THAPALIYA, E. R. et al. "Cyanine Masking: A Strategy to Test Functional Group Effects on Antibody Conjugate Targeting"<br>*Bioconjugate Chemistry,* Vol. 33, No. 4, 20 April 2022 (2022-04-20),<br>pages 718-725 | 1-137 |

Form PCT/ISA/210 (second sheet) (July 2022)

**INTERNATIONAL SEARCH REPORT**

International application No.
**PCT/CN2024/103480**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/103480**

| Patent document cited in search report | Publication date (day/month/year) | Patent family member(s) | Publication date (day/month/year) |
|---|---|---|---|
| WO 2022199237 A1 | 29 September 2022 | None | |
| CN 113816990 A | 21 December 2021 | None | |
| WO 2022228493 A1 | 03 November 2022 | JP 2024522388 A | 19 June 2024 |
| | | KR 20240006028 A | 12 January 2024 |
| | | TW 202309045 A | 01 March 2023 |
| | | CA 3173758 A1 | 03 November 2022 |
| | | TW 202308701 A | 01 March 2023 |
| | | US 2024245795 A1 | 25 July 2024 |
| | | WO 2022228495 A1 | 03 November 2022 |
| | | EP 4331613 A1 | 06 March 2024 |
| | | EP 4331611 A1 | 06 March 2024 |
| | | WO 2022228494 A1 | 03 November 2022 |
| | | IL 308025 A | 01 December 2023 |
| | | JP 2024522389 A | 19 June 2024 |
| | | US 2024245799 A1 | 25 July 2024 |
| | | KR 20240006029 A | 12 January 2024 |
| | | CA 3173902 A1 | 03 November 2022 |
| | | US 2024238440 A1 | 18 July 2024 |
| | | JP 2024522312 A | 14 June 2024 |
| | | TW 202308700 A | 01 March 2023 |
| | | AU 2022263700 A1 | 16 November 2023 |
| | | CA 3173511 A1 | 28 October 2023 |
| | | EP 4331612 A1 | 06 March 2024 |
| WO 2011146595 A2 | 24 November 2011 | None | |
| WO 2021160155 A1 | 19 August 2021 | JP 2023513401 A | 30 March 2023 |
| | | JP 7522482 B2 | 25 July 2024 |
| | | CA 3167349 A1 | 19 August 2021 |
| | | AU 2021218927 A1 | 22 September 2022 |
| | | EP 4105238 A1 | 21 December 2022 |
| | | EP 4105238 A4 | 27 March 2024 |
| | | US 2023096452 A1 | 30 March 2023 |
| | | KR 20220140786 A | 18 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023106385 W
- WO 2023280227 A
- WO 2022228493 A
- US 9907854 B
- WO 2022058548 A1
- WO 2011146595 A2
- WO 2005114193 A
- CN 115521998
- WO 2021160155 A
- WO 2021225892 A

### Non-patent literature cited in the description

- *Mol Cancer Ther*, 2017, vol. 16 (1), 116-123
- *Nat. Biotech.*, 2015, vol. 33, 733-735
- *Toxicol. App. Pharmacol.*, 2020, vol. 392, 114932
- *Chem. Sci*, 2019, vol. 10 (14), 4048-4053
- *Cancer Res*, 2018, vol. 78 (13), 755
- *CHEMICAL ABSTRACTS*, 78276-19-4
- *CHEMICAL ABSTRACTS*, 1349808-80-5
- *CHEMICAL ABSTRACTS*, 853798-56-8
- Bioconjugate Chem.. Shanghai Haoyuan Pharmaceutical Co., Ltd., 2022, vol. 33, 718-725
- *CHEMICAL ABSTRACTS*, 1881221-47-1
- *CHEMICAL ABSTRACTS*, 2170240-98-7
- *CHEMICAL ABSTRACTS*, 6118-51-0
- *CHEMICAL ABSTRACTS*, 121492-06-6
- *CHEMICAL ABSTRACTS*, HY-114233
- *CHEMICAL ABSTRACTS*, 646502-53-6
- *CHEMICAL ABSTRACTS*, HY-13631E